# EUROPEAN PATENT APPLICATION

(11) **EP 4 269 403 A1**
(43) Date of publication of application: **01.11.2023**
(21) Application number: 21908645.1
(22) Date of filing: 27.08.2021
(51) Int. Cl.: C07D 401/14, C07D 401/00, A61K 31/506, A61K 31/505, A61K 31/495, C07D 471/04, A61P 35/00

(54) **AROMATIC HETEROCYCLIC COMPOUND, AND PHARMACEUTICAL COMPOSITION AND APPLICATION THEREOF**

(30) Priority: 24.12.2020 CN 202011552478
(71) Applicant: GT Apeiron Therapeutics Limited, Hong Kong 999077 (HK); Exscientia Al Limited, Dundee, DD1 3JT (GB)
(72) Inventor: GU, Xiaohui, Shanghai 201203 (CN); BAI, Haiyun, Shanghai 201203 (CN); BARBEAU, Olivier Rémy, Oxford Oxfordshire OX4 4GE (GB); BESNARD, Jérémy, Oxford Oxfordshire OX4 4GE (GB)
(74) Representative: Keltie LLP
(86) International application number: PCT/CN2021/115078
(87) International publication number: WO 2022/134642

(57) **Abstract**

An aromatic heterocyclic compound, and a pharmaceutical composition and an application thereof. Specifically disclosed are a compound as represented by formula I-A, a stereoisomer thereof, a diastereoisomer thereof, or a pharmaceutically acceptable salt of any one of the described substances, or a crystalline form or a solvate of any one of the described substances. The aromatic heterocyclic compound is novel in structure, and has good CDK7 inhibitory activity and good selectivity.

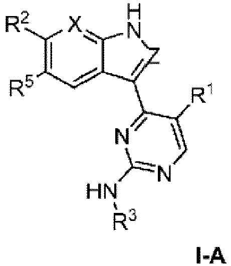

## Description

The present application claims the priority to Chinese Patent Application 2020115524787 filed on December 24, 2020. The present application refers to the above-mentioned Chinese patent application in its entirety.

### Technical Field

The invention relates to an aromatic heterocyclic compound, and a pharmaceutical composition and an application thereof.

### Background Art

Members of the cyclin-dependent kinase (CDK) family play a key regulatory role in proliferation. CDK7, which is unique among mammalian CDKs, has the effects of integrating the kinase activity and regulating cell cycle and transcription. In cytosol, CDK7 exists as a heterotrimeric complex and is considered to function as a CDK1/2-activating kinase (CAK), whereby the phosphorylation of conserved residues in CDK1/2 by CDK7 is essential for full catalytic CDK activity and cell cycle progression. In a cell nucleus, CDK7 forms a kinase core of an RNA polymerase (RNAP) II general transcription factor complex and is responsible for phosphorylating a C-terminal domain (CTD) of RNAP II, which is an essential step of the initiation of gene transcription. Two functions of CDK7 (i.e., CAK and CTD phosphorylation) together support key aspects of cell proliferation, cell cycle, and transcription.

Destruction of RNAP IICTD phosphorylation has been shown to preferentially affect proteins with short half-lives, including proteins of the anti-apoptotic BCL-2 family. Cancer cells have demonstrated the ability to evade pro-cell death signaling by upregulating BCL-2 family members. Therefore, inhibition of human CDK7 kinase activity may result in an antiproliferative activity.

The high sequences and the structural similarity of the kinase domains of the CDK family members hamper the discovery of CDK7 selective inhibitors. Therefore, it is needed to discover and develop selective CDK7 inhibitors. Such CKD7 inhibitors hold promise as a therapeutic agent for treating CLL and other cancers.

The prerequisite for an oral drug to exert pharmacological effects in vivo is that the oral drug needs to be absorbed and distributed to reach a respective site of action. Membrane permeability of a drug can reflect the in-vivo absorption and transport ability of the drug. Passive diffusion of a drug is positively correlated with biomembrane permeability of the drug. A drug with good biomembrane permeability is more easily absorbed by the gastrointestinal tract. The efflux rate of an oral drug is an important parameter for characterizing its absorption, with a lower efflux rate indicating that the drug is better absorbed in the gastrointestinal tract.

The patent WO2018013867A1 discloses a CDK7 inhibitor, based on which the researchers of the present invention have found that the compound disclosed in this patent document had the problems of poor membrane permeability and high efflux rate in a Caco-2 monolayer permeation test model, which would affect the absorption of a drug in the gastrointestinal tract. However, the researchers of the present invention surprisingly found that after inventive structural modifications of the compound disclosed in this patent document, the resulting compound of the present invention could have higher membrane permeability and lower efflux rate in the Caco-2 monolayer permeation test model, which would be more beneficial to oral absorption, while maintaining high biological activity.

### Summary of the Invention

The technical problem to be solved by the present invention is to provide a CDK7 inhibitor which is novel in structure, has high CDK7 inhibitory activity, and meanwhile has better membrane permeability and lower efflux rate in view of the defects of low membrane permeability and high efflux rate present in existing CDK7 inhibitors. A compound of the present invention can solve the problems of low oral availability and low gastrointestinal absorption rate present in the existing CDK7 inhibitors.

The present invention solves the above-mentioned technical problems by the following technical schemes.

The present invention provides a compound as represented by formula I-A, a stereoisomer thereof, a diastereoisomer thereof, a pharmaceutically acceptable salt of any one of the described substances (referring to the described compound as represented by formula I-A, the stereoisomer thereof, or the diastereoisomer thereof), or a crystalline form or a solvate of any one of the described substances (referring to the described compound as represented by formula I-A, the stereoisomer thereof, the diastereoisomer thereof, or the pharmaceutically acceptable salt thereof): 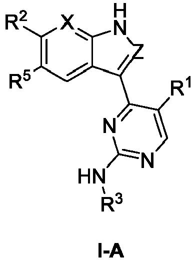
R¹ is CF₃, CHF₂, F, Cl, Br, C₁-C₆ alkyl, -C(=O)NH₂, or CN;
R⁵ is H, halogen, C₁-C₆ alkyl, or C₁-C₆ alkoxy;
X is N or C(R⁴), and R⁴ is -P(=O)Me₂;
Z is N or CH;
R² is H, halogen, "5- to 10-membered heteroaryl having 1-4 heteroatoms selected from one or more of N, O, and S," "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, "4- to 12-membered heterocycloalkenyl having 1-4 heteroatoms selected from one or more of N, O, and S," "4- to 12-membered heterocycloalkenyl having 1-4 heteroatoms selected from one or more of N, O, and S" and substituted with one or more R^{a-3}, C₂-C₆ alkynyl, C₁-C₆ alkyl, C₁-C₆ alkoxy, or CN;
R^{a-1}, R^{a-2}, and R^{a-3} are independently CN, oxo, C₁-C₆ alkyl substituted with one or more R^{a-1-1}, NH₂, OH, or C₁-C₆ alkyl; R^{a-1-1} is independently CN, OH, or halogen;
R³ is C₃-C₈ cycloalkyl substituted with one or more R^{b-1}, C₁-C₆ alkyl substituted with one or more R^{b-2}, 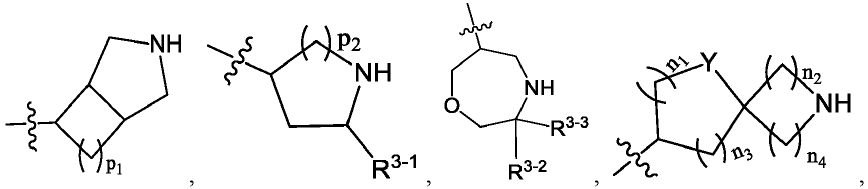 "4- to 12-membered heterocycloalkyl having 1-4 heteroatoms being O" and substituted with one or more R^{b-3}, 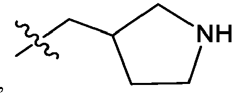 or "5- to 10-membered heteroaryl having 1-4 heteroatoms selected from one or more of N, O, and S";
R^{b-1} is independently halogen, OH, -NR^{b-1-1}R^{b-1-2}, C₁-C₆ alkyl, or "C₁-C₆ alkyl substituted with one or more R^{b-1-3}";
R^{b-1-1} and R^{b-1-2} are independently H or C₁-C₆ alkyl;
R^{b-1-3} is independently OH or NR^{b-1-4}R^{b-1-5}; R^{b-1-4} and R^{b-1-5} are independently H or C₁-C₆ alkyl;
R^{b-2} is independently OH, halogen, C₃-C₈ cycloalkyl, C₃-C₈ cycloalkyl substituted with one or more R^{b-2-1}, 4- to 12-membered heterocycloalkyl having 1-4 heteroatoms being O, or "4- to 12-membered heterocycloalkyl having 1-4 heteroatoms being O and substituted with one or more R^{b-2-2}_{"};
R^{b-2-1} and R^{b-2-2} are independently OH, C₁-C₆ alkyl, or "C₁-C₆ alkyl substituted with one or more OH";
p₁ is 0, 1, 2, or 3;
p₂ is 2 or 3, and R³⁻¹ is H or C₁-C₆ alkyl;
R³⁻² and R³⁻³ are independently H, C₁-C₆ alkyl, or "C₁-C₆ alkyl substituted with one or more halogens";
Y is O or CH₂, n₁ is 1 or 2, n₂, n₃, and n₄ are independently 0, 1, 2, or 3, and n₂ and n₄ are not 0 at the same time;
R^{b-3} is independently halogen, OH, C₁-C₆ alkyl, or "C₁-C₆ alkyl substituted with one or more OH."

In certain preferred embodiments of the present invention, certain groups of the compound as represented by formula I-A, a stereoisomer thereof, a diastereoisomer thereof, or a pharmaceutically acceptable salt of any one of the described substances, or a crystalline form or a solvate of any one of the described substances are as defined below, the unmentioned groups are the same as described in any scheme of the present application (simply referred to as "in a certain scheme of the present invention"),
and when R¹ is C₁-C₆ alkyl, the C₁-C₆ alkyl is C₁-C₃ alkyl, for example, methyl.

In a certain scheme of the present invention, when R⁵ is halogen, the halogen is F, Cl, Br, or I, for example, F.

In a certain scheme of the present invention, when R⁵ is C₁-C₆ alkyl, the C₁-C₆ alkyl is C₁-C₃ alkyl, preferably methyl, ethyl, n-propyl, or isopropyl, for example, methyl.

In a certain scheme of the present invention, when R⁵ is C₁-C₆ alkoxy, the C₁-C₆ alkoxy is C₁-C₃ alkoxy, preferably methoxy, ethoxy, n-propoxy, or isopropoxy, for example, methoxy.

In a certain scheme of the present invention, when R² is halogen, the halogen is F, Cl, Br, or I, for example, F.

In a certain scheme of the present invention, when R² is "5- to 10-membered heteroaryl having 1-4 heteroatoms selected from one or more of N, O, and S," the "5- to 10-membered heteroaryl having 1-4 heteroatoms selected from one or more of N, O, and S" is "5- to 6-membered heteroaryl having 1-4 heteroatoms selected from one or more of N and O," for example, 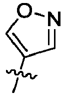 or

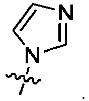

In a certain scheme of the present invention, when R² is "5- to 10-membered heteroaryl having 1-4 heteroatoms selected from one or more of N, O, and S" and substituted with one or more R^{a-1}, the "5- to 10-membered heteroaryl having 1-4 heteroatoms selected from one or more of N, O, and S" and substituted with one or more R^{a-1} is "5- to 6-membered heteroaryl having 1-4 heteroatoms selected from one or more of N and O" and substituted with one or 2 R^{a-1}, for example,

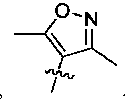

In a certain scheme of the present invention, when R² is "4- to 12-membered heterocycloalkyl having 1-4 heteroatoms selected from one or more of N, O, and S" or "4- to 12-membered heterocycloalkyl having 1-4 heteroatoms selected from one or more of N, O, and S" and substituted with one or more R^{a-2}," the "4- to 12-membered heterocycloalkyl having 1-4 heteroatoms selected from one or more of N, O, and S" is a monocyclic ring, a bicyclic ring, or a bridged ring, and the bicyclic ring includes a spiro ring or a fused ring.

In a certain scheme of the present invention, when R² is "4- to 12-membered heterocycloalkyl having 1-4 heteroatoms selected from one or more of N, O, and S," the "4- to 12-membered heterocycloalkyl having 1-4 heteroatoms selected from one or more of N, O, and S" is "4- to 12-membered heterocycloalkyl having 1-2 heteroatoms selected from one or more of N, O, and S," for example, 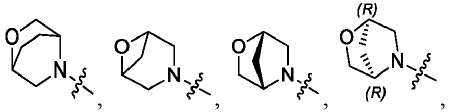 azetidinyl, oxazepanyl, tetrahydrofuranyl, tetrahydropyranyl, piperidyl, pyrrolidinyl, piperazinyl, thiomorpholinyl, or morpholinyl, for another example, 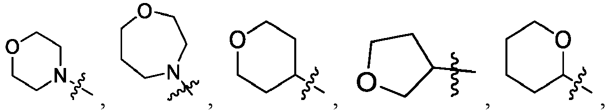 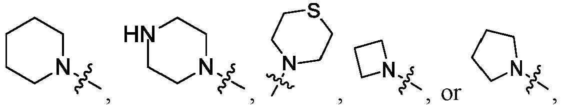 and still for another example, 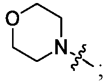 preferably "4- to 12-membered heterocycloalkyl having 2 heteroatoms selected from one or more of N, O, and S."

In a certain scheme of the present invention, when R² is "4- to 12-membered heterocycloalkyl having 1-4 heteroatoms selected from one or more of N, O, and S" and substituted with one or more R^{a-2}, the "4- to 12-membered heterocycloalkyl having 1-4 heteroatoms selected from one or more of N, O, and S" and substituted with one or more R^{a-2} is "4- to 12-membered heterocycloalkyl having 1-2 heteroatoms selected from one or more of N, O, and S" and substituted with 1 or 2 R^{a-2}, for example, morpholinyl substituted with 1 or 2 R^{a-2}, azetidinyl substituted with 1 or 2 R^{a-2}, tetrahydrofuranyl substituted with 1 or 2 R^{a-2}, tetrahydropyranyl substituted with 1 or 2 R^{a-2}, piperidyl substituted with 1 or 2 R^{a-2}, pyrrolidinyl substituted with 1 or2 R^{a-2}, piperazinyl substituted with 1 or 2 R^{a-2}, or "thiomorpholinyl substituted with 1 or 2 R^{a-2}," for another example, 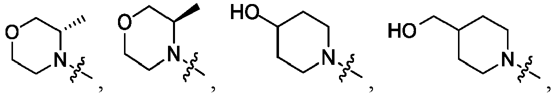 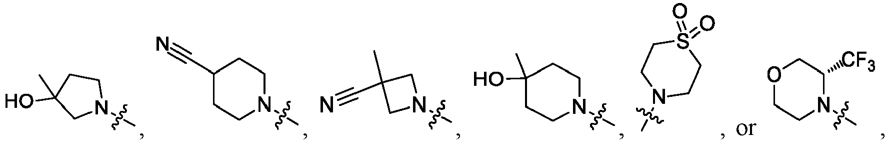 and still for another example, 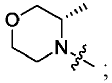 preferably "4- to 12-membered heterocycloalkyl having 2 heteroatoms selected from one or more of N, O, and S" and substituted with 1 or 2 R^{a-2}.

In a certain scheme of the present invention, when R² is "4- to 12-membered heterocycloalkenyl having 1-4 heteroatoms selected from one or more of N, O, and S," the "4- to 12-membered heterocycloalkenyl having 1-4 heteroatoms selected from one or more of N, O, and S" is "4- to 12-membered heterocycloalkenyl having 1-2 heteroatoms selected from one or more of N, O, and S," for example, dihydrofuranyl, for another example,

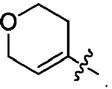

In a certain scheme of the present invention, when R^{a-1}, R^{a-2}, and R^{a-3} are independently C₁-C₆ alkyl substituted with one or more R^{a-1-1}, the C₁-C₆ alkyl is C₁-C₃ alkyl; preferably methyl, ethyl, n-propyl, or isopropyl, for example, methyl.

In a certain scheme of the present invention, when R^{a-1}, R^{a-2}, and R^{a-3} are independently C₁-C₆ alkyl, the C₁-C₆ alkyl is C₁-C₃ alkyl; preferably methyl, ethyl, n-propyl, or isopropyl, for example, methyl.

In a certain scheme of the present invention, when R^{a-1-1} is halogen, the halogen is F, Cl, Br, or I, for example, F.

In a certain scheme of the present invention, when R² is C₁-C₆ alkyl, the C₁-C₆ alkyl is C₁-C₃ alkyl, preferably methyl, ethyl, n-propyl, or isopropyl, for example, methyl.

In a certain scheme of the present invention, when R² is C₁-C₆ alkoxy, the C₁-C₆ alkoxy is C₁-C₃ alkoxy, preferably methoxy, ethoxy, n-propoxy, or isopropoxy, for example, methoxy.

In a certain scheme of the present invention, when R³ is C₃-C₈ cycloalkyl substituted with one or more R^{b-1}, the C₃-C₈ cycloalkyl substituted with one or more R^{b-1} is C₃-C₆ cycloalkyl substituted with 1, 2, or 3 R^{b-1}, for example, cyclopropyl substituted with 1, 2, or 3 R^{b-1}, cyclobutyl substituted with 1, 2, or 3 R^{b-1}, or "cyclopentyl substituted with 1, 2, or 3 R^{b-1}," for another example, 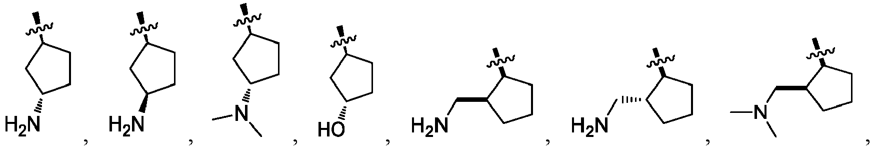 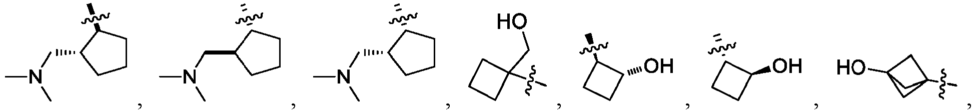 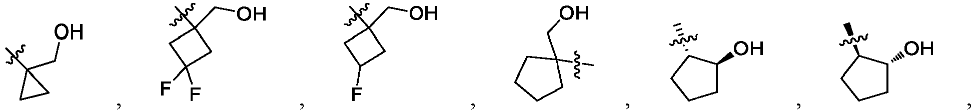 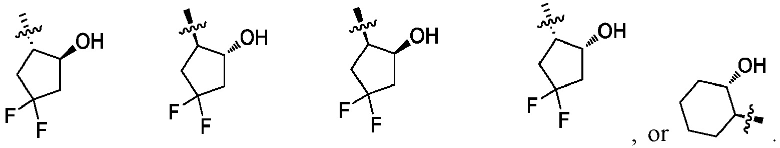 Preferably, the C₃-C₈ cycloalkyl substituted with one or more R^{b-1} is C₃-C₅ cycloalkyl substituted with 1, 2, or 3 R^{b-1}.

In a certain scheme of the present invention, when R^{b-1} is independently halogen, the halogen is F, Cl, Br, or I, for example, F.

In a certain scheme of the present invention, when R^{b-1} is independently C₁-C₆ alkyl, the C₁-C₆ alkyl is C₁-C₃ alkyl; preferably methyl, ethyl, n-propyl, or isopropyl, for example, methyl.

In a certain scheme of the present invention, when R^{b-1} is independently "C₁-C₆ alkyl substituted with one or more R^{b-1-3}," the C₁-C₆ alkyl is C₁-C₃ alkyl; preferably methyl, ethyl, n-propyl, or isopropyl, for example, methyl.

In a certain scheme of the present invention, when R^{b-1-1} and R^{b-1-2} are independently C₁-C₆ alkyl, the C₁-C₆ alkyl is C₁-C₃ alkyl; preferably methyl, ethyl, n-propyl, or isopropyl, for example, methyl.

In a certain scheme of the present invention, when R^{b-1-4} and R^{b-1-5} are independently C₁-C₆ alkyl, the C₁-C₆ alkyl is C₁-C₃ alkyl; preferably methyl, ethyl, n-propyl, or isopropyl, for example, methyl.

In a certain scheme of the present invention, when R³ is C₁-C₆ alkyl substituted with one or more R^{b-2}, the C₁-C₆ alkyl is methyl, ethyl, n-propyl, isopropyl, n-butyl, isobutyl, or sec-butyl; preferably, the C₁-C₆ alkyl substituted with one or more R^{b-2} is

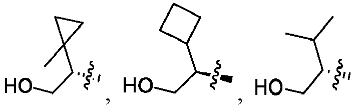

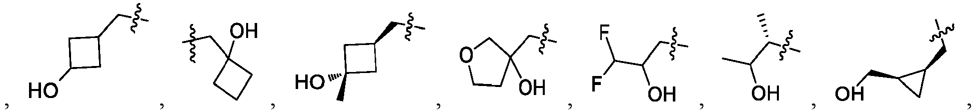

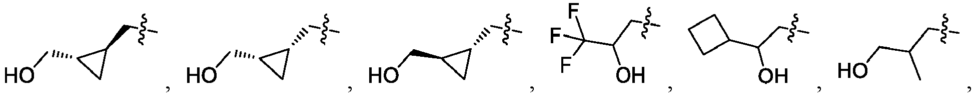

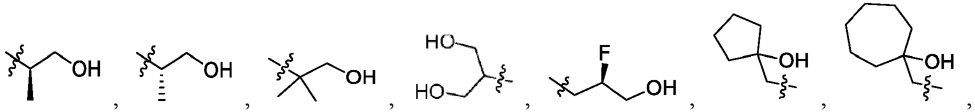

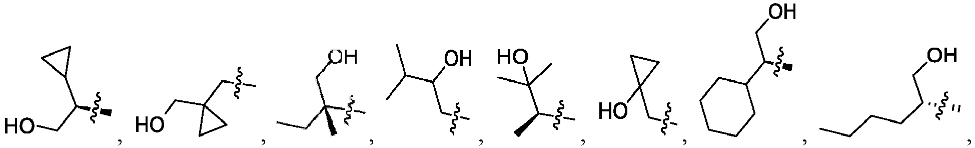

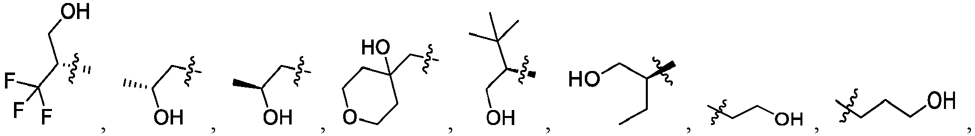

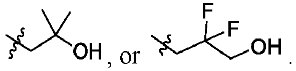

In a certain scheme of the present invention, when R^{b-2} is independently halogen, the halogen is F, Cl, Br, or I, for example, F.

In a certain scheme of the present invention, when R^{b-2} is independently C₃-C₈ cycloalkyl, the C₃-C₈ cycloalkyl is C₃-C₇ cycloalkyl, for example, cyclopropyl, cyclobutyl, cyclopentyl, cyclohexyl, or cycloheptyl.

In a certain scheme of the present invention, when R^{b-2} is independently C₃-C₈ cycloalkyl substituted with one or more R^{b-2-1}, the C₃-C₈ cycloalkyl substituted with one or more R^{b-2-1} is C₃-C₇ cycloalkyl substituted with 1 or 2 R^{b-2-1}, for example, cyclopropyl substituted with 1 or 2 R^{b-2-1}, cyclobutyl substituted with 1 or 2 R^{b-2-1}, cyclopentyl substituted with 1 or 2 R^{b-2-1}, cyclohexyl substituted with 1 or 2 R^{b-2-1}, or cycloheptyl substituted with 1 or 2 R^{b-2-1}, for another example,

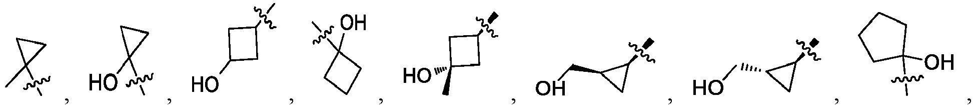

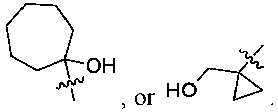

In a certain scheme of the present invention, when R^{b-2} is independently "C₃-C₈ cycloalkyl substituted with one or more OH," the C₃-C₈ cycloalkyl is C₃-C₆ cycloalkyl, for example, cyclobutyl.

In a certain scheme of the present invention, when R^{b-2} is independently 4- to 12-membered heterocycloalkyl having 1-4 heteroatoms being O, the "4- to 12-membered heterocycloalkyl having 1-4 heteroatoms being O" is "4- to 6-membered heterocycloalkyl having 1 heteroatom being O"; for example, tetrahydrofuranyl or tetrahydropyranyl, for another example, 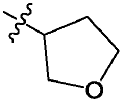 or

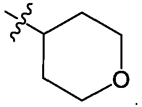

In a certain scheme of the present invention, when R^{b-2} is independently "4- to 12-membered heterocycloalkyl having 1-4 heteroatoms being O and substituted with one or more R^{b-2-2}," the "4- to 12-membered heterocycloalkyl having 1-4 heteroatoms being O and substituted with one or more R^{b-2-2}" is "4- to 6-membered heterocycloalkyl having 1 heteroatom being O and substituted with 1 or 2 R^{b-2-2}"; for example, "tetrahydrofuranyl substituted with 1 or 2 R^{b-2-2}" or "tetrahydropyranyl substituted with 1 or 2 R^{b-2-2}," for another example, 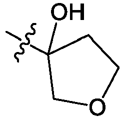 or

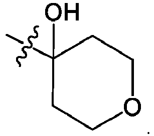

In a certain scheme of the present invention, when R^{b-2-1} and R^{b-2-2} are independently C₁-C₆ alkyl, the C₁-C₆ alkyl is C₁-C₃ alkyl; preferably methyl, ethyl, n-propyl, or isopropyl, for example, methyl.

In a certain scheme of the present invention, when R^{b-2-1} and R^{b-2-2} are independently "C₁-C₆ alkyl substituted with one or more OH," the C₁-C₆ alkyl is C₁-C₃ alkyl; preferably methyl, ethyl, n-propyl, or isopropyl, for example, methyl.

In a certain scheme of the present invention, when R³⁻¹ is C₁-C₆ alkyl, the C₁-C₆ alkyl is C₁-C₃ alkyl; preferably methyl, ethyl, n-propyl, or isopropyl, for example, methyl.

In a certain scheme of the present invention, when R³⁻² and R³⁻³ are independently C₁-C₆ alkyl, the C₁-C₆ alkyl is C₁-C₃ alkyl; preferably methyl, ethyl, n-propyl, or isopropyl, for example, methyl.

In a certain scheme of the present invention, when R³ is 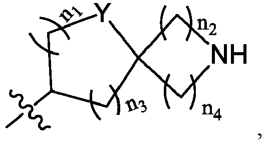 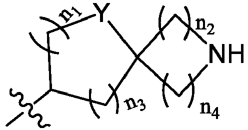 is

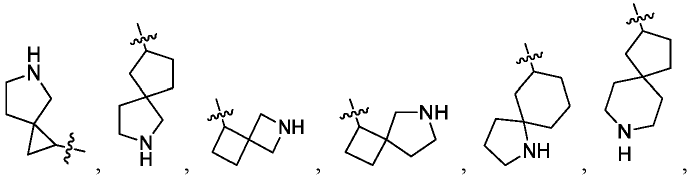

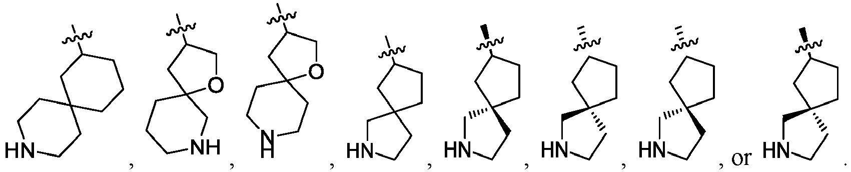

In a certain scheme of the present invention, when R³ is "4- to 12-membered heterocycloalkyl having 1-4 heteroatoms being O" and substituted with one or more R^{b-3}, the "4-to 12-membered heterocycloalkyl having 1-4 heteroatoms being O" and substituted with one or more R^{b-3} is "4- to 6-membered heterocycloalkyl having 1-2 heteroatoms being O" and substituted with 1 or 2 R^{b-3}, for example, oxetanyl substituted with 1 or 2 R^{b-3}, tetrahydrofuranyl substituted with 1 or 2 R^{b-3}, or "tetrahydropyranyl substituted with 1 or 2 R^{b-3}," for another example,

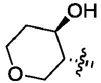

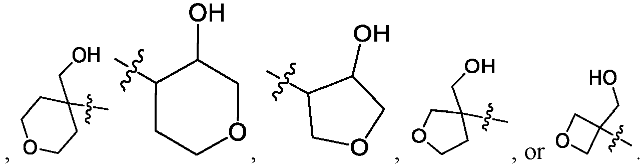

In a certain scheme of the present invention, when R^{b-3} is independently "C₁-C₆ alkyl substituted with one or more OH," the C₁-C₆ alkyl is C₁-C₃ alkyl; preferably methyl, ethyl, n-propyl, or isopropyl, for example, methyl.

In a certain scheme of the present invention, when R³ is "4- to 12-membered heterocycloalkyl having 1-4 heteroatoms being O" and substituted with one or more OH, the "4-to 12-membered heterocycloalkyl having 1-4 heteroatoms being O" and substituted with one or more OH is "4- to 6-membered heterocycloalkyl having 1-2 heteroatom being O" and substituted with one OH, for example, tetrahydrofuranyl substituted with one OH or "tetrahydropyranyl substituted with one OH," for another example,

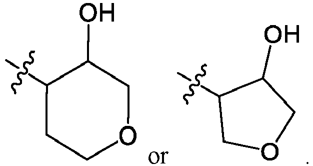

In a certain scheme of the present invention, when R³ is "5- to 10-membered heteroaryl having 1-4 heteroatoms selected from one or more of N, O, and S," the "5- to 10-membered heteroaryl having 1-4 heteroatoms selected from one or more of N, O, and S" is "5- to 6-membered heteroaryl having 1-4 heteroatoms selected from one or more of N," for example, pyrazolyl, for another example,

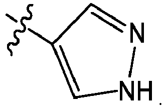

In a certain scheme of the present invention, wherein R³ represents -(CR^{M1}R^{M2})ₘ-(L)ₛ-(CR^{N1}R^{N2})ₜ-M;
wherein R^{M1}, R^{M2}, R^{N1}, and R^{N2} each independently represent hydrogen, and C₁-C₆ alkyl and C₃-C₆ cycloalkyl substituted with 0, 1, 2 selected from hydroxyl, C₁-C₆ alkyl, and halogen; or R^{M1}, R^{M2}, R^{N1}, and R^{N2} each independently form a 3- to 6-membered ring together with carbon atoms to which they are jointly attached, and the ring optionally contains 0, 1, 2 heteroatoms selected from O, N, and S; further, the ring can also be optionally substituted with 0, 1, 2 substituents selected from halogen, C₁-C₆ alkyl, and hydroxyl;
wherein L represents -CR^{Q1}R^{Q2}- or -C₃-C₆ cycloalkyl-; wherein R^{Q1} and R^{Q2} each independently represent hydrogen, and C₁-C₆ alkyl and C₃-C₆ cycloalkyl substituted with 0, 1, 2 selected from hydroxyl, C₁-C₆ alkyl and halogen; or R^{Q1} and R^{Q2} each independently form a 3- to 6-membered ring together with carbon atoms to which they are jointly attached, and the ring optionally contains 0, 1, 2 heteroatoms selected from O, N, and S; further, the ring can also be optionally substituted with 0, 1, 2 substituents selected from halogen, C₁-C₆ alkyl, and hydroxyl;
wherein M represents hydrogen, hydroxyl, or C₁-C₆ alkyl and C₃-C₆ cycloalkyl substituted with 0, 1, 2 selected from hydroxyl, C₁-C₆ alkyl, and halogen;
wherein m, s, and t each independently represent 0, 1, 2, 3;
wherein at least one group in -(CR^{M1}R^{M2})ₘ-(L)ₛ-(CR^{N1}R^{N2})ₜ-M is substituted with hydroxyl.

In a certain scheme of the present invention, R¹ is CF₃, Cl, Br, or CN.

In a certain scheme of the present invention, R¹ is CF₃.

In a certain scheme of the present invention, R⁵ is H, halogen (F), or C₁-C₆ alkoxy (OCH₃).

In a certain scheme of the present invention, R⁵ is H.

In a certain scheme of the present invention, X is C(R⁴).

In a certain scheme of the present invention, Z is CH.

In a certain scheme of the present invention, R² is H, halogen, "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, "4- to 12-membered heterocycloalkenyl having 1-4 heteroatoms selected from one or more of N, O, and S," C₁-C₆ alkoxy, or CN.

In a certain scheme of the present invention, R² is H, "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N and O" and substituted with one or more R^{a-2}, or CN.

In a certain scheme of the present invention, R^{a-1} and R^{a-2} are independently C₁-C₆ alkyl.

In a certain scheme of the present invention, R² is H, F, CN, -OCH₃,

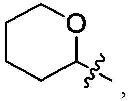

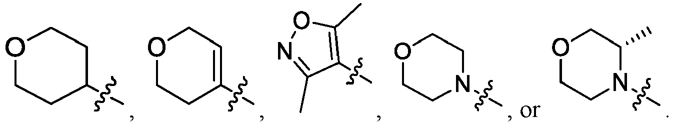

In a certain scheme of the present invention, R² is H, CN,

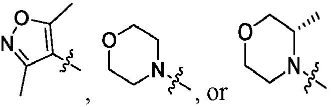

In a certain scheme of the present invention, p₁ is 0 or 3, for example, 3.

In a certain scheme of the present invention, when Y is O, n₁ is 1 and n₃ is 0 or 1; preferably, when Y is O, n₁ is 1 and n₃ is 0.

In a certain scheme of the present invention, when the number of R^{b-1} is one, R^{b-1} is OH, - NR^{b-1-1}R^{b-1-2}, or "C₁-C₆ alkyl substituted with one or more R^{b-1-3}"; when the number of R^{b-1} is plural, at least one R^{b-1} is OH or "C₁-C₆ alkyl substituted with one or more OH."

In a certain scheme of the present invention, when the number of R^{b-2} is one, R^{b-2} is OH, C₃-C₈ cycloalkyl substituted with one or more R^{b-2-1}, or "4- to 12-membered heterocycloalkyl having 1-4 heteroatoms being O and substituted with one or more R^{b-2-2}"; when the number of R^{b-2} is plural, at least one R^{b-2} is OH, C₃-C₈ cycloalkyl substituted with one or more R^{b-2-1}, or "4- to 12-membered heterocycloalkyl having 1-4 heteroatoms being O and substituted with one or more R^{b-2-2}"; wherein, when the number of R^{b-2-1} is one, R^{b-2-1} is OH or "C₁-C₆ alkyl substituted with one or more OH"; when the number of R^{b-2-1} is plural, at least one R^{b-2-1} is OH or "C₁-C₆ alkyl substituted with one or more OH"; when the number of R^{b-2-2} is one, R^{b-2-2} is OH or "C₁-C₆ alkyl substituted with one or more OH"; when the number of R^{b-2-2} is plural, at least one R^{b-2-2} is OH or "C₁-C₆ alkyl substituted with one or more OH."

In a certain scheme of the present invention, when the number of R^{b-3} is one, R^{b-3} is OH, or "C₁-C₆ alkyl substituted with one or more OH"; when the number of R^{b-3} is plural, at least one R^{b-3} is OH or "C₁-C₆ alkyl" substituted with one or more OH."

In a certain scheme of the present invention, R³ is C₁-C₆ alkyl substituted with one or more R^{b-2}, 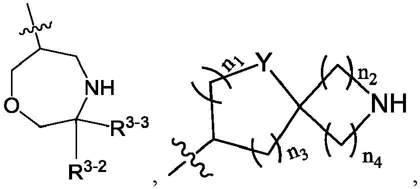 or "4- to 12-membered heterocycloalkyl having 1-4 heteroatoms being O" and substituted with one or more R^{b-3}.

In a certain scheme of the present invention, R³ is

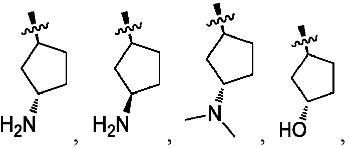

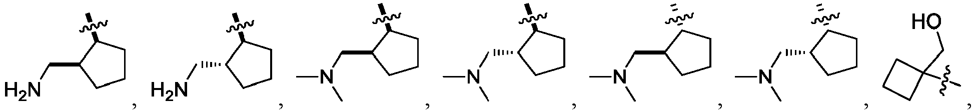

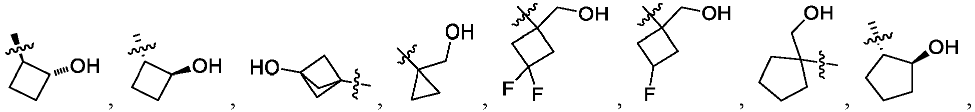

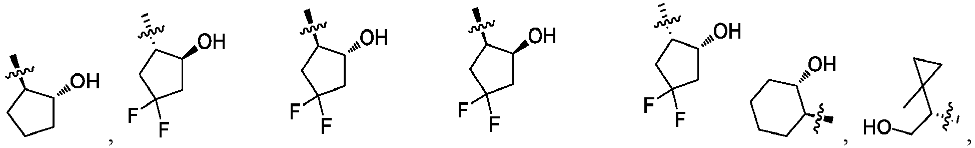

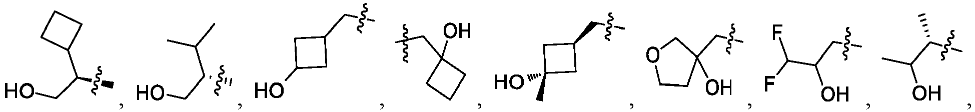

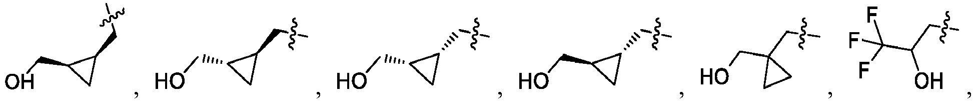

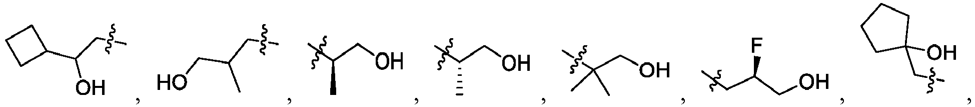

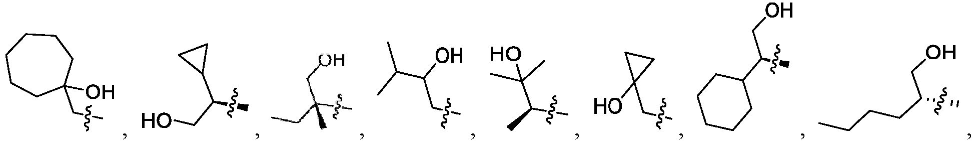

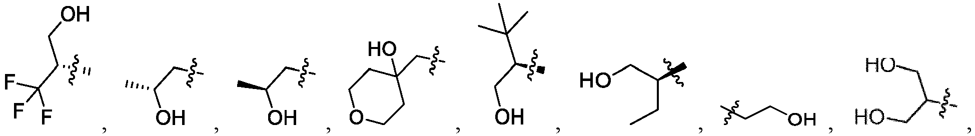

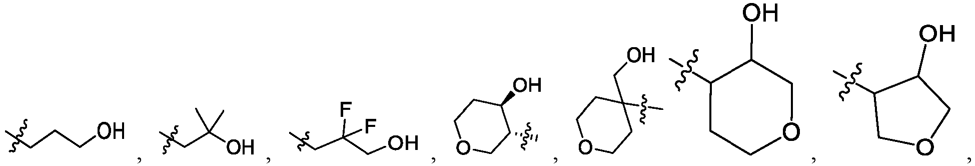

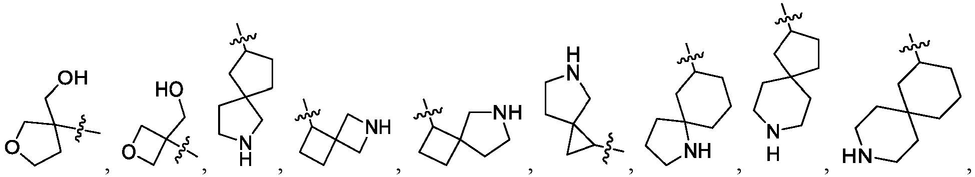

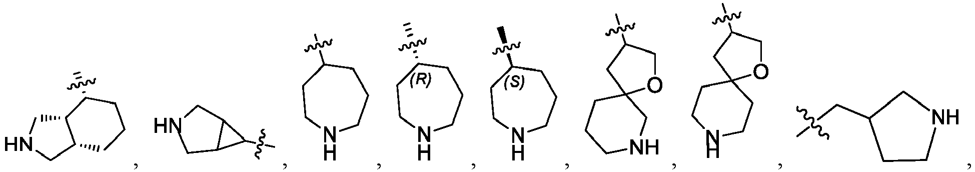

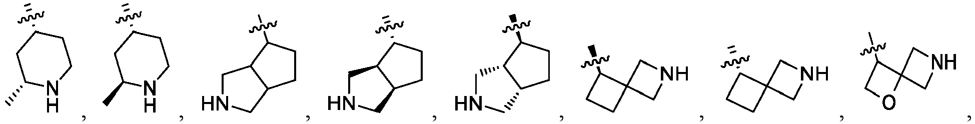

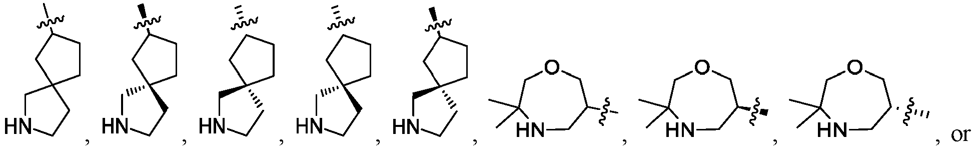

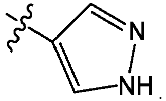

In a certain scheme of the present invention, R³ is

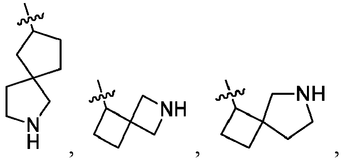

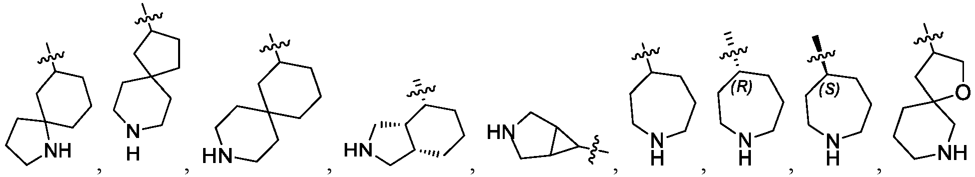

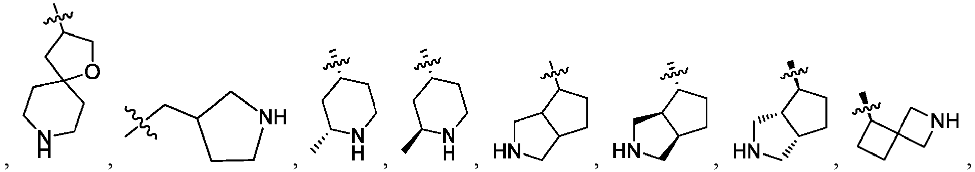

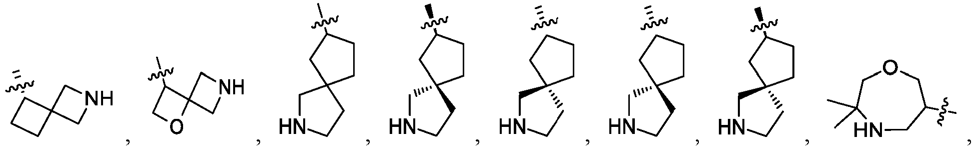

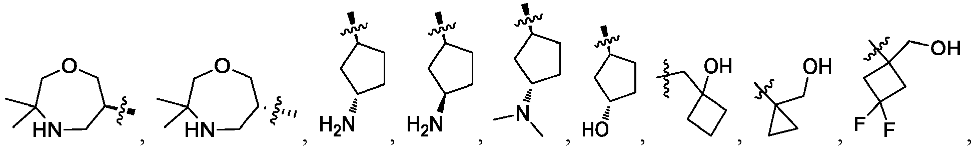

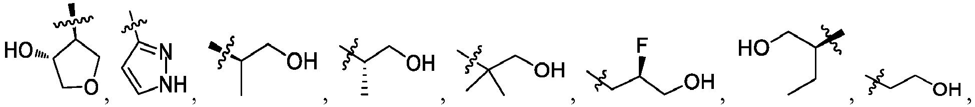

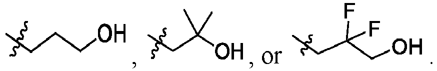

In a certain scheme of the present invention, the compound as represented by formula I-A is 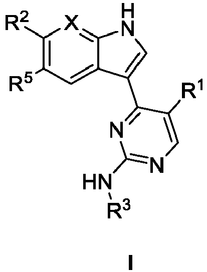 wherein,
R¹ is CF₃, F, Cl, Br, or CN;
R⁵ is H or halogen;
X is N or C(R⁴), and R⁴ is -P(=O)Me₂;
R² is H, halogen, "5- to 10-membered heteroaryl having 1-4 heteroatoms selected from one or more of N, O, and S," "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, C₂-C₆ alkynyl, or CN;
R^{a-1} and R^{a-2} are independently CN, oxo, C₁-C₆ alkyl substituted with one or more R^{a-1-1}, NH₂, OH, or C₁-C₆ alkyl; R^{a-1-1} is independently CN, OH, or halogen;
R³ is C₃-C₈ cycloalkyl substituted with one or more R^{b-1}, C₁-C₆ alkyl substituted with one or more R^{b-2}, 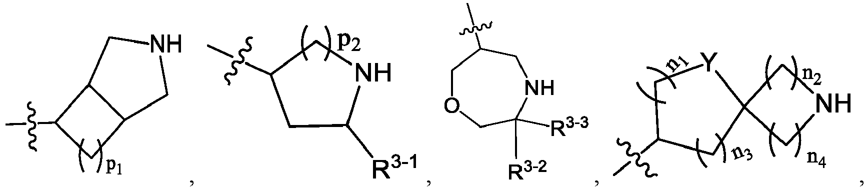 "4- to 12-membered heterocycloalkyl having 1-4 heteroatoms being O" and substituted with one or more OH, 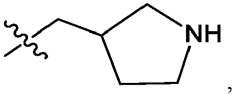 or "5- to 10-membered heteroaryl having 1-4 heteroatoms selected from one or more of N, O, and S";
R^{b-1} is independently halogen, OH, -NR^{b-1-1}R^{b-1-2}, or "C₁-C₆ alkyl substituted with one or more R^{b-1-3}";
R^{b-1-1} and R^{b-1-2} are independently H or C₁-C₆ alkyl;
R^{b-1-3} is independently OH or NR^{b-1-4}R^{b-1-5}; R^{b-1-4} and R^{b-1-5} are independently H or C₁-C₆ alkyl;
R^{b-2} is independently OH, halogen, or "C₃-C₈ cycloalkyl substituted with one or more OH";
p₁ is 0, 1, 2, or 3;
p₂ is 2 or 3, and R³⁻¹ is H or C₁-C₆ alkyl;
R³⁻² and R³⁻³ are independently H, C₁-C₆ alkyl, or "C₁-C₆ alkyl substituted with one or more halogens";
Y is O or CH₂, n₁ is 1 or 2, n₂, n₃, and n₄ are independently 0, 1, 2, or 3, and n₂ and n₄ are not 0 at the same time.

In a certain scheme of the present invention, when X is C(R⁴) and R⁴ is -P(=O)Me₂, R² is H, halogen, C₁-C₆ alkoxy or CN; when X is N, R² is "5- to 10-membered heteroaryl having 1-4 heteroatoms selected from one or more of N, O, and S" and substituted with one or more R^{a-1}, "4-to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, "4- to 12-membered heterocycloalkenyl having 1-4 heteroatoms selected from one or more of N, O, and S," or CN.

In a certain scheme of the present invention, when X is C(R⁴) and R⁴ is -P(=O)Me₂, R² is H or CN; when X is N, R² is "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N and O" and substituted with one or more R^{a-2}, or CN.

In a certain scheme of the present invention, when X is C(R⁴) and R⁴ is -P(=O)Me₂, R³ is C₃-C₈ cycloalkyl substituted with one or more R^{b-1}, 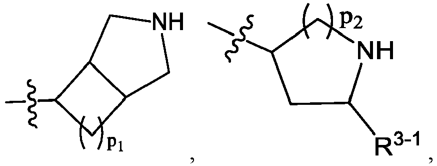 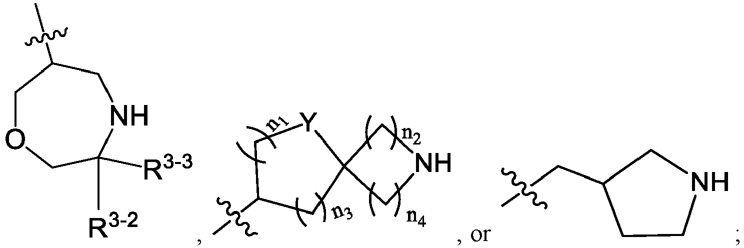 when X is N, R³ is C₃-C₈ cycloalkyl substituted with one or more R^{b-1}, C₁-C₆ alkyl substituted with one or more R^{b-2},

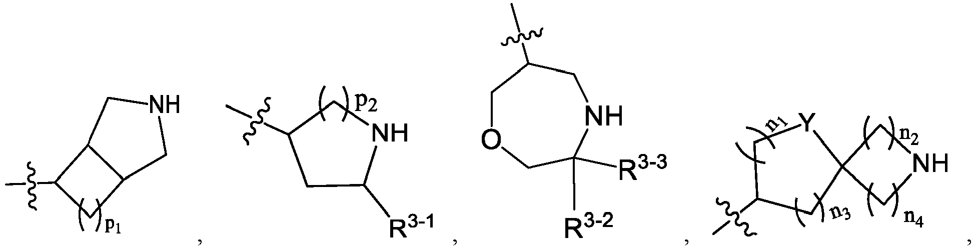

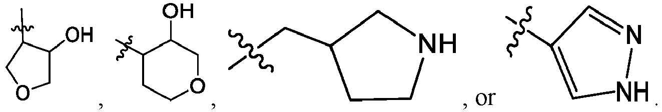

In a certain scheme of the present invention, R³ is C₃-C₈ cycloalkyl substituted with one or more R^{b-1}, C₁-C₆ cycloalkyl substituted with one or more R^{b-2}, 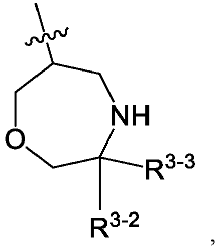 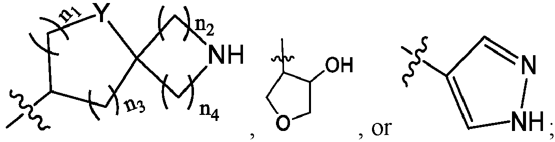 when Y is O, n₁ is 1, and n₃ is 0.

In a certain scheme of the present invention, the compound as represented by formula I-A is not the following compounds: 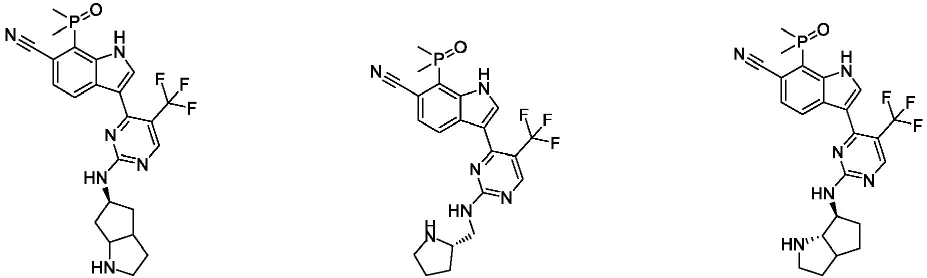 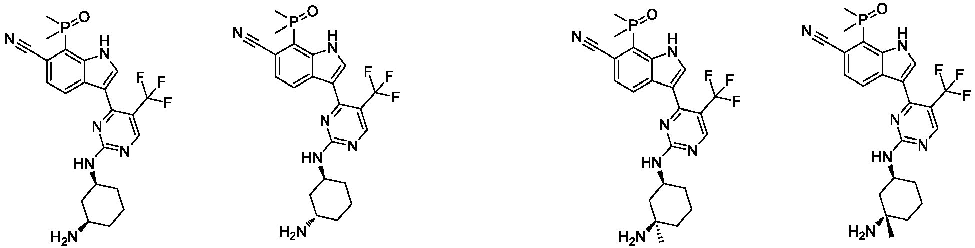 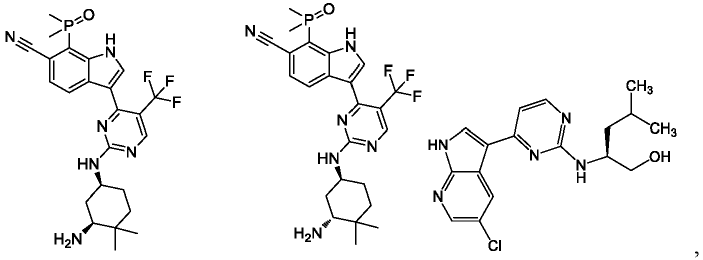 or a pharmaceutically acceptable salt, a stereoisomer, a tautomer, a hydrate, a solvate, an isotopic compound, or a prodrug of any one of the above-mentioned substances.

In a certain scheme of the present invention, the compound as represented by formula I-A is any one of the following compounds:

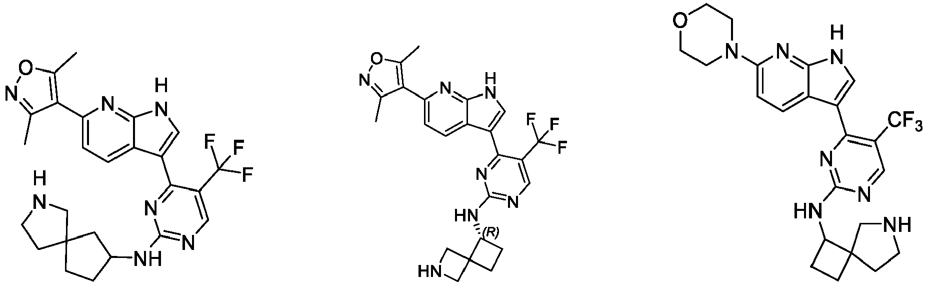

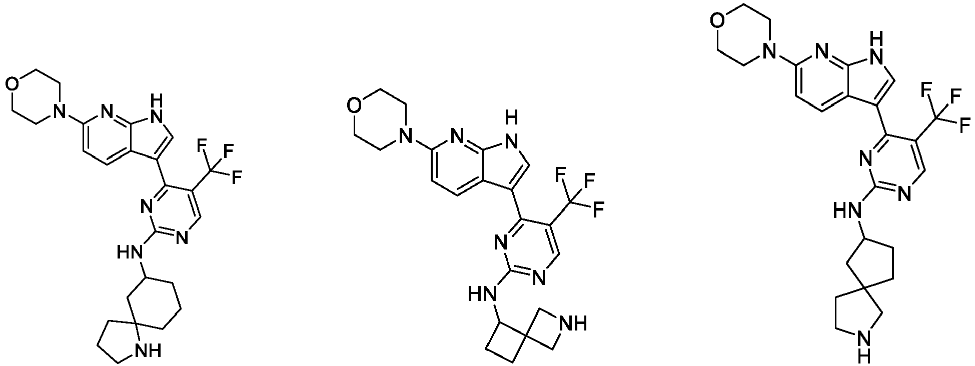

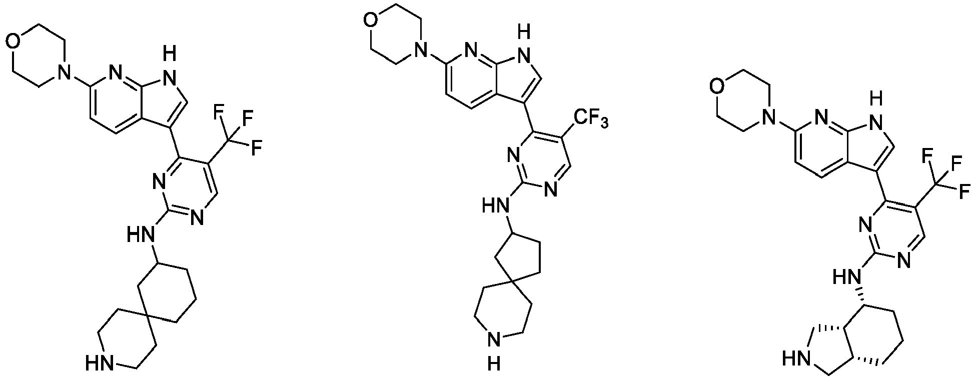

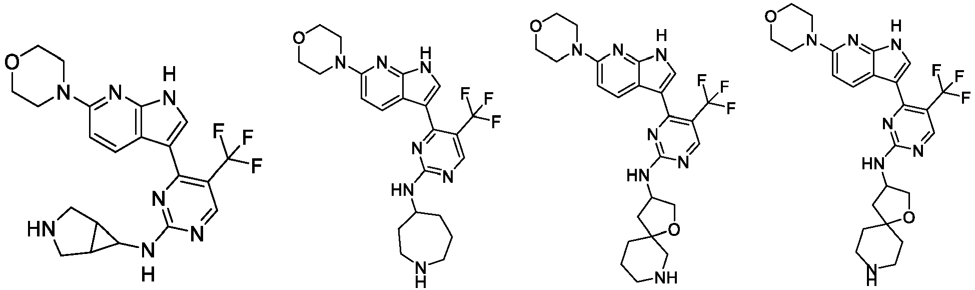

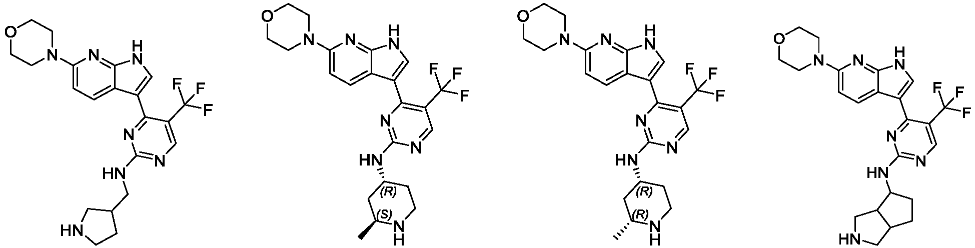

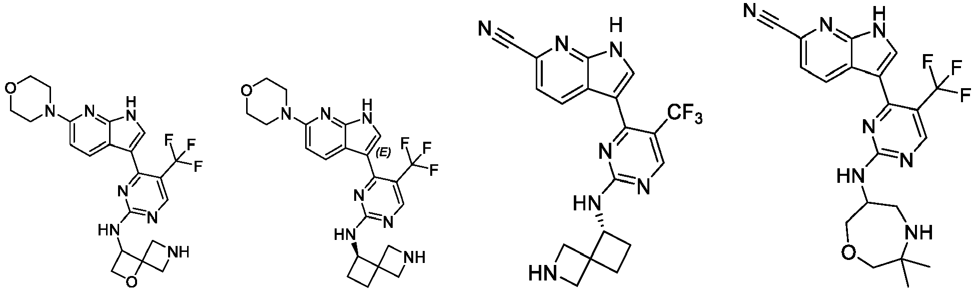

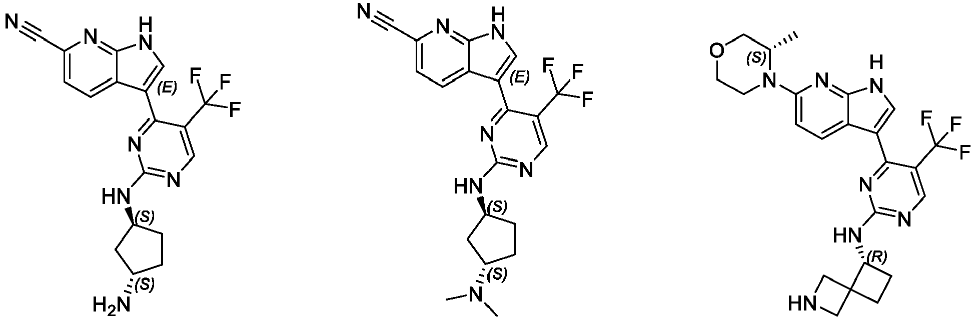

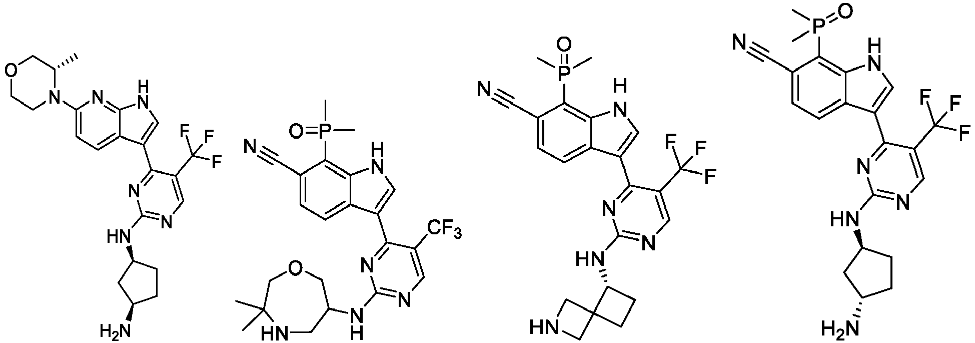

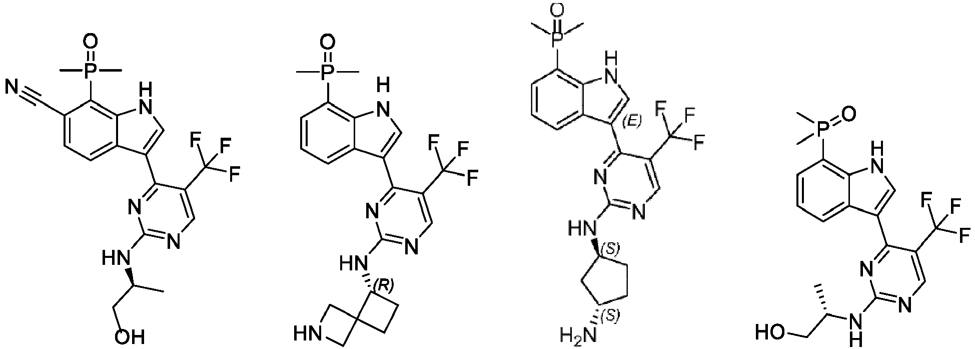

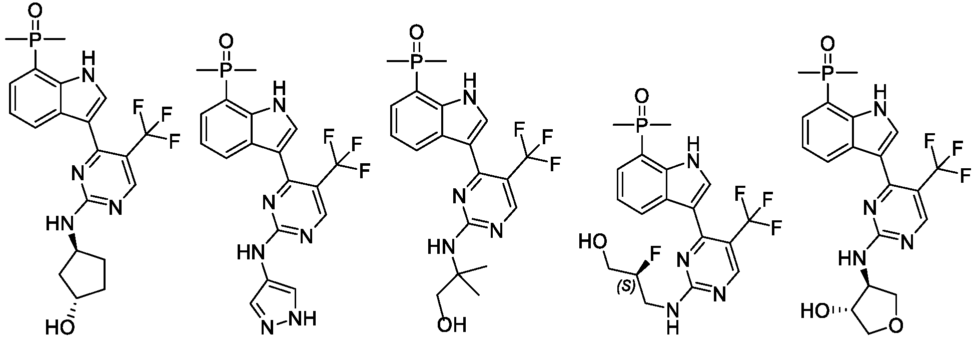

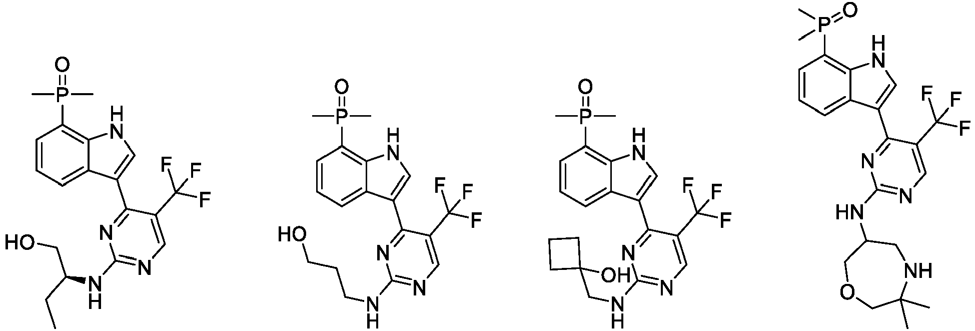

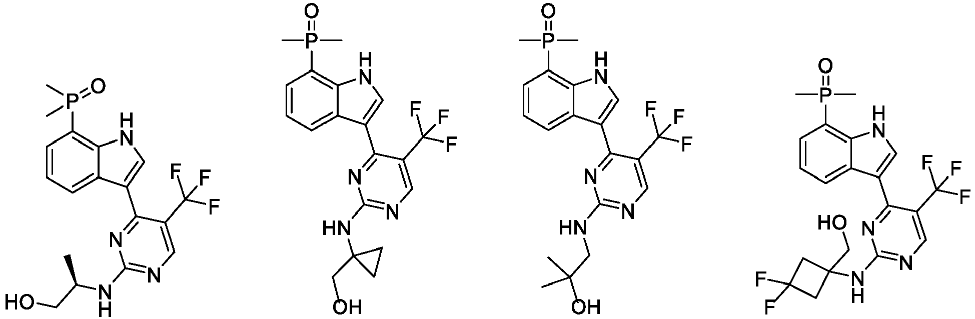

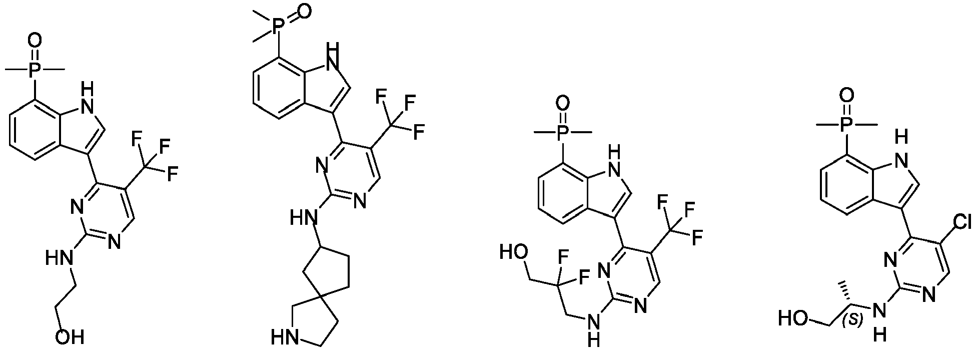

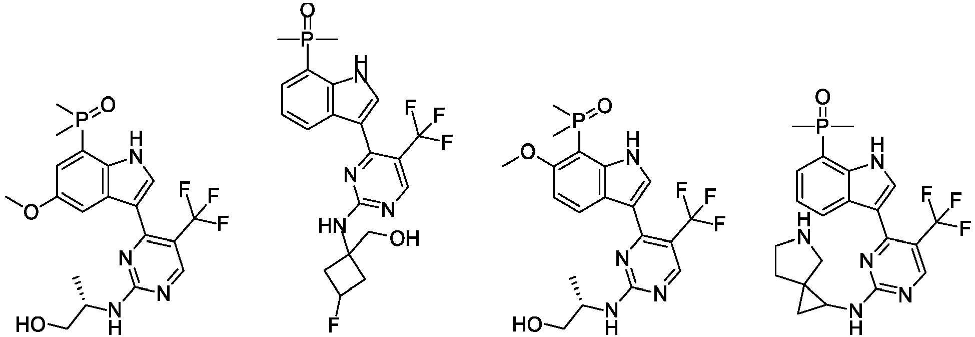

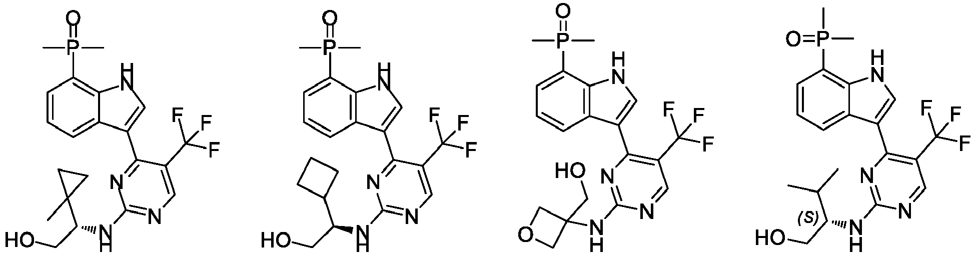

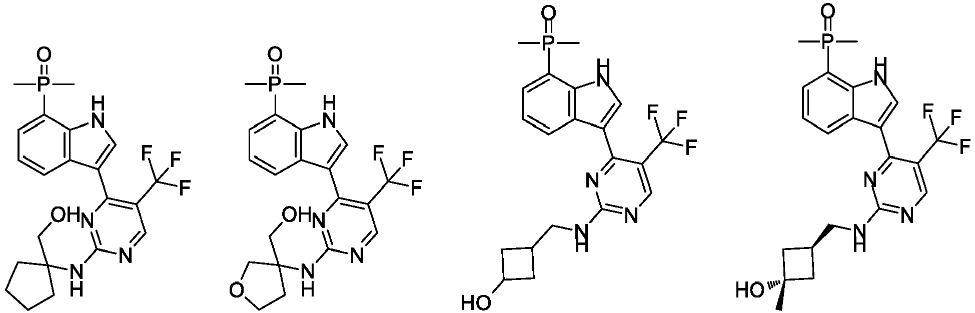

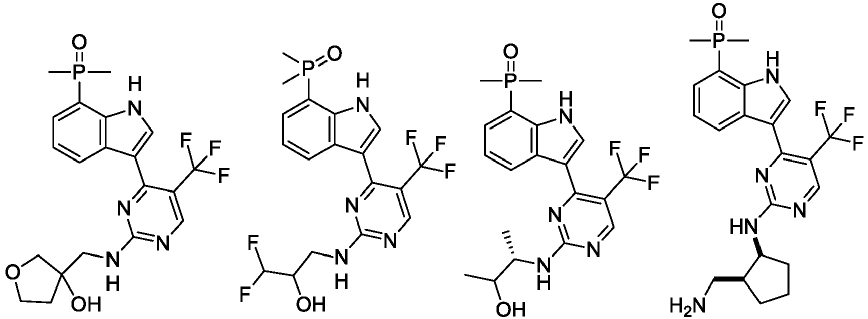

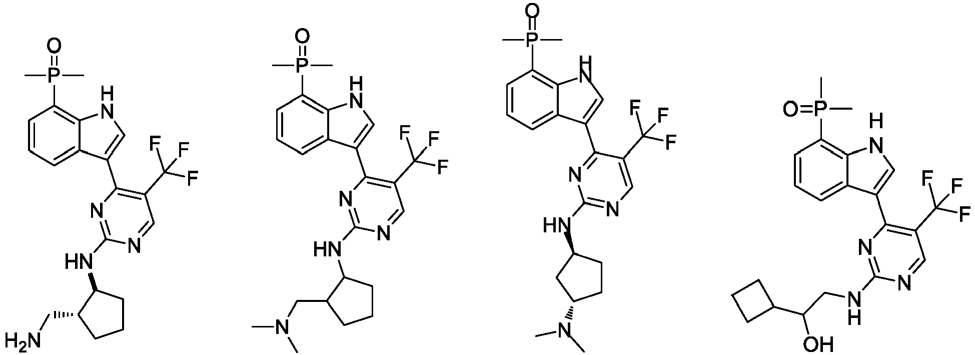

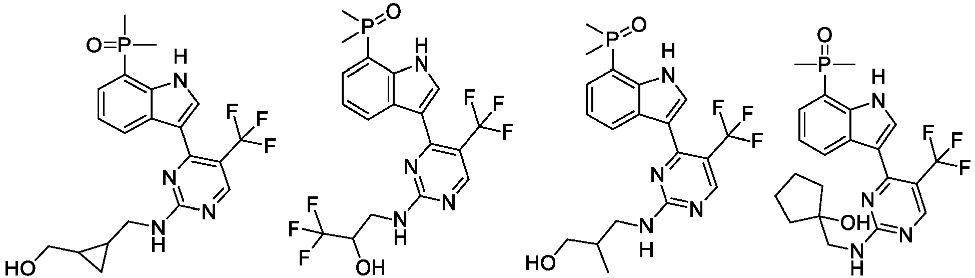

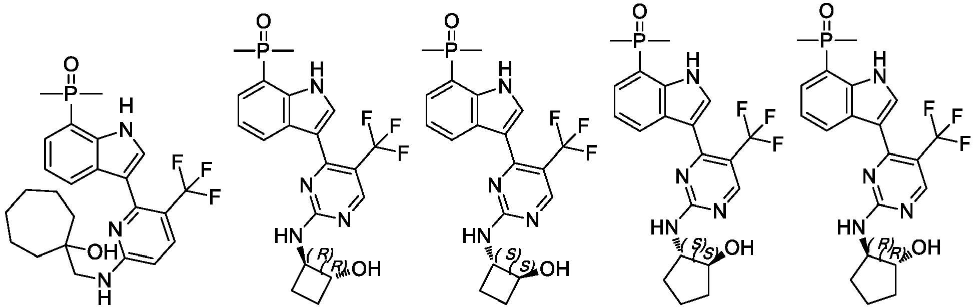

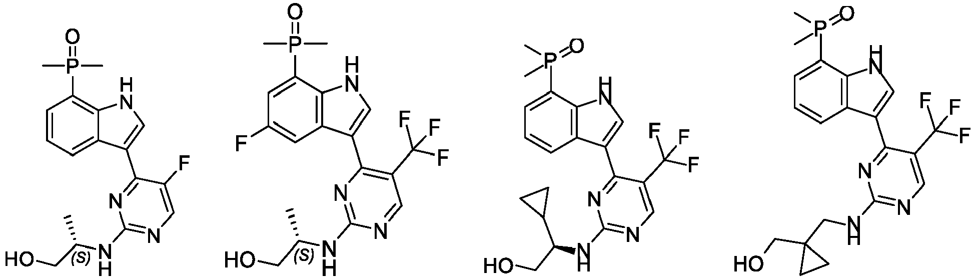

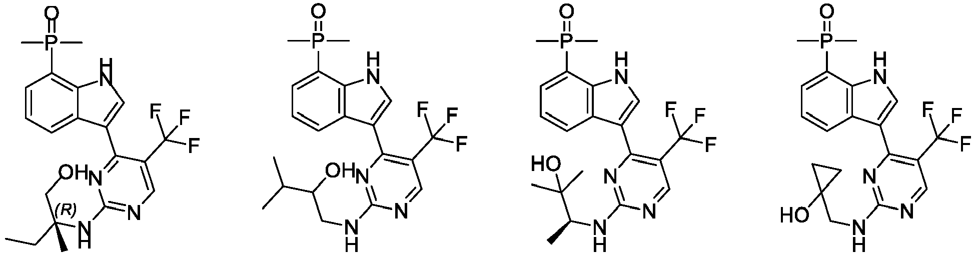

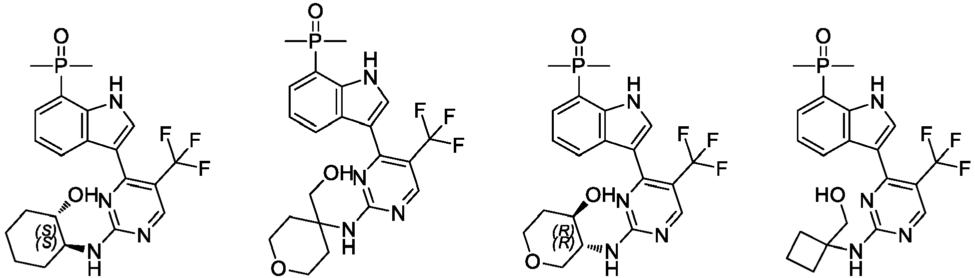

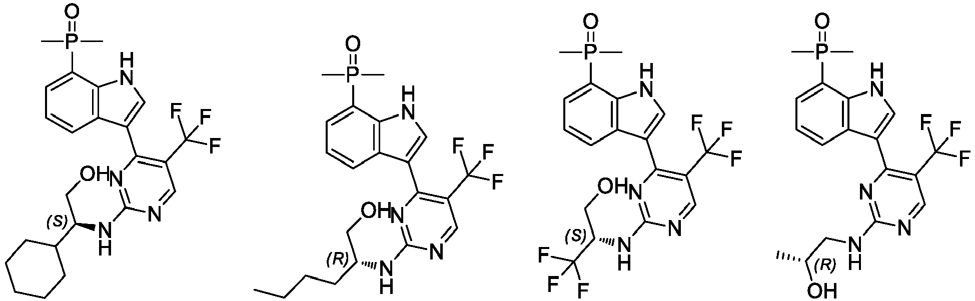

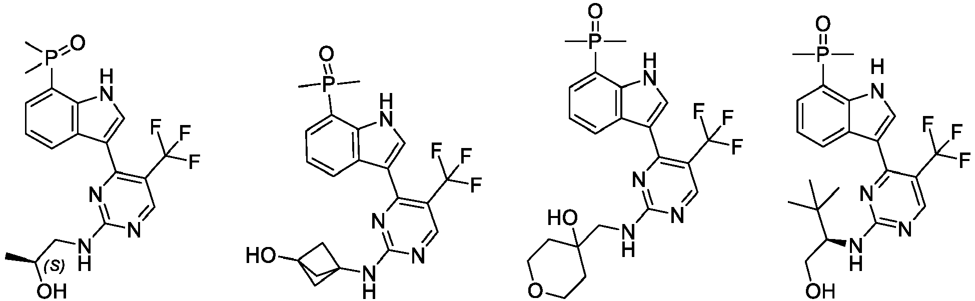

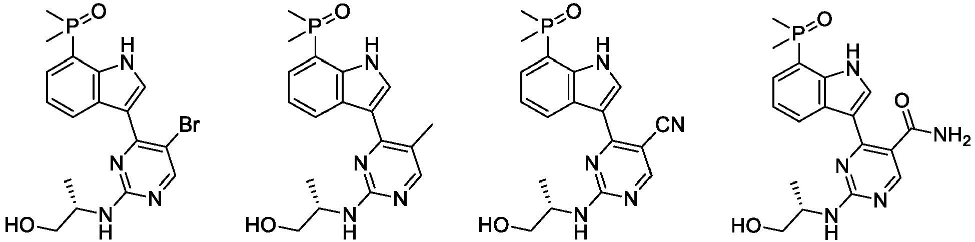

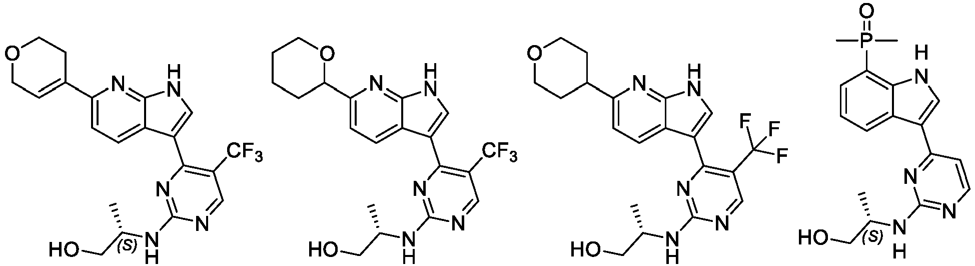

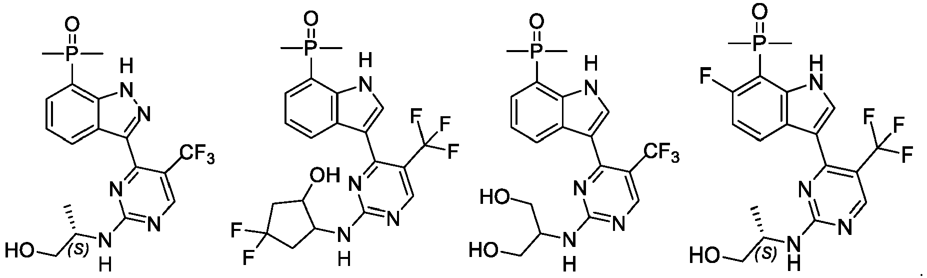

Preferably, the compound as represented by formula I-A is any one of the following compounds:
a compound with a retention time of 2.187 min under the following conditions, which is one stereoisomer in 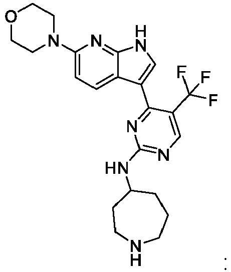 chromatographic column: Chiralpak AD-3, 250 mm × 30 mm, 10 µm; mobile phases: Phase A of carbon dioxide, and Phase B of 0.05% diethylamine/isopropanol, gradient: as for Phase B, from 5% to 40% in 2 minutes, holding Phase B at 40% for 1.2 minutes, and then holding Phase B at 5% for 0.8 minutes; flow rate: 2.5 mL/min;
a compound with a retention time of 2.877 min under the following conditions, which is one stereoisomer in 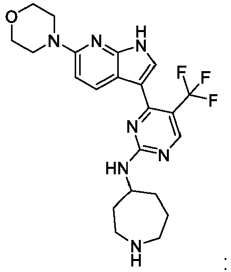 chromatographic column: Chiralpak AD-3, 250 mm × 30 mm, 10 µm; mobile phases: Phase A of carbon dioxide, and Phase B of 0.05% diethylamine/isopropanol, gradient: as for Phase B, from 5% to 40% in 2 minutes, holding Phase B at 40% for 1.2 minutes, and then holding Phase B at 5% for 0.8 minutes; flow rate: 2.5 mL/min;
a compound with a retention time of 2.343 min under the following conditions, which is one stereoisomer in 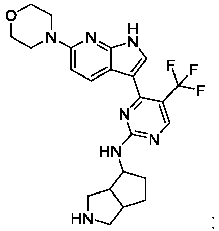 chromatographic column: Chiralpak IG-3, 250 mm × 30 mm, 10 µm; mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes; flow rate: 4 mL/min;
a compound with a retention time of 2.847 min under the following conditions, which is one stereoisomer in 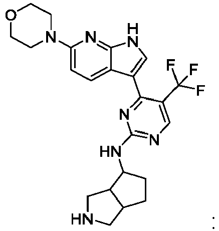 chromatographic column: Chiralpak IG-3, 250 mm × 30 mm, 10 µm; mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes; flow rate: 4 mL/min;
a compound with a retention time of 3.934 min under the following conditions, which is one stereoisomer in 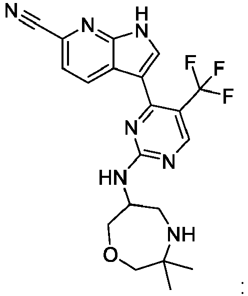 chromatographic column: Chiralpak AD-3, 250 mm × 30 mm, 10 µm; mobile phases: Phase A of carbon dioxide, and Phase B of 0.05% diethylamine/ethanol; gradient: as for Phase B, from 5% to 40% in 4.5 minutes, and holding Phase B at 5% for 1.5 minutes; flow rate: 2.5 mL/min;
a compound with a retention time of 4.355 min under the following conditions, which is one stereoisomer in 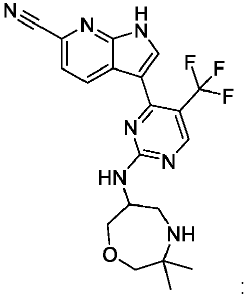 chromatographic column: Chiralpak AD-3, 250 mm × 30 mm, 10 µm; mobile phases: Phase A of carbon dioxide, and Phase B of 0.05% diethylamine/ethanol; gradient: as for Phase B, from 5% to 40% in 4.5 minutes, and holding Phase B at 5% for 1.5 minutes; flow rate: 2.5 mL/min;
a compound with a retention time of 3.788 min under the following conditions, which is one stereoisomer in 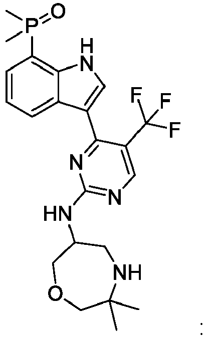 chromatographic column: chromatographic column: Phenomenex-Cellulose-2, 250 mm × 30 mm, 10 µm; mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 mL/min;
a compound with a retention time of 4.110 min under the following conditions, which is one stereoisomer in 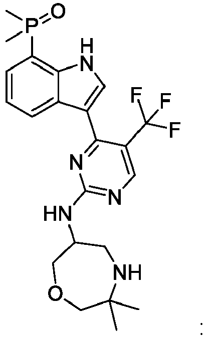 chromatographic column: chromatographic column: Phenomenex-Cellulose-2, 250 mm × 30 mm, 10 µm; mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 mL/min;
a compound with a retention time of 0.887 min under the following conditions, which is one stereoisomer in 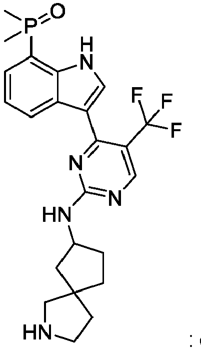 chromatographic column: DAICEL CHIRALPAK IG, 250 mm × 30 mm, 10 µm; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 1.00 min under the following conditions, which is one stereoisomer in 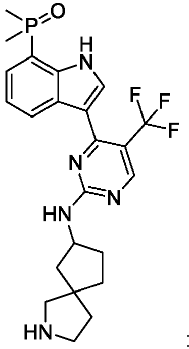 chromatographic column: DAICEL CHIRALPAK IG, 250 mm × 30 mm, 10 µm; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 1.979 min under the following conditions, which is one stereoisomer in 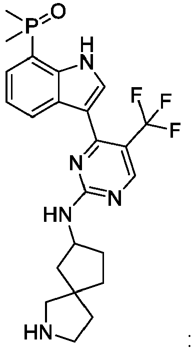 chromatographic column: DAICEL CHIRALPAK IG, 250 mm × 30 mm, 10 µm; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 2.643 min under the following conditions, which is one stereoisomer in 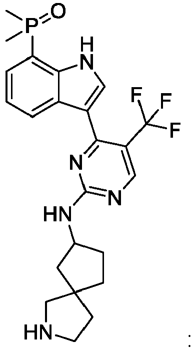 chromatographic column: DAICEL CHIRALPAK IG, 250 mm × 30 mm, 10 µm; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 5.650 min under the following conditions, which is one stereoisomer in 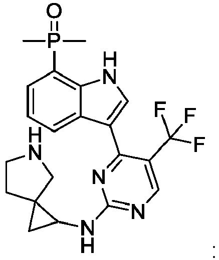 chromatographic column: ChiralPak IG-3, 100 × 4.6 mm, 3 µm; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 mL/min;
a compound with a retention time of 5.985 min under the following conditions, which is one stereoisomer in 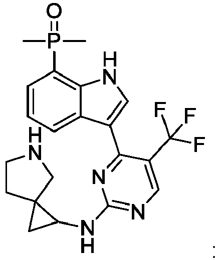 chromatographic column: ChiralPak IG-3, 100 × 4.6 mm, 3 µm; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 mL/min;
a compound with a retention time of 6.338 min under the following conditions, which is one stereoisomer in 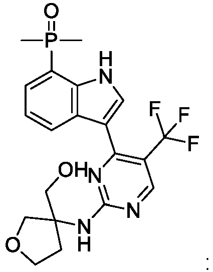 chromatographic column: Chiralpak AD-3, 150 mm * 4.6 mm, 3 um; mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 mL/min;
a compound with a retention time of 7.132 min under the following conditions, which is one stereoisomer in 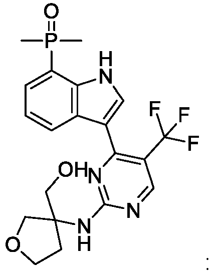 chromatographic column: Chiralpak AD-3, 150 mm * 4.6 mm, 3 um; Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 mL/min;
a compound with a retention time of 1.798 min under the following conditions, which is one stereoisomer in 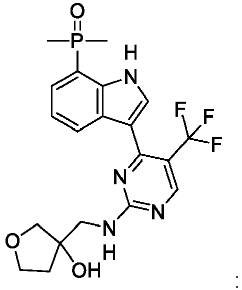 chromatographic column: Chiralpak AD-3; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 2.023 min under the following conditions, which is one stereoisomer in 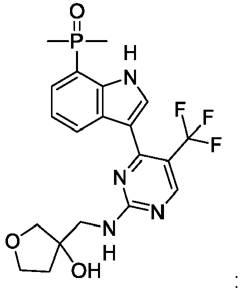 chromatographic column: Chiralpak AD-3; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 5.096 min under the following conditions, which is one stereoisomer in 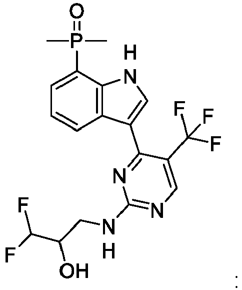 chromatographic column: Chiralpak AD-3; mobile phases: Phase A of carbon dioxide, and Phase B of 0.05% diethylamine/ethanol; gradient: as for Phase B, from 5% to 40% in 5 minutes, then from 40% to 5% in 0.5 minutes, and then holding Phase B at 5% for 1.5 minutes; flow rate: 2.5 mL/min;
a compound with a retention time of 5.388 min under the following conditions, which is one stereoisomer in 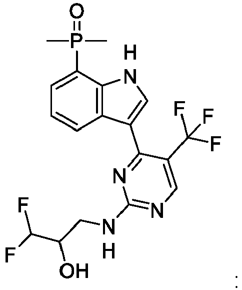 chromatographic column: Chiralpak AD-3; mobile phases: Phase A of carbon dioxide, and Phase B of 0.05% diethylamine/ethanol; gradient: as for Phase B, from 5% to 40% in 5 minutes, then from 40% to 5% in 0.5 minutes, and then holding Phase B at 5% for 1.5 minutes; flow rate: 2.5 mL/min;
a compound with a retention time of 2.177 min under the following conditions, which is one stereoisomer in 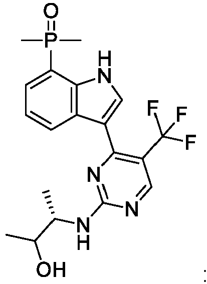 chromatographic column: Chiralpak AS-3, 100 mm * 4.6 mm, 3 µm; mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 mL/min;
a compound with a retention time of 2.318 min under the following conditions, which is one stereoisomer in 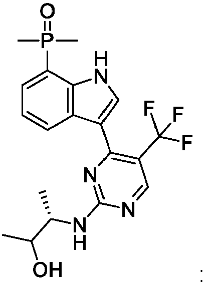 chromatographic column: Chiralpak AS-3, 100 mm * 4.6 mm, 3 µm; mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 mL/min;
a compound with a retention time of 4.512 min under the following conditions, which is one stereoisomer in 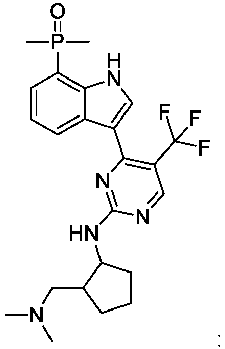 chromatographic column: Chiralpak AD-3, 150 mm * 4.6 mm, 3 µm; mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 milliliter/minute; a compound with a retention time of 6.985 min under the following conditions, which is one stereoisomer in 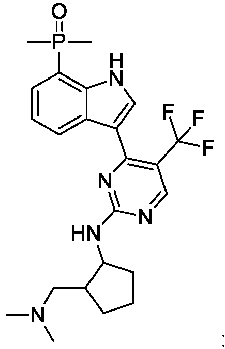 chromatographic column: Chiralpak AD-3, 150 mm * 4.6 mm, 3 µm; mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 milliliter/minute; a compound with a retention time of 6.809 min under the following conditions, which is one stereoisomer in 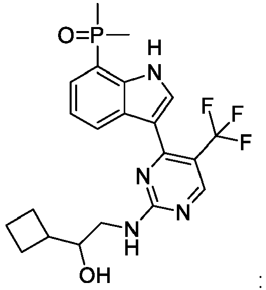 chromatographic column: Chiralpak AD-3, 150 * 4.6 mm, 3 um; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 mL/min;
a compound with a retention time of 7.460 min under the following conditions, which is one stereoisomer in 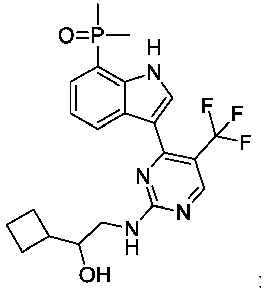 chromatographic column: Chiralpak AD-3, 150 * 4.6 mm, 3 um; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 mL/min;
a compound with a retention time of 6.744 min under the following conditions, which is one stereoisomer in 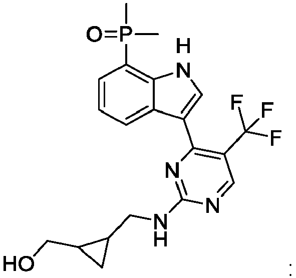 chromatographic column: Chiralpak AD-3, 150 × 4.6 mm, 3 um; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 mL/min;
a compound with a retention time of 7.642 min under the following conditions, which is one stereoisomer in 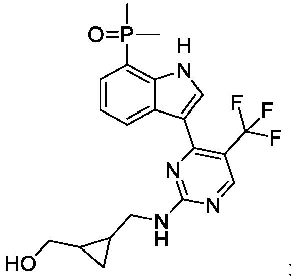 chromatographic column: Chiralpak AD-3, 150 × 4.6 mm, 3 um; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 mL/min;
a compound with a retention time of 4.114 min under the following conditions, which is one stereoisomer in 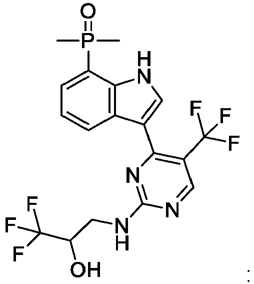 chromatographic column: DAICEL CHIRALPAK AD, 250 mm * 30 mm, 10 µm; mobile phases: Phase A of carbon dioxide; and Phase B of 0.05% diethylamine/ethanol; gradient: as for Phase B, from 5% to 40% in 4.5 minutes, then holding Phase B at 5% for 1.5 minutes; flow rate: 2.5 milliliter/minute;
a compound with a retention time of 4.316 min under the following conditions, which is one stereoisomer in 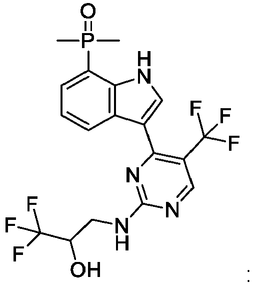 chromatographic column: DAICEL CHIRALPAK AD, 250 mm * 30 mm, 10 µm; mobile phases: Phase A of carbon dioxide; and Phase B of 0.05% diethylamine/ethanol; gradient: as for Phase B, from 5% to 40% in 4.5 minutes, then holding Phase B at 5% for 1.5 minutes; flow rate: 2.5 milliliter/minute;
a compound with a retention time of 4.156 min under the following conditions, which is one stereoisomer in 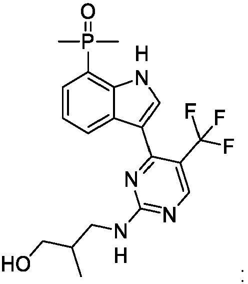 chromatographic column: Chiralpak IG-3, 100 mm * 4.6 mm * 3 µm; mobile phases: Phase A of carbon dioxide, and Phase B of 0.05% diethylamine/methanol; gradient: as for Phase B, from 5% to 40% in 4 minutes, holding Phase B at 40% for 2.5 minutes, and then holding Phase B at 5% for 2.5 minutes; flow rate: 2.8 mL/min;
a compound with a retention time of 4.543 min under the following conditions, which is one stereoisomer in 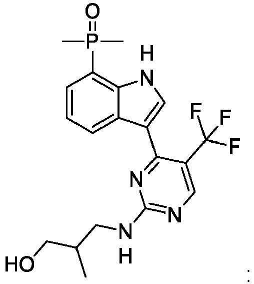 chromatographic column: Chiralpak IG-3, 100 mm * 4.6 mm * 3 µm; mobile phases: Phase A of carbon dioxide, and Phase B of 0.05% diethylamine/methanol; gradient: as for Phase B, from 5% to 40% in 4 minutes, holding Phase B at 40% for 2.5 minutes, and then holding Phase B at 5% for 2.5 minutes; flow rate: 2.8 mL/min;
a compound with a retention time of 1.017 min under the following conditions, which is one stereoisomer in 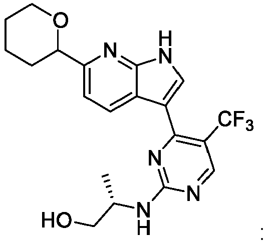 chromatographic column: DAICEL CHIRALCEL OJ, 250 mm * 30 mm, 10 µm; mobile phases: Phase A of carbon dioxide, and Phase B of 0.05% diethylamine/ethanol; gradient: holding Phase B at 40%; flow rate: 2.8 milliliter/minute;
a compound with a retention time of 2.833 min under the following conditions, which is one stereoisomer in 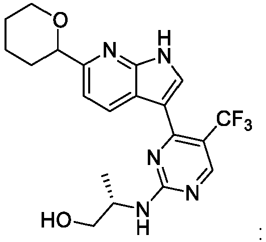 chromatographic column: DAICEL CHIRALCEL OJ, 250 mm * 30 mm, 10 µm; mobile phases: Phase A of carbon dioxide, and Phase B of 0.05% diethylamine/ethanol; gradient: holding Phase B at 40%; flow rate: 2.8 milliliter/minute;
a compound with a retention time of 1.615 min under the following conditions, which is one stereoisomer in 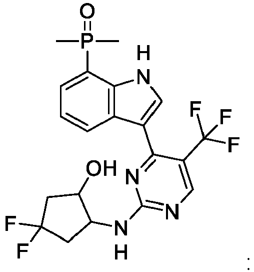 Chiralpak AD-3 50 mm * 4.6 mm, 3 um; mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 1.917 min under the following conditions, which is one stereoisomer in 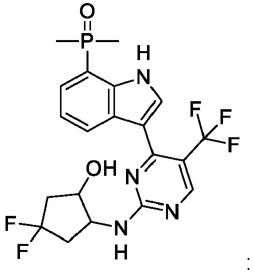 Chiralpak AD-3 50 mm * 4.6 mm, 3 um; mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes, flow rate: 4 mL/min;
a compound with a retention time of 5.293 min under the following conditions, which is one stereoisomer in 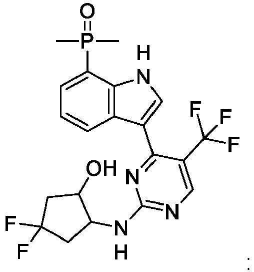 Chiralpak AD-3 150 mm * 4.6 mm, 3 um; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute;
a compound with a retention time of 5.960 min under the following conditions, which is one stereoisomer in 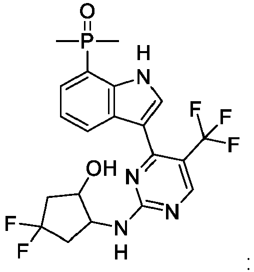 Chiralpak AD-3 150 mm * 4.6 mm, 3 um; mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute.

The above-mentioned testing conditions for the retention time are not limiting for the compound. As long as the retention time obtained by using the above-mentioned testing conditions for measurement is the same as that described above or within an error range, and this compound is one stereoisomer in the compound defined by the above-mentioned retention time, this compound shall fall within the protection scope of the present invention.

The compound of the present invention can be prepared by applying synthetic methods known in the art and synthetic methods summarized in the schemes set forth below.

### General synthetic method 1:

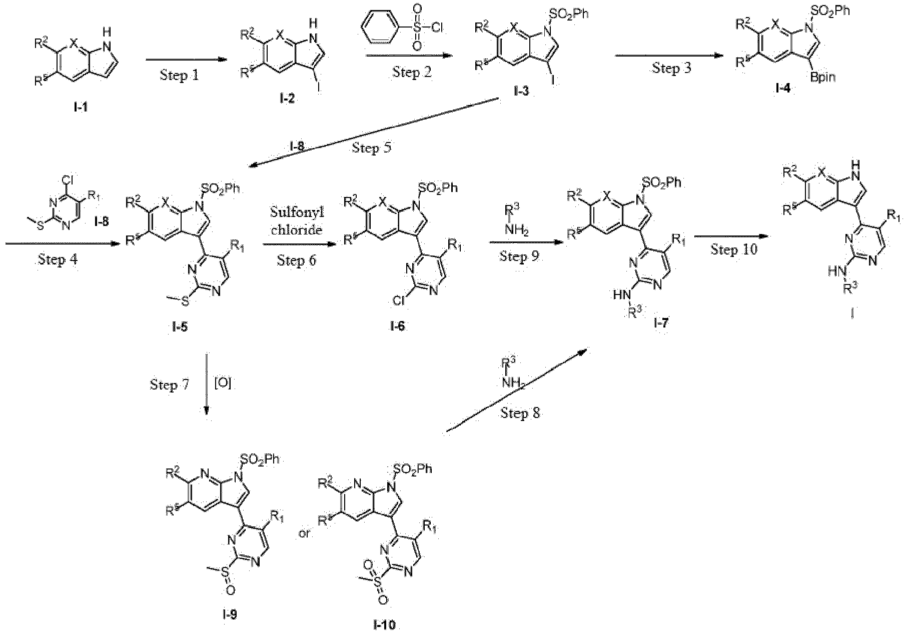

As shown in general synthetic method 1, a compound represented by formula (I-1) reacts with a suitable halogenating reagent (such as but not limited to elementary iodine) to afford a halogenated product I-2, the product I-2 selects a suitable protecting group (such as but not limited to benzenesulfonyl) to protect an NH functional group in the structure, and a compound having chemical formula (I-2) can be protected by the benzenesulfonyl at a low temperature (for example, 0°C) to afford a compound I-3. Subsequently, under a suitable catalyst, a compound I-5 is generated by a reaction with a compound I-8 by one-pot stile coupling, or the compound I-3 is firstly converted from halogenation into a corresponding borate (or boric acid) compound I-4 under suitable reaction conditions, and the compound I-4 reacts with the compound I-8 under a suitable catalyst by Suzuki coupling to afford the compound I-5; the compound I-5 is heated under a suitable chlorinating agent (such as but not limited to SO₂Cl₂) condition to afford a chlorinated intermediate I-6, or oxidizes thiomethyl ether into sulphone I-10 (sulfoxide I-9 or "a mixture of sulphone I-10 and sulfoxide I-9") by a suitable oxidizing agent (such as but not limited to m-CPBA). The chlorinated intermediate I-6 (I-10, or a mixture of I-9/I-10) is heated under a suitable basic condition (such as but not limited to DIEA) to react with formula R³NH₂ to afford a compound of formula (I-7), and the compound of formula (I-7) is heated under a suitable basic condition (such as but not limited to NaOH) to be deprotected to afford a final product of formula (I). If R³ group contains other protecting groups (such as but not limited to a Boc protecting group), the compound I affords a final compound under a suitable acidic condition (such as but not limited to TFA/DCM).

General synthetic method 2: in a compound as represented by formula I, when X is N, we inventively synthesize a novel key intermediate compound II-6, by which a final compound II (corresponding to a compound I with X being N) of the present invention can be conveniently synthesized.

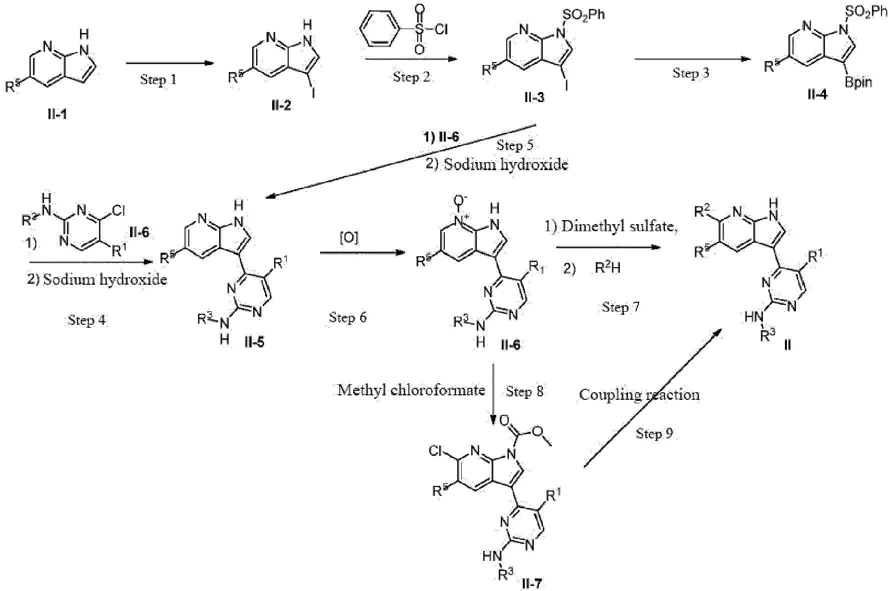

As shown in the general synthetic method 2, a compound represented by formula (II-1) reacts with a suitable halogenating reagent (such as but not limited to elementary iodine) to afford a halogenated product II-2, and the product II-2 selects a suitable protecting group (such as but not limited to benzenesulfonyl) to protect an NH functional group in the structure, for example, the product II-2 can be protected by the benzenesulfonyl under a reaction with benzenesulfonyl chloride at a low temperature (for example, 0°C) to afford a compound II-3. Subsequently, under a suitable catalyst, a compound II-5 is generated by a reaction with a compound II-6 by one-pot stile coupling, or the compound II-3 is firstly converted from halogenation into a corresponding borate (or boric acid) compound II-4 under suitable reaction conditions, and the compound II-4 reacts with the compound II-6 by Suzuki coupling under a suitable catalyst to afford the compound II-5; the compound II-5 affords a nitrogen-oxygen compound II-6 under a suitable oxidation condition (such as but not limited to m-CPBA), the nitrogen-oxygen compound II-6 and a suitable activating reagent (such as but not limited to dimethyl sulfate) generate active pyridine azoxymethyl ether under a heating condition, and the active intermediate reacts with an amino compound R²H under the presence of a suitable base (such as but not limited to DIEA) to afford a compound as represented by formula (II). If R³ group contains other protecting groups (such as but not limited to a Boc protection group), the compound II affords a final product under a suitable acidic condition (such as but not limited to TFA/DCM).

The compound II-5 is heated under a suitable halogenation condition and under a suitable chlorinating reagent or brominating reagent (such as but not limited to methyl chloroformate) condition to afford a chlorinated intermediate II-7 or a corresponding brominated intermediate. Under a suitable catalytic condition, the chlorinated intermediate II-7 reacts with a corresponding borate/boronic acid or amino compound R²H by a suitable coupling condition (such as but not limited to Suzuki coupling or Buchwald coupling) to afford a compound of formula (II). If R³ group contains other protecting groups (such as but not limited to a Boc protecting group), the compound II affords a final compound under a suitable acidic condition (such as but not limited to TFA/DCM).

General synthetic method 3: in a compound as represented by formula I, when X is C(R⁴) and R⁴ is -P(=O)Me₂, we inventively synthesize a novel key intermediate compound III-4, by which a final compound III (corresponding to a compound I with X being C(R⁴) and R⁴ being - P(=O)Me₂) of the present invention can be conveniently synthesized with a simple substitution reaction.

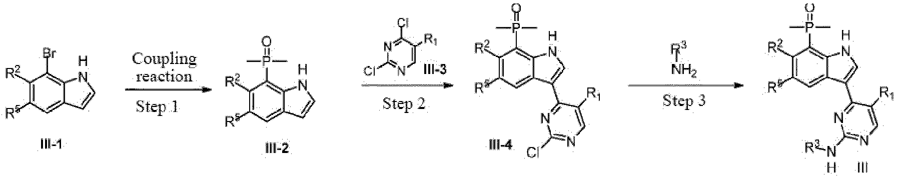

As shown in the general synthetic method 3, a compound (such as but not limited to a brominated compound) represented by formula (III-1) and a suitable reagent (such as but not limited to dimethylphosphine oxide) afford a product III-2 through a coupling reaction under a suitable catalyst; and under the condition of the presence of a suitable acidic reagent (such as but not limited to trifluoromethanesulfonic acid or aluminum trichloride), the product III-2 for which a suitable solvent (such as but not limited to 1,1,1,3,3,3-hexafluoropropan-2-ol or dichloromethane) is selected reacts with a compound (III-3) at a suitable temperature (such as but not limited to 60°C or 0°C) to afford a compound III-4. The chlorinated intermediate III-4 is heated under a suitable basic condition (such as but not limited to DIEA) to react with formula R³NH₂ to afford a compound of formula (III). If R³ group contains other protecting groups (such as but not limited to a Boc protecting group), the compound I affords a final compound under a suitable acidic condition (such as but not limited to TFA/DCM).

The present invention further provides a pharmaceutical composition, comprising the above-mentioned compound as represented by formula I-A, a stereoisomer thereof, a diastereoisomer thereof, a pharmaceutically acceptable salt of any one of the described substances (referring to the described compound as represented by formula I-A, the stereoisomer thereof, or the diastereoisomer thereof), or a crystalline form or a solvate of any one of the described substances (referring to the described compound as represented by formula I-A, the stereoisomer thereof, the diastereoisomer thereof, or the pharmaceutically acceptable salt thereof), and a pharmaceutical excipient.

The present invention further provides an application of the above-mentioned compound as represented by formula I-A, a stereoisomer thereof, a diastereoisomer thereof, a pharmaceutically acceptable salt of any one of the described substances (referring to the described compound as represented by formula I-A, the stereoisomer thereof, or the diastereoisomer thereof), or a crystalline form or a solvate of any one of the described substances (referring to the described compound as represented by formula I-A, the stereoisomer thereof, the diastereoisomer thereof, or the pharmaceutically acceptable salt thereof), or the above-mentioned pharmaceutical composition in preparing a drug. Preferably, the drug is used for preventing and/or treating proliferative diseases.

The present invention further provides a method for preventing and/or treating proliferative diseases, and the method comprises: administering to a patient a therapeutically effective amount of the above-mentioned compound as represented by formula I, a pharmaceutically acceptable salt thereof or solvates of the described substances (referring to the described compound as represented by formula I or the pharmaceutically acceptable salt thereof), or the above-mentioned pharmaceutical composition.

Preferably, the proliferative diseases are cancers (for example, leukemia, acute myeloid leukemia, acute lymphoblastic leukemia, breast cancer, ovarian cancer, brain cancer, lung cancer, liver cancer, small cell lung cancer, melanoma, bladder cancer, colon cancer, esophageal cancer, bone cancer, neuroblastoma, ovarian cancer, pancreatic cancer, prostate cancer, testicular cancer epithelial sarcoma, soft tissue sarcoma, multiple myeloma), benign neoplasms, angiogenesis, inflammatory diseases, autoinflammatory diseases, or autoimmune diseases.

The compound of the present invention, a stereoisomer thereof, a diastereoisomer thereof, or a pharmaceutically acceptable salt of any one of the described substances, or a crystalline form or a solvate of any one of the described substances, and a pharmaceutical composition can be administered locally or systematically, for example, used for intra-intestinal administration, such as rectal administration or oral administration; or used for parenteral administration to mammals (especially humans). Exemplary combinations for rectal administration include suppositories, and the suppositories may contain, for example, suitable non-irritating excipients, for example, cocoa butter, synthetic glyceride, or polyethylene glycols, which are solid at normal temperature, but are thawed and/or dissolved in the rectum cavity to release drugs. The compound of the present invention may also be administered parenterally, for example, by inhalation, injection, or infusion, such as by intravenous, intraarterial, intrabony, intramuscular, intracerebral, extraventricular, intrasynovial, intrasternal, intrathecal, intralesional, intracranial, intratumoral, intradermal, and subcutaneous injection or infusion.

A therapeutically effective amount of an active ingredient is as defined in the context and depends on the mammal species, body weight, age, individual conditions, individual pharmacokinetic parameters, diseases to be treated, and modes of administration. For intra-intestinal administration, such as an oral medicament, the compound of the present invention can be formulated into a wide variety of dosage forms.

An effective amount of the compound of the present invention, a pharmaceutically acceptable salt thereof, solvates of the described substances, or a pharmaceutical composition thereof can be easily determined by routine experiments, and the most effective and convenient administration route and the most appropriate preparation can also be determined by routine experiments.

Unless otherwise specified, the terms used in the present invention have the following meanings:

Those skilled in the art may understand that, according to the conventions used in the art, 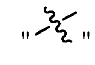 used in the structural formulas describing groups in the present invention means that a corresponding group is linked to other fragments and groups in the compound by this site.

A carbon atom with "*" represents a chiral carbon atom, which is in an S configuration or an R configuration.

The term "pharmaceutically acceptable salt" refers to a salt prepared by a compound of the present invention and a relatively non-toxic, pharmaceutically acceptable acid or base. When the compound of the present invention contains a relatively acidic functional group, a base addition salt can be obtained by using a sufficient amount of pharmaceutically acceptable bases to contact a neutral form of such compound in a pure solution or in a suitable inert solvent. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compound of the present invention contains a relatively basic functional group, an acid addition salt can be obtained by using a sufficient amount of pharmaceutically acceptable acids to contact a neutral form of such compound in a pure solution or in a suitable inert solvent. The pharmaceutically acceptable acids include inorganic acids, the inorganic acids or organic acids. When the compound of the present invention contains relatively acidic functional groups and relatively basic functional groups, the compound can be converted into the base addition salt or the acid addition salt. For details, see Berge et al., "Pharmaceutical Salts," Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present invention or a pharmaceutically acceptable salt thereof with a stoichiometric or non-stoichiometric solvent. Solvent molecules in the solvate may exist in the form of an ordered or non-ordered arrangement. The solvents include, but are not limited to: water, methanol, ethanol, etc.

If there is a stereoisomer, the terms "compound," "pharmaceutically acceptable salt," "solvate," and "solvate of pharmaceutically acceptable salt" may exist in the form of a single stereoisomer or a mixture (for example, a racemate) thereof. The term "stereoisomer" refers to cis-trans isomers or optical isomers. These stereoisomers can be separated, purified, and enriched by asymmetric synthetic methods or chiral separation methods (including but not limited to thin layer chromatography, rotary chromatography, column chromatography, gas chromatography, high pressure liquid chromatography, etc.), and can further be obtained by chiral resolution by bond formation (chemical bonding, etc.) or salification (physical bonding, etc.) with other chiral compounds and other manners. The term "single stereoisomer" means that the mass content of one stereoisomer of the compound of the present invention relative to all stereoisomers of the compound is not less than 95%.

If there is a tautomer, the terms "compound," "pharmaceutically acceptable salt," "solvate," and "solvate of pharmaceutically acceptable salt" may exist in the form of a single tautomer or a mixture thereof, and preferably exist mainly in the form of a relatively stable tautomer.

The atoms in the terms "compound," "pharmaceutically acceptable salt," "solvate," and "solvate of pharmaceutically acceptable salt" may exist in the form of natural abundance or unnatural abundance thereof. Hydrogen atoms are taken as an example, the form of natural abundance thereof refers to about 99.985% of protium and about 0.015% of deuterium therein; and the form of unnatural abundance thereof refers to about 95% of deuterium therein. That is, one or more atoms in the terms "compound," "pharmaceutically acceptable salt," "solvate," and "solvate of pharmaceutically acceptable salt" may be atoms existing in the form of unnatural abundance.

When any variable (for example, R^{a-1}) appears multiple times in a definition of a compound, the definition of the variable appearing at each position is irrelevant to the definitions appearing at the other positions, and their meanings are independent of each other and do not affect each other. Therefore, if a certain group is substituted with 1, 2, or 3 R^{a-1} groups, that is to say, the group may be substituted with up to 3 R^{a-1} groups, the definition of R^{a-1} at this position is mutually independent of the definitions of R^{a-1} at the other positions. Furthermore, a combination of substituents and/or variables is permissible only when the combination results in a stable compound.

The term "a plurality of" refers to 2, 3, 4, or 5, preferably 2 or 3.

The term "alkyl" refers to straight or branched chain alkyl having a designated number of carbon atoms. Examples of alkyl include methyl, ethyl, n-propyl, isopropyl, n-butyl, tert-butyl, isobutyl, sec-butyl, n-pentyl, n-hexyl, n-heptyl, n-octyl, and similar alkyl groups thereof.

The term "cycloalkyl" refers to a saturated monocyclic, polycyclic, or bridged carbocyclic substituent consisting of carbon atoms and hydrogen atoms, and the cycloalkyl may be attached to the remainder of a molecule via any suitable carbon atom by a single bond; when the cycloalkyl is polycyclic, it may be a condensed ring system or a spiro ring system in parallel ring linkage or spiro ring linkage (that is, two geminal hydrogens on a carbon atom are substituted with alkylene). A cycloalkyl substituent may be attached to a center molecule via any suitable carbon atom. In some embodiments, a ring having 3-8 carbon atoms can be represented as C₃-C₈ cycloalkyl. In some embodiments, C₃-C₆ cycloalkyl includes cyclopropyl (C₃), cyclobutyl (C₄), cyclopentyl (C₅), bicyclo[1.1.1]pentane, and cyclohexyl (C₆).

The term "heterocycloalkyl" refers to a saturated cyclic group having heteroatoms, including cases of monocyclic ring, polycyclic ring, or bridged ring. When the heterocycloalkyl is a polycyclic ring, it may be a condensed ring system or a spiro ring system in parallel ring linkage or spiro ring linkage. A 4- to 12-membered saturated cyclic group containing 1-4 ring heteroatoms independently selected from N, O, and S is preferred. Exemplary 4-membered heterocyclyl groups include, but are not limited to, azetidinyl, epoxypropyl, thietanyl, or isomers and stereoisomers thereof; exemplary 5-membered heterocyclyl groups include, but are not limited to, tetrahydrofuranyl, tetrahydrothienyl, pyrrolidinyl, thiazolidinyl, isothiazolidinyl, oxazolidinyl, isoxazolidinyl, imidazolidinyl, pyrazolidinyl, dioxolanyl, oxathiofuryl, dithiofuryl, or isomers and stereoisomers thereof. Exemplary 6-membered heterocyclyl groups include, but are not limited to, piperidyl, tetrahydropyranyl, thianyl, morpholinyl, thiomorpholinyl, dithianyl, dioxanyl, piperazinyl, triazinyl, or isomers and stereoisomers thereof; exemplary 7-membered heterocyclyl groups include, but are not limited to, azepanyl, oxepanyl, thiepanyl, oxazepanyl, and diazepanyl, or isomers and stereoisomers thereof.

The term "heteroaryl" refers to an aromatic group containing heteroatoms, preferably an aromatic 5- to 6-membered monocyclic ring or 9- to 10-membered bicyclic ring containing 1-4 heteroatoms independently selected from nitrogen, oxygen, and sulfur; when the heteroaryl is a bicyclic ring, at least one ring has aromaticity, for example, furyl, pyridyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzisothiazolyl, benzazolyl, benzisoxazolyl, quinolinyl, isoquinolyl, etc.

The term "pharmaceutical excipient" refers to excipients and additives used when drugs are produced and prescriptions are formulated, which are all substances contained in a pharmaceutical preparation except for active ingredients. See Pharmacopoeia of the People's Republic of China (2015 Edition), Volume IV; or Handbook of Pharmaceutical Excipients (Raymond C Rowe, 2009 Sixth Edition).

The term "treating" refers to therapeutic therapy. When a specific condition is involved, treating refers to: (1) palliation of one or more biological manifestations of a disease or a condition; (2) interference with (a) one or more points in the biological cascade resulting in or causing the condition or (b) one or more biological manifestations of the condition; (3) amelioration of one or more symptoms, effects, or side effects related to the condition, or one or more symptoms, effects, or side effects related to the condition or the treating thereof; or (4) slowing of the development of the condition or one or more biological manifestations of the condition.

The term "preventing" refers to a reduction in the risk of acquiring or developing a disease or disorder.

The term "therapeutically effective amount" refers to an amount of a compound sufficient to effectively treat a disease or condition described herein when administered to a patient. The "therapeutically effective amount" will vary according to compounds, conditions, and severities thereof, as well as the ages of patients to be treated, but may be adjusted by those skilled in the art as needed.

The term "patient" refers to any animal that is about to receive or has already received the administration of the compound or composition according to the embodiments of the present invention, preferably mammals, most preferably humans. The term "mammals" includes any mammal. Examples of the mammals include, but are not limited to, cows, horses, sheep, pigs, cats, dogs, mice, rats, rabbits, guinea pigs, monkeys, humans, etc., most preferably humans.

On the basis of not violating common knowledge in the art, the above-mentioned preferred conditions may be combined arbitrarily to namely obtain various preferred examples of the present invention.

The reagents and raw materials used in the present invention are all commercially available.

The positive progress effects of the present invention lie in that: the present invention provides an aromatic heterocyclic compound, and the aromatic heterocyclic compound is novel in structure, has good CDK7 inhibitory activity and good selectivity (relative to CDK2, CDK9, and CDK12), has excellent inhibitory effect on human breast cancer cells HCC70 and ovarian cancer A2780, and meanwhile has better membrane permeability and lower efflux rate.

### Detailed Description of Embodiments

The present invention is further illustrated below by means of embodiments, but the present invention will not be thereby limited in the scope of the embodiments. For the experimental methods without specific conditions specified in the following embodiments, selection shall be made according to conventional methods and conditions or according to product instructions.

### Preparation of Intermediate C: (S)-3-methyl-4-(3-(2-(methylsulfonyl)-5-(trifluoromethyl) pyrimidin-4-yl)-1-(benzenesulfonyl)-1H-pyrrolo[2,3-b]pyridin-6-yl)morpholine

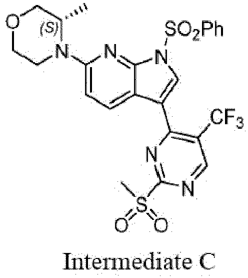

### Step 1: 1H-pyrrolo[2,3-b]pyridine-7-oxide

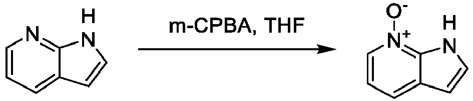

A compound 1H-pyrrolo[2,3-b]pyridine (130.00 g, 1.10 mol) was dissolved in tetrahydrofuran (1.20 L), m-chloroperoxybenzoic acid (80% purity, 356.05 g, 1.65 mol) was added, and then stirring was carried out for 16 hours under 20°C; and the system was a yellow suspension. The reaction solution was concentrated to spin-remove half of the solvent, and a solid was filtered off, washed with tetrahydrofuran (50 mL), and dried under vacuum to afford a crude product 1H-pyrrolo[2,3-b]pyridine-7-oxide (50% purity, 275.00 g), which was a white solid. The crude product was used directly in the next step without purification. LCMS (ESI): [M+H]⁺ = 135.1.

### Step 2: (S)-3-methyl-4-(1H-pyrrolo[2,3-b]pyridin-6-yl)-morpholine

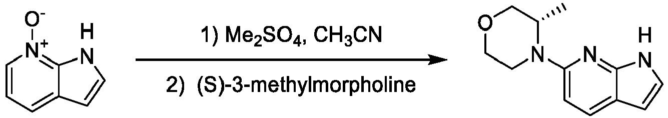

1H-pyrrolo[2,3-b]pyridine-7-oxide (50% purity, 55.00 g, 205.07 mmol) was dissolved in acetonitrile (535 mL), and dimethyl sulfate (21 mL, 225.51 mmol) was added. The mixture was heated to 60°C and stirred for 16 hours. Cooling to 0°C was carried out, and (3S)-3-methylmorpholine (103.68 g, 1.03 mol) was added. Heating to 60°C was carried out, and stirring was carried out for 20 hours. Cooling and concentration were carried out, and the residue was subjected to fractional liquid extraction with dichloromethane (200 mL) and a 10% aqueous sodium carbonate solution (200 mL). An aqueous phase was extracted with dichloromethane (200 mL * 2). Organic phases were combined, dried over anhydrous magnesium sulfate, and concentrated. The residue was purified by flash column chromatography (silica gel, tetrahydrofuran/petroleum ether with 0-25% gradient) to afford a yellow solid (S)-3-methyl-4-(1H-pyrrolo[2,3-b]pyridin-6-yl)-morpholine (8.64 g, 39.76 mmol, 19% yield). LCMS (ESI): [M+H]⁺ = 218.1.

¹H NMR (400 MHz, CD₃OD) δ ppm 7.75 (d, *J* = 8.5 Hz, 1H), 7.03 (d, *J* = 3.3 Hz, 1H), 6.58 (d, *J* = 8.5 Hz, 1H), 6.29 (d, *J* = 3.5 Hz, 1H), 4.31 (q, *J* = 6.5 Hz, 1H), 4.00 (dd, *J* = 3.1, 11.2 Hz, 1H), 3.84 - 3.71 (m, 3H), 3.65 (dt, *J* = 3.0, 11.4 Hz, 1H), 3.22 (dt, *J* = 3.8, 12.3 Hz, 1H), 1.18 (d, *J* = 6.8 Hz, 3H)

### Step 3: (S)-4-(3-iodo-1H-pyrrolo[2,3-b]pyridin-6-yl)-3-methylmorpholine

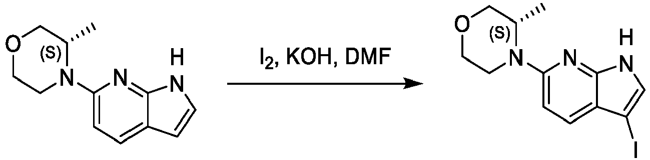

A compound (S)-3-methyl-4-(1H-pyrrolo[2,3-b]pyridin-6-yl)-morpholine (8.34 g, 38.39 mmol) was dissolved in dimethylformamide (40 mL), potassium hydroxide (5.37 g, 95.96 mmol) was added, a solution of iodine (9.75 g, 38.39 mmol) in dimethylformamide (40 mL) was added under 0°C, and the reaction mixture was stirred for 1 hour at 25°C. Concentration was carried out, and the residue was added with water (100 mL) and extracted with dichloromethane (100 mL * 3). The organic phases were combined and subsequently dried over magnesium sulfate. Filtration and concentration were carried out to afford a crude compound (S)-4-(3-iodo-1H-pyrrolo[2,3-b]pyridin-6-yl)-3-methylmorpholine (14.70 g). LCMS (ESI): [M+H]⁺ = 344.0.

### Step 4: (S)-4-(3-iodo-1-benzenesulfonyl-1H-pyrrolo[2,3-b]pyridin-6yl)-3-methylmorpholine

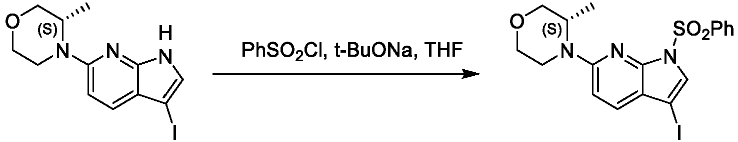

A compound (S)-4-(3-iodo-1H-pyrrolo[2,3-b]pyridin-6-yl)-3-methylmorpholine (14.70 g, 34.27 mmol) was dissolved in tetrahydrofuran (150 mL), sodium tert-butoxide (4.94 g, 51.40 mmol) was added under 0°C, and stirring was carried out for 30 minutes at 0°C. Benzenesulfonyl chloride (6.6 mL, 51.40 mmol) was added, and the reaction mixture was stirred for 2 hours at 20°C. Concentration was carried out, and the residue was purified by flash column chromatography (silica gel, tetrahydrofuran/petroleum ether with 0-40% gradient) to afford a yellow solid compound (S)-4-(3-iodo-1-benzenesulfonyl-1H-pyrrolo[2,3-b]pyridin-6yl)-3-methylmorpholine (4.60 g, 9.52 mmol, 28% yield). LCMS (ESI): [M+H]⁺ = 484.0.

### Step 5: (S)-3-methyl-4-(3-(2-(methylthio)-5-(trifluoromethyl)pyrimidin-4-yl)-1-(benzenesulfonyl)-1H-pyrrolo[2,3-b]pyridin-6-yl)morpholine

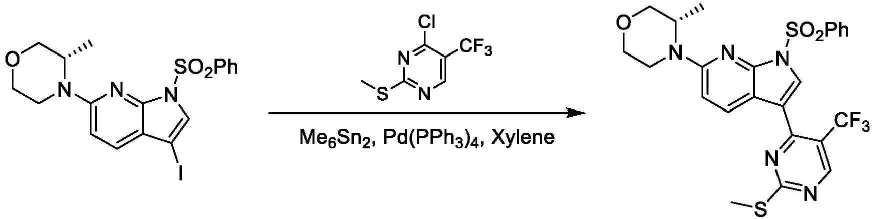

Compounds (S)-4-(3-iodo-1-benzenesulfonyl-1H-pyrrolo[2,3-b]pyridin-6-yl)-3-methylmorpholine (500 mg, 1.03 mmol) and 4-chloro-2-(methylthio)-5-(trifluoromethyl)pyrimidine (512 mg, 1.35 mmol) were dissolved in xylene (10 mL), and under nitrogen protection, tetrakis(triphenylphosphine)palladium (120 mg, 0.10 mmol) and hexamethylditin (407 mg, 1.24 mmol) were added under room temperature. The reaction mixture was stirred for 2 hours at 100°C, and then heated to 140°C to continue to react for 12 hours. The reaction solution was concentrated, and the residue was purified by flash column chromatography (silica gel, ethyl acetate/petroleum ether with 0-50% gradient) to afford a yellow solid compound (S)-3-methyl-4-(3-(2-(methylthio)-5-(trifluoromethyl)pyrimidin-4-yl)-1-(benzenesulfonyl)-1H-pyrrolo[2,3-b]pyridin-6-yl)morpholine (220 mg). LCMS (ESI): [M+H]⁺ = 550.3

### Step 6: (S)-3-methyl-4-(3-(2-(methylsulfonyl)-5-(trifluoromethyl)pyrimidin-4-yl)-1-(benzenesulfonyl)-1H-pyrrolo[2,3-b]pyridin-6-yl)morpholine

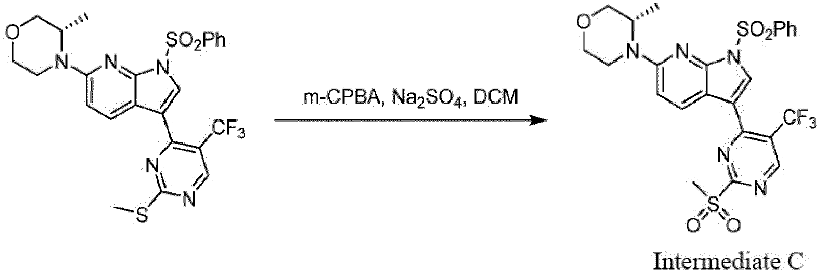

M-chloroperoxybenzoic acid (85% purity, 650 mg, 3.20 mmol) was added to a suspension of a compound (S)-3-methyl-4-(3-(2-(methylthio)-5-(trifluoromethyl)pyrimidin-4-yl)-1-(benzenesulfonyl)-1H-pyrrolo[2,3-b]pyridin-6-yl)morpholine (800 mg, 1.46 mmol) and sodium sulfate (202 mg, 1.60 mmol) in dichloromethane (59 mL) in batches under 0°C, and a resulting reaction system was stirred for 40 minutes at 25^{°C}. Filtration was carried out, and the filtrate was directly used in the next-step reaction. LCMS (ESI): [M+H]⁺ = 582.1.

### Preparation of Intermediate D: 4-(3-(2-chloro-5-(trifluoromethyl)pyrimidin-4-yl)-1H-pyrrolo[2,3-b]pyridin-6-yl)morpholine

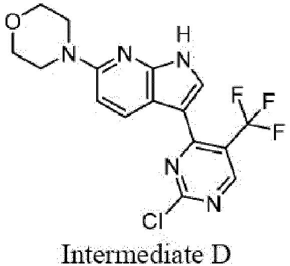

### Step 1: 4-(1H-pyrrolo[2,3-b]pyridin-6-yl)morpholine

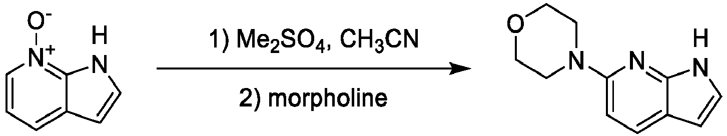

A compound 1H-pyrrolo[2,3-b]pyridine-7-oxide (50% purity, 35.00 g, 0.13 mol) was dissolved in acetonitrile (250 mL), dimethyl sulfate (14 mL, 0.14 mol) was added, and then stirring was carried out for 16 hours under 60°C. The reaction system was cooled to 0°C, morpholine (230 mL, 2.61 mol) was subsequently added, and then, stirring was carried out for 20 hours under 60°C; and the system was a yellow solution. The reaction system was cooled and concentrated, and dichloromethane (300 mL) and a 10% aqueous sodium carbonate solution (200 mL) were added to the residue. After organic phases were separated, an aqueous phase was extracted with dichloromethane (200 mL * 2), the combined organic phases were dried over magnesium sulfate and filtered, and the filtrate was spin-dried to afford 80 g of residue, and the residue was purified by flash column chromatography (C18, acetonitrile/water with 0-100% gradient) to afford 4-(1H-pyrrolo[2,3-b]pyridin-6-yl)morpholine, which was a yellow solid (13.50 g, 66.44 mmol, 51% yield). LCMS (ESI): [M+H]⁺ = 204.2.

### Step 2: 4-(3-iodo-1H-pyrrolo[2,3-b]pyridin-6-yl)morpholine

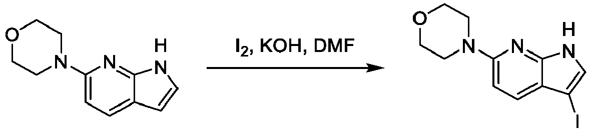

A compound 4-(1H-pyrrolo[2,3-b]pyridin-6-yl)morpholine (9.70 g, 47.74 mmol) was dissolved in dimethylformamide (50 mL), potassium hydroxide (6.66 g, 118.72 mmol) was added, and a reaction was stirred for 30 minutes. Under 0°C, a solution of elementary iodine (12.10 g, 47.68 mmol) in dimethylformamide (50 mL) was added dropwise to the reaction solution; and a reaction was conducted for 1 hour under 20°C. The reaction solution was added with water (200 mL) for dilution, and extracted with ethyl acetate (200 mL * 3). The organic phases were combined, dried over magnesium sulfate, and filtered, and the filtrate was spin-dried to afford a crude product 4-(3-iodo-1H-pyrrolo[2,3-b]pyridin-6-yl)morpholine (11.80 g), which was a tan oily substance. The crude product was directly subjected to the next-step reaction without purification. LCMS (ESI): [M+H]⁺ = 330.0.

### Step 3: 4-(3-iodo-1-(benzenesulfonyl)-1H-pyrrolo[2,3-b]pyridin-6-yl)morpholine

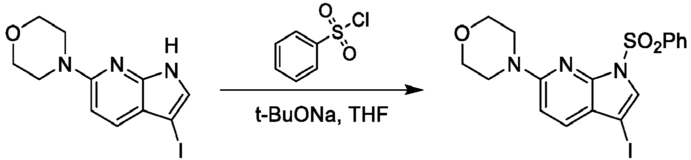

A compound 4-(3-iodo-1H-pyrrolo[2,3-b]pyridin-6-yl)morpholine (14.50 g, 44.06 mmol) was dissolved in tetrahydrofuran (145 mL), sodium tert-butoxide (6.35 g, 66.08 mmol) was added therein under 0°C, and stirring was carried out for 30 minutes. Then, benzenesulfonyl chloride (15.50 g, 87.76 mmol) was added under 0°C, and the reaction solution was reacted for 2 hours under 20°C. Afterwards, concentration under reduced pressure was carried out to spin-remove the solvent, and the residue was purified by flash column chromatography (silica gel, tetrahydrofuran/petroleum ether with 0-25% gradient) to afford a compound 4-(3-iodo-1-(benzenesulfonyl)-1H-pyrrolo[2,3-b]pyridin-6-yl)morpholine (12.70 g, 27.06 mmol, 61% yield). LCMS (ESI): [M+H]⁺ = 470.0.

### Step 4: 4-(3-(2-(methylthio)-5-(trifluoromethyl)pyrimidin-4-yl)-1-(benzenesulfonyl)-1H-pyrrolo[2,3-b]pyridin-6-yl)morpholine

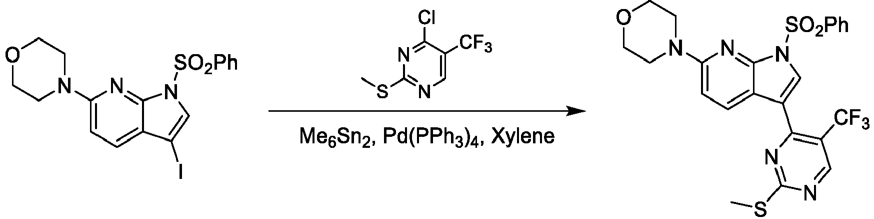

A compound 4-(3-iodo-1-(benzenesulfonyl)-1H-pyrrolo[2,3-b]pyridin-6-yl)morpholine (5.50 g, 11.72 mmol) and a compound 4-chloro-2-(methylthio)-5-(trifluoromethyl)pyrimidine (3.48 g, 15.24 mmol) were dissolved in xylene (100 mL), and tetrakis(triphenylphosphine)palladium (1.35 g, 1.17 mmol) and hexamethylditin (3 mL, 15.24 mmol) were added under a nitrogen atmosphere; and under nitrogen protection, the reaction was conducted for 2 hours under 100°C, and then the reaction was conducted for 14 hours under 140°C. The system was a black suspension. The system was concentrated under reduced pressure to afford a brown solid which was purified by flash column chromatography (silica gel, ethyl acetate/petroleum ether with 0-30% gradient) to afford a compound 4-(3-(2-(methylthio)-5-(trifluoromethyl)pyrimidin-4-yl)-1-(benzenesulfonyl)-1H-pyrrolo[2,3-b]pyridin-6-yl)morpholine (4.10 g, 6.12 mmol, 52% yield). LCMS (ESI): [M+H]⁺ = 536.1.

### Step 5: 4-(6-morpholinyl-1H-pyrrolo[2,3-b]pyridin-3-yl)-5-(trifluoromethyl)pyrimidin-2-ol hydrochloride

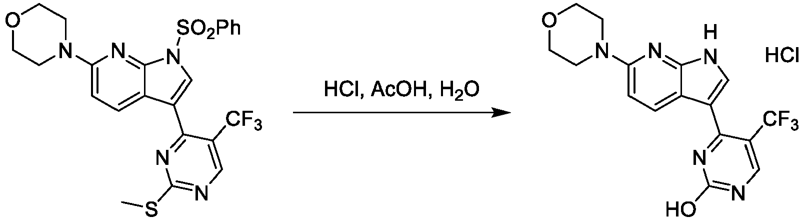

A compound 4-(3-(2-(methylthio)-5-(trifluoromethyl)pyrimidin-4-yl)-1-(benzenesulfonyl)-1H-pyrrolo[2,3-b]pyridin-6-yl)morpholine (8.00 g, 14.94 mmol) was dissolved in a mixture of glacial acetic acid (100 mL) and water (50 mL), and concentrated hydrochloric acid (50 mL) was added at room temperature. The resulting reaction solution was stirred for 16 hours under 100°C. The reaction solution was concentrated, a resulting residue was added in ethyl acetate (40 mL), and stirring was carried out for one hour under room temperature. The solid was filtered off and dried in vacuum to afford a crude compound 4-(6-morpholinyl-1H-pyrrolo[2,3-b]pyridin-3-yl)-5-(trifluoromethyl)pyrimidin-2-ol hydrochloride (6.80 g). The compound was directly used in the next-step reaction. LCMS (ESI): [M+H]⁺ = 366.1.

### Step 6: 4-(3-(2-chloro-5-(trifluoromethyl)pyrimidin-4-yl)-1H-pyrrolo[2,3-b]pyridin-6-yl)morpholine

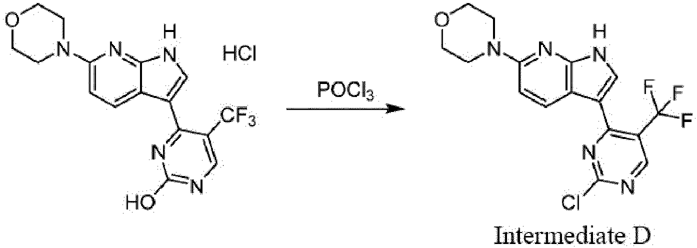

A compound 4-(6-morpholinyl-1H-pyrrolo[2,3-b]pyridin-3-yl)-5-(trifluoromethyl)pyrimidin-2-ol hydrochloride (2.20 g, 6.02 mmol) was dissolved in phosphorus oxychloride (40 mL), and stirring was carried out for 16 hours under 80°C. The reaction solution was concentrated, and a resulting residue was purified by flash column chromatography (silica gel, ethyl acetate/petroleum ether with 0-90% gradient) to afford a compound 4-(3-(2-chloro-5-(trifluoromethyl)pyrimidin-4-yl)-1H-pyrrolo[2,3-b]pyridin-6-yl)morpholine (1.40 g, 2.92 mmol, 48% two-step yield), which was a yellow solid. LCMS (ESI): [M+H]⁺ = 384.1.

¹H NMR (400 MHz, DMSO-*d*₆) δ ppm 9.00 (s, 1H), 8.58 - 8.42 (m, 1H), 7.84 (d, *J* = 1.8 Hz, 1H), 6.99 - 6.81 (m, 1H), 3.79 - 3.66 (m, 4H), 3.57 - 3.40 (m, 4H).

### Preparation of Intermediate E: (3-(2-chloro-5-(trifluoromethyl)pyrimidin-4-yl)-1H-indole-7-yl)dimethylphosphine oxide

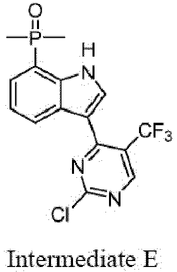

### Step 1: (1H-indole-7-yl)dimethylphosphine oxide

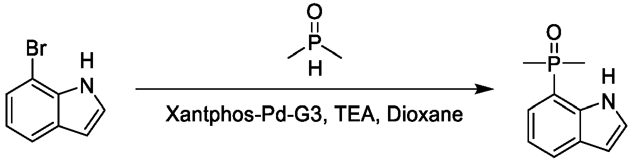

A compound 7-bromo-1H-indole (2.00 g, 10.20 mmol) and dimethylphosphine oxide (2.39 g, 30.60 mmol) were dissolved in 1,4-dioxane (50 mL); under nitrogen protection, triethylamine (7 mL, 51.00 mmol) and a [9,9-dimethyl-4,5-bis(diphenylphosphino)xanthene][2-amino-1,1-diphenyl]palladium(II) mesylate dichloromethyl adduct (20 mg, 0.02 mmol) were added under 25°C. The reaction system was warmed up to 100°C and reacted for 16 hours. Cooling to room temperature was carried out, filtration was carried out, the filtrate was concentrated, and a resulting residue was purified by flash column chromatography (silica gel, tetrahydrofuran/petroleum ether with 0-100% gradient) to afford a compound (1H-indole-7-yl)dimethylphosphine oxide (220 mg, 1.08 mmol, 11% yield), which was a yellow solid. LCMS (ESI): [M+H]⁺ = 194.1.

### Step 2: (3-(2-chloro-5-(trifluoromethyl)pyrimidin-4-yl)-1H-indole-7-yl)dimethylphosphine oxide

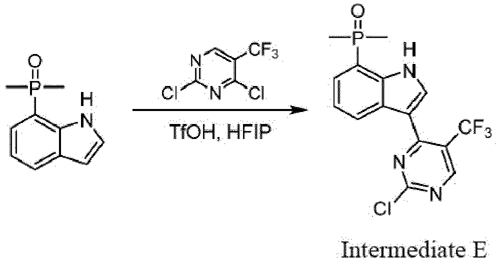

A compound (1H-indole-7-yl)dimethylphosphine oxide (220 mg, 1.08 mmol) and a compound 2,4-dichloro-5-(trifluoromethyl)pyrimidine (220 uL, 1.63 mmol) were dissolved in hexafluoroisopropanol (10 mL), trifluoromethanesulfonic acid (106 uL, 1.20 mmol) was added dropwise under 0°C, and the reaction system was stirred for 16 hours under 60°C. Cooling to room temperature was carried out, the reaction system was poured in a saturated aqueous sodium bicarbonate solution (20 mL), and ethyl acetate (15 mL * 2) was used for extraction. Organic phases were combined, dried over magnesium sulfate, and filtered, the filtrate was concentrated, and the residue was purified by a preparative silica gel plate (petroleum ether/tetrahydrofuran at a volume ratio of 1:2) to afford a yellow oily compound (3-(2-chloro-5-(trifluoromethyl)pyrimidin-4-yl)-1H-indole-7-yl)dimethylphosphine oxide (200 mg, 0.38 mmol, 34% yield). LCMS (ESI): [M+H]⁺ = 374.0.

### Preparation of Intermediate F: The following intermediate F (7-bromo-3-(2-chloro-5-(trifluoromethyl)pyrimidin-4-yl)-1H-indole-6-carbonitrile) was prepared by using a method the same as that in embodiment 4 of patent WO2020093011A1.

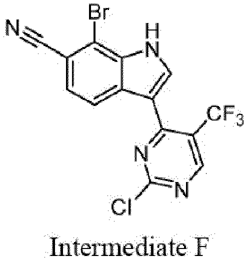

The compound intermediate F was a light yellow solid. LCMS (ESI): [M+H]⁺ = 401.2;
¹H NMR (400 MHz, DMSO-*d*₆) δ ppm 13.00 (br s, 1H), 9.16 (s, 1H), 8.36 (d, *J* = 8.4 Hz, 1H), 8.16 (d, *J* = 2.4 Hz, 1H), 7.71 (d, *J* = 8.4 Hz, 1H)

### Preparation Example of Intermediate G: 3-(2-(methylsulfonyl)-5-(trifluoromethyl)pyrimidin-4-yl)-1-(benzenesulfonyl)-1H-pyrrolo[2,3-b]pyridine-6-carbonitrile

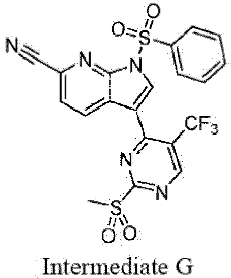

### Step 1: 1H-pyrrolo[2,3-b]pyridine-6-carbonitrile

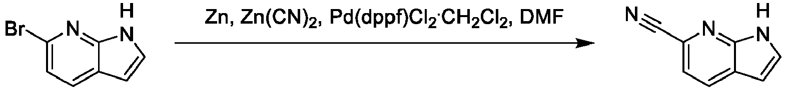

A mixture of 6-bromo-1H-pyrrolo[2,3-b]pyridine (4.00 g, 20.30 mmol), zinc powder (133 mg, 2.03 mmol), zinc cyanide (1.67 g, 14.21 mmol), and a 1,1-bis(diphenylphosphino)ferrocene palladium dichloride dichloromethane mixture (829 mg, 1.02 mmol) in dimethylformamide (10 mL) was degassed and purged with nitrogen for 3 times, and then the mixture was stirred for 5 hours at 140°C under nitrogen protection. The reaction mixture was diluted with ethyl acetate (50 mL), washed successively with a saturated aqueous sodium bicarbonate solution (100 mL) and saturated brine (100 mL * 2), dried over sodium sulfate, and filtered, and the filtrate was spin-dried. The residue was purified by flash column chromatography (silica gel, ethyl acetate/petroleum ether with 10-33% gradient) to afford a compound 1H-pyrrolo[2,3-b]pyridine-6-carbonitrile (85% purity, 1.50 g, 8.91 mmol, 44% yield), which was a white solid. LCMS (ESI): [M+H]⁺ = 144.2.

### Step 2: 3-iodo-1H-pyrrolo[2,3-b]pyridine-6-carbonitrile

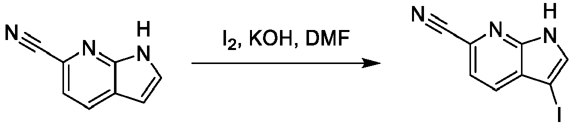

A solution of 1H-pyrrolo[2,3-b]pyridine-6-carbonitrile (85% purity, 26.00 g, 154.44 mmol) and potassium hydroxide (22.93 g, 408.66 mmol) in dimethylformamide (150 mL) was cooled to 0°C, and then a solution of elementary iodine (41.49 g, 163.46 mmol) in dimethylformamide (150 mL) was added dropwise. The reaction mixture was stirred for 1 hour under 25°C. The reaction mixture was subjected to suction filtration, the filtrate was spin-dried, and a resulting crude product was washed with water (100 mL * 3) for three times and dried in vacuum to afford a crude compound 3-iodo-1H-pyrrolo[2,3-b]pyridine-6-carbonitrile (53.00 g). LCMS (ESI): [M+H]⁺ = 270.0.

### Step 3: 3-iodo-1-(benzenesulfonyl)-1H-pyrrolo[2,3-b]pyridine-6-carbonitrile

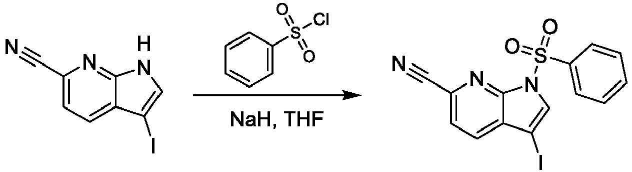

After a solution of 3-iodo-1H-pyrrolo[2,3-b]pyridine-6-carbonitrile (50.00 g, 148.68 mmol) in tetrahydrofuran (2.50 L) was cooled to 0°C, sodium hydride (60% purity, 10.71 g, 267.63 mmol) was added under nitrogen protection. Then, benzenesulfonyl chloride (28 mL, 223.01 mmol) was added. The mixture was stirred for 3 hours under 25°C, and then acetic acid (20 mL) and water (200 mL) were added under 0°C for quenching. Tetrahydrofuran in the solution was removed by rotary evaporation, and the precipitated solid was filtered off and dried in vacuum to afford a crude product. The crude product was subjected to beating with methyl tert-butyl ether (100 mL), and filtered to afford a white solid compound 3-iodo-1-(benzenesulfonyl)-1H-pyrrolo[2,3-b]pyridine-6-carbonitrile (45.00 g, 110.04 mmol, 63% yield). LCMS (ESI): [M+H]⁺ = 410.0.

### Step 4: 3-(2-methylthio-5-trifluoromethylpyrimidin-4-yl)-1-(benzenesulfonyl)-1H-pyrrolo[2,3-b]pyridine-6-carbonitrile

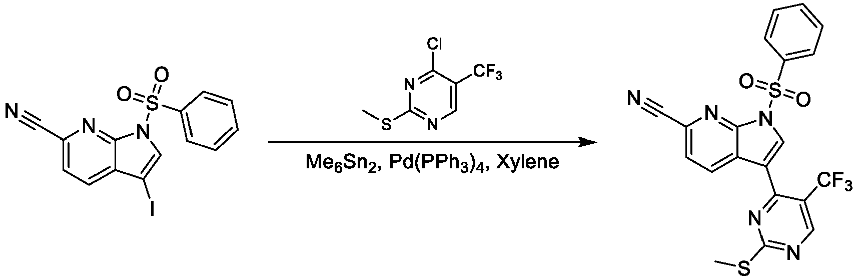

Under nitrogen protection, tetrakis(triphenylphosphine)palladium (1.27 g, 1.10 mmol) was added to a solution of 3-iodo-1-(benzenesulfonyl)-1H-pyrrolo[2,3-b]pyridine-6-carbonitrile (5.00 g, 11.00 mmol), 4-chloro-2-methylthio-5-trifluoromethylpyrimidine (3.27 g, 14.30 mmol), and hexamethylditin (4.72 g, 14.30 mmol) in xylene (100 mL). Under nitrogen protection, the reaction mixture was stirred for 2 hours at 100°C, and then was warmed up to 140°C to react for 16 hours. The reaction mixture was spin-dried, and the residue was purified by flash column chromatography (silica gel, tetrahydrofuran/petroleum ether with 5-10% gradient) to afford a yellow solid compound 3-(2-methylthio-5-trifluoromethylpyrimidin-4-yl)-1-(benzenesulfonyl)-1H-pyrrolo[2,3-b]pyridine-6-carbonitrile (3.10 g, 6.52 mmol, 59% yield). LCMS (ESI): [M+H]⁺ = 476.2.

### Step 5: 3-(2-(methylsulfonyl)-5-(trifluoromethyl)pyrimidin-4-yl)-1-(benzenesulfonyl)-1H-pyrrolo[2,3-b]pyridine-6-carbonitrile

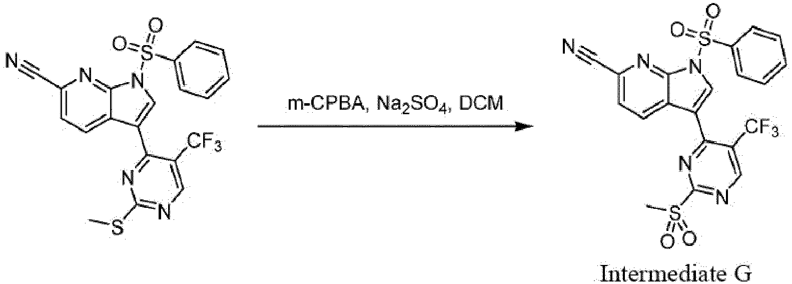

A compound 3-(2-methylthio-5-trifluoromethylpyrimidin-4-yl)-1-(benzenesulfonyl)-1H-pyrrolo[2,3-b]pyridine-6-carbonitrile (200 mg, 0.42 mmol) was dissolved in dichloromethane (4 mL), m-chloroperoxybenzoic acid (80% purity, 181 mg, 0.84 mmol) and sodium sulfate (50 mg, 0.35 mmol) were added, and the resulting reaction system was stirred for 2 hours under 20°C. A saturated sodium sulfite solution (1 mL) and a saturated sodium bicarbonate solution (5 mL) were added in the reaction solution, and ethyl acetate (10 mL * 3) was used for extraction. Organic phases were combined and concentrated to afford a crude compound 3-(2-(methylsulfonyl)-5-(trifluoromethyl)pyrimidin-4-yl)-1-(benzenesulfonyl)-1H-pyrrolo[2,3-b]pyridine-6-carbonitrile (230 mg), which was a yellow oily substance, and the crude product was directly used in the next-step reaction. LCMS (ESI): [M+H]⁺ = 508.0.

### Preparation of Intermediate H: 3,5-dimethyl-4-(3-(2-methylsulfonyl-5-trifluoromethylpyrimidin-4-yl)-1-benzenesulfonyl-1H-pyrrolo[2,3-b]pyridin-6-yl)isoxazole

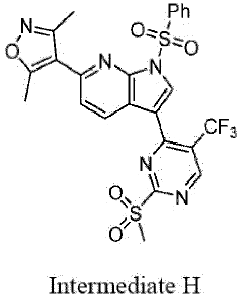

With reference to embodiment **11** of patent WO2019143719, the intermediate H was prepared by using the same synthetic method.

### Embodiment 35. N-(4-(6-(3,5-dimethylisoxazol-4-yl)-1H-pyrrolo[2,3-b]pyridin-3-yl)-5-(trifluoromethyl)pyrimidin-2-yl)-2-azaspiro[4.4]non-7-amine (compound 35)

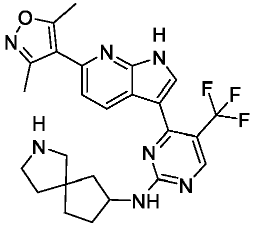

### Step 1: tert-butyl 7-((4-(6-(3,5-dimethylisoxazol-4-yl)-1-(benzenesulfonyl)-1H-pyrrolo[2,3-b] pyridin-3-yl)-5-(trifluoromethyl)pyrimidin-2-yl)amino)-2-azaspiro[4.4]nonane-2-carboxylate

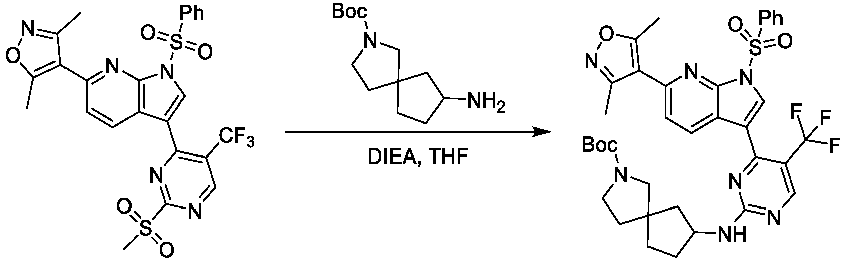

A compound tert-butyl 7-amino-2-azaspiro[4.4]nonane-2-carboxylate (30 mg, 0.125 mmol) and diisopropylethylamine (103 uL, 0.62 mmol) were dissolved in tetrahydrofuran (100 uL), and a compound 3,5-dimethyl-4-(3-(2-methylsulfonyl-5-trifluoromethylpyrimidin-4-yl)-1-benzenesulfonyl-1H-pyrrolo[2,3-b]pyridin-6-yl)isoxazole (87 mg, 0.15 mmol) was added. A reaction proceeded with stirring for 12 hours at 25°C. Water (2 mL) was added, and ethyl acetate (2 mL * 3) was used for extraction. Organic phases were combined, dried over magnesium sulfate, and filtered, and the filtrate was concentrated to afford a crude compound tert-butyl 7-((4-(6-(3,5-dimethylisoxazol-4-yl)-1-(benzenesulfonyl)-1H-pyrrolo[2,3-b]pyridin-3-yl)-5-(trifluoromethyl)pyrimidin-2-yl)amino)-2-azaspiro[4.4]nonane-2-carboxylate (100 mg), which was a yellow oily liquid. LCMS (ESI): [M+H]⁺ = 738.3.

### Step 2: 7-((4-(6-(3,5-dimethylisoxazol-4-yl)-1H-pyrrolo[2,3-b]pyridin-3-yl)-5-(trifluoromethyl)pyrimidin-2-yl)amino)-2-azaspiro[4.4]nonane-2-carboxylate

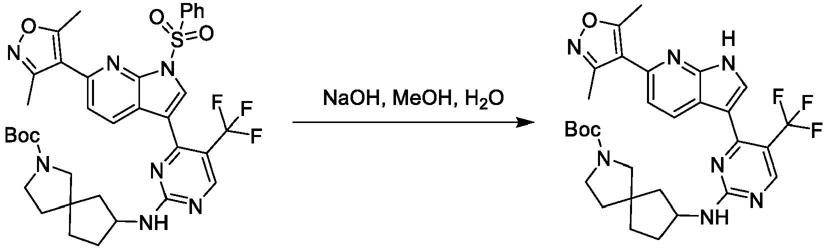

A compound tert-butyl 7-((4-(6-(3,5-dimethylisoxazol-4-yl)-1-(benzenesulfonyl)-1H-pyrrolo[2,3-b]pyridin-3-yl)-5-(trifluoromethyl)pyrimidin-2-yl)amino)-2-azaspiro[4.4]nonane-2-carboxylate (90% purity, 100 mg, 0.12 mmol) was dissolved in methanol (1 mL), an aqueous sodium hydroxide solution (4 M, 153 uL, 0.61 mmol) was added, and a reaction proceeded with stirring for 1 hour at 25°C. After water (2 mL) was added for dilution, ethyl acetate (2 mL * 3) was used for extraction. The organic phases were combined and dried over magnesium sulfate, and after filtration, the filtrate was concentrated to afford a crude compound 7-((4-(6-(3,5-dimethylisoxazol-4-yl)-1H-pyrrolo[2,3-b]pyridin-3-yl)-5-(trifluoromethyl)pyrimidin-2-yl)amino)-2-azaspiro[4.4]nonane-2-carboxylate (100 mg), which was a yellow oily liquid. LCMS (ESI): [M+H]⁺ = 612.3.

### Step 3: N-(4-(6-(3,5-dimethylisoxazol-4-yl)-1H-pyrrolo[2,3-b]pyridin-3-yl)-5-(trifluoromethyl)pyrimidin-2-yl)-2-azaspiro[4.4]non-7-amine

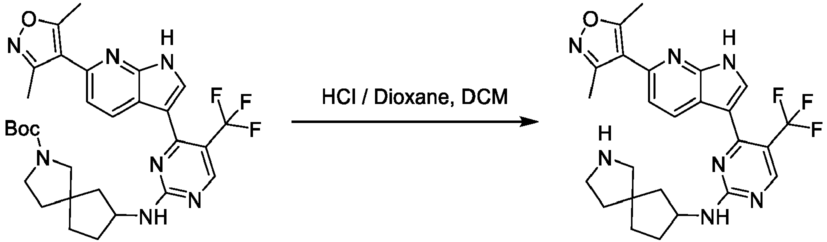

A compound 7-((4-(6-(3,5-dimethylisoxazol-4-yl)-1H-pyrrolo[2,3-b]pyridin-3-yl)-5-(trifluoromethyl)pyrimidin-2-yl)amino)-2-azaspiro[4.4]nonane-2-carboxylate (100 mg, 0.17 mmol) was dissolved in dichloromethane (2 mL), a hydrogen chloride/dioxane solution (4 M, 1 mL, 4.00 mmol) was added under 0°C, and a reaction proceeded with stirring for 1 hour at 25°C. Concentration was carried out, and the residue was purified by preparative HPLC to afford a compound N-(4-(6-(3, 5-dimethylisoxazol-4-yl)-1H-pyrrolo[2,3-b]pyridin-3-yl)-5-(trifluoromethyl)pyrimidin-2-yl)-2-azaspiro[4.4]non-7-amine (formate, 12.11 mg, 21 umol, 12% yield), which was a white solid. LCMS (ESI): [M+H]⁺ = 498.3,

¹H NMR (400 MHz, CD₃OD) δ ppm 8.85 (m, 1H), 8.56 (m, 2H), 8.03 (s, 1H), 7.36 (m, 1H), 4.61 (m, 1H), 3.36 (m, 2H), 3.28-3.15 (m, 2H), 2.63 (s, 3H), 2.47 (s, 3H), 2.31 (m, 2H), 2.13-1.92 (m, 3H), 1.90-1.71 (m, 3H).

We used the same method for synthesizing the compound 35 and used 3,5-dimethyl-4-(3-(2-methylsulfonyl-5-trifluoromethylpyrimidin-4-yl)-1-benzenesulfonyl-1H-pyrrolo[2,3-b]pyridin-6-yl)isoxazole to react with a corresponding amine to synthesize the following compound:

### Embodiment 36. (R)-N-(4-(6-(3,5-dimethylisoxazol-4-yl)-1H-pyrrolo[2,3-b]pyridin-3-yl)-5-(trifluoromethyl)pyrimidin-2-yl)-2-azaspiro[3.3]heptan-5-amine (compound 36)

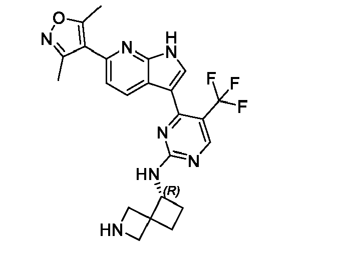

The compound **36** (2.02 mg, yellow solid). LC-MS (ESI): [M+H]+⁼470.2;
¹H NMR (400 MHz, CD₃OD) δ ppm 9.20-8.50 (m, 2H), 8.09 (m, 1H), 7.57-7.28 (m, 1H), 4.64 (m, 2H), 4.28 (m, 1H), 4.12-3.66 (m, 2H), 2.70 (s, 3H), 2.50 (s, 3H), 2.35 (m, 1H), 2.21 (m, 2H), 2.12-1.98 (m, 1H).

### Embodiment 37. N-(4-(6-morpholinyl-1H-pyrrolo[2,3-b]pyridin-3-yl)-5-(trifluoromethyl) pyrimidin-2-yl)-6-azaspiro[3.4]octan-1-amine (compound 37)

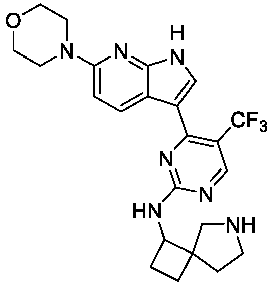

### Step 1: benzyl 1-((tert-butoxycarbonyl)amino)-6-azaspiro[3.4]octane-6-carboxylate

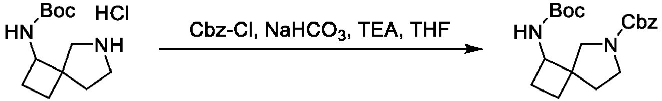

A compound tert-butyl (6-azaspiro[3.4]octan-1-yl)carbamate hydrochloride (100 mg, 0.44 mmol) was dissolved in tetrahydrofuran (500 µL); and sodium bicarbonate (111 mg, 1.32 mmol), benzyl chloroformate (500 µL), and triethylamine (92 µL, 0.66 mmol) were added under 25°C, a reaction was conducted for 4 hours under 25°C, and the system was a yellow suspension. The reaction mixture was diluted with water (2 mL), and extracted with ethyl acetate (2 mL * 4). Organic phases were combined, dried over magnesium sulfate, and filtered, and the filtrate was spin-dried to afford a crude product benzyl 1-((tert-butoxycarbonyl)amino)-6-azaspiro[3.4]octane-6-carboxylate (134 mg). The crude product was directly subjected to the next step without purification. LCMS (ESI): [M-56+H]⁺ = 305.1.

### Step 2: benzyl-1-amino-6-azaspiro[3.4]octane-6-carboxylate

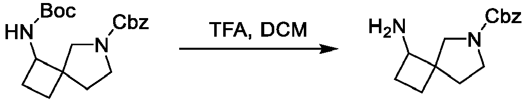

A compound benzyl 1-((tert-butoxycarbonyl)amino)-6-azaspiro[3.4]octane-6-carboxylate (134 mg, 0.37 mmol) was dissolved in dichloromethane (1 mL), trifluoroacetic acid (276 µL, 3.72 mmol) was added under 0°C, a reaction proceeded with stirring for 2 hours under 25°C, and the system was a yellow suspension. The reaction mixture was concentrated to afford a crude product benzyl 1-amino-6-azaspiro[3.4]octane-6-carboxylate (185 mg), and the crude product was directly subjected to the next step without purification. LCMS (ESI): [M+H]⁺ = 261.1.

### Step 3: 4-(3-(2-(methylsulfonyl)-5-(trifluoromethyl)pyrimidin-4-yl)-1-(benzenesulfonyl)-1H-pyrrolo[2,3-b]pyridin-6-yl)morpholine

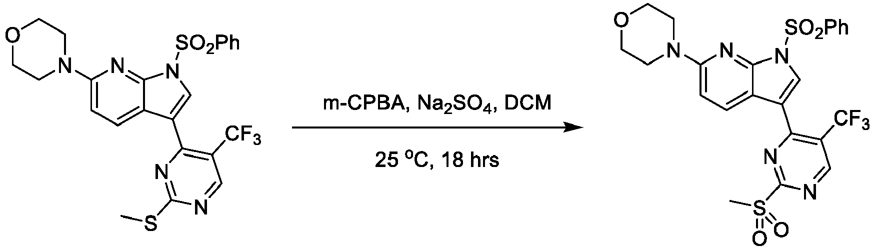

A suspension of a compound 4-(3-(2-(methylthio)-5-(trifluoromethyl)pyrimidin-4-yl)-1-(benzenesulfonyl)-1H-pyrrolo[2,3-b]pyridin-6-yl)morpholine (80% purity, 3.50 g, 5.23 mmol) and sodium sulfate (725 mg, 5.75 mmol) in dichloromethane (59 mL) was cooled to 0°C, and m-chloroperoxybenzoic acid (85% purity, 2.34 g, 11.50 mmol) was added in batches. After the addition was completed, a reaction was conducted for 40 minutes under 25°C. The system was a yellow suspension, the mixture was filtered, and the filtrate was directly used in the next-step reaction. LCMS (ESI): [M+H]⁺ = 568.1.

### Step 4: benzyl 1-((4-(6-morpholinyl-1-(benzenesulfonyl)-1H-pyrrolo[2,3-b]pyridin-3-yl)-5-(trifluoromethyl)pyrimidin-2-yl)amino)-6-azaspiro[3.4]octane-6-carboxylate

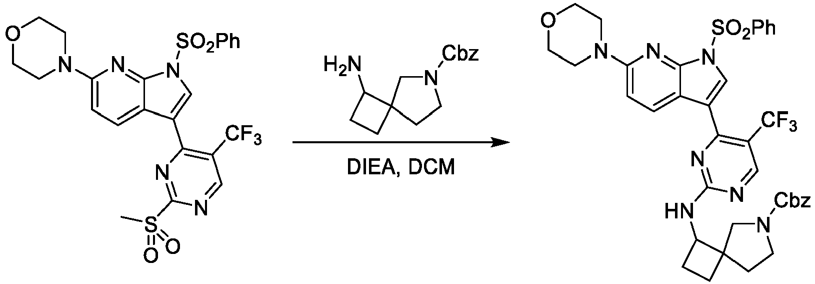

After a solution of a compound 4-(3-(2-(methylsulfonyl)-5-(trifluoromethyl)pyrimidin-4-yl)-1-(benzenesulfonyl)-1H-pyrrolo[2,3-b]pyridin-6-yl)morpholine (180 mg, 0.32 mmol) in dichloromethane (2 mL) was cooled to 0°C, diisopropylethylamine (1048 uL, 6.34 mmol) was added, and then a solution of a compound benzyl 1-amino-6-azaspiro[3.4]octane-6-carboxylate (50% purity, 185 mg, 0.35 mmol) in dichloromethane (700 µL) was added dropwise; and after the addition was completed, a reaction was conducted for 18 hours under 25°C. The system was a yellow suspension, and the mixture was concentrated to be dry and purified by flash column chromatography (silica gel, ethyl acetate/petroleum ether with 0-26% gradient) to afford a white solid benzyl 1-((4-(6-morpholinyl-1-(benzenesulfonyl)-1H-pyrrolo[2,3-b]pyridin-3-yl)-5-(trifluoromethyl)pyrimidin-2-yl)amino)-6-azaspiro[3.4]octane-6-carboxylate (132 mg, 0.18 mmol, 56% yield). LCMS (ESI): [M+H]⁺ = 748.3.

### Step 5: benzyl 1-((4-(6-morpholinyl-1H-pyrrolo[2,3-b]pyridin-3-yl)-5-(trifluoromethyl) pyrimidin-2-yl)amino)-6-azaspiro[3.4]octane-6-carboxylate

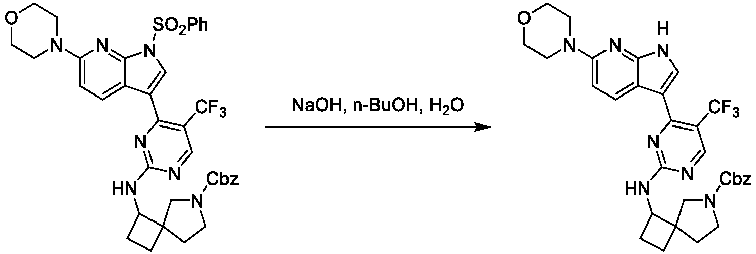

An aqueous sodium hydroxide solution (4 M, 221 µL, 0.88 mmol) was added to a solution of a compound benzyl 1-((4-(6-morpholinyl-1-(benzenesulfonyl)-1H-pyrrolo[2,3-b]pyridin-3-yl)-5-(trifluoromethyl)pyrimidin-2-yl)amino)-6-azaspiro[3.4]octane-6-carboxylate (132 mg, 0.18 mmol) in n-butanol (1200 uL), and the reaction solution was reacted for 18 hours under 25°C. The system was a yellow suspension. The reaction solution was diluted with water (2 mL) and subsequently extracted with ethyl acetate (2 mL * 3). The organic phases were combined, subsequently dried over magnesium sulfate, and filtered, and the filtrate was spin-dried to afford a crude product compound benzyl 1-((4-(6-morpholinyl-1H-pyrrolo[2,3-b]pyridin-3-yl)-5-(trifluoromethyl)pyrimidin-2-yl)amino)-6-azaspiro[3.4]octane-6-carboxylate (85 mg). LCMS (ESI): [M+H]⁺ = 608.3.

### Step 6: N-(4-(6-morpholinyl-1H-pyrrolo[2,3-b]pyridin-3-yl)-5-(trifluoromethyl)pyrimidin -2-yl)-6-azaspiro[3.4]octan-1-amine

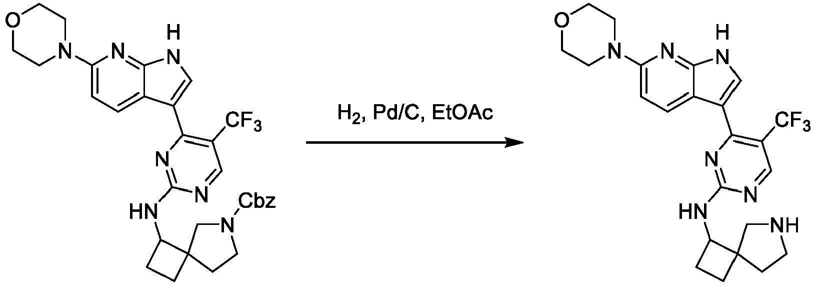

Under nitrogen protection, the compound benzyl 1-((4-(6-morpholinyl-1H-pyrrolo[2,3-b]pyridin-3-yl)-5-(trifluoromethyl)pyrimidin-2-yl)amino)-6-azaspiro[3.4]octane-6-carboxylate (98 mg, 0.16 mmol) was dissolved in ethyl acetate (2 mL), and dry Pd/C (10%, 50 mg) was added; and hydrogen balloon replacement was carried out, and stirring was carried out under a hydrogen atmosphere for 18 hours at 24°C. The reaction solution was filtered, the filtrate was concentrated, and the residue was purified by preparative HPLC to afford a white solid N-(4-(6-morpholinyl-1H-pyrrolo[2,3-b]pyridin-3-yl)-5-(trifluoromethyl)pyrimidin-2-yl)-6-azaspiro[3.4]octan-1-amine (0.89 mg, 2.00 umol, 1.17% two-step yield). LCMS (ESI): [M+H] ⁺ = 474.2;
¹H NMR (400 MHz, CD₃OD) δ ppm 8.69-8.43 (m, 2H), 7.72 (m, 1H), 6.95-6.69 (m, 1H), 4.20-4.10 (m, 1H), 3.90-3.76 (m, 4H), 3.62-3.48 (m, 4H), 2.33-2.15 (m, 3H), 2.04 (m, 5H), 1.62 (m, 2H).

We used the same method for synthesizing the compound **37,** and used 4-(3-(2-methylsulfonyl-5-(trifluoromethyl)pyrimidin-4-yl)-1H-pyrrolo[2,3-b]pyridin-6-yl) morpholine to react with a commercially available or simply synthesized amino compound to afford the following compounds through deprotection (chloroformic benzyl or tert-butoxycarbonyl):

### Embodiment 38. N-(4-(6-morpholinyl-1H-pyrrolo[2,3-b]pyridin-3-yl)-5-(trifluoromethyl) pyrimidin-2-yl)-1-azaspiro[4.5]decan-7-amine (compound 38)

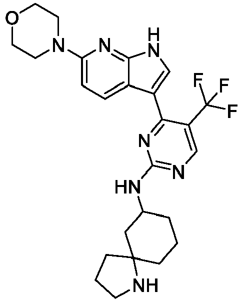

The compound **38** (4.16 mg, yellow solid). LCMS (ESI): [M+H]⁺ = 502.2;
¹H NMR (400 MHz, CD₃OD): δ ppm 8.62-8.47 (m, 2H), 7.75 (br s, 1H), 6.80 (d, *J* = 8.8 Hz, 1H), 3.89-3.85 (m, 5H), 3.55 (br d, *J* = 4.0 Hz, 4H), 3.21 (br t, *J* = 6.8 Hz, 2H), 2.03-1.92 (m, 4H), 1.86 (brt, *J* = 7.2 Hz, 2H), 1.80-1.74 (m, 2H), 1.70 (br d, *J* = 15.2 Hz, 4H).

### Embodiment 39. N-(4-(6-morpholinyl-1H-pyrrolo[2,3-b]pyridin-3-yl)-5-(trifluoromethyl) pyrimidin-2-yl)-2-azaspiro[3.3]heptan-5-amine (compound 39)

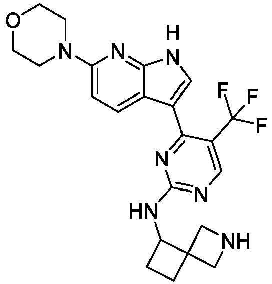

The compound **39** (10.74 mg, yellow solid). LCMS (ESI): [M+H]⁺ = 460.2;
¹H NMR (400 MHz, CD₃OD) δ ppm 8.80-8.37 (m, 2H), 7.75 (m, 1H), 6.81 (m, 1H), 4.24 (m, 1H), 3.95 (m, 2H), 3.88-3.80 (m, 6H), 3.61- 3.50 (m, 4H), 2.24-2.10 (m, 2H), 2.10-1.93 (m, 2H).

### Embodiment 40. N-(4-(6-morpholinyl-1H-pyrrolo[2,3-b]pyridin-3-yl)-5-(trifluoromethyl) pyrimidin-2-yl)-2-azaspiro[4.4]non-7-amine (compound 40)

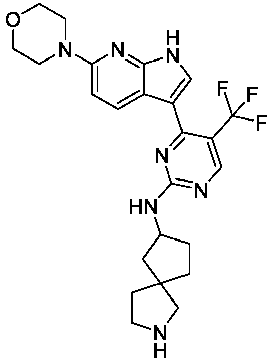

The compound **40** (5.05 mg, white solid). LCMS (ESI): [M+H]⁺ = 488.3;
¹H NMR (400 MHz, CD₃OD): δ ppm 8.62-8.42 (m, 2H), 7.74 (m, 1H), 6.79 (m, 1H), 3.93-3.82 (m, 5H), 3.55 (m, 4H), 3.16 (m, 2H), 3.02 (s, 2H), 2.25 (m, 2H), 1.90 (m, 3H), 1.80-1.69 (m, 3H)

### Embodiment 41. N-(4-(6-morpholinyl-1H-pyrrolo[2,3-b]pyridin-3-yl)-5-(trifluoromethyl) pyrimidin-2-yl)-3-azaspiro[5.5]undecan-8-amine, formate (compound 41)

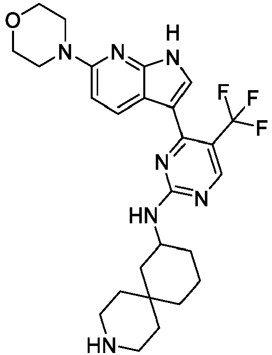

The compound **41** (2.22 mg, white solid). LCMS (ESI): [M+H]⁺ = 460.2;
¹H NMR (400 MHz, CD₃OD) δ ppm 8.61-8.44 (m, 3 H), 7.72 (m, 1H), 6.78 (m, 1H), 4.32-4.03 (m, 1H), 3.91-3.81 (m, 4H), 3.55 (m, 4H), 3.26-2.77 (m, 4 H), 2.17 (m, 2H), 1.85-1.52 (m, 6H), 1.45-1.10 (m, 4H).

### Embodiment 42. N-(4-(6-morpholinyl-1H-pyrrolo[2,3-b]pyridin-3-yl)-5-(trifluoromethyl) pyrimidin-2-yl)-8-azaspiro[4.5]decan-2-amine (compound 42)

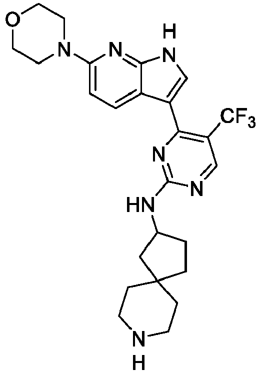

The compound **42** (6.76 mg, white solid). LCMS (ESI): [M+H]⁺ = 502.3;
¹H NMR (400 MHz, CD₃OD) δ ppm 9.03-8.74 (m, 1H), 8.56 (s, 1H), 8.02 (m, 1H), 7.05 (m, 1H), 4.76-4.38 (m, 1H), 3.96-3.83 (m, 4H), 3.78-3.63 (m, 4H), 3.29-3.12 (m, 4H), 2.38-2.19 (m, 2H), 1.96-1.62 (m, 8H).

### Embodiment 45. (3aR,4R,7aS)-N-(4-(6-morpholinyl-1H-pyrrolo[2,3-b]pyridin-3-yl)-5-(trifluoromethyl)pyrimidin-2-yl)octahydro-1H-isoindole-4-amine (compound 45)

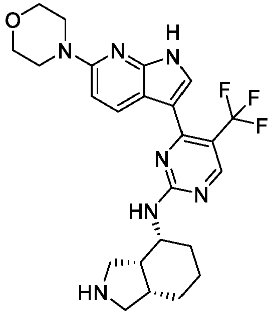

The compound **45** (5.20 mg, white solid). LCMS (ESI): [M+H]⁺ = 488.2;
¹H NMR (400 MHz, CD₃OD) δ ppm 8.51 (m, 2H), 7.74 (s, 1H), 6.80 (m, 1H), 3.85 (m, 4H), 3.54 (br s, 4H), 3.47-3.36 (m, 1H), 3.19 (m, 2H), 2.45 (br s, 1H), 1.99-1.82 (m, 5H), 1.80-1.78 (m, 1H), 1.78-1.41 (m, 2H), 1.35-1.17 (m, 1H).

### Embodiment 46. N-(4-(6-morpholinyl-1H-pyrrolo[2,3-b]pyridin-3-yl)-5-(trifluoromethyl) pyrimidin-2-yl)-3-azabicyclo[3.1.0]n-hexan-6-amine (compound 46)

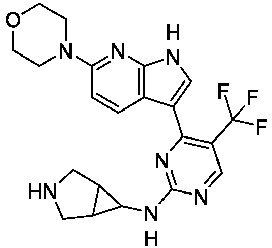

The compound **46** (20.45 mg, yellow solid). LCMS (ESI): [M+H] ⁺ = 446.2;
¹H NMR (400 MHz, CD₃OD) δ ppm 8.71-8.45 (m, 2H), 7.74 (s, 1H), 6.80 (m, 1H), 4.62 (m, 1H), 3.90-3.78 (m, 4H), 3.61-3.41 (m, 6H), 2.02 (br s, 2H), 1.94 (s, 2H).

### Embodiment 47. N-(4-(6-morpholinyl-1H-pyrrolo[2,3-b]pyridin-3-yl)-5-(trifluoromethyl) pyrimidin-2-yl)azepan-4-amine, formate (compound 47)

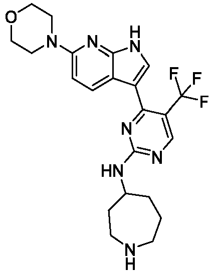

The compound **47** (51 mg, white solid). LCMS (ESI): [M+H]⁺ = 462.2;
¹H NMR (400 MHz, CD₃OD) δ ppm 8.73-8.35 (m, 3H) 7.75 (s, 1 H) 6.79 (m, 1H) 4.29 (br s, 1H) 3.93-3.80 (m, 4H) 3.61-3.50 (m, 4H) 3.46-3.34 (m, 2H) 3.29-3.19 (m, 2H) 2.33 (br s, 2H) 2.16-1.76 (m, 4H).

The compound **47** was separated by SFC (column: DAICEL CHIRALPAK AD (250 mm * 30 mm, 10 µm); mobile phases: Phase A of carbon dioxide, and Phase B of 0.1% ammonia water/isopropanol; holding Phase B at 65%; flow rate: 60 milliliter/minute) to afford an optically pure target compound **48** and an optically pure target compound **49.**

### Embodiment 48. A chiral monomer compound (compound 48) with a short peak time after the compound 47 was separated by SFC

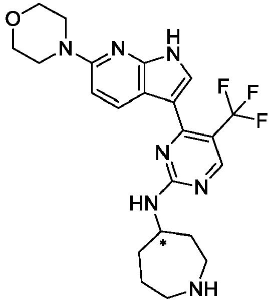

(The carbon atom with "*" represents a chiral carbon atom, which was in an S configuration or an R configuration)
The compound **48** (9.70 mg, white solid). LCMS (ESI): [M+H]⁺ = 462.2; SFC analysis (column: Chiralpak AD-3 (250 mm * 30 mm, 10 um); mobile phases: Phase A of carbon dioxide, and Phase B of 0.05% diethylamine/isopropanol, gradient: as for Phase B, from 5% to 40% in 2 minutes, holding Phase B at 40% for 1.2 minutes, and then holding Phase B at 5% for 0.8 minutes; flow rate: 2.5 milliliter/minute): RT = 2.187 min; ee = 98.98%.

¹H NMR (400 MHz, CD₃OD) δ ppm 8.45-8.35 (m, 2H), 7.60 (s, 1H), 6.65 (br d, *J* = 8.40 Hz, 1H), 4.15 (m, 1H), 3.74-3.71 (m, 4H), 3.44-3.41 (m, 4H), 2.90-2.83 (m, 4H), 2.20-2.00 (m, 2H), 1.76-1.66 (m, 4H).

### Embodiment 49. A chiral monomer compound (compound 49) with a long peak time after the compound 47 was separated by SFC

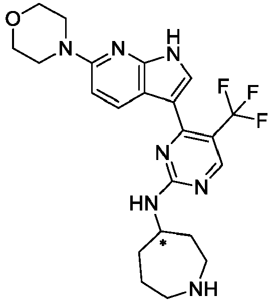

The compound **49** (11.70 mg, white solid). LCMS (ESI): [M+H]⁺ = 462.2; SFC analysis (column: Chiralpak AD-3 (250 mm * 30 mm, 10 um); mobile phases: Phase A of carbon dioxide, and Phase B of 0.05% diethylamine/isopropanol, gradient: as for Phase B, from 5% to 40% in 2 minutes, holding Phase B at 40% for 1.2 minutes, and then holding Phase B at 5% for 0.8 minutes; flow rate: 2.5 milliliter/minute): RT = 2.877 min, ee = 98.06%.

¹H NMR (400 MHz, CD₃OD) δ ppm 8.45-8.35 (m, 2H), 7.60 (s, 1H), 6.65 (br d, *J* = 8.4 Hz, 1H), 4.15 (m, 1H), 3.74-3.71 (m, 4H), 3.44-3.41 (m, 4H), 2.90-2.83 (m, 4H), 2.20-2.00 (m, 2H), 1.76-1.66 (m, 4H).

### Embodiment 50. N-(4-(6-morpholinyl-1H-pyrrolo[2,3-b]pyridin-3-yl)-5-(trifluoromethyl)pyrimidin-2-yl)-1-oxa-7-azaspiro[4.5]decan-3-amine (compound 50)

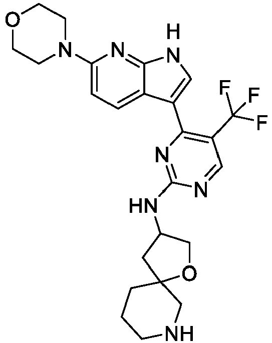

The compound **50** (36.73 mg, yellow solid). LCMS (ESI): [M+H]⁺ = 504.2;
¹H NMR (400 MHz, DMSO-*d*₆) δ ppm 11.88 (br s, 1H), 8.74-8.35 (m, 2 H), 8.09 (br d, *J* = 6.7 Hz, 1H), 7.71-7.47 (m, 1H), 6.93-6.64 (m, 1H), 4.75-4.41 (m, 1H), 3.98-4.11 (m, 1H), 3.82-3.65 (m, 4H), 3.48 (m, 2H), 2.81-2.54 (m, 4H), 2.39-2.27 (m, 1H), 1.89 (s, 4H), 1.79-1.51 (m, 4H), 1.38 (br s, 1H).

### Embodiment 51. N-(4-(6-morpholinyl-1H-pyrrolo[2,3-b]pyridin-3-yl)-5-(trifluoromethyl)pyrimidin-2-yl)-1-oxa-8-azaspiro[4.5]decan-3-amine (compound 51)

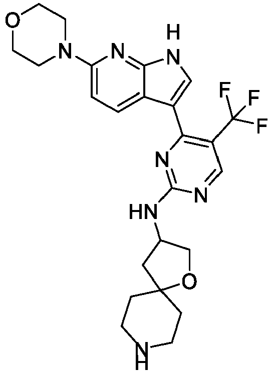

The compound **51** (60.55 mg, yellow solid). LCMS (ESI): [M+H]⁺ = 504.2;
¹H NMR (400 MHz, DMSO-*d*₆) δ ppm 11.88 (br s, 1H), 8.68-8.45 (m, 2H), 8.41 (m, 1H), 7.61 (m, 1H), 6.78 (m, 1H), 4.75-4.41 (m, 1H), 4.11-3.98 (m, 1H), 3.75 (m, 5H), 3.48 (m, 4H), 2.81-2.45 (m, 4H), 2.27-2.39 (m, 1H), 1.89 (m, 1H), 1.66-1.55 (m, 4H).

### Embodiment 52. 4-(6-morpholinyl-1H-pyrrolo[2,3-b]pyridin-3-yl)-N-(pyrrolidin-3-ylmethyl)-5-(trifluoromethyl)pyrimidin-2-amine, formate (compound 52)

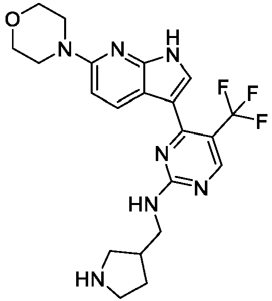

The compound **52** (2.77 mg, white solid). LCMS (ESI): [M+H]⁺ = 448.2;
¹H NMR (400 MHz, CD₃OD) δ ppm 8.85-8.24 (m, 3H), 7.73 (s, 1H), 6.80 (m, 1H), 3.96-3.82 (m, 3H), 3.98-3.76 (m, 1H), 3.72-3.49 (m, 6H), 3.42 (m, 2H), 3.29 (br s, 1H), 3.08 (br s, 1H), 2.81 (br s, 1H), 2.22 (m, 1H), 1.86 (m, 1H).

### Embodiment 53. N-((2S,4R)-2-methylpiperidin-4-yl)-4-(6-morpholinyl-1H-pyrrolo[2,3-b]pyridin-3-yl)-5-(trifluoromethyl)pyrimidin-2-amine, formate (compound 53)

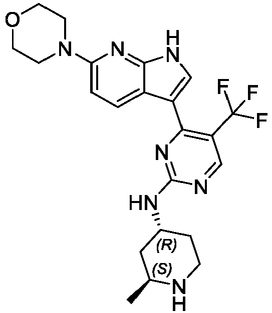

The compound **53** (110.74 mg, yellow solid). LCMS (ESI): [M+H]⁺ = 462.2;
¹H NMR (400 MHz, CD₃OD) δ ppm 8.55 (m, 3H), 7.73 (br s, 1 H), 6.78 (d, *J* = 8.8 Hz, 1H), 4.44 (m, 1H), 3.85 (m, 4H), 3.62 (m, 1H), 3.54 (m, 4H), 3.34 (m, 2H), 2.24 (m, 2H), 2.07 (m, 1H), 1.90 (m, 1H), 1.37 (m, 3H).

### Embodiment 54. N-((2R,4R)-2-methylpiperidin-4-yl)-4-(6-morpholinyl-1H-pyrrolo[2,3-b]pyridin-3-yl)-5-(trifluoromethyl)pyrimidin-2-amine (compound 54)

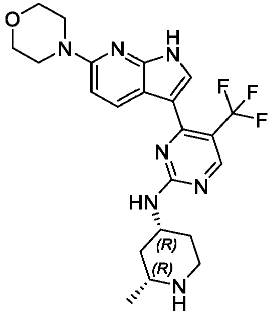

The compound **54** (29.62 mg, yellow solid). LCMS (ESI): [M+H]⁺ = 462.2
¹H NMR (400 MHz, DMSO-*d*₆) δ ppm 11.87 (m, 1H), 8.71-8.40 (m, 2H), 7.87 (m, 1H), 7.62 (m, 1H), 6.78 (m, 1H) 3.94 (m, 1H), 3.75 (br s, 4H), 3.47 (br s, 4H), 3.03 (m, 1H), 2.76-2.54 (m, 3H), 1.88 (m, 2H), 1.48-1.29 (m, 1H), 1.21-0.97 (m, 4H).

### Embodiment 55. N-(4-(6-morpholinyl-1H-pyrrolo[2,3-b]pyridin-3-yl)-5-(trifluoromethyl)pyrimidin-2-yl)octahydrocyclopenta[c]pyrrol-4-amine, formate (compound 55)

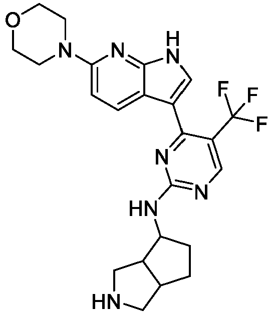

The compound **55** (16 mg, yellow solid). LCMS (ESI): [M+H]⁺ = 474.2
¹H NMR (400 MHz, CD₃OD) δ ppm 8.60-8.54 (m, 3H), 7.73 (s, 1H), 6.79 (s, 1H), 4.48 (s, 1H), 3.86 (m, 4H), 3.55 (m, 6H), 3.13-2.83 (m, 4H), 2.11 (m, 1H), 1.91 (m, 2H), 1.68 (m, 1H).

The compound **55** was separated by SFC (column: DAICEL CHIRALPAK IG (250 mm * 30 mm, 10 µm); mobile phases: Phase A of carbon dioxide, and Phase B of 0.1% ammonia water/ethyl acetate; holding Phase B at 50%; flow rate: 60 milliliter/minute), and two components were further purified by preparative HPLC respectively to afford a target compound **56** and a target compound **57.**

### Embodiment 56. A chiral monomer (compound 56) with a short peak time after chiral resolution of the compound 55

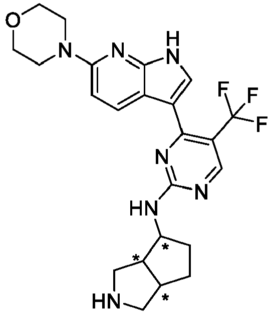

The compound **56** (1.91 mg, yellow solid, formate). LCMS (ESI): [M+H]⁺ = 474.2; SFC analysis (column: Chiralpak IG-3 (50 mm * 4.6 mm, 3 um); mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT = 2.343 min, ee = 100%.

¹H NMR (400 MHz, CD₃OD) δ ppm 8.70-8.50 (m, 3H), 7.72 (m, 1H), 6.79 (m, 1H), 4.46 (m, 1H), 3.86 (m, 4H), 3.55 (m, 6H), 3.10-2.80 (m, 4H), 2.10 (m, 1H), 1.91 (m, 2H), 1.68 (m, 1H).

### Embodiment 57. A chiral monomer (compound 57) with a long peak time after chiral resolution of the compound 55

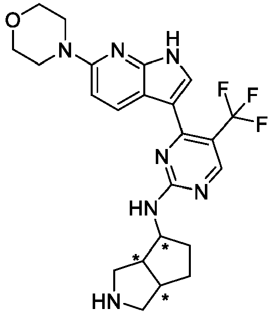

The compound **57** (1.01 mg, yellow solid, formate). LCMS (ESI): [M+H]⁺ = 474.2; SFC analysis (column: Chiralpak IG-3 (50 mm * 4.6 mm, 3 um); mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT = 2.847 min, ee = 96.88%.

¹H NMR (400 MHz, CD₃OD) *δ* ppm 8.70-8.50 (m, 3H), 7.72 (s, 1H), 6.79 (m, 1H), 4.46 (m, 1H), 3.86 (m, 4H), 3.55 (m, 6H), 3.10-2.80 (m, 4H), 2.10 (m, 1H), 1.91 (m, 2H), 1.68 (m, 1H).

### Embodiment 58. N-(4-(6-morpholinyl-1H-pyrrolo[2,3-b]pyridin-3-yl)-5-(trifluoromethyl)pyrimidin-2-yl)-1-oxa-6-azaspiro[3.3]heptan-3-amine (compound 58)

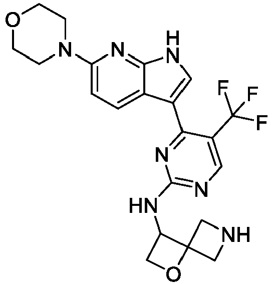

The compound **58** (25.64 mg, yellow solid). LCMS (ESI): [M+H]⁺ = 462.2;
¹H NMR (400 MHz, CD₃OD) δ ppm 8.87-8.38 (m, 2H), 7.73 (s, 1H), 6.80 (s, 1H), 5.36-5.11 (m, 1H), 4.78 (s, 1H), 4.55 (s, 1H), 4.17 (s, 1H), 4.01 (m, 1H), 3.90-3.79 (m, 5H), 3.75 (s, 1H), 3.61-3.50 (m, 4H).

### Embodiment 59. (R)-N-(4-(6-morpholinyl-1H-pyrrolo[2,3-b]pyridin-3-yl)-5-(trifluoromethyl)pyrimidin-2-yl)-2-azaspiro[3.3]heptan-5-amine (compound 59)

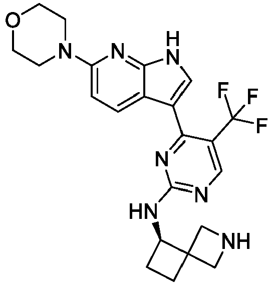

The compound **59** (10.74 mg, yellow solid). LCMS (ESI): [M+H]⁺ = 460.2;
¹H NMR (400 MHz, CD₃OD) δ ppm 8.71-8.67 (m, 2H), 7.75 (m, 1H), 6.80 (br s, 1H), 4.24 (m, 1H), 4.10-3.78 (m, 8H), 3.61-3.50 (m, 4H), 2.30 (m, 1H), 2.24-2.10 (m, 2H), 2.10-1.93 (m, 1H).

### Embodiment 61. (R)-3-(2-((2-azaspiro[3.3]heptan-5-yl)amino)-5-(trifluoromethyl)pyrimidin-4-yl)-1H-pyrrolo[2,3-b]pyridine-6-carbonitrile (compound 61)

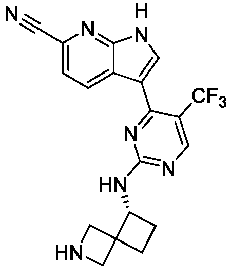

### Step 1: tert-butyl (R)-5-((4-(6-cyano-1-(benzenesulfonyl)-1H-pyrrolo[2,3-b]pyridin-3-yl)-5-(trifluoromethyl)pyrimidin-2-yl)amino)-2-azaspiro[3.3]heptane-2-carboxylate

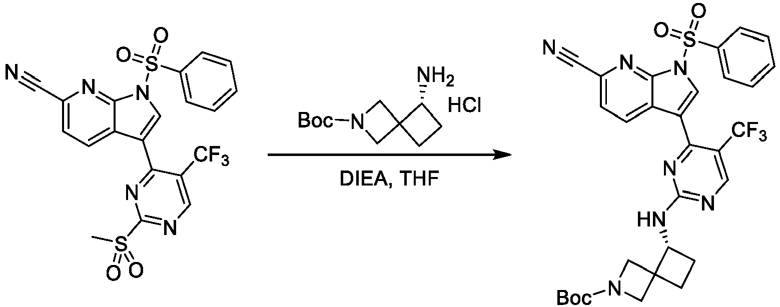

A compound 3-(2-(methylsulfonyl)-5-(trifluoromethyl)pyrimidin-4-yl)-1-(benzenesulfonyl)-1H-pyrrolo[2,3-b]pyridine-6-carbonitrile (230 mg, 0.32 mmol) and a compound tert-butyl (R)-5-amino-2-azaspiro[3.3]heptane-2-carboxylate hydrochloride (79 mg, 0.32 mmol) were dissolved in tetrahydrofuran (3 mL), diisopropylethylamine (524 µL, 3.17 mmol) was added under 20^{°}C, and a resulting reaction system was stirred for 1 hour at 20°C; concentration was carried out, the residue was purified by flash column chromatography (silica gel, 0-30 % gradient of ethyl acetate/petroleum ether) to afford a yellow solid compound tert-butyl (R)-5-((4-(6-cyano-1-(benzenesulfonyl)-1H-pyrrolo[2,3-b]pyridin-3-yl)-5-(trifluoromethyl)pyrimidin-2-yl)amino)-2-azaspiro[3.3]heptane-2-carboxylate (140 mg, 0.22 mmol, 69% yield). LCMS (ESI): [M-56+H]⁺ = 584.1;

### Step 2: tert-butyl (R)-5-((4-(6-cyano-1H-pyrrolo[2,3-b]pyridin-3-yl)-5-(trifluoromethyl)pyrimidin-2-yl)amino)-2-azaspiro[3.3]heptane-2-carboxylate

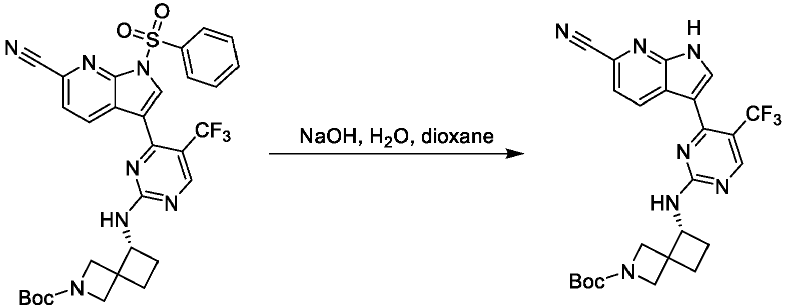

A compound tert-butyl (R)-5-((4-(6-cyano-1-(benzenesulfonyl)-1H-pyrrolo[2,3-b]pyridin-3-yl)-5-(trifluoromethyl)pyrimidin-2-yl)amino)-2-azaspiro[3.3]heptane-2-carboxylate (120 mg, 0.19 mmol) was dissolved in 1,4-dioxane (2 mL), an aqueous sodium hydroxide solution (4 M, 375 µL, 1.50 mmol) was added, and a resulting reaction system was reacted for 2 hours at 20°C. Water (2 mL) was added, ethyl acetate (3 mL * 3) was used for extraction, and organic phases were combined, dried over anhydrous sodium sulfate, and concentrated to afford a crude product tert-butyl (R)-5-((4-(6-cyano-1H-pyrrolo[2,3-b]pyridin-3-yl)-5-(trifluoromethyl)pyrimidin-2-yl)amino)-2-azaspiro[3.3]heptane-2-carboxylate (122 mg). LCMS (ESI): [M-100+H]⁺ = 400.1.

### Step 3: (R)-3-(2-((2-azaspiro[3.3]heptan-5-yl)amino)-5-(trifluoromethyl)pyrimidin-4-yl)-1H-pyrrolo[2,3-b]pyridine-6-carbonitrile

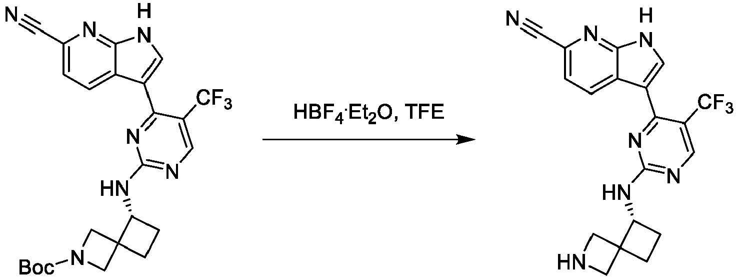

A compound tert-butyl (R)-5-((4-(6-cyano-1H-pyrrolo[2,3-b]pyridin-3-yl)-5-(trifluoromethyl)pyrimidin-2-yl)amino)-2-azaspiro[3.3]heptane-2-carboxylate (122 mg, 0.17 mmol) was dissolved in trifluoroethanol (2 mL), a tetrafluoroboric acid - diethyl ether adduct (50% content, 111 mg, 0.34 mmol) was added under 0°C, and a resulting reaction system was stirred for 1 hour under 20°C. Concentration was carried out, a residue was purified with preparative HPLC to afford a white solid compound (R)-3-(2-((2-azaspiro[3.3]heptan-5-yl)amino)-5-(trifluoromethyl)pyrimidin-4-yl)-1H-pyrrolo[2,3-b]pyridine-6-carbonitrile (20.64 mg, 47 umol, 27% yield). LCMS (ESI): [M+H]⁺ = 399.9;

¹H NMR (400 MHz, CD₃OD) δ ppm 9.07 (m, 1H), 8.73 (m, 1H) 8.27 (m, 1H), 7.68 (br d, *J* = 8.28 Hz, 1H), 4.64-4.48 (m, 2H), 4.24 (m, 1H), 4.00 (br s, 2H), 2.31 (m, 1H), 2.23-2.14 (m, 2H), 2.06-1.98 (m, 1H).

We used the same method for synthesizing the compound **61** for synthesis, and an intermediate 7 (3-(2-(methylsulfonyl)-5-(trifluoromethyl)pyrimidin-4-yl)-1-(benzenesulfonyl)-1H-pyrrolo[2,3-b]pyridine-6-carbonitrile) reacted with a corresponding amine to synthesize the following compound:

### Embodiment 62. 3-(2-((3,3-dimethyl-1,4-oxazepan-6-yl)amino)-5-(trifluoromethyl)pyrimidin-4-yl)-1H-pyrrolo[2,3-b]pyridine-6-carbonitrile (a compound 62)

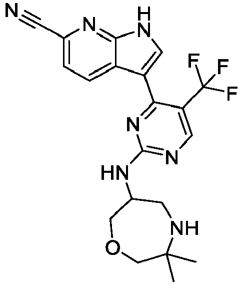

The compound **62** (6.64 mg, yellow solid). LCMS (ESI): [M+H]⁺ = 432.1;
¹H NMR (400 MHz, CD₃OD) δ ppm 8.73 (m, 1H), 8.60 (br s, 1H), 8.21 (br s, 1H), 7.68 (br s, 1H), 4.44 (m, 1H), 4.06 (m, 1H), 3.76 (m, 1H), 3.56 (m, 2H), 3.07 (m, 2H), 1.14 (m, 6H)

The compound **62** was separated by SFC (column: DAICEL CHIRALPAK AD (250 mm * 30 mm, 10 µm); mobile phases: Phase A of carbon dioxide; Phase B of 0.1% ammonia water/ethanol; holding Phase B at 70%; flow rate: 70 milliliter/minute) to afford a compound **63** and a compound **64.**

### Embodiment 63. A chiral monomer (compound 63) with a short peak time after SFC resolution of the compound 62

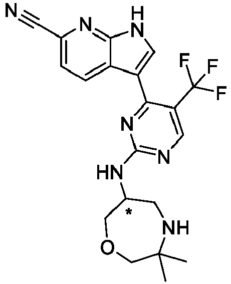

The compound **63** (7.24 mg, yellow solid). LCMS (ESI): [M+H]⁺ = 432.1; SFC analysis (column: ChiralPak AD-3 (150 mm * 4.6 mm), 3 um; mobile phases: Phase A of carbon dioxide, and Phase B of 0.05% diethylamine/ethanol; gradient: as for Phase B, from 5% to 40% in 4.5 minutes, and holding Phase B at 5% for 1.5 minutes; flow rate: 2.5 milliliter/minute): RT = 3.934 min, ee = 100%
¹H NMR (400 MHz, CD₃OD) δ ppm 8.73 (m, 1H), 8.60 (br s, 1H), 8.21 (br s, 1H), 7.68 (br s, 1H), 4.44 (m, 1H), 4.06 (m, 1H), 3.76 (m, 1H), 3.56 (m, 2H), 3.07 (m, 2H), 1.14 (m, 6 H)

### Embodiment 64. A chiral monomer (compound 64) with a long peak time after SFC resolution of the compound 62

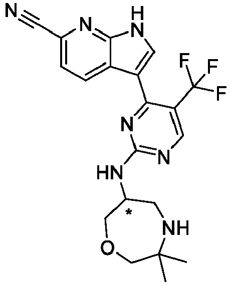

The compound **64** (7.10 mg, yellow solid). LCMS (ESI): [M+H]⁺ = 432.2; SFC analysis (column: ChiralPak AD-3 (150 mm * 4.6 mm), 3 um; mobile phases: Phase A of carbon dioxide, and Phase B of 0.05% diethylamine/ethanol; gradient: as for Phase B, from 5% to 40% in 4.5 minutes, and holding Phase B at 5% for 1.5 minutes; flow rate: 2.5 milliliter/minute): RT = 4.355 min, ee = 99.33%.

¹H NMR (400 MHz, CD₃OD) δ ppm 8.78 (m, 1H), 8.59 (br s, 1H), 8.21 (br s, 1H), 7.67 (br s, 1H), 4.39 (m, 1H), 4.06 (m, 1H), 3.75 (m, 1H), 3.55 (m, 2H), 3.07 (m, 2H), 1.14 (m, 6H).

### Embodiment 65. 3-(2-(((1S,3S)-3-aminocyclopentyl)amino)-5-(trifluoromethyl)pyrimidin-4-yl)-1H-pyrrolo[2,3-b]pyridine-6-carbonitrile (compound 65)

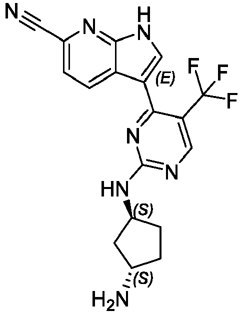

The compound **65** (12.10 mg, white solid). LCMS (ESI): [M+H]⁺ = 388.1;
¹H NMR (400 MHz, CD₃OD) δ ppm 8.78 (m, 1H), 8.59 (br s, 1H), 8.20 (br s, 1H), 7.67 (br s, 1H), 4.58 (m, 1H), 3.58 (m, 1H), 2.31 (m, 2H), 2.01 (m, 2H), 1.95 (m, 1H), 1.72 (m, 1H).

### Embodiment 66. 3-(2-((1S,3S)-3-(dimethylamino)cyclopentyl)amino)-5-(trifluoromethyl)pyrimidin-4-yl)-1H-pyrrolo[2,3-b]pyridine-6-carbonitrile (compound 66)

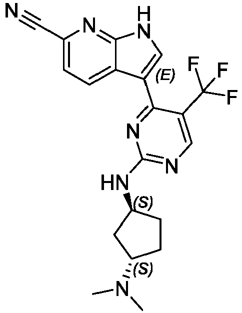

The compound **66** (8.64 mg, white solid). LCMS (ESI): [M+H]⁺ = 416.1;
¹H NMR (400 MHz, CD₃OD) δ ppm 8.98-8.97 (m, 1H), 8.56 (br s, 1H), 8.20 (br s, 1H), 7.67 (br s, 1H), 4.54 (m, 1H), 3.32 (m, 1H), 2.61-2.56 (m, 6H), 2.31-2.29 (m, 2H), 2.01 (m, 2H) 1.77-1.74 (m, 2H).

### Embodiment 67. (R)-N-(4-(6-((S)-3-methylmorpholine)-1H-pyrrolo[2,3-b]pyridin-3-yl)-5-(trifluoromethyl)pyrimidin-2-yl)-2-azaspiro[3.3]heptan-5-amine (compound 67)

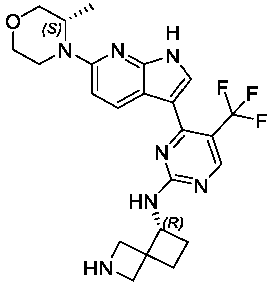

### Step 1: tert-butyl (R)-5-((4-(6-((S)-3-methylmorpholinyl)-1-(benzenesulfonyl)-1H-pyrrolo[2,3-b ]pyridin-3-yl)-5-(trifluoromethyl)pyrimidin-2-yl)amino)-2-azaspiro[3.3]heptane-2-carboxylate

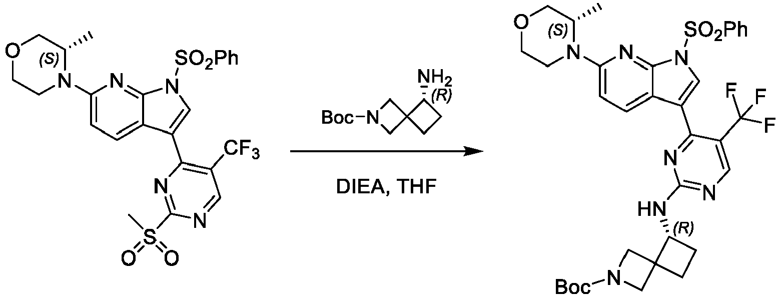

A compound (S)-3-methyl-4-(3-(2-(methylsulfonyl)-5-(trifluoromethyl)pyrimidin-4-yl)-1-(benzenesulfonyl)-1H-pyrrolo[2,3-b]pyridin-6-yl)morpholine (2.3 g, 1.58 mmol) and diisopropylethylamine (5 mL, 31.64 mmol) were dissolved in tetrahydrofuran (20 mL), a compound tert-butyl (R)-5-amino-2-azaspiro[3.3]heptane-2-carboxylate (390 mg, 1.58 mmol) was added, and a resulting reaction system was stirred for 12 hours under 25°C. The reaction system was concentrated to spin-remove tetrahydrofuran, added with water (20 mL) and extracted with ethyl acetate (30 mL * 3). Organic phases were combined, dried over anhydrous sodium sulfate, and filtered, and the filtrate was concentrated. The residue was purified by flash column chromatography (silica gel, ethyl acetate/petroleum ether with 0-30% gradient) to afford an umber solid tert-butyl (R)-5-((4-(6-((S)-3-methylmorpholinyl)-1-(benzenesulfonyl)-1H-pyrrolo[2,3-b]pyridin-3-yl)-5-(trifluoromethyl)pyrimidin-2-yl)amino)-2-azaspiro[3.3]heptane-2-carboxylate (340 mg, 0.48 mmol, 30% yield). LCMS (ESI): [M+H]⁺ = 714.2.

### Step 2: tert-butyl (R)-5-((4-(6-((S)-3-methylmorpholinyl)-1H-pyrrolo[2,3-b]pyridin-3-yl)-5-(trifluoromethyl)pyrimidin-2-yl)amino)-2-azaspiro[3.3]heptane-2-carboxylate

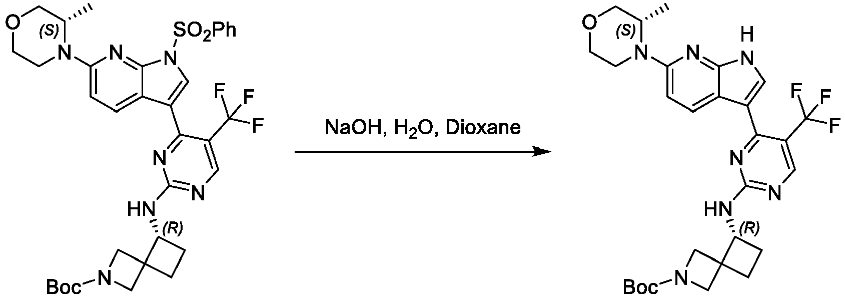

An aqueous sodium hydroxide solution (4 M, 953 uL, 3.81 mmol) was added to a compound tert-butyl (R)-5 -((4-(6-((S)-3-methylmorpholinyl)-1-(benzenesulfonyl)-1H-pyrrolo[2,3-b] pyridin-3-yl)-5-(trifluoromethyl)pyrimidin-2-yl)amino)-2-azaspiro[3.3]heptane-2-carboxylate (340 mg, 0.48 mmol) in 1,4-dioxane (14 mL). A resulting reaction system was stirred for 0.5 hours under 100°C. The reaction system was added with water (5 mL) for dilution, and extracted with ethyl acetate (10 mL * 3). Organic phases were combined, dried over anhydrous sodium sulfate, and filtered, and the filtrate was concentrated to afford a crude compound tert-butyl (R)-5-((4-(6-((S)-3-methylmorpholinyl)-1H-pyrrolo[2,3-b]pyridin-3-yl)-5-(trifluoromethyl)pyrimidin-2-yl)amino)-2-azaspiro[3.3]heptane-2-carboxylate (310 mg). LCMS (ESI): [M+H]⁺ = 574.3.

### Step 3: (R)-N-(4-(6-((S)-3-methylmorpholine)-1H-pyrrolo[2,3-b]pyridin-3-yl)-5-(trifluoromethyl)pyrimidin-2-yl)-2-azaspiro[3.3]heptan-5-amine

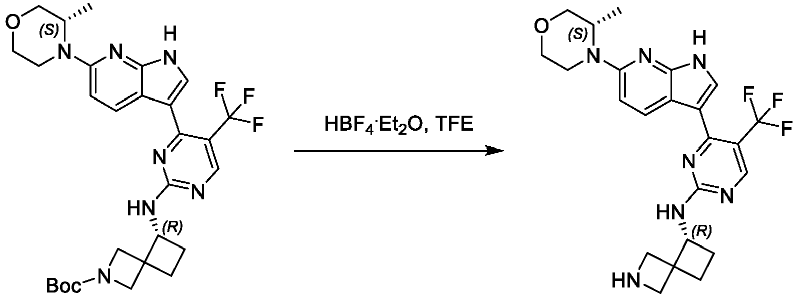

A compound tert-butyl (R)-5-((4-(6-((S)-3-methylmorpholinyl)-1H-pyrrolo[2,3-b]pyridin-3-yl)-5-(trifluoromethyl)pyrimidin-2-yl)amino)-2-azaspiro[3.3]heptane-2-carboxylate (310 mg, 0.54 mmol) was dissolved in trifluoroethanol (6 mL), a tetrafluoroboric acid - diethyl ether adduct (50% content, 2.63 g, 0.81 mmol) was added under 0°C, and the reaction system was stirred for 1 hour under 25°C. The reaction system was added with water (10 mL) for dilution and lyophilization, and the residue was purified by preparative HPLC to afford a yellow solid (R)-N-(4-(6-((S)-3-methylmorpholine)-1H-pyrrolo[2,3-b]pyridin-3-yl)-5-(trifluoromethyl)pyrimidin-2-yl)-2-azaspiro[3.3]heptan-5-amine (34 mg, 0.072 mmol, 13% yield). LCMS (ESI): [M+H]⁺ = 474.2;
¹H NMR (400 MHz, CD₃OD) δ ppm 8.77-8.36 (m, 2H), 7.70 (s, 1H), 6.74 (d, *J* = 8.8 Hz, 1H), 4.52 (s, 1H), 4.42 (s, 1H), 4.21-3.85 (m, 4H), 3.82 (d, *J* = 1.8 Hz, 2H), 3.72-3.36 (m, 3H), 3.26 (dt, *J* = 3.6, 12.6 Hz, 1H), 2.27 (s, 1H), 2.18-2.09 (m, 1H), 2.08-1.88 (m, 2H), 1.24 (d, *J* = 6.8 Hz, 3H).

We used the same method for synthesizing the compound 67, and used (S)-3-methyl-4-(3-(2-(methylsulfonyl)-5-(trifluoromethyl)pyrimidin-4-yl)-1-(benzenesulfonyl)-1H-pyrrolo[2,3-b]pyridin-6-yl)morpholine as a raw material to react with a corresponding amine to synthesize the following compounds:

### Embodiment 68. (1S,3R)-N1-(4-(6-((S)-3-methylmorpholinyl)-1H-pyrrolo[2,3-b]pyridin-3-yl)-5-(trifluoromethyl)pyrimidin-2-yl)cyclopentan-1,3-diamine, formate (compound 68)

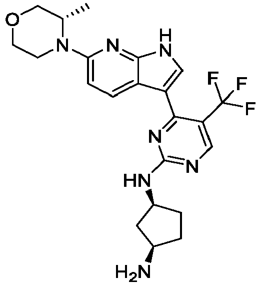

The compound **68** (15.10 mg, white solid). LCMS (ESI): [M+H]⁺ = 462.2;
¹H NMR (400 MHz, CD₃OD) δ ppm 8.54 (m, 3H), 7.71 (s, 1H), 6.74 (m, 1H), 4.44 (m, 2H), 4.02 (m, 1H), 3.83 (m, 3H), 3.66 (m, 2H), 3.25 (m, 1H), 2.65 (m, 1H), 2.18 (m, 2H), 1.86 (m, 2H), 1.65(m, 1H), 1.23(m, 3H).

### Embodiment 76. 3-(2-((3,3-dimethyl-1,4-oxazepan-6-yl)amino)-5-(trifluoromethyl) pyrimidin-4-yl)-7-(dimethylphosphoryl)-1H-indole-6-carbonitrile (compound 76)

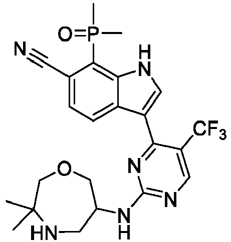

### Step 1: tert-butyl 6-((4-(7-bromo-6-cyano-1H-indole-3-yl)-5-(trifluoromethyl)pyrimidin-2-yl)amino)-3,3-dimethyl-1,4-oxazepane-4-carboxylate

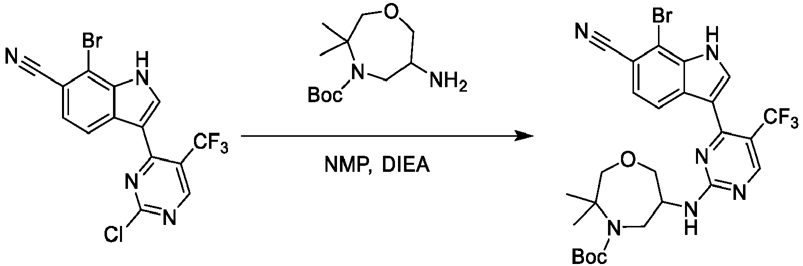

A compound 7-bromo-3-(2-chloro-5-(trifluoromethyl)pyrimidin-4-yl)-1H-indole-6-carbonitrile (160 mg, 0.40 mmol) and a compound tert-butyl 6-amino-3,3-dimethyl-1,4-oxazepane-4-carboxylate (107 mg, 0.44 mmol) were dissolved in 1-methyl-2-pyrrolidone (4 mL), and diisopropylethylamine (493 µL, 2.99 mmol) was added under 25°C. The temperature was raised to 130°C, and a reaction was conducted for 3 hours under this temperature. The reaction system was cooled to room temperature, and the residue was purified by flash column chromatography (C18, acetonitrile/water with 0-40% gradient) to afford a white solid compound tert-butyl 6-((4-(7-bromo-6-cyano-1H-indole-3-yl)-5-(trifluoromethyl)pyrimidin-2-yl)amino)-3,3-dimethyl-1,4-oxazepane-4-carboxylate (120 mg, 0.20 mmol, 50% yield). LCMS (ESI): [M+H]⁺ = 609.2.

### Step 2: tert-butyl 6-((4-(6-cyano-7-(dimethylphosphoryl)-1H-indole-3-yl)-5-(trifluoromethyl)pyrimidin-2-yl)amino)-3,3-dimethyl-1,4-oxazepane-4-carboxylate

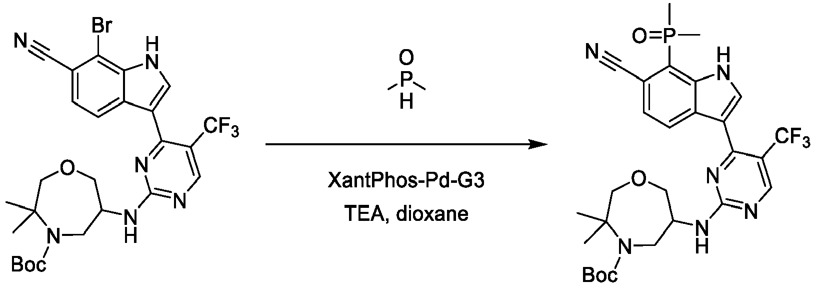

A compound tert-butyl 6-((4-(7-bromo-6-cyano-1H-indole-3-yl)-5-(trifluoromethyl)pyrimidin-2-yl)amino)-3,3-dimethyl-1,4-oxazepane-4-carboxylate (100 mg, 0.21 mmol) and dimethylphosphine oxide (15 mg, 0.20 mmol) were dissolved in 1,4-dioxane (2 mL); under nitrogen protection, triethylamine (68 µL, 0.49 mmol) and a methanesulfonato[9,9-dimethyl-4,5-bis(diphenylphosphino)xanthene][2-amino-1,1-biphenyl]palladium(II) dichloromethane adduct (16 mg, 16.00 umol) were added under 25°C. The reaction system was stirred for 2 hours under 100°C. Cooling to room temperature was carried out, concentration was carried out, and the residue was purified by flash column chromatography (C18, acetonitrile/water with 0-95% gradient) to afford a yellow solid compound tert-butyl 6-((4-(6-cyano-7-(dimethylphosphoryl)-1H-indole-3-yl)-5-(trifluoromethyl)pyrimidin-2-yl)amino)-3,3-dimethyl-1,4-oxazepane-4-carboxylate (58 mg, 86 umol, 41% yield). LCMS (ESI): [M+H]⁺ = 607.3;

### Step 3: 3-(2-((3,3-dimethyl-1,4-oxazepan-6-yl)amino)-5-(trifluoromethyl)pyrimidin-4-yl)-7-(dimethylphosphoryl)-1H-indole-6-carbonitrile

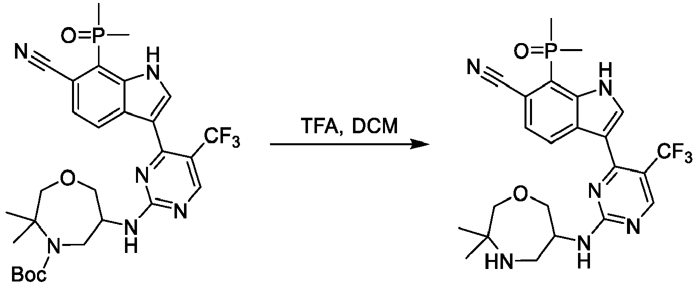

A compound tert-butyl 6-((4-(6-cyano-7-(dimethylphosphoryl)-1H-indole-3-yl)-5-(trifluoromethyl)pyrimidin-2-yl)amino)-3,3-dimethyl-1,4-oxazepane-4-carboxylate (110 mg, 0.18 mmol) was dissolved in dichloromethane (2 mL), and trifluoroacetic acid (135 µL, 1.81 mmol) was dropwise added under 0°C. The reaction system was stirred for 1 hours under 0^{°}C. The residue was purified by preparative HPLC to afford a white solid 3-(2-((3,3-dimethyl-1,4-oxazepan-6-yl)amino)-5-(trifluoromethyl)pyrimidin-4-yl)-7-(dimethylphosphoryl)-1H-indole-6-carbonitrile (2.16 mg, 4 umol, 2.2% yield). LCMS (ESI): [M+H]⁺ = 507.1.

¹H NMR (400 MHz, CD₃OD) δ ppm 8.46-8.81 (m, 2H), 8.19 (br s, 1H), 7.68 (br s, 1H), 4.48 (br s, 1H), 4.09 (br s, 1H), 3.76 (br s, 1H), 3.61 (br s, 2H), 3.08-3.29 (m, 2H), 2.16 (s, 3H), 2.13 (s, 3H), 1.21 (br s, 6H).

### Embodiment 77. (R)-3-(2-((2-azaspiro[3.3]heptan-5-yl)amino)-5-(trifluoromethyl)pyrimidin-4-yl)-7-(dimethylphosphoryl)-1H-indole-6-carbonitrile (compound 77)

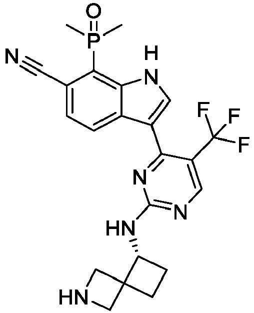

### Step 1: tert-butyl (R)-5-((4-(7-bromo-6-cyano-1H-indole-3-yl)-5-(trifluoromethyl)pyrimidin-2-yl)amino)-2-azaspiro[3.3]heptane-2-carboxylate

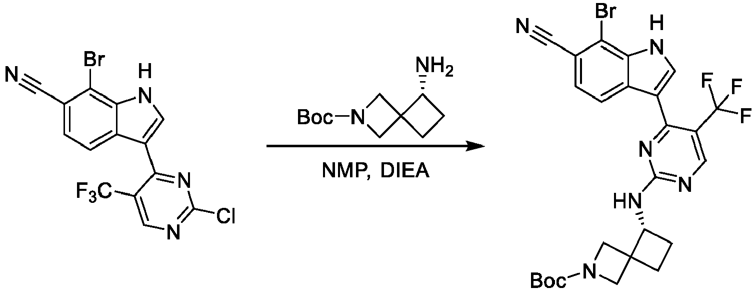

A compound 7-bromo-3-(2-chloro-5-(trifluoromethyl)pyrimidin-4-yl)-1H-indole-6-carbonitrile (200 mg, 0.50 mmol) and a compound tert-butyl (R)-5-amino-2-azaspiro[3.3]heptane-2-carboxylate (136 mg, 0.55 mmol) were dissolved in 1-methyl-2-pyrrolidone (8 mL), and diisopropylethylamine (610 uL, 3.74 mmol) was added under 25°C. The reaction system was reacted for 3 hours under 130°C. Cooling to room temperature was carried out, filtration was carried out, the residue was purified by flash column chromatography (C18, acetonitrile/water with 0-40% gradient) to afford a white solid compound tert-butyl (R)-5-((4-(7-bromo-6-cyano-1H-indole-3-yl)-5-(trifluoromethyl)pyrimidin-2-yl)amino)-2-azaspiro[3.3]heptane-2-carboxylate (270 mg, 0.47 mmol, 93% yield). LCMS (ESI): [M-56+H]⁺ = 520.0.

### Step 2: tert-butyl (R)-5-((4-(6-cyano-7-(dimethylphosphoryl)-1H-indole-3-yl)-5-(trifluoromethyl)pyrimidin-2-yl)amino)-2-azaspiro[3.3]heptane-2-carboxylate

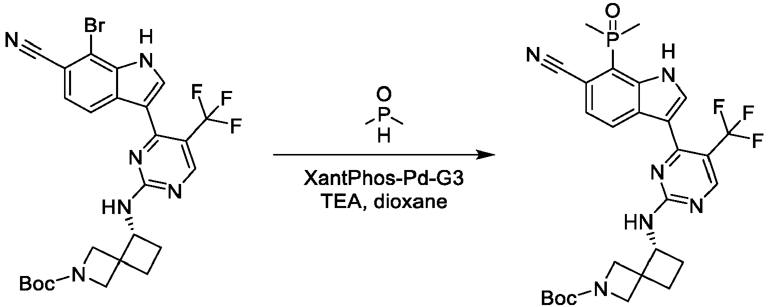

A compound tert-butyl (R)-5-((4-(7-bromo-6-cyano-1H-indole-3-yl)-5-(trifluoromethyl)pyrimidin-2-yl)amino)-2-azaspiro[3.3]heptane-2-carboxylate (270 mg, 0.47 mmol) and a compound dimethylphosphine oxide (44 mg, 0.56 mmol) were dissolved in 1,4-dioxane (2.5 mL), and under nitrogen protection, triethylamine (195 µL, 1.40 mmol) and a methanesulfonato[9,9-dimethyl-4,5-bis(diphenylphosphino)xanthene][2-amino-1,1-biphenyl]palladium(II) dichloromethane adduct (44 mg, 47 umol) were added at 25°C. A resulting reaction system was reacted for 4 hours under 100°C. Cooling to room temperature was carried out, water (2 mL) is added, and extraction is carried out with ethyl acetate (3 mL * 3). Organic phases were combined, dried over anhydrous sodium sulfate, and concentrated, and the residue was purified by flash column chromatography (silica gel, ethyl acetate/petroleum ether with 0-30% gradient) to afford a yellow solid compound tert-butyl (R)-5-( (4-(6-cyano-7-(dimethylphosphoryl)-1H-indole-3-yl)-5-(trifluoromethyl)pyrimidin-2-yl)amino)-2-azaspiro[3.3]heptane-2-carboxylate (50 mg, 87 umol, 18% yield). LCMS (ESI): [M+H]⁺ = 575.3.

### Step 3: (R)-3-(2-((2-azaspiro[3.3]heptan-5-yl)amino)-5-(trifluoromethyl)pyrimidin-4-yl)-7-(dimethylphosphoryl)-1H-indole-6-carbonitrile

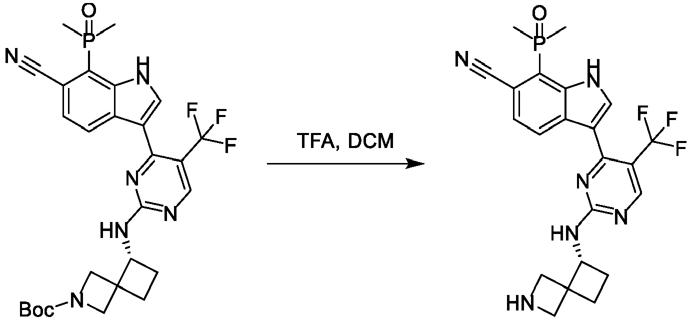

A compound tert-butyl (R)-5-((4-(6-cyano-7-(dimethylphosphoryl)-1H-indole-3-yl)-5-(trifluoromethyl)pyrimidin-2-yl)amino)-2-azaspiro[3.3]heptane-2-carboxylate (50 mg, 0.09 mmol) was dissolved in dichloromethane (5 mL), trifluoroacetic acid (129 µL, 1.74 mmol) was added under 20°C, and a resulting reaction system was reacted for 16 hours at 20°C. The reaction solution was concentrated, and the resulting residue was purified by preparative HPLC to afford a title yellow solid compound (R)-3-(2-((2-azaspiro[3.3]heptan-5-yl)amino)-5-(trifluoromethyl)pyrimidin-4-yl)-7-(dimethylphosphoryl)-1H-indole-6-carbonitrile (2.58 mg, 3 umol, 3.4% yield). LCMS (ESI): [M+H]⁺ = 475.2.

¹H NMR (400 MHz, CD₃OD) δ ppm 8.52-8.91 (m, 2H) 8.08-8.33 (m, 1H) 7.69 (br s, 1H) 4.62 (br s, 4H) 4.25 (br d, *J* = 10.8 Hz, 1H) 3.65-4.08 (m, 2H) 2.30 (br s, 1H) 2.15 (d, *J* = 13.8 Hz, 6H) 1.97-2.08 (m, 2H) 1.62 (br s, 1H).

By using the same method for synthesizing the compound 77, the following compounds were synthesized:

### Embodiment 78. 3-(2-(((1S,3S)-3-aminocyclopentyl)amino)-5-(trifluoromethyl)pyrimidin-4-yl)-7-(dimethylphosphoryl)-1H-indole-6-carbonitrile, formate (compound 78)

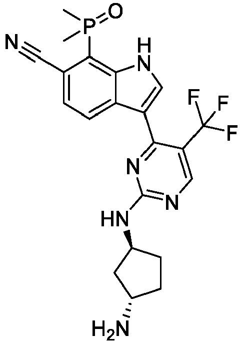

The compound **78** (18.30 mg, white solid). LCMS (ESI): [M+H]⁺ = 463.2.

¹H NMR (400 MHz, CD₃OD) δ ppm 8.74-8.55 (m, 3H), 8.20 (br s, 1H), 7.66 (m, 1H), 4.63 (m, 1H), 3.80 (m, 1H), 2.36 (m, 2H), 2.23-2.13 (m, 8H), 1.83-1.70 (m, 2H).

### Embodiment 79. (S)-7-(dimethylphosphoryl)-3-(2-((1-hydroxy-propan-2-yl)amino)-5-(trifluoromethyl)pyrimidin-4-yl)-1H-indole-6-carbonitrile (compound 79)

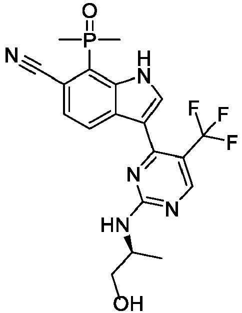

The compound **79** (41.44 mg, white solid). LCMS (ESI): [M+H]⁺ = 438.1.

¹H NMR (400 MHz, CD₃OD) δ ppm 8.74-8.50 (m, 2H), 8.17 (br s, 1H), 7.64 (br s, 1H), 4.28 (br s, 1H), 3.63 (m, 2H), 2.14 (s, 3H), 2.11 (s, 3H), 1.28 (d, *J* = 6.8 Hz, 3H).

### Embodiment 80. (R)-(3-(2-(2-azaspiro[3.3]heptan-5-yl)amino)-5-(trifluoromethyl)pyrimidin-4-yl)-1H-indole-7-yl)dimethylphosphine oxide (compound 80)

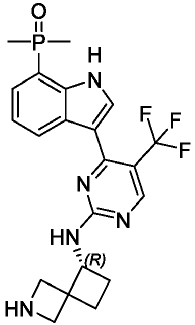

### Step 1: tert-butyl (R)-5-((4-(7-(dimethylphosphoryl)-1H-indole-3-yl)-5-(trifluoromethyl)pyrimidin-2-yl)amino)-2-azaspiro[3.3]heptane-2-carboxylate

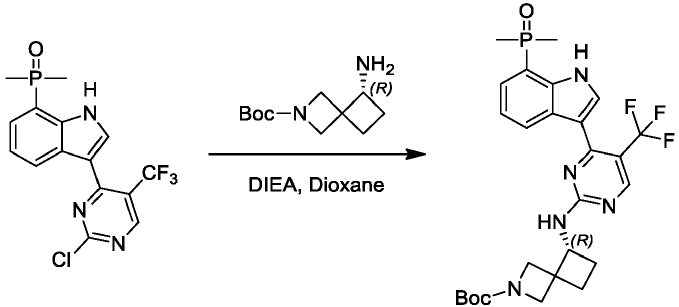

A compound (3-(2-chloro-5-(trifluoromethyl)pyrimidin-4-yl)-1H-indole-7-yl)dimethylphosphine oxide (168 mg, 0.45 mmol) and a compound tert-butyl (R)-5-amino-2-azaspiro[3.3]heptane-2-carboxylate (123 mg, 0.49 mmol) were dissolved in 1,4-dioxane (100 uL), and diisopropylethylenediamine (446 uL, 2.70 mmol) was added under 25°C. The reaction system was heated to 100°C and stirred for 3 hours. The reaction was detected by LCMS to be completed, and the reaction system was cooled to room temperature and concentrated to afford a crude compound tert-butyl (R)-5-((4-(7-(dimethylphosphoryl)-1H-indole-3-yl)-5-(trifluoromethyl)pyrimidin-2-yl)amino)-2-azaspiro[3.3]heptane-2-carboxylate (150 mg), which was a yellow oily liquid. LCMS (ESI): [M+H]⁺ = 450.0.

### Step 2: (R)-(3-(2-((2-azaspiro[3.3]heptan-5-yl)amino)-5-(trifluoromethyl)pyrimidin-4-yl)-1H-indole-7-yl)dimethylphosphine oxide

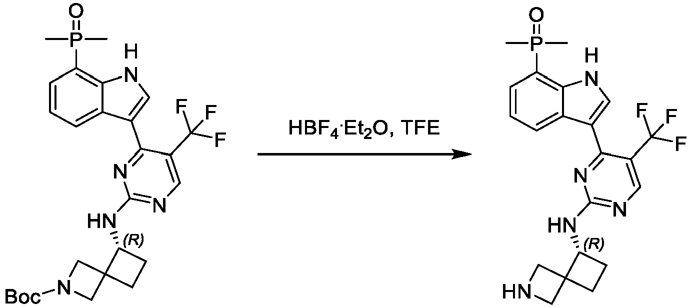

A compound tert-butyl (R)-5-((4-(7-(dimethylphosphoryl)-1H-indole-3-yl)-5-(trifluoromethyl)pyrimidin-2-yl)amino)-2-azaspiro[3.3]heptane-2-carboxylate (200 mg, 0.36 mmol) was dissolved in trifluoroethanol (2 mL), and a tetrafluoroboric acid - diethyl ether adduct (50% content, 236 mg, 0.73 mmol) was added under 0°C, an ice-salt bath was removed, and the reaction solution was stirred for 1 hour at 20°C. The reaction solution was lyophilized to afford a crude product, and the crude product was purified by preparative HPLC to afford a title compound (R)-(3-(2-((2-azaspiro[3.3]heptan-5-yl)amino)-5-(trifluoromethyl)pyrimidin-4-yl)-1H-indole-7-yl)dimethylphosphine oxide (67.85 mg, 0.15 mmol, 39% yield), which was a yellow solid. LCMS (ESI): [M+H]⁺ = 450.1.

¹H NMR (400 MHz, CD₃OD) δ ppm 8.48-8.75 (m, 2H), 7.98 (br s, 1H), 7.52 (m, 1H), 7.35 (br t, *J* = 6.3 Hz, 1H), 4.54 (br s, 1H), 3.90-4.35 (m, 2H), 3.39-3.80 (m, 2H), 2.00-2.34 (m, 3H), 1.93 (m, 7H).

By using the same method for synthesizing the compound 80, 3-(2-chloro-5-(trifluoromethyl)pyrimidin-4-yl)-1H-indole-7-yl)dimethylphosphine oxide was reacted with a corresponding amino compound to synthesize the following compounds:

### Embodiment 81. (3-(2-((1S,3S)-3-aminocyclopentyl)amino)-5-(trifluoromethyl)pyrimidin-4-yl)-1H-indole-7-yl)dimethylphosphine oxide (compound 81)

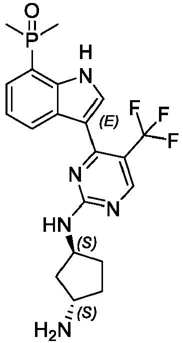

The compound **81** (4.52 mg, yellow solid). LCMS (ESI): [M+H]⁺ = 438.2.

¹H NMR (400 MHz, CD₃OD) δ ppm 8.57 (m, 2H), 8.06 (br s, 1H), 7.53 (m, 1H), 7.34 (m, 1H), 4.64 (m, 1H), 3.81 (m, 1H), 2.36 (m, 2H), 2.33-2.19 (m, 2H), 1.95 (s, 3H), 1.92 (s, 3H), 1.83-1.71 (m, 2H).

### Embodiment 82. (S)-(3-(2-((1-hydroxypropyl-2-yl)amino)-5-(trifluoromethyl)pyrimidin-4-yl)-1H-indole-7-yl)dimethylphosphine oxide (compound 82)

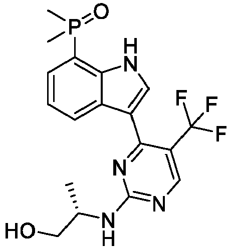

The compound **82** (11.12 mg, white solid). LCMS (ESI): [M+H]⁺ = 413.1.

¹H NMR (400 MHz, DMSO-*d*₆) δ ppm 11.56 (br s, 1H), 8.67-8.55 (m, 2H), 7.91 (m, 1H), 7.68 (m, 1H), 7.49 (m, 1H), 7.25 (m, 1H), 4.78 (m, 1H), 4.15 (m, 1H), 3.51 (m, 2H), 1.80 (s, 6H), 1.18 (d, *J* = 6.8 Hz, 3H)

### Embodiment 83. 3-(2-(((1S,3S)-3-hydroxycyclopentyl)amino)-5-(trifluoromethyl)pyrimidin-4-yl)-1H-indole-7-yl)dimethylphosphine oxide (compound 83)

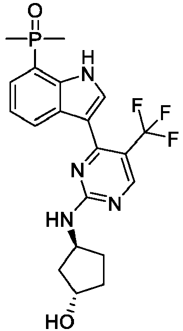

The compound **83** (13.02 mg, white solid). LCMS (ESI): [M+H]⁺ = 439.1.

¹H NMR (400 MHz, CD₃OD) δ ppm 8.67-8.51 (m, 2H), 7.97 (m, 1H), 7.51 (m, 1H), 7.35 (m, 1H), 4.71 (m, 1H), 4.41 (m, 1H), 2.32 (m, 1H), 2.13 (m, 2H), 1.95 (s, 3H), 1.91 (s, 3H), 1.87 (m, 1H), 1.65 (m, 2H).

### Embodiment 84. (3-(2-((1H-pyrazol-4-yl)amino)-5-(trifluoromethyl)pyrimidin-4-yl)-1H-indole-7-yl)dimethylphosphine oxide (compound 84)

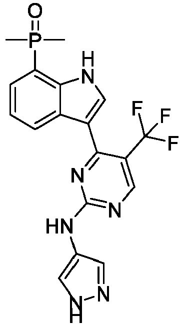

The compound **84** (23.12 mg, white solid). LCMS (ESI): [M+H]⁺ = 421.1.

¹H NMR (400 MHz, DMSO-*d*₆) δ ppm 12.64 (br s, 1H), 11.64 (br s, 1H), 10.12 (br s, 1H), 8.80-8.00 (m, 2H), 7.97-7.60 (m, 3H), 7.57 (m, 1H), 7.37-7.27 (m, 1H), 1.91 (s, 3H), 1.87 (s, 3H).

### Embodiment 85. (3-(2-((1-hydroxy-2-methylpropan-2-yl)amino)-5-(trifluoromethyl)pyrimidin-4-yl)-1H-indole-7-yl)dimethylphosphine oxide (compound 85)

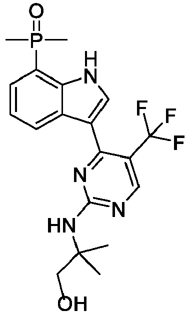

The compound **85** (25.21 mg, white solid). LCMS (ESI): [M+H]⁺ = 427.1.

¹H NMR (400 MHz, DMSO-*d*₆) δ ppm 11.51 (br s, 1H), 8.59-7.70 (m, 3H), 7.49 (m, 1H), 7.26 (m, 1 H), 7.18 (br s, 1H), 4.93 (br s, 1 H), 3.56 (br s, 2 H), 1.83 (s, 3H), 1.80 (s, 3H), 1.37 (br s, 6H).

### Embodiment 86. (S)-(3-(2-((2-fluoro-3-hydroxypropyl)amino)-5-(trifluoromethyl)pyrimidin-4-yl)-1H-indole-7-yl)dimethylphosphine oxide (compound 86)

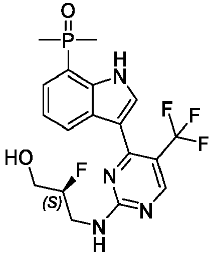

The compound **86** (25.21 mg, white solid). LCMS (ESI): [M+H]⁺ = 431.1.

¹H NMR (400 MHz, DMSO-*d*₆) δ ppm 11.56 (br s, 1H), 8.65-8.58 (m, 2H), 8.14-8.05 (m, 1H), 7.94 (m, 1H), 7.50 (m, 1 H), 7.27 (br s, 1H), 5.04 (br s, 1H), 4.77 (m, 1 H), 3.80-3.40 (m, 4H), 1.83 (s, 3H), 1.80 (s, 3H)

### Embodiment 87. (3-(2-((3S,4R)-4-hydroxytetrahydrofuran-3-yl)amino)-5-(trifluoromethyl)pyrimidin-4-yl)-1H-indole-7-yl)dimethylphosphine oxide (compound 87)

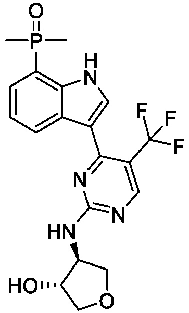

The compound **87** (23.90 mg, white solid). LCMS (ESI): [M+H]⁺ = 441.1.

¹H NMR (400 MHz, DMSO-*d*₆) δ ppm 11.56 (br s, 1H), 8.70-8.38 (m, 2H), 8.13 (m, 1H), 7.94 (m, 1H), 7.51 (m, 1H), 7.29 (m, 1H), 5.29 (m, 1H), 4.35-4.25 (m, 2H), 4.06 (m, 1H), 4.04 (m, 1H), 3.71 (m, 1H), 3.58 (m, 1H), 1.83 (s, 3H), 1.80 (s, 3H).

### Embodiment 88. (S)-(3-(2-((1-hydroxybutan-2-yl)amino)-5-(trifluoromethyl)pyrimidin-4-yl)-1H-indole-7-yl)dimethylphosphine oxide (compound 88)

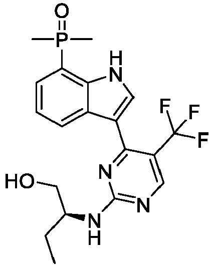

The compound **88** (27.13 mg, white solid). LCMS (ESI): [M+H]⁺ = 427.1.

¹H NMR (400 MHz, DMSO-*d*₆) δ ppm 11.53 (br s, 1H), 8.65-8.40 (m, 2H), 7.94-7.89 (m, 1H), 7.59-7.46 (m, 2H), 7.27 (m, 1H), 4.72 (t, *J* = 5.6 Hz, 1H), 4.02 (m, 1 H), 3.50-3.30 (m, 2H), 1.82 (s, 3H), 1.79 (s, 3H), 1.68 (m, 1H), 1.50 (m, 1H), 0.91 (m, 3H).

### Embodiment 89. (3-(2-((3-hydroxypropyl)amino)-5-(trifluoromethyl)pyrimidin-4-yl)-1H-indole-7-yl)dimethylphosphine oxide (compound 89)

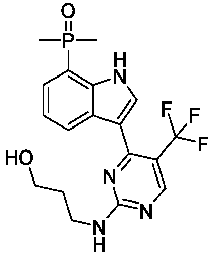

The compound **89** (21.97 mg, white solid). LCMS (ESI): [M+H]⁺ = 413.0.

¹H NMR (400 MHz, DMSO-*d*₆) δ ppm 11.56 (br s, 1H), 8.61-8.48 (m, 2H), 7.93-7.85 (m, 2H), 7.50 (m, 1H), 7.27 (m, 1H), 4.50 (t, *J* = 5.2 Hz, 1H), 3.53-3.30 (m, 4H), 1.83 (s, 3H), 1.80 (s, 3H), 1.75 (m, 2H)

### Embodiment 90. (3-(2-((1-hydroxycyclobutyl)methyl)amino)-5-(trifluoromethyl)pyrimidin-4-yl)-1H-indole-7-yl)dimethylphosphine oxide (compound 90)

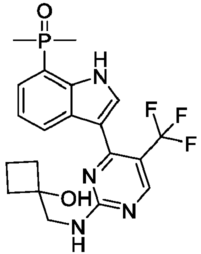

The compound **90** (26.17 mg, white solid). LCMS (ESI): [M+H]⁺ = 439.1.

¹H NMR (400 MHz, DMSO-*d*₆) δ ppm 11.54 (br s, 1H), 8.68-8.45 (m, 2H), 7.95-7.89 (m, 1H), 7.55-7.46 (m, 2H), 7.26 (m, 1H), 5.24 (m, 1H), 3.63-3.57 (m, 2H), 2.06 (m, 2H), 1.96 (m, 2H), 1.83 (s, 3H), 1.80 (s, 3H), 1.65 (m, 1H), 1.48 (m, 1H).

By using the same method for synthesizing the compound **80,** 3-(2-chloro-5-(trifluoromethyl)pyrimidin-4-yl)-1H-indole-7-yl)dimethylphosphine oxide was reacted with 3,3-dimethyl-1,4-oxazin-6-amine to synthesize a racemic compound (3-(2-((3,3-dimethyl-1,4-oxazin-6-yl)amino)-5-(trifluoromethyl)pyrimidin-4-yl)-1H-indole-7-yl)dimethylphosphine oxide, and the compound was separated by SFC (column: DAICEL CHIRALPAK AD (250 mm * 30 mm, 10 um); mobile phases: Phase A of carbon dioxide, and Phase B of 0.1% ammonia water/methanol; holding Phase B at 65%; flow rate: 70 milliliter/minute) to afford a compound **91** and a compound **92.**

### Embodiment 91. A chiral monomer (compound 91) with a short peak time after SFC resolution of (3-(2-((3,3-dimethyl-1,4-oxazepan-6-yl)amino)-5-(trifluoromethyl)pyrimidin-4-yl)-1H-indole-7-yl)dimethylphosphine oxide

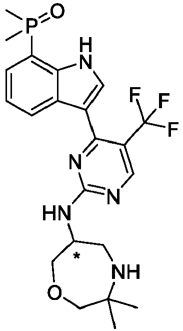

The compound **91** (19.72 mg, yellow solid). LCMS (ESI): [M+H]⁺ = 482.2; SFC analysis (column: Cellulose-2 (100 mm * 4.6 mm, 3 um); mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 milliliter/minute): RT = 3.788 min, ee = 93.88%.

¹H NMR (400 MHz, CD₃OD) δ ppm 8.62-8.40 (m, 2H), 7.97 (br s, 1H), 7.52 (m, 1H), 7.36 (m, 1H), 4.63 (m, 1H), 4.17 (m, 1H), 3.83-3.57 (m, 3H), 3.40 (m, 2H), 1.98 (s, 3H), 1.95 (s, 3H), 1.36 (m, 6H).

### Embodiment 92. A chiral monomer (compound 92) with a long peak time after SFC resolution of (3-(2-((3,3-dimethyl-1,4-oxazepan-6-yl)amino)-5-(trifluoromethyl)pyrimidin-4-yl)-1H-indole-7-yl)dimethylphosphine oxide

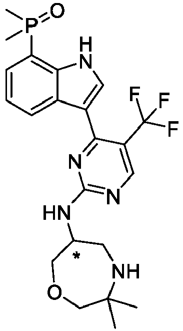

The compound **92** (22.30 mg, yellow solid). LCMS (ESI): [M+H]⁺ = 482.2; SFC analysis (column: Cellulose-2 (100 mm * 4.6 mm, 3 um); mobile phases: Phase A of carbon dioxide, and Phase B of 0.05% diethylamine/ethanol; gradient: as for Phase B, from 5% to 40% in 2 minutes, holding Phase B at 40% for 2.5 minutes, and then holding Phase B at 5% for 1.5 minutes; flow rate: 2.8 milliliter/minute): RT =4.110 min, ee = 99.32%.

¹H NMR (400 MHz, CD₃OD) δ ppm 8.62-8.40 (m, 2H), 7.97 (br s, 1H), 7.52 (m, 1H), 7.36 (m, 1H), 4.63 (m, 1H), 4.17 (m, 1H), 3.83-3.57 (m, 3H), 3.40 (m, 2H), 1.98 (s, 3H), 1.95 (s, 3H), 1.36 (m, 6H).

### Embodiment 93. (R)-(3-(2-((1-hydroxypropyl-2-yl)amino)-5-(trifluoromethyl)pyrimidin-4-yl)-1H-indole-7-yl)dimethylphosphine oxide (compound 93)

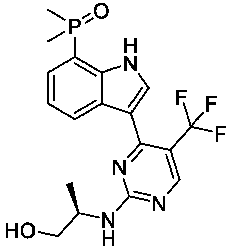

The compound **93** (21.22 mg, white solid). LCMS (ESI): [M+H]⁺ = 413.1.

¹H NMR (400 MHz, DMSO-*d*₆) δ ppm 11.52 (br s, 1H), 8.67-8.55 (m, 2H), 7.92 (m, 1H), 7.69 (m, 1H), 7.50 (m, 1H), 7.28 (m, 1H), 4.79 (m, 1H), 4.15 (m, 1H), 3.52 (m, 2H), 1.83 (s, 3H), 1.80 (s, 3H), 1.18 (d, *J* = 6.4 Hz, 3H)

### Embodiment 94. (3-(2-((1-(hydroxymethyl)cyclopropyl)amino)-5-(trifluoromethyl)pyrimidin-4-yl)-1H-indole-7-yl)dimethylphosphine oxide (compound 94)

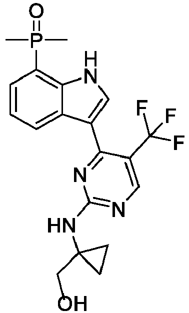

The compound **94** (27.21 mg, white solid). LCMS (ESI): [M+H]⁺ = 425.1.

¹H NMR (400 MHz, DMSO-*d*₆) δ ppm 11.58 (br s, 1H), 8.70-8.50 (m, 2H), 8.26-8.13 (m, 1H), 7.95 (s, 1H), 7.49 (m, 1H), 7.28 (m, 1H), 4.79-4.69 (m, 1H), 3.59 (m, 2H), 1.83 (s, 3H), 1.80 (s, 3H), 0.81 (m, 4H).

### Embodiment 95. (3-(2-((2-hydroxy-2-methylpropyl)amino)-5-(trifluoromethyl)pyrimidin-4-yl)-1H-indole-7-yl)dimethylphosphine oxide (compound 95)

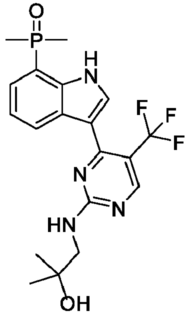

The compound **95** (21.40 mg, white solid). LCMS (ESI): [M+H]⁺ = 427.2.

¹H NMR (400 MHz, DMSO-*d*₆) δ ppm 11.56 (br s, 1H), 8.67-8.47 (m, 2H), 7.95-7.89 (m, 1H), 7.68-7.61 (m, 1H), 7.49 (m, 1H), 7.27 (m, 1H), 4.62-4.57 (m, 1H), 3.44 (m, 2H), 1.83 (s, 3H), 1.80 (s, 3H), 1.15 (s, 6H).

### Embodiment 96. (3-(2-((3,3-difluoro-1-(hydroxymethyl)cyclobutyl)amino)-5-(trifluoromethyl)pyrimidin-4-yl)-1H-indole-7-yl)dimethylphosphine oxide (compound 96)

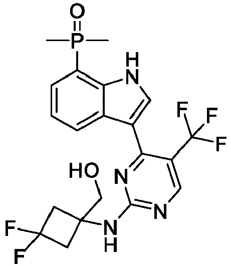

The compound **96** (26.10 mg, white solid). LCMS (ESI): [M+H]⁺ = 475.1.

¹H NMR (400 MHz, DMSO-*d*₆) δ ppm 11.56 (m, 1H), 8.71-7.83 (m, 4H), 7.50 (m, 1H), 7.28 (m, 1H), 5.19 (m, 1H), 3.70 (br s, 2H), 2.89 (m, 4H), 1.83 (s, 3H), 1.80 (s, 3H).

### Embodiment 97. (3-(2-((2-hydroxyethyl)amino)-5-(trifluoromethyl)pyrimidin-4-yl)-1H-indole-7-yl)dimethylphosphine oxide (compound 97)

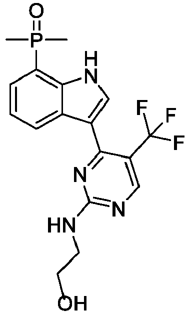

The compound **97** (31.10 mg, white solid). LCMS (ESI): [M+H]⁺ = 399.0.

¹H NMR (400 MHz, DMSO-*d*₆) δ ppm 11.54 (br s, 1H), 8.65-8.40 (m, 2H), 7.95-7.75 (m, 2H), 7.49 (m, 1H), 7.27 (m, 1 H), 4.79 (m, 1 H), 3.58 (m, 2H), 3.51-3.30 (m, 2H), 1.83 (s, 3H), 1.80 (s, 3H).

By using the same method for synthesizing the compound **80,** 3-(2-chloro-5-(trifluoromethyl)pyrimidin-4-yl)-1H-indole-7-yl)dimethylphosphine oxide was reacted with tert-butyl 7-amino-2-azaspiro[4.4]nonane-2-carboxylate to afford a racemic compound (3-(2-((2-azaspiro[4.4]non-7-yl)amino)-5-(trifluoromethyl)pyrimidin-4-yl)-1H-indole-7-yl)dimethylphosphine oxide after deprotection, and the compound was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 45%; flow rate: 80 milliliter/minute) to afford a target compound **98,** a target compound **99,** and a target compound **100.**

### Embodiment 98. A mixture (1:1) (mixed compound 98) of compounds with first peak time and second peak time after SFC resolution of a compound (3-(2-(((5R,7S)-2-azaspiro[4.4]non-7-yl)amino)-5-(trifluoromethyl)pyrimidin-4-yl)-1H-indole-7-yl)dimethylphosphine oxide and a compound (3-(2-(((5S,7R)-2-azaspiro[4.4]non-7-yl)amino)-5-(trifluoromethyl)pyrimidin-4-yl)-1H-indole-7-yl)dimethylphosphine oxide

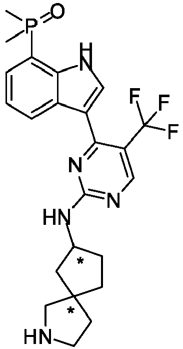

The mixed compound 98 (3.10 mg, yellow solid). LCMS (ESI): [M+H]⁺ = 478.2; chromatographic column: DAICEL CHIRALPAK IG, 250 mm x 30 mm, 10 µm; mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes, flow rate: 4 mL/min; RT = 0.887min, 1.00 min, with the sum of e.e values of the two being 90%.

¹H NMR (400 MHz, CD₃OD) δ ppm 8.58-8.52 (m, 2H), 7.95 (br s, 1H), 7.51 (m, 1H), 7.33 (m, 1H), 4.54 (m, 1H), 3.24 (m, 2H), 2.92 (m, 2H), 2.22 (m, 2H), 1.93-1.70 (m, 12H).

### Embodiment 99. (3-(2-(((5R,7R)-2-azaspiro[4.4]non-7-yl)amino)-5-(trifluoromethyl)pyrimidin-4-yl)-1H-indole-7-yl)dimethylphosphine oxide (compound 99)

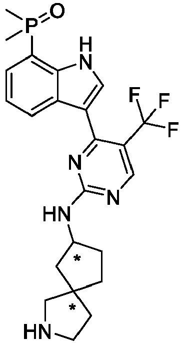

The compound **99** (10.10 mg, yellow solid). LCMS (ESI): [M+H]⁺ = 478.2;
SFC analysis (column: Chiralpak IG-3 (250 mm * 30 mm, 10 um); mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT =1.979 min, ee = 100%.

¹H NMR (400 MHz, CD₃OD) δ ppm 8.61-8.56 (m, 2H), 7.97 (br s, 1H), 7.53 (m, 1H), 7.35 (m, 1H), 4.58 (m, 1H), 3.15 (m, 2H), 3.02 (m, 2H), 2.28 (m, 2H), 1.96-1.70 (m, 12H).

### Embodiment 100. (3-(2-(((5S,7S)-2-azaspiro[4.4]non-7-yl)amino)-5-(trifluoromethyl)pyrimidin-4-yl)-1H-indole-7-yl)dimethylphosphine oxide compound (compound 100)

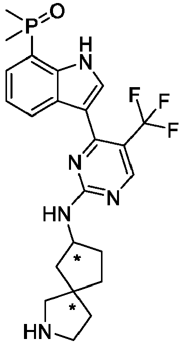

The compound **100** (11.05 mg, yellow solid). LCMS (ESI): [M+H]⁺ = 478.2;

SFC analysis (column: Chiralpak IG-3 (250 mm * 30 mm, 10 um); mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT =2.643 min, ee = 100%.

¹H NMR (400 MHz, CD₃OD) δ ppm 8.61-8.56 (m, 2H), 7.98 (br s, 1H), 7.54 (m, 1H), 7.35 (m, 1H), 4.58 (m, 1H), 3.11 (m, 2H), 2.98 (m, 2H), 2.28 (m, 2H), 1.96-1.70 (m, 12H).

### Embodiment 101. (S)-N-(piperidin-3-yl)-4-(6-(pyrrolidin-1-yl)-1H-indole-3-yl)-5-(trifluoromethyl)pyrimidin-2-amine (compound 101, compound 213 of patent CN201780057760.8)

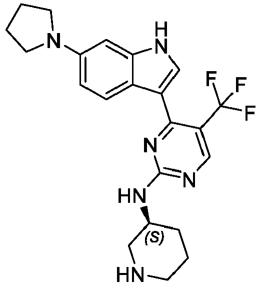

According to a synthetic method the same as that in embodiment 49 of patent CN201780057760.8, we synthesized the compound 213 (116.75 mg, light yellow solid) of patent CN201780057760.8. LC-MS: [M+H]⁺ = 431.2.
¹H NMR (400MHz, CD₃OD): *δ* ppm 8.35 (br s, 1H), 8.29 (s, 1H), 7.65 (br s, 1H), 6.72 (br s, 1H), 6.63 (d, *J* = 8.4 Hz, 1H), 4.22 (m, 1H), 3.37-3.30 (m, 5H), 3.10 (m, 1H), 2.80 (m, 2H), 2.21-1.86 (m, 6H), 1.73-1.52 (m, 2H)

By using the same method for synthesizing the compound **80,** 3-(2-chloro-5-(trifluoromethyl)pyrimidin-4-yl)-1H-indole-7-yl)dimethylphosphine oxide was reacted with a corresponding amino compound to synthesize the following compounds:

### Embodiment 102. (3-(2-((2,2-difluoro-3-hydroxypropyl)amino)-5-(trifluoromethyl)pyrimidin-4-yl)-1H-indole-7-yl)dimethylphosphine oxide (compound 102)

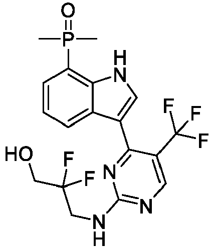

The compound **102** (15.21 mg, white solid). LCMS (ESI): [M+H]⁺ = 449.0.
¹H NMR (400 MHz, DMSO-*d*₆) δ ppm 8.70-8.45 (m, 2H), 8.23-8.16 (m, 1H), 7.99-7.92 (m, 1H), 7.50 (m, 1H), 7.27 (br s, 1H), 5.57 (br s, 1H), 4.00 (m, 2H), 3.69 (t, *J* = 13.6 Hz, 2H), 1.83 (s, 3H), 1.80 (s, 3H)

### Embodiment 103. (S)-(3-(5-chloro-2-((1-hydroxypropan-2-yl)amino)pyrimidin-4-yl)-1H-indole-7-yl)dimethylphosphine oxide (compound 103)

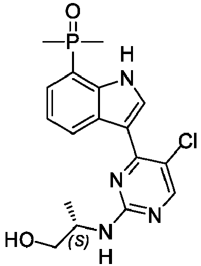

The compound **103** (4.52 mg, yellow solid). LCMS (ESI): [M+H]⁺ = 438.2.

¹H NMR (400 MHz, CD₃OD) δ ppm 8.88 (m, 1H), 8.57 (s, 1H), 8.24 (s, 1H), 7.54-7.36 (m, 2H), 4.25 (m, 1H), 3.68 (m, 2H), 1.93 (br d, *J* = 13.5Hz, 6H), 1.32 (br d, *J* = 7.20Hz, 3H).

### Embodiment 104. (S)-(3-(2-(((1-hydroxypropan-2-yl)amino)-5-(trifluoromethyl)pyrimidin-4-yl)-5-methoxy-1H-indole-7-yl)dimethylphosphine oxide (compound 104)

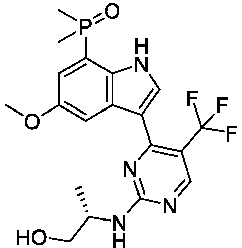

### Step 1: 1-(2-amino-3-bromo-5-methoxyphenyl)-2-chloroethanone

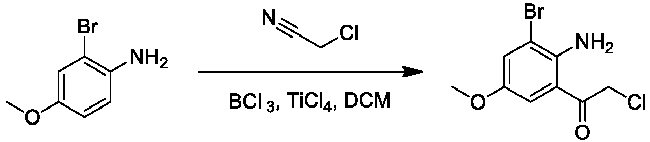

2-bromo-4-methoxyaniline (3.30 g, 16.33 mmol) was dissolved in dichloromethane (120 mL), and 2-chloroacetonitrile (2 mL, 31.03 mmol) and a solution (1 M, 25 mL, 25.00 mmol) of boron tribromide in dichloromethane were respectively added dropwise at 0°C; then, titanium tetrachloride (3 mL, 24.50 mmol) was added dropwise at 0°C, and the temperature was raised to 40°C for a reaction for 72 hours. The reaction solution was slowly added in a mixture of hydrochloric acid (2 M, 40 mL) and ice, and sodium hydroxide was added to adjust the pH to 5; ethyl acetate (150 mL * 3) was used for extraction for three times, and organic phases were concentrated to afford a crude product; and the crude product was purified by flash column chromatography (silica gel, ethyl acetate/petroleum ether with 0-10% gradient) to afford a compound 1-(2-amino-3-bromo-5-methoxyphenyl)-2-chloroethanone (1.20 g, 4.24 mmol, 26% yield), which was a yellow solid. LCMS (ESI): [M+H]⁺ = 277.0.

### Step 2: 7-bromo-5-methoxy-1H-indole

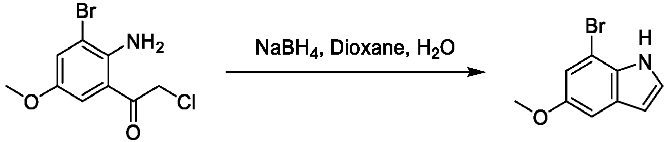

1-(2-amino-3-bromo-5-methoxyphenyl)-2-chloroethanone (1.20 g, 4.31 mmol) was dissolved in dioxane (54 mL) and water (9 mL), sodium borohydride (0.23 g, 6.03 mmol) was added, and the temperature was raised to 100°C for a reaction for 16 hours. After cooling, hydrochloric acid (0.1 M, 10 mL) was added to the reaction solution, dichloromethane (20 mL * 3) was used for extraction for three times, and organic phases were combined to afford a crude product; and the crude product was purified by flash column chromatography (silica gel, ethyl acetate/petroleum ether with 0-40% gradient) to afford a compound 7-bromo-5-methoxy-1H-indole (0.39 g, 1.72 mmol, 40% yield), which was a yellow oily compound. LCMS (ESI): [M+H]⁺ = 226.0;

### Step 3: 7-bromo-3-(2-chloro-5-(trifluoromethyl)pyrimidin-4-yl)-5-methoxy-1H-indole

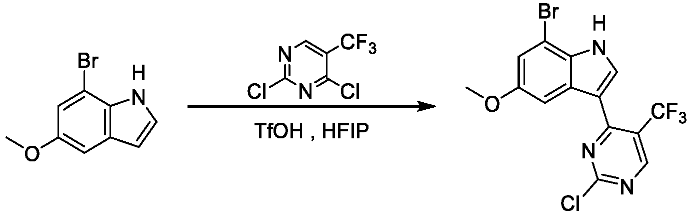

7-bromo-5-methoxy-1H-indole (320 mg, 1.42 mmol) and 2,4-dichloro-5-(trifluoromethyl)pyrimidine (460 mg, 2.12 mmol) were dissolved in hexafluoroisopropanol (3 mL), trifluoromethanesulfonic acid (138 uL, 1.56 mmol) was added dropwise at 0°C, and the reaction solution was reacted for 16 hours at 60°C; 3 milliliter of ethyl acetate was added for beating, and a solid obtained by filtration was dried in vacuum to afford a crude product compound 7-bromo-3-(2-chloro-5-(trifluoromethyl)pyrimidin-4-yl)-5-methoxy-1H-indole (270 mg), which was a yellow solid. LCMS (ESI): [M+H]⁺ = 406.0.

### Step 4: 7-bromo-5-methoxy-3-(2-(2,2,2-trifluoroethoxy)-5-(trifluoromethyl)pyrimidin-4-yl)-1H-indole

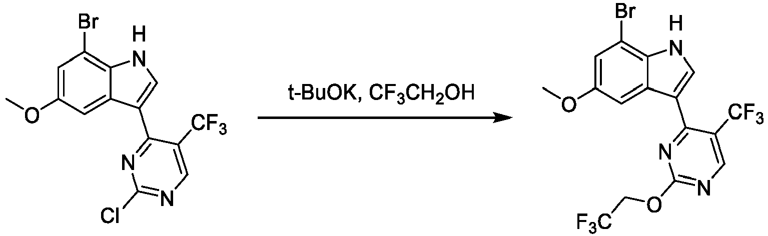

7-bromo-3-(2-chloro-5-(trifluoromethyl)pyrimidin-4-yl)-5-methoxy-1H-indole (260 mg, 0.64 mmol) and potassium tert-butoxide (358 mg, 3.20 mmol) were added in trifluoroethanol (3 mL), and the temperature was raised to 60°C for a reaction for 16 hours; the reaction solution was concentrated, the residue was washed with water and dried in vacuum to afford a crude compound 7-bromo-5-methoxy-3-(2-(2,2,2-trifluoroethoxy)-5-(trifluoromethyl)pyrimidin-4-yl)-1H-indole (120 mg), which was a white solid. LCMS (ESI): [M+H]⁺ = 470.0;

### Step 5: (5-methoxy-3-(2-(2,2,2-trifluoroethoxy)-5-(trifluoromethyl)pyrimidin-4-yl)-1H-indole-7-yl)dimethylphosphine oxide

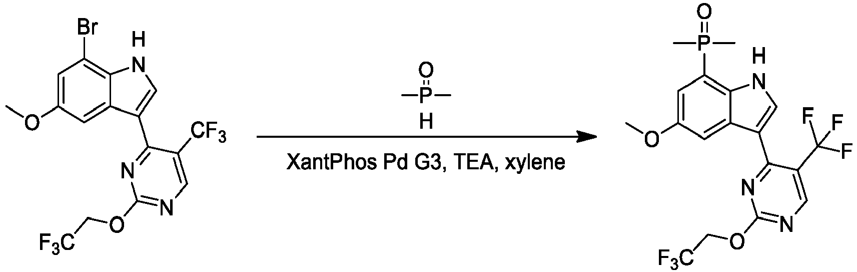

7-bromo-5-methoxy-3-(2-(2,2,2-trifluoroethoxy)-5-(trifluoromethyl)pyrimidin-4-yl)-1H-indole (100 mg, 0.21 mmol), dimethylphosphine oxide (0.03 g, 0.43 mmol) and triethylamine (90 uL, 0.64 mmol) were dissolved in xylene (1 mL), and in a glove box, a methanesulfonato[9,9-dimethyl-4,5-bis(diphenylphosphino)xanthene][2-amino-1,1-biphenyl]palladium(II) dichloromethane adduct (20 mg, 0.02 mmol) was added in the reaction solution; and the temperature was raised to 140°C for a reaction for 16 hours. The reaction solution was concentrated to afford a crude compound (5-methoxy-3-(2-(2,2,2-trifluoroethoxy)-5-(trifluoromethyl)pyrimidin-4-yl)-1H-indole-7-yl)dimethylphosphine oxide (150 mg). LCMS (ESI): [M+H]⁺ = 468.1;

### Step 6: (S)-(3-(2-(((1-hydroxypropan-2-yl)amino)-5-(trifluoromethyl)pyrimidin-4-yl)-5-methoxy-1H-indole-7-yl)dimethylphosphine oxide

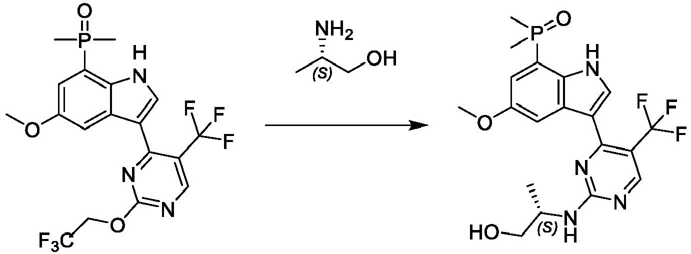

(5-methoxy-3-(2-(2,2,2-trifluoroethoxy)-5-(trifluoromethyl)pyrimidin-4-yl)-1H-indole-7-yl)dimethylphosphine oxide (30% purity, 100 mg, 64 umol) and (S)-2-(methylamino)propan-1-ol (1 mL) were added in a reaction flask, and the temperature was raised to 100°C for a reaction for 4 hours. The residue was purified by preparative HPLC to afford a title compound (S)-(3-(2-(((1-hydroxypropan-2-yl)amino)-5-(trifluoromethyl)pyrimidin-4-yl)-5-methoxy-1H-indole-7-yl)dimethylphosphine oxide (7 mg, 16 umol, 25% yield), which was a white solid. LCMS (ESI): [M+H]⁺ = 443.1.

¹H NMR (400 MHz, CD₃OD) δ ppm 8.53 (br s, 1H), 8.10 (br s, 1H), 7.93 (br s, 1H), 7.15 (dd, *J* = 2.4, 14.4 Hz, 1H), 4.53-4.19 (m, 1H), 3.92 (s, 3H), 3.71-3.61 (m, 2H), 1.96-1.89 (m, 6H), 1.31 (d, *J* = 6.8 Hz, 3H).

By using the same method for synthesizing the compound 80, 3-(2-chloro-5-(trifluoromethyl)pyrimidin-4-yl)-1H-indole-7-yl)dimethylphosphine oxide was reacted with a corresponding amino compound to synthesize the following compounds:

### Embodiment 105. (3-(2-((3-fluoro-1-(hydroxymethyl)cyclobutyl)amino)-5-(trifluoromethyl)pyrimidin-4-yl)-1H-indole-7-yl)dimethylphosphine oxide (compound 105)

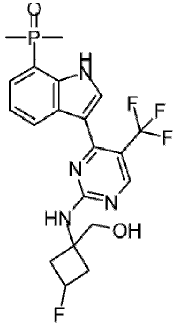

The compound **105** (6.45 mg, umber solid). LCMS (ESI): [M+H]⁺ = 457.1.

1H NMR (400 MHz, CD₃OD) δ ppm 8.57 (d, *J* = 3.9 Hz, 2H), 7.92 (br s, 1H), 7.50 (dd, *J* = 13.6, 7.2 Hz, 1H), 7.34 (td, *J* = 7.7, 2.4 Hz, 1H), 5.39-4.93 (m, 1H), 3.90 (s, 1H), 3.76 (s, 1H), 2.95-2.74 (m, 2H), 2.60-2.40 (m, 2H), 1.93 (d, *J* =13.3 Hz, 6H).

### Embodiment 106. (S)-(3-(2-((1-hydroxypropan-2-yl)amino)-5-(trifluoromethyl)pyrimidin-4-yl)-6-methoxy-1H-indole-7-yl)dimethylphosphine oxide (compound 106)

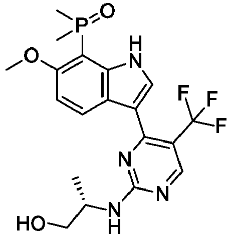

### Step 1: 1-(2-amino-3-bromo-4-methoxyphenyl)-2-chloroethan-1-one

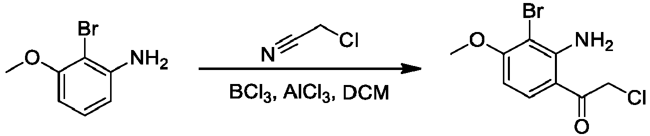

A solution (1 M, 109 mL, 108.88 mmol) of boron trichloride in dichloromethane was added dropwise at 0°C in a solution of 2-bromo-3-methoxyaniline (20.00 g, 98.99 mmol) in dichloromethane (60 mL); then chloroacetonitrile (8 mL, 118.78 mmol) was added dropwise under 0°C, and aluminum trichloride (total 14.52 g, 108.89 mmol) was added in three times. The reaction mixture system was stirred for 12 hours at 50°C. Then, the reaction mixture was cooled to 0°C, hydrochloric acid (1 M, 300 mL) was added slowly, a white precipitate appeared, and subsequently the mixture system was stirred for 1 hour at 50°C. Filtration was carried out, a mother liquor was extracted with dichloromethane (200 mL * 3), and organic phases were dried over anhydrous sodium sulfate, filtered, and spin-dried. 1-(2-amino-3-bromo-4-methoxyphenyl)-2-chloroethan-1-one (18.00 g, 64.63 mmol, 65% yield), which was a gray solid was afforded. LCMS (ESI): [M+H]⁺ = 277.9.

### Step 2: 7-bromo-6-methoxy-1H-indole

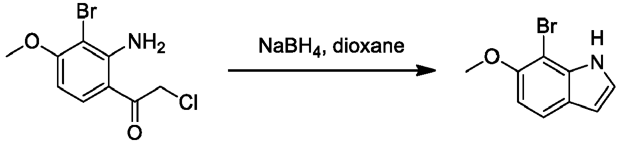

Sodium borohydride (total 2.44 g, 64.62 mmol) was added to a solution of 1-(2-amino-3-bromo-4-methoxyphenyl)-2-chloroethan-1-one (18.00 g, 64.63 mmol) in 1,4-dioxane (270 mL) and water (27 mL) in three times under 0°C, and the reaction mixture system was stirred for 12 hours at 100°C. Water (100 mL) is added, ethyl acetate (100 mL * 3) was used for extraction, anhydrous sodium sulfate was used for drying, and filtration and spin-drying were carried out. A crude product was purified by flash column chromatography (silica gel, ethyl acetate/petroleum ether with 0-50% gradient) to afford 7-bromo-6-methoxy-1H-indole (8.50 g, 37.59 mmol, 58% yield), which was a white solid. LCMS (ESI): [M+H]⁺ = 227.9.

### Step 3: 7-bromo-3-(2-chloro-5-(trifluoromethyl)pyrimidin-4-yl)-6-methoxy-1H-indole

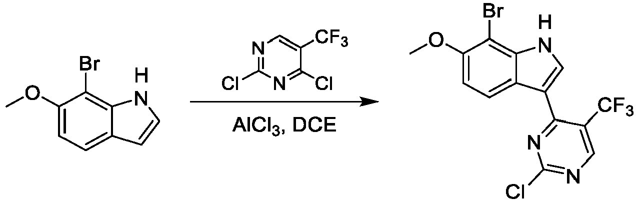

Aluminum trichloride (total 7.52 g, 56.40 mmol) was added to a solution of 7-bromo-6-methoxy-1H-indole (8.50 g, 37.59 mmol) and 2,4-dichloro-5-(trifluoromethyl)pyrimidine (12.24 g, 56.40 mmol) in dichloroethane (85 mL) in three times under 0°C, and a reaction mixture system was stirred for 45 minutes at 60°C. It was added in 50 mL of water under 0°C, dichloromethane (50 mL * 3) was used for extraction, and organic phases were dried over anhydrous sodium sulfate, and filtration and spin-drying were carried out. The crude product was purified by flash column chromatography (silica gel, ethyl acetate/petroleum ether with 0-50% gradient) to afford 7-bromo-3-(2-chloro-5-(trifluoromethyl)pyrimidin-4-yl)-6-methoxy-1H-indole (5.20 g, 12.79 mmol, 34% yield), which was a white solid. LCMS (ESI): [M+H]⁺ = 405.9.

### Step 4: 7-bromo-6-methoxy-3-(2-(2,2,2-trifluoroethoxy)-5-(trifluoromethyl)pyrimidin-4-yl)-1H-indole

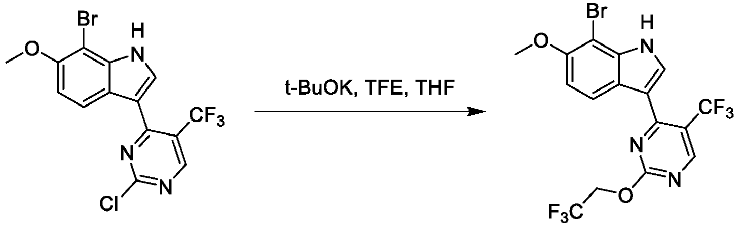

Potassium tert-butoxide (0.82 g, 7.38 mmol) was added to a solution of 7-bromo-3-(2-chloro-5-(trifluoromethyl)pyrimidin-4-yl)-6-methoxy-1H-indole (1.00 g, 2.46 mmol) in trifluoroethanol (8 mL, 49.19 mmol) and tetrahydrofuran (8 mL) under 0°C, and the reaction mixture system was stirred for 16 hours at 60^{°C}. Spin-drying was carried out, the crude product was added with water (2 mL) for beating and filtering, and a solid was dried to afford 7-bromo-6-methoxy-3-(2-(2,2,2-trifluoroethoxy)-5-(trifluoromethyl)pyrimidin-4-yl)-1H-indole (1.00 g, 2.13 mmol, 86% yield), which was a white solid. LCMS (ESI): [M+H]⁺ = 470.0.

### Step 5: (6-methoxy-3-(2-(2,2,2-trifluoroethoxy)-5-(trifluoromethyl)pyrimidin-4-yl)-1H-indole-7-yl)dimethylphosphine oxide

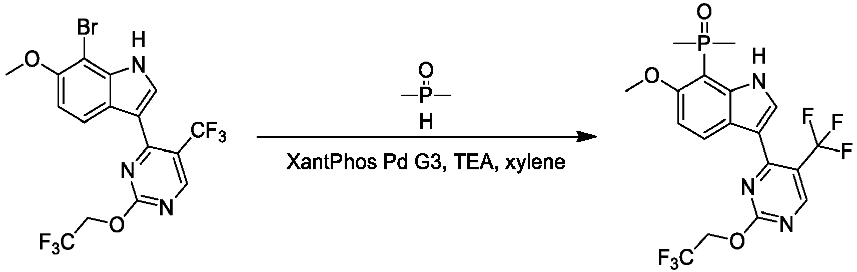

Under nitrogen protection in a glove box, a methanesulfonato[9,9-dimethyl-4,5-bis(diphenylphosphino)xanthene][2-amino-1,1-biphenyl]palladium(II) dichloromethane adduct (110 mg, 0.11 mmol) was added to a solution of 7-bromo-6-methoxy-3-(2-(2,2,2-trifluoroethoxy)-5-(trifluoromethyl)pyrimidin-4-yl)-1H-indole (500 mg, 1.06 mmol), diisopropylethylenediamine (526 uL, 3.19 mmol), and dimethylphosphine oxide (163 mg, 2.13 mmol) in xylene (10 mL), and the reaction mixture system was stirred for 12 hours at 140^{°}C under nitrogen. Spin-drying was carried out, the crude product was added with ethyl acetate (2 mL) and methyl tert-butyl ether (2 mL) for beating and filtering, and drying was carried out to afford a crude compound (6-methoxy-3-(2-(2,2,2-trifluoroethoxy)-5-(trifluoromethyl)pyrimidin-4-yl)-1H-indole-7-yl)dimethylphosphine oxide (540 mg), which was a yellow solid. LCMS (ESI): [M+H]⁺ = 468.2;

### Step 6: (S)-(3-(2-(((1-hydroxypropan-2-yl)amino)-5-(trifluoromethyl)pyrimidin-4-yl)-6-methoxy-1H-indole-7-yl)dimethylphosphine oxide

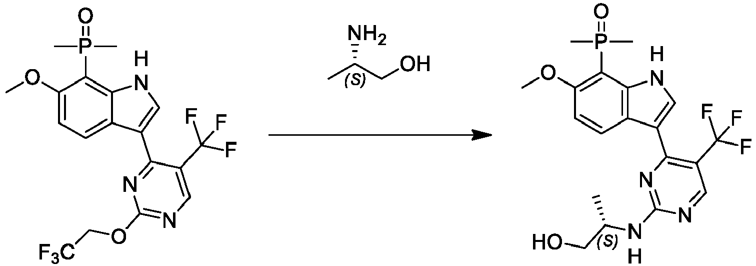

(6-methoxy-3-(2-(2,2,2-trifluoroethoxy)-5-(trifluoromethyl)pyrimidin-4-yl)-1H-indole-7-yl)dimethylphosphine oxide (100 mg, 0.21 mmol) and (S)-2-aminopropan-1-ol (321 mg, 4.28 mmol) were mixed, and the mixture was stirred for 1 hour under 100°C under nitrogen. Purification was carried out by preparative HPLC to afford a title compound (S)-(3-(2-(((1-hydroxypropan-2-yl)amino)-5-(trifluoromethyl)pyrimidin-4-yl)-6-methoxy-1H-indole-7-yl)dimethylphosphine oxide (47 mg, 0.11 mmol, 50% yield). LCMS (ESI): [M+H]⁺ = 443.1.

¹H NMR (400MHz, CD₃OD) δ ppm 8.73-8.34 (m, 2H), 7.87 (s, 1H), 7.08 (s, 1H), 4.50-4.16 (m, 1H), 4.00 (s, 3H), 3.74-3.58 (m, 2H), 1.91 (d, *J* =14.1 Hz, 6H), 1.31 (d, *J* = 6.7 Hz, 3H)

By using the same method for synthesizing the compound **80,** 3-((2-chloro-5-(trifluoromethyl)pyrimidin-4-yl)-1H-indole-7-yl)dimethylphosphine oxide was reacted with a corresponding amino compound to synthesize the following compounds:

### Embodiment 107. (3-(2-((5-azaspiro[2.4]heptan-1-yl)amino)-5-(trifluoromethyl)pyrimidin-4-yl)-1H-indole-7-yl)dimethylphosphine oxide (compound 107)

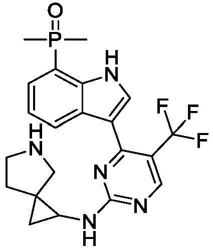

The compound **107** (150 mg, white solid). LCMS (ESI): [M+H]⁺ = 450.0.

The compound **107** was separated by SFC (column: DAICEL CHIRALPAK IG (250 mm * 30 mm, 10 um); mobile phases: Phase A of carbon dioxide, and Phase B of 0.1% ammonia water/ethanol; holding Phase B at 35%; flow rate: 70 milliliter/minute) to afford an optically pure target compound: a chiral monomer (compound **108**) with the shortest peak time ; a chiral monomer (compound **109**) with the second shortest peak time; a chiral monomer (compound **110**) with the third shortest peak time; and a chiral monomer (compound **111**) with a long peak time.

### Embodiment 108. The chiral monomer (compound 108) with the shortest peak time after SFC resolution of the compound 107

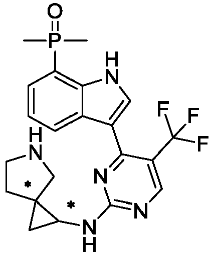

The compound **108** (14.17 mg, white solid). LCMS (ESI): [M+H]⁺ = 450.0;
SFC analysis (column: ChiralPak IG-3 (100 mm * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT =5.650 min, chiral purity 100%.

¹H NMR (400 MHz, CD₃OD) δ ppm 8.72-8.51 (m, 2 H), 8.00 (s, 1 H), 7.51 (br d, *J*= 13.3 Hz, 1 H), 7.36 (br s, 1 H), 3.26-2.56 (m, 5 H), 1.93 (br d, *J* = 13.6 Hz, 6 H), 1.84-1.53 (m, 2 H), 1.34-1.18 (m, 1 H), 0.97 (br s, 1 H).

### Embodiment 109. The chiral monomer (compound 109) with the second shortest peak time after SFC resolution of the compound 107

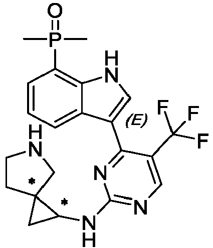

The compound **109** (24.22 mg, yellow solid). LCMS (ESI): [M+H]⁺ =450.0;
SFC analysis (column: ChiralPak IG-3 (100 mm * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 5.985 min, chiral purity 98.94%.

¹H NMR (400 MHz, CD₃OD) δ ppm 8.75-8.54 (m, 2H), 8.05-7.95 (m, 1H), 7.60-7.46 (m, 1H), 7.35 (br s, 1H), 3.21-2.25 (m, 5H), 1.97-1.90 (m, 6H), 1.84 (m, 2H), 1.24 (m, 1H), 0.96-0.85 (m, 1H).

### Embodiment 110. The chiral monomer (compound 110) with the third shortest peak time after SFC resolution of the compound 107

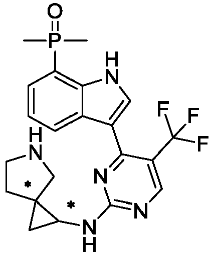

The compound **110** (38.84 mg, white solid). LCMS (ESI): [M+H]⁺ = 450.0;
SFC analysis (column: ChiralPak IG-3 (100 mm * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 6.307 min, chiral purity 98.53%.

¹H NMR (400 MHz, CD₃OD) δ ppm 8.75-8.54 (m, 2H), 8.05-7.95 (m, 1H), 7.60-7.46 (m, 1H), 7.35 (br s, 1H), 3.21-2.25 (m, 5H), 1.97-1.75 (m, 7H), 1.31-1.24 (m, 2H), 0.91 (br d, *J* = 7.0 Hz, 1H).

### Embodiment 111. The chiral monomer (compound 111) with a longer peak time after SFC resolution of the compound 107

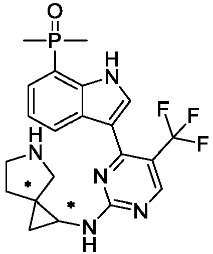

The compound **111** (16.52 mg, white solid); LCMS (ESI): [M+H]⁺ = 499.9;
SFC analysis (column: ChiralPak IG-3 (100 mm * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 6.843 min, chiral purity 98.85%.

¹H NMR (400 MHz, CD₃OD) δ ppm 8.72-8.51 (m, 2H), 8.00 (s, 1H), 7.51 (br d, *J* = 13.3 Hz, 1H), 7.36 (br s, 1H), 3.26-2.56 (m, 5H), 1.93-1.75 (m, 7H), 1.34-1.18 (m, 2H), 0.97 (br s, 1H)

### Embodiment 112. (S)-(3-(2-((2-hydroxy-1-(1-methylcyclopropyl)ethyl)amino)-5-(trifluoromethyl)pyrimidin-4-yl)-1H-indole-7-yl)dimethylphosphine oxide (compound 112)

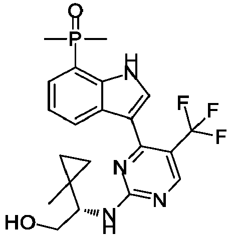

The compound **112** (11.57 mg, white solid). LCMS (ESI): [M+H]⁺ = 452.9.

¹H NMR (400 MHz, CD₃OD) δ ppm 8.65-8.46 (m, 2H), 7.95 (s, 1H), 7.50 (dd, *J* = 13.5, 7.2 Hz, 1H), 7.41-7.27 (m, 1H), 4.03 (br s, 1H), 3.87 (br dd, *J* = 11.3, 4.0 Hz, 1H), 3.82-3.70 (m, 1H), 1.93 (d, *J* = 13.3 Hz, 6H), 1.16 (s, 3H), 0.70 (br s, 1H), 0.53 (br s, 1H), 0.41-0.16 (m, 2H).

### Embodiment 113. (R)-(3-(2-((1-cyclobutyl-2-hydroxyethyl)amino)-5-(trifluoromethyl)pyrimidin-4-yl)-1H-indole-7-yl)dimethylphosphine oxide (compound 113)

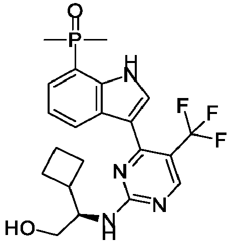

The compound **113** (63.08 mg, white solid). LCMS (ESI): [M+H]⁺ = 452.9.

¹H NMR (400 MHz, DMSO-*d*₆) δ ppm 11.55 (br s, 1H), 8.73-8.40 (m, 2H), 7.98-7.85 (m, 1H), 7.68-7.44 (m, 2H), 7.27 (br t, *J* = 7.4 Hz, 1H), 4.62 (br s, 1H), 4.33-4.10 (m, 1H), 3.55-3.38 (m, 2H), 2.57 (br d, *J* = 5.8 Hz, 1H), 2.03-1.66 (m, 12H).

### Embodiment 114. (3-(2-((3-(hydroxymethyl)oxetan-3-yl)amino)-5-(trifluoromethyl)pyrimidin-4-yl)-1H-indole-7-yl)dimethylphosphine oxide (compound 114)

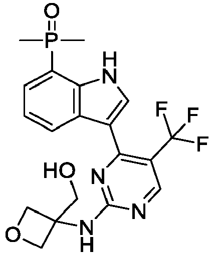

The compound **114** (8.57 mg, white solid). LCMS (ESI): [M+H]⁺ = 441.1.

¹H NMR (400 MHz, DMSO-*d*₆) δ ppm 11.65-11.47 (m, 1H), 8.78-8.36 (m, 3H), 8.13-7.76 (m, 1H), 7.50 (dd, *J* = 7.2, 12.9 Hz, 1H), 7.28 (dt, *J* = 2.1, 7.6 Hz, 1H), 5.17 (br s, 1H), 4.66 (br d, *J* = 6.0 Hz, 2H), 4.62-4.50 (m, 2H), 3.83 (br s, 2H), 1.82 (d, *J* = 13.6 Hz, 6H).

### Embodiment 115 (S)-(3-(2-((1-hydroxy-3-methylbutan-2-yl)amino)-5-(trifluoromethyl)pyrimidin-4-yl)-1H-indole-7-yl)dimethylphosphine oxide (compound 115)

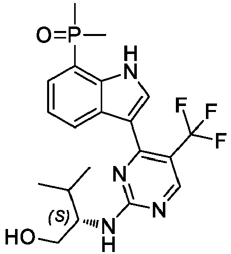

The compound **115** (20.45 mg, white solid). LCMS (ESI): [M+H]⁺ = 441.1.

¹H NMR (400 MHz, DMSO-*d*₆) δ ppm 11.55 (br s, 1H), 8.71-8.38 (m, 2H), 7.92 (br d, *J* = 18.8 Hz, 1H), 7.61 (br dd, *J=* 4.5, 9.0 Hz, 1H), 7.50 (dd, *J* = 7.0, 12.8 Hz, 1H), 7.30-7.24 (m, 1H), 4.62 (t, *J* = 5.4 Hz, 1H), 4.01 (br dd, *J* = 7.5, 13.8 Hz, 1H), 3.62-3.49 (m, 2H), 2.03-1.93 (m, 1H), 1.82 (d, *J* = 13.6 Hz, 6H), 0.98-0.90 (m, 6H).

### Embodiment 116. (3-(2-((1-(hydroxymethyl)cyclopentyl)amino)-5-(trifluoromethyl)pyrimidin-4-yl)-1H-indole-7-yl)dimethylphosphine oxide (compound 116)

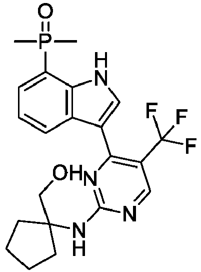

The compound **116** (32.93 mg, white solid). LCMS (ESI): [M+H]⁺ = 452.9.

¹H NMR (400 MHz, DMSO-*d*₆) δ ppm 11.60-11.46 (m, 1H), 8.72-8.15 (m, 2H), 7.98-7.74 (m, 1H), 7.53-7.42 (m, 2H), 7.26 (t, *J* = 6.7 Hz, 1H), 4.94-4.74 (m, 1H), 3.74-3.60 (m, 2H), 2.14-2.00 (m, 2H), 1.82 (d, *J* = 13.3 Hz, 10H), 1.56 (br s, 2H).

### Embodiment 117. (3-(2-((3-(hydroxymethyl)tetrahydrofuran-3-yl)amino)-5-(trifluoromethyl)pyrimidin-4-yl)-1H-indole-7-yl)dimethylphosphine oxide (compound 117)

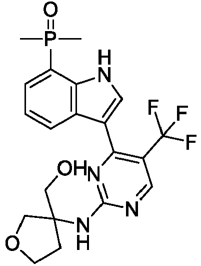

The compound **117** (2.46 mg, white solid). LCMS (ESI): [M+H]⁺ = 454.9.

¹H NMR (400 MHz, DMSO-*d*₆) δ ppm 11.57 (br s, 1H), 8.78-8.54 (m, 2H), 8.21-7.76 (m, 2H), 7.50 (dd, *J* = 6.8, 13.1 Hz, 1H), 7.27 (dt, *J* = 2.3, 7.7 Hz, 1H), 5.04-4.94 (m, 1H), 3.98 (br d, *J* = 8.0 Hz, 1H), 3.88-3.67 (m, 5H), 2.31 (br d, *J* = 16.3 Hz, 1H), 2.16-2.04 (m, 1H), 1.82 (d, *J*= 13.6 Hz, 6H).

The compound **117** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 45%; flow rate: 80 milliliter/minute) to afford an optically pure target compound: a chiral monomer (compound **118**) with a short peak time and a chiral monomer (compound **119**) with a long peak time

### Embodiment 118. The chiral monomer (compound 118) with a short peak time after SFC resolution of the compound 117

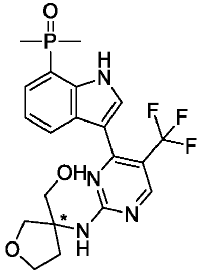

The compound **118** (10.80 mg, white solid). LCMS (ESI): [M+H]⁺ =455.0.

SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 milliliter/minute): RT = 6.338 min, ee = 99.96%.

¹H NMR (400 MHz, DMSO-*d*₆) δ ppm 11.30 (br s, 1H), 8.94-8.48 (m, 2H), 8.08-7.79 (m, 2H), 7.55 (dd, *J* = 7.1, 12.9 Hz, 1H), 7.41-7.21 (m, 1H), 5.06 (br s, 1H), 4.02 (br s, 1H), 3.93-3.70 (m, 5H), 2.36 (br d, *J* = 13.6 Hz, 1H), 2.24-2.08 (m, 1H), 1.87 (d, *J* = 13.4 Hz, 6H).

### Embodiment 119. The chiral monomer (compound 119) with a long peak time after SFC resolution of the compound 117

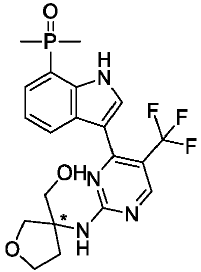

The compound **119** (9.84 mg, white solid). LCMS (ESI): [M+H]⁺ =455.1.

SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 milliliter/minute): RT = 7.132 min, ee = 99.50%.

¹H NMR (400 MHz, DMSO-*d*₆) δ ppm 11.60-11.35 (m, 1H), 8.72-8.47 (m, 2H), 7.94-7.68 (m, 2H), 7.43 (dd, *J* = 7.1, 12.8 Hz, 1H), 7.20 (dt, *J* = 2.2, 7.7 Hz, 1H), 4.95 (br s, 1H), 3.96-3.85 (m, 1H), 3.70 (br d, *J* = 17.9 Hz, 5H), 2.22 (br s, 1H), 2.10-1.96 (m, 1H), 1.75 (d, *J* = 13.5 Hz, 6H).

### Embodiment 120. (3-(2-((3-hydroxytetrahydrofuran-3-yl)methyl)amino)-5-(trifluoromethyl)pyrimidin-4-yl)-1H-indole-7-yl)dimethylphosphine oxide (compound 120)

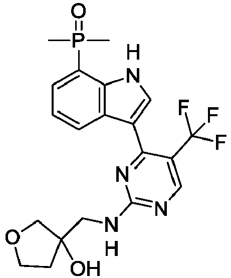

The compound **120** (37 mg, white solid). LCMS (ESI): [M+H]⁺ = 455.2.

¹H NMR (400 MHz, DMSO-*d*₆) δ ppm 11.40 (br s, 1H), 8.72-8.40 (m, 2H), 8.05-7.74 (m, 2H), 7.50 (dd, *J* = 6.8, 13.1 Hz, 1H), 7.27 (br t, *J* = 7.5 Hz, 1H), 5.10 (s, 1H), 3.87-3.72 (m, 2H), 3.71-3.61 (m, 3H), 3.51 (br dd, *J* = 4.5, 8.8 Hz, 1H), 2.04-1.91 (m, 1H), 1.82 (d, *J* = 13.6 Hz, 7H).

The compound **120** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 40%; flow rate: 80 milliliter/minute) to afford an optically pure target compound: a chiral monomer (compound **121**) with a short peak time and a chiral monomer (compound **122**) with a long peak time

### Embodiment 121. A chiral monomer (compound 121) with a short peak time after SFC resolution of the compound 120

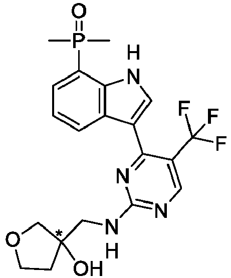

The compound **121** (9.69 mg, white solid). LCMS (ESI): [M+H]⁺ =455.1.

SFC analysis (column: Chiralpak AD-3 (50 mm * 4.6 mm, 3 um); mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT = 1.798 min, ee = 99.16%.

¹H NMR (400 MHz, DMSO-*d*₆) δ ppm 11.57 (br s, 1H), 8.68-8.41 (m, 2H), 7.96 (br s, 1H), 7.99-7.78 (m, 1H), 7.50 (dd, *J* = 6.7, 12.9 Hz, 1H), 7.31-7.24 (m, 1H), 5.10 (s, 1H), 3.86-3.74 (m, 2H), 3.72-3.61 (m, 3H), 3.51 (br dd, *J* = 4.8, 9.0 Hz, 1H), 1.97 (br d, *J* = 7.5 Hz, 1H), 1.82 (d, *J* = 13.3 Hz, 7H).

### Embodiment 122. The chiral monomer (compound 122) with a long peak time after SFC resolution of the compound 120

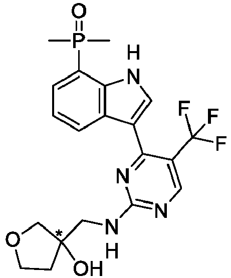

The compound **122** (11.69 mg, white solid). LCMS (ESI): [M+H]⁺ =455.2.

SFC analysis (column: Chiralpak AD-3 (50 mm * 4.6 mm, 3 um); mobile phases: Phase A of carbon dioxide, and Phase B of 0.05% diethylamine/isopropanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT = 2.023 min, ee = 98.70%.

¹H NMR(400 MHz, DMSO-*d*₆) δ ppm 11.56 (br s, 1H), 8.68-8.41 (m, 2H), 7.99-7.75 (m, 2H), 7.50 (dd, *J* = 6.8, 13.1 Hz, 1H), 7.31-7.24 (m, 1H), 5.10 (s, 1H), 3.86-3.74 (m, 2H), 3.71-3.62 (m, 3H), 3.51 (br dd, *J* = 4.8, 8.3 Hz, 1H), 2.02-1.91 (m, 1H), 1.82 (d, *J* = 13.3 Hz, 7H).

### Embodiment 123. (3-(2-((3,3-difluoro-2-hydroxypropyl)amino)-5-(trifluoromethyl)pyrimidin-4-yl)-1H-indole-7-yl)dimethylphosphine oxide (compound 123)

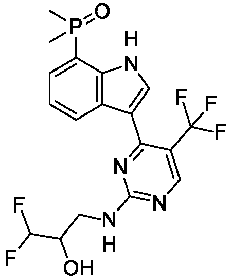

The compound **123** (51 mg, white solid). LCMS (ESI): [M+H]⁺ = 449.1.

¹H NMR (400 MHz, DMSO-*d*₆) δ ppm 11.58 (br s, 1H), 8.72-8.42 (m, 2H), 8.03-7.88 (m, 2H), 7.50 (dd, *J* = 7.2, 12.9 Hz, 1H), 7.33-7.21 (m, 1H), 6.16-5.77 (m, 2H), 4.05-3.92 (m, 1H), 3.73-3.58 (m, 1H), 3.54-3.40 (m, 1H), 1.82 (d, *J=* 13.6 Hz, 6H).

The compound **123** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 35%; flow rate: 70 milliliter/minute) to afford an optically pure target compound: a chiral monomer (compound **124)** with a short peak time and a chiral monomer (compound **125)** with a long peak time

### Embodiment 124. A chiral monomer (compound 124) with a short peak time after SFC resolution of the compound 123

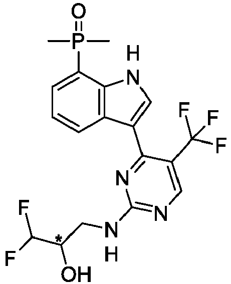

The compound **124** (2.34 mg, white solid). LCMS (ESI): [M+H]⁺= 449.1.

SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5 minutes and then from 40% to 5% in 0.5 minutes, and then holding Phase B at 5% for 1.5 minutes; flow rate: 2.5 milliliter/minute): RT = 5.096 min, ee = 100%

¹H NMR (400 MHz, DMSO-*d*₆) δ ppm 11.58 (br s, 1H), 8.72-8.42 (m, 2H), 8.03-7.88 (m, 2H), 7.50 (dd, *J* = 7.2, 12.9 Hz, 1H), 7.33-7.21 (m, 1H), 6.16-5.77 (m, 2H), 4.05-3.92 (m, 1H), 3.73-3.58 (m, 1H), 3.54-3.40 (m, 1H), 1.82 (d, *J=* 13.6 Hz, 6H).

### Embodiment 125. The chiral monomer (compound 125) with a long peak time after SFC resolution of the compound 123

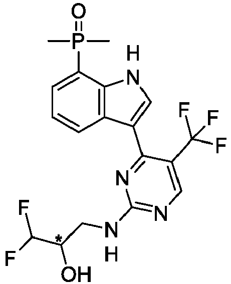

The compound **125** (12.28 mg, white solid). LCMS (ESI): [M+H]⁺= 449.0.

SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5 minutes and then from 40% to 5% in 0.5 minutes, and then holding Phase B at 5% for 1.5 minutes; flow rate: 2.5 milliliter/minute): RT = 5.388 min, ee = 98.64%.

¹H NMR (400 MHz, DMSO-*d*₆) δ ppm 11.58 (br s, 1H), 8.73-8.38 (m, 2H), 8.04-7.85 (m, 2H), 7.50 (dd, *J* = 7.1, 12.9 Hz, 1H), 7.36-7.14 (m, 1H), 6.17-5.74 (m, 2H), 3.98 (br s, 1H), 3.73-3.56 (m, 1H), 3.54-3.39 (m, 1H), 1.82 (d, *J=* 13.5 Hz, 6H).

### Embodiment 126. (3-(2-(((3-hydroxycyclobutyl)methyl)amino)-5-(trifluoromethyl)pyrimidin-4-yl)-1H-indole-7-yl)dimethylphosphine oxide (compound 126)

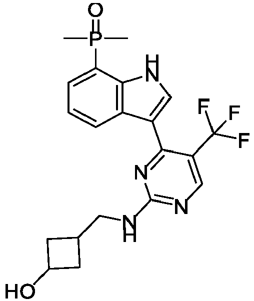

The compound **126** (40.84 mg, white solid). LCMS (ESI): [M+H]⁺= 439.2.

¹H NMR (400 MHz, DMSO-*d*₆) δ ppm 11.57 (br s, 1H), 8.66-8.40 (m, 2H), 8.19-7.89 (m, 2H), 7.50 (br dd, *J* = 7.3, 12.8 Hz, 1H), 7.31-7.21 (m, 1H), 4.97 (br s, 1H), 3.91 (quin, *J* = 7.5 Hz, 1H), 3.49-3.42 (m, 2H), 2.36-2.23 (m, 2H), 2.04 (td, *J* = 7.6, 14.6 Hz, 1H), 1.82 (d, *J* = 13.6 Hz, 6H), 1.56 (br d, *J* = 8.0 Hz, 2H).

### Embodiment 127. (3-(2-(((trans-3-hydroxy-3-methylcyclobutyl)methyl)amino)-5-(trifluoromethyl)pyrimidin-4-yl)-1H-indole-7-yl)dimethylphosphine oxide (compound 127)

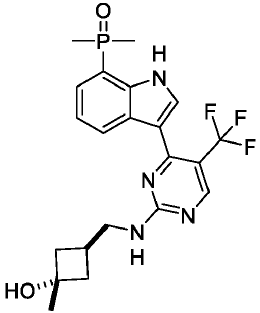

The compound **127** (22.99 mg, white solid). LCMS (ESI): [M+H]⁺= 453.2.

¹H NMR (400 MHz, DMSO-*d*₆) δ ppm 11.57 (br s, 1H), 8.65-8.41 (m, 2H), 8.04-7.89 (m, 2H), 7.50 (br dd, *J* = 7.4, 12.7 Hz, 1H), 7.31-7.18 (m, 1H), 4.80 (s, 1H), 3.52-3.40 (m, 2H), 2.68-2.53 (m, 1H), 2.16-2.00 (m, 2H), 1.82 (d, *J=* 13.6 Hz, 6H), 1.79-1.73 (m, 2H), 1.29-1.16 (m, 3H).

### Embodiment 128. (3-(2-(((2S)-3-hydroxybutan-2-yl)amino)-5-(trifluoromethyl)pyrimidin-4-yl)-1H-indole-7-yl)dimethylphosphine oxide (compound 128)

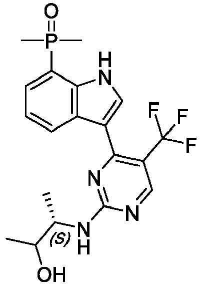

The compound **128** (103 mg, white solid). LCMS (ESI): [M+H]⁺= 427.2.

¹H NMR (400 MHz, CD₃OD) δ ppm 8.54 (br s, 2H), 7.97 (s, 1H), 7.51 (dd, *J* = 7.3, 13.6 Hz, 1H), 7.34 (dt, *J* = 2.4, 7.7 Hz, 1H), 4.25 (br s, 1H), 3.98-3.87 (m, 1H), 1.95 (s, 3H), 1.92 (s, 3H), 1.32-1.27 (m, 3H), 1.24 (dd, *J =* 3.6, 6.4 Hz, 3H).

The compound **128** was separated by SFC (column: DAICEL CHIRALPAK AS (250 mm * 30 mm, 10 um); mobile phases: Phase A of carbon dioxide, and Phase B of 0.1% ammonia water/ethanol; holding Phase B at 15%; flow rate: 60 milliliter/minute) to afford: a chiral monomer (compound **129)** with a short peak time and a chiral monomer (compound **130)** with a long peak time

### Embodiment 129. A chiral monomer (compound 129) with a short peak time after SFC resolution of the compound 128

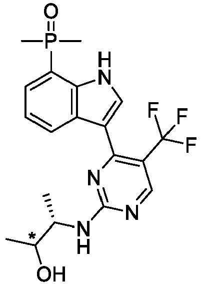

The compound **129** (36.61 mg, white solid). LCMS (ESI): [M+H]⁺= 427.2.

SFC analysis (column: Chiralpak AS-3 (100 mm * 4.6 mm, 3 um); mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 milliliter/minute): RT = 2.177 min, de = 100%.

¹H NMR (400 MHz, CD₃OD) δ ppm 8.53 (br s, 2H), 7.97 (s, 1H), 7.51 (dd, *J =* 13.6, 7.3 Hz, 1H), 7.34 (td, *J* = 7.7, 2.5 Hz, 1H), 4.30-4.09 (m, 1H), 3.94 (br s, 1H), 1.93 (d, *J* = 13.6 Hz, 6H), 1.26 (dd, *J =* 15.7, 6.7 Hz, 6H).

### Embodiment 130. The chiral monomer (compound 130) with a long peak time after SFC resolution of the compound 128

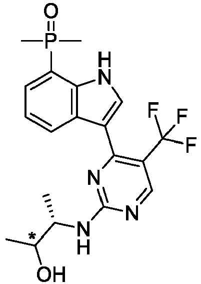

The compound **130** (17.37 mg, white solid). LCMS (ESI): [M+H]⁺= 427.1.

SFC analysis (column: Chiralpak AS-3 (100 mm * 4.6 mm, 3 um); mobile phases: Phase A of carbon dioxide, and Phase B of 0.05% diethylamine/ethanol; gradient: as for Phase B, from 5% to 40% in 4 minutes, holding Phase B at 40% for 2.5 minutes, and then holding Phase B at 5% for 1.5 minutes; flow rate: 2.8 milliliter/minute): RT = 2.318 min, de = 87.76%.

¹H NMR (400 MHz, CD₃OD) δ ppm 8.55 (br s, 2H), 7.96 (s, 1H), 7.51 (dd, *J* = 13.6, 6.8 Hz, 1H), 7.34 (td, *J =* 7.7, 2.5 Hz, 1H), 4.34-4.12 (m, 1H), 3.93-3.84 (m, 1H), 1.93 (d, *J =* 13.3 Hz, 6H), 1.33-1.20 (m, 6H).

### Embodiment 131. (3-(2-((cis-2-(aminomethyl)cyclopentyl)amino)-5-(trifluoromethyl)pyrimidin-4-yl)-1H-indole-7-yl)dimethylphosphine oxide (compound 131)

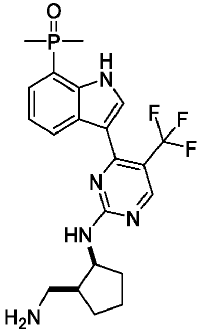

### Step 1: 2-aminocyclopentane-1-carbonitrile

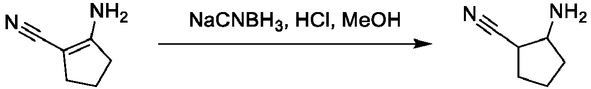

A hydrochloric acid/methanol solution (4 M, 10 mL) was added to a solution of a compound 2-aminocyclopent-1-ene-1-carbonitrile (8.60 g, 79.53 mmol) in methanol (50 mL), and then sodium cyanoborohydride (10.46 g, 166.45 mmol) was slowly added in 4 times. In this process, a 4 M hydrochloric acid/methanol solution was added in several times to maintain the pH value of the reaction solution below 7. The reaction mixture was stirred for 1 hour under 25°C. Then, the reaction solution was spin-dried under reduced pressure, an aqueous sodium hydroxide solution (1 M, 200 mL) was added, and sodium chloride (20.00 g) was then added; the reaction mixture was extracted with dichloromethane (100 mL * 3), organic phases were combined and extracted with hydrochloric acid (2 M, 50 mL * 3), and an aqueous phase of hydrochloric acid was combined and adjusted to be alkaline with a sodium hydroxide aqueous solution (4 M) under ice bath cooling, and then dichloromethane (100 mL * 4) was used for extraction; and the organic phases were dried over anhydrous sodium sulfate, filtered under reduced pressure, and spin-dried to afford a colorless liquid compound 2-aminocyclopentane-1-carbonitrile (7.90 g, 71.82 mmol, 90% yield).

¹H NMR (400 MHz, CDCl₃) δ ppm 3.59-3.46 (m, 1H), 2.88-2.34 (m, 1H), 2.24-2.00 (m, 2H), 1.99-1.48 (m, 4H), 1.47-1.40 (m, 2H).

### Step 2: benzyl (2-cyanocyclopentyl)carbamate

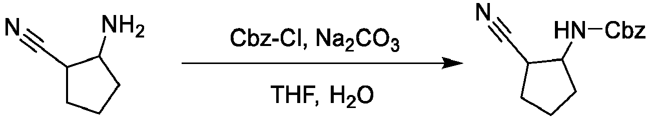

A mixed solution of a compound 2-aminocyclopentane-1-carbonitrile (2.00 g, 18.16 mmol) and sodium carbonate (3.76 g, 27.23 mmol) in tetrahydrofuran (40 mL) and water (40 mL) was added dropwise in benzyl chloroformate (3.41 g, 19.97 mmol) under an ice bath. The mixture was stirred for 12 hours under 25°C, and then the reaction mixture was extracted with ethyl acetate (40 mL * 3); organic phases were dried over anhydrous sodium sulfate, subjected to suction filtration, and spin-dried, and the residue was purified by flash column chromatography (silica gel, ethyl acetate/petroleum ether with 0-50% gradient) to afford a compound benzyl (2-cyanocyclopentyl)carbamate (3.60 g, 14.75 mmol, 81% yield), which was a colorless liquid. LCMS (ESI): [M+H]⁺= 245.1.

### Step 3: benzyl (2-(aminomethyl)cyclopentyl)carbamate

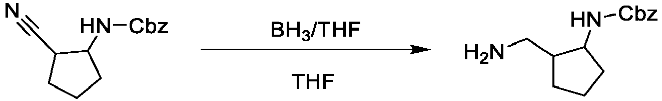

A solution of a compound benzyl (2-cyanocyclopentyl)carbamate (3.60 g, 14.75 mmol) in tetrahydrofuran (50 mL) was added dropwise in borane/tetrahydrofuran solution (1 M, 29.47 mL, 29.47 mmol) under the ice bath at 0°C. The mixture was stirred for 12 hours at 25°C. Then, the reaction mixture was spin-dried to afford a crude compound benzyl (2-(aminomethyl)cyclopentyl) carbamate (3.90 g), which was a colorless liquid. LCMS (ESI): [M+H]⁺= 249.1.

### Step 4: benzyl cis-2-((((tert-butoxycarbonyl)amino)methyl)cyclopentyl)carbamate and benzyl trans-2-((((tert-butoxycarbonyl)amino)methyl)cyclopentyl)carbamate

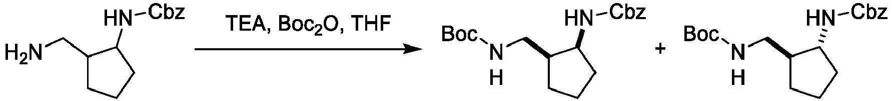

A solution of a compound benzyl (2-(aminomethyl)cyclopentyl)carbamate (3.90 g, 15.71 mmol) and triethylamine (5 mL, 39.26 mmol) in tetrahydrofuran (40 mL) was added dropwise in di-tert-butyl dicarbonate (4.11 g, 18.85 mmol) under the ice batch. The mixture was stirred for 12 hours under 25°C, and then the reaction mixture was added with water (40 mL) and extracted with ethyl acetate (40 mL * 3); organic phases were dried over anhydrous sodium sulfate, subjected to suction filtration, and spin-dried, and the residue was purified by flash column chromatography (silica gel, ethyl acetate/petroleum ether with 0-30% gradient) to afford two epimers. Through 2D NMR identification, the compound with a large polarity was trans.

The compound which has a small polarity and was afforded first: benzyl cis-2-((((tert-butoxycarbonyl)amino)methyl)cyclopentyl)carbamate (1.00 g, 2.87 mmol, 18% yield), which was a white solid. LCMS (ESI): [M-100+H]⁺= 249.1.

¹H NMR (400 MHz, CDCl₃) δ ppm 7.41-7.30 (m, 5H), 5.46 (s, 1H), 5.21-5.01 (m, 2H), 4.74 (m, 1H), 4.20-4.04 (m, 1H), 3.43 (m, 1H), 2.73 (m, 1H), 2.11-1.50 (m, 6H), 1.45 (s, 9H), 1.27-1.11 (m, 1H).

The compound which has a large polarity and was afforded later: benzyl trans-2-((((tert-butoxycarbonyl)amino)methyl)cyclopentyl)carbamate (1.20 g, 3.44 mmol, 22% yield), which was a white solid. LCMS (ESI): [M-100+H]⁺ = 249.1.

¹H NMR (400 MHz, CDCl₃) δ ppm 7.47-7.29 (m, 5H), 5.37 (s, 1H), 5.22-4.98 (m, 2H), 4.76 (m, 1H), 3.80-3.57 (m, 1H), 3.29-2.96 (m, 2H), 2.11-1.98 (m, 1H), 1.91-1.77 (m, 2H), 1.69-1.58 (m, 2H), 1.52-1.38 (m, 10H), 1.37-1.26 (m, 1H).

### Step 5: tert-butyl cis-((2-aminocyclopentyl)methyl)carbamate

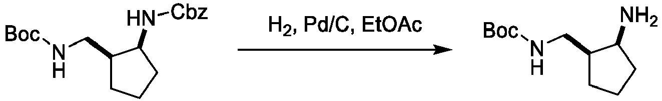

A solution of a compound benzyl cis-2-((((tert-butoxycarbonyl)amino)methyl) cyclopentyl)carbamate (0.25 g, 0.72 mmol) in ethyl acetate (25 mL) was added in wet palladium-on-carbon (10% content, 0.10 g). The mixture was stirred under a 15 psi hydrogen balloon for 12 hours under 25°C. Then, the reaction mixture was filtered and spin-dried to afford a compound tert-butyl cis-((2-aminocyclopentyl)methyl)carbamate (0.13 g, 0.61 mmol, 85% yield), which was a colorless liquid. LCMS (ESI): [M+H]⁺= 215.1.

### Step 6: tert-butyl ((cis-2-((4-(7-(dimethylphosphoryl)-1H-indole-3-yl)-5-(trifluoromethyl)pyrimidin-2-yl)amino) cyclopentyl)methyl)carbamate

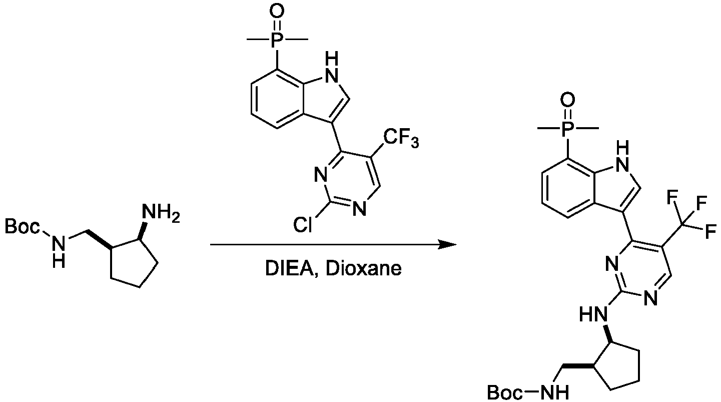

A compound tert-butyl cis-((2-aminocyclopentyl)methyl)carbamate (0.13 g, 0.61 mmol) was added to a solution of a compound (3-(2-chloro-5-(trifluoromethyl)pyrimidin-4-yl)-1H-indole-7-yl)dimethylphosphine oxide (0.21 g, 0.55 mmol) and diisopropylethylamine (0.91 mL, 5.51 mmol) in 1,4-dioxane (3 mL). The mixture was stirred for 4 hours under 100°C; then, the reaction mixture was spin-dried, and the residue was purified by flash column chromatography (C18, acetonitrile/water with 0-60% gradient) to afford a compound tert-butyl ((cis-2-((4-(7-(dimethylphosphoryl)-1H-indole-3-yl)-5-(trifluoromethyl)pyrimidin-2-yl)amino)cyclopentyl) methyl)carbamate (0.23 g, 0.42 mmol, 76% yield), which was a white solid. LCMS (ESI): [M+H]⁺ = 552.2.

### Step 7: (3-(2-((cis-2-(aminomethyl)cyclopentyl)amino)-5-(trifluoromethyl)pyrimidin-4-yl)-1H-indole-7-yl)dimethylphosphine oxide, formate

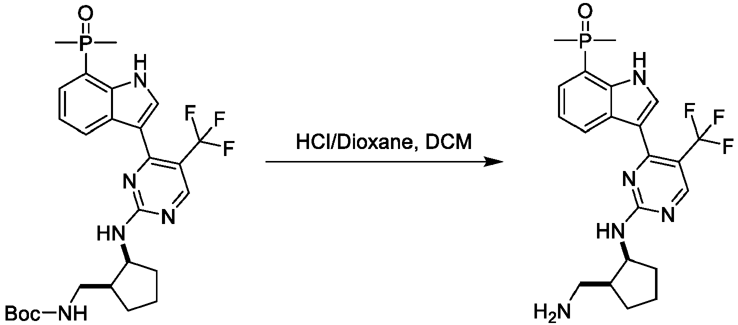

A solution of a compound tert-butyl ((cis-2-((4-(7-(dimethylphosphoryl)-1H-indole-3-yl)-5-(trifluoromethyl)pyrimidin-2-yl)amino)cyclopentyl)methyl)carbamate (35 mg, 0.06 mmol) in dichloromethane (1 mL) was added in a hydrogen chloride/dioxane solution (4 M, 320 uL, 1.28 mmol) under 0°C. The mixture was stirred for 1 hour under 25°C. The reaction mixture was spin-dried, and the residue was purified by preparative HPLC to afford a white solid compound (3-(2-((cis-2-(aminomethyl)cyclopentyl)amino)-5-(trifluoromethyl)pyrimidin-4-yl)-1H-indole-7-yl)dimethylphosphine oxide (formate, 14 mg, 31 umol, 49% yield), which was a white solid. LCMS (ESI): [M+H]⁺ = 452.2.

¹H NMR (400 MHz, CD₃OD) δ ppm 8.84-8.36 (m, 3H), 8.01 (s, 1H), 7.53 (s, 1H), 7.37 (s, 1H), 4.65-4.39 (m, 1H), 3.12-2.71 (m, 2H), 2.39-2.13 (m, 2H), 2.03 (s, 1H), 1.93 (d, *J=* 13.3 Hz, 8H), 1.85-1.72 (m, 1H), 1.61-1.44 (m, 1H).

### Embodiment 132. (3-(2-((trans-2-(aminomethyl)cyclopentyl)amino)-5-(trifluoromethyl)pyrimidin-4-yl)-1H-indole-7-yl)dimethylphosphine oxide (compound 132)

An intermediate benzyl trans-2-((((tert-butoxycarbonyl)amino)methyl)cyclopentyl) carbamate was used as a starting material to afford a compound **132** by methods the same as those in above steps 5, 6, and 7.

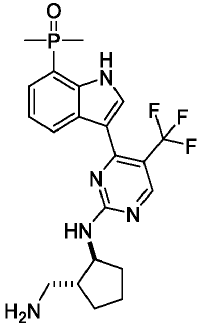

The compound **132** (formate, 20 mg, white solid). LCMS (ESI): [M+H]⁺ = 452.2.

¹H NMR (400 MHz, CD₃OD) δ ppm 8.82-8.25 (m, 3H), 8.07-7.87 (m, 1H), 7.53 (s, 1H), 7.35 (s, 1H), 4.15 (s, 1H), 3.17-2.68 (m, 2H), 2.36-2.00 (m, 3H), 1.93 (m, 9H), 1.47 (s, 1H).

### Embodiment 133. (3-(2-((cis-2-((dimethylamino)methyl)cyclopentyl)amino)-5-(trifluoromethyl)pyrimidin-4-yl)-1H-indole-7-yl)dimethylphosphine oxide (compound 133)

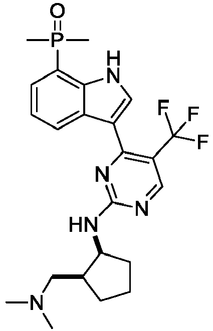

### Step 1: (3-(2-((cis-2-((dimethylamino)methyl)cyclopentyl)amino)-5-(trifluoromethyl) pyrimidin-4-yl)-1H-indole-7-yl)dimethylphosphine oxide, formate

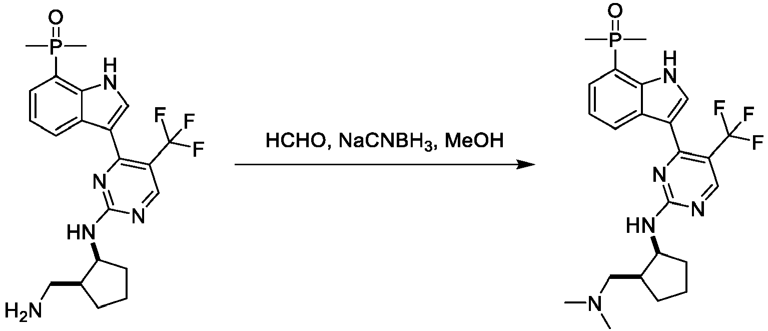

A solution of a compound (3-(2-((cis-2-(aminomethyl)cyclopentyl)amino)-5-(trifluoromethyl)pyrimidin-4-yl)-1H-indole-7-yl)dimethylphosphine oxide (130 mg, 0.29 mmol) and an aqueous formaldehyde solution (37% content, 234 mg, 2.88 mmol) in methanol (5 mL) was added in sodium cyanoborohydride (36 mg, 0.58 mmol). The mixture was stirred for 1 hour under 25°C. The reaction mixture was spin-dried. The residue was purified by preparative HPLC to afford a compound (3-(2-((cis-2-((dimethylamino)methyl)cyclopentyl)amino)-5-(trifluoromethyl)pyrimidin-4-yl)-1H-indole-7-yl)dimethylphosphine oxide (formate, 20 mg, 0.04 mmol, 14% yield), which was a yellow solid. LCMS (ESI): [M+H]⁺= 480.3.

¹H NMR (400 MHz, CD₃OD) δ ppm 8.72 (s, 1H), 8.56 (s, 1H), 8.37 (s, 1H), 7.98 (s, 1H), 7.66-7.31 (m, 2H), 4.47-4.34 (m, 1H), 2.90 (s, 4H), 2.59-2.16 (m, 2H), 2.08-1.91 (m, 12H), 1.88-1.76 (m, 2H), 1.50 (s, 1H).

### Embodiment 134. (3-(2-((trans-2-((dimethylamino)methyl)cyclopentyl)amino)-5-(trifluoromethyl)pyrimidin-4-yl)-1H-indole-7-yl)dimethylphosphine oxide, formate (compound 134)

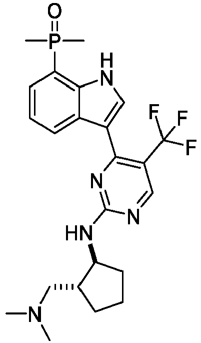

### Step 1:

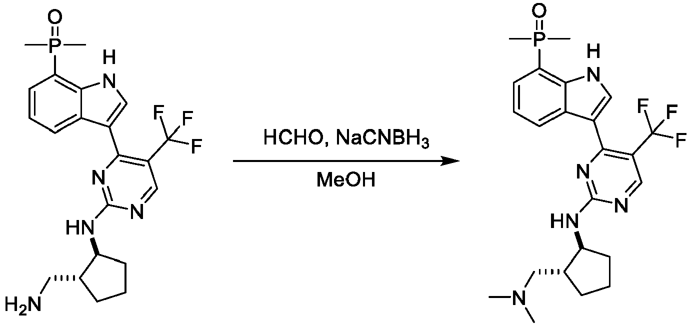

Sodium cyanoborohydride (36 mg, 0.58 mmol) was added to a solution of a compound (3-(2-((trans-2-(aminomethyl)cyclopentyl)amino)-5-(trifluoromethyl)pyrimidin-4-yl)-1H-indole-7-yl)dimethylphosphine oxide (130 mg, 0.29 mmol) and an aqueous formaldehyde solution (37% content, 234 mg, 2.88 mmol) in methanol (5 mL). The mixture was stirred for 1 hour under 25°C. The reaction mixture was spin-dried. The residue was purified by preparative HPLC to afford a compound (3-(2-((trans-2-((dimethylamino)methyl)cyclopentyl)amino)-5-(trifluoromethyl) pyrimidin-4-yl)-1H-indole-7-yl)dimethylphosphine oxide (formate, 24 mg, 0.05 mmol, 17% yield), which was a yellow solid. LCMS (ESI): [M+H]⁺= 480.4.

¹H NMR (400 MHz, CD₃OD) δ ppm 8.75-8.24 (m, 3H), 8.14-7.83 (m, 1H), 7.53 (s, 1H), 7.37 (s, 1H), 4.12 (d, *J =* 5.8 Hz, 1H), 3.20-2.95 (m, 1H), 2.84 (s, 3H), 2.49-2.03 (m, 6H), 2.01-1.77 (m, 10H), 1.42 (m, 1H)
(3-(2-((trans-2-((dimethylamino)methyl)cyclopentyl)amino)-5-(trifluoromethyl) pyrimidin-4-yl)-1H-indole-7-yl)dimethylphosphine oxide (compound **134)** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 40%; flow rate: 80 milliliter/minute) to afford a chiral monomer compound **135** with a short peak time and a chiral monomer compound **136** with a long peak time.

### Embodiment 135. A chiral monomer (compound 135) with a short peak time after SFC resolution of the compound 134

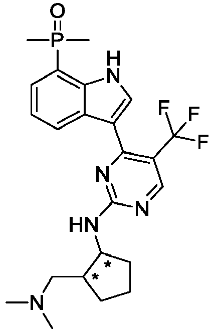

The compound **135** (41.50 mg, white solid). LCMS (ESI): [M+H]⁺= 480.2.

SFC analysis (column: Chiralpak AD-3 (150 mm * 4.6 mm,3 um); mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 milliliter/minute): RT = 4.512 min, ee = 100%
¹H NMR (400 MHz, CD₃OD) δ ppm 8.53 (s, 2H), 7.96 (s, 1H), 7.51 (dd, *J=* 7.3, 13.6 Hz, 1H), 7.34 (dt, *J* = 2.5, 7.7 Hz, 1H), 4.26-3.99 (m, 1H), 2.53 (dd, *J =* 4.1, 12.2 Hz, 1H), 2.46-2.02 (m, 10H), 1.93 (d, *J* = 13.3 Hz, 6H), 1.84-1.51 (m, 3H), 1.42 (s, 1H)

### Embodiment 136. A chiral monomer (compound 136) with a long peak time after chiral resolution of the compound 134

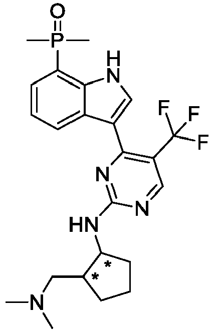

The compound **136** (36.26 mg, white solid). LCMS (ESI): [M+H]⁺= 480.3.

SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5 minutes, holding Phase B at 40% for 2.5 minutes, and then holding Phase B at 5% for 2.5 minutes; flow rate: 2.5 milliliter/minute): RT =6.895 min, ee = 100%
¹H NMR (400 MHz, CD₃OD) δ ppm 8.74-8.31 (m, 2H), 7.96 (s, 1H), 7.51 (dd, *J* = 7.3, 13.6 Hz, 1H), 7.34 (dt, *J =* 2.5, 7.8 Hz, 1H), 4.13 (br d, *J* = 14.8 Hz, 1H), 2.56 (s, 1H), 2.49-2.01 (m, 10H), 1.93 (d, *J* = 13.3 Hz, 6H), 1.86-1.55 (m, 3H), 1.45-1.36 (m, 1H)

With reference to the synthetic method of the compound 133, the following compound was prepared:

### Embodiment 137. (3-(2-(((1S,3S)-3-(dimethylamino)cyclopentyl)amino)-5-(trifluoromethyl)pyrimidin-4-yl)-1H-indole-7-yl)dimethylphosphine oxide, formate (compound 137)

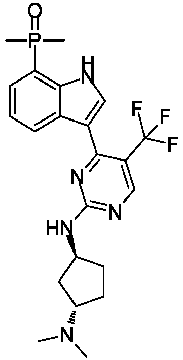

The compound **137** (52.92 mg, gray solid). LCMS (ESI): [M+H]⁺= 466.0;
¹H NMR (400 MHz, CD₃OD) δ ppm 8.56 (br d, *J=* 11.86 Hz, 3H), 7.96 (s, 1H), 7.51 (dd, *J=* 13.45, 7.21 Hz, 1H), 7.34 (td, *J=* 7.67, 2.38 Hz, 1H), 4.60 (br s, 1H), 3.62 (br s, 1H), 2.77 (br s, 5H), 2.42-2.26 (m, 2H), 2.25-2.11 (m, 2H), 1.93 (d, *J* = 13.45 Hz, 7H), 1.88-1.69 (m, 2H).

With reference to the synthetic method of the compound 80, the following compounds were prepared:

### Embodiment 138. (3-(2-((2-cyclobutyl-2-hydroxyethyl)amino)-5-(trifluoromethyl) pyrimidin-4-yl)-1H-indole-7-yl)dimethylphosphine oxide (compound 138)

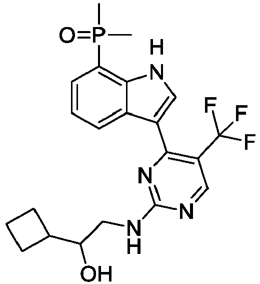

The compound **138** (50.31 mg, gray solid). LCMS (ESI): [M+H]⁺= 453.1;
¹H NMR (400MHz, DMSO-*d*₆) δ ppm 11.56 (br s, 1H), 8.68-8.41 (m, 2H), 7.92 (br d, *J=* 14.6 Hz, 1H), 7.83-7.64 (m, 1H), 7.50 (dd, *J* = 7.2, 12.8 Hz, 1H),
7.26 (q, *J* = 7.1 Hz, 1H), 4.77 (br d, *J=* 4.9 Hz, 1H), 3.63 (br d, *J=* 5.1 Hz, 1H), 3.50-3.39 (m, 1H), 3.20 (td, *J* = 6.5, 12.9 Hz, 1H), 2.42-2.27 (m, 1H), 2.00-1.60 (m, 12H).

The compound **138** was separated by SFC (column: DAICEL ChiralPak AD (250 * 30 mm, 10 um); mobile phases: Phase A of carbon dioxide, and Phase B of 0.1% ammonia water/ethanol; holding Phase B at 45%; flow rate: 60 milliliter/minute) to afford a target compound: a chiral monomer (compound **139)** with a short peak time and a chiral monomer (compound **140)** with a long peak time.

### Embodiment 139. The chiral monomer (compound 139) with a short peak time after chiral resolution of the compound 138

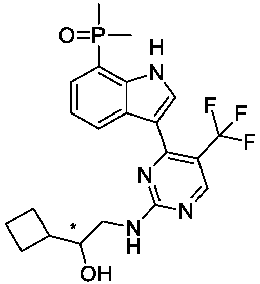

The compound **139** (25.31 mg, white solid), LCMS (ESI): [M+H]⁺ = 453.1;
SFC analysis (column: Chiralpak AD-3 (150 * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 6.809 min, ee = 100%.

¹H NMR (400 MHz, DMSO-*d*₆) δ ppm 11.56 (br s, 1H), 8.68-8.41 (m, 2H), 7.92 (br d, *J* = 14.6 Hz, 1H), 7.83-7.64 (m, 1H), 7.50 (dd, *J* = 7.2, 12.8 Hz, 1H), 7.26 (q, *J* = 7.1 Hz, 1H), 4.77 (br d, *J=* 4.9 Hz, 1H), 3.63 (br d, *J=* 5.1 Hz, 1H), 3.50-3.39 (m, 1H), 3.20 (td, *J=* 6.5, 12.9 Hz, 1H), 2.42-2.27 (m, 1H), 2.00-1.60 (m, 12H).

### Embodiment 140. A chiral monomer (compound 140) with a long peak time after chiral resolution of the compound 138

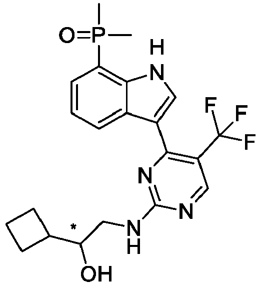

The compound **140** (25.49 mg, white solid). LCMS (ESI): [M+H]⁺ = 453.1;
SFC analysis (column: Chiralpak AD-3 (150 * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 7.460 min, ee = 100%.

¹H NMR (400 MHz, DMSO-*d*₆) δ ppm 11.56 (br s, 1H), 8.74-8.38 (m, 2H), 7.93 (br d, *J=* 14.6 Hz, 1H), 7.83-7.62 (m, 1H), 7.50 (dd, *J* = 7.2, 12.8 Hz, 1H), 7.26 (q, *J* = 7.1 Hz, 1H), 4.77 (br d, *J=* 4.9 Hz, 1H), 3.63 (br d, *J=* 4.5 Hz, 1H), 3.51-3.40 (m, 1H), 3.27-3.13 (m, 1H), 2.44-2.28 (m, 1H), 1.99-1.63 (m, 12H).

With reference to the synthetic method of the compound 80, the following compound was prepared:

### Embodiment 141. (3-(2-((((1S,2R)-2-(hydroxymethyl)cyclopropyl)methyl)amino)-5-(trifluoromethyl)pyrimidin-4-yl)-1H-indole-7-yl)dimethylphosphine oxide (compound 141)

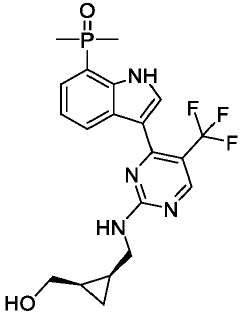

The compound **141** (3.12 mg, white solid). LCMS (ESI): [M+H]⁺= 439.2;
¹H NMR (400 MHz, DMSO-*d*₆) δ ppm 11.54 (br s, 1H), 8.70-8.34 (m, 2H), 8.02-7.72 (m, 2H), 7.49 (dd, *J* = 7.0, 13.3 Hz, 1H), 7.27 (br s, 1H), 4.69 (br s, 1H), 3.84-3.58 (m, 4H), 1.81 (d, *J* = 13.6 Hz, 6H), 1.30-1.00 (m, 2H), 0.67 (br s, 1H), 0.19 (br s, 1H).

With reference to the synthetic method of the compound 80, the following compounds were prepared:

### Embodiment 142. (3-(2-(((trans-2-(hydroxymethyl)cyclopropyl)methyl)amino)-5-(trifluoromethyl)pyrimidin-4-yl)-1H-indole-7-yl)dimethylphosphine oxide (compound 142)

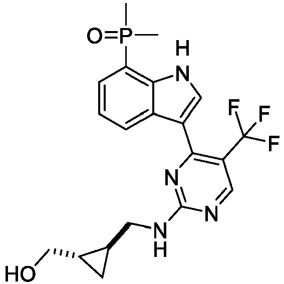

The compound **142** (39.14 mg, white solid). LCMS (ESI): [M+H]⁺= 439.1.

¹H NMR (400 MHz, DMSO-*d*₆) δ ppm 11.54 (br s, 1H), 8.66-8.40 (m, 2H), 8.07-7.86 (m, 1H), 8.07-7.86 (m, 1H), 7.50 (dd, *J* = 7.1, 12.9 Hz, 1H), 7.32-7.18 (m, 1H), 4.52-4.40 (m, 1H), 3.30-3.19 (m, 4H), 1.82 (d, *J* = 13.5 Hz, 6H), 1.01-0.76 (m, 2H), 0.50-0.30 (m, 2H).

The compound 142 was separated by SFC (column: DAICEL ChiralPak AD (250 * 30 mm, 10 um); mobile phases: Phase A of carbon dioxide, and Phase B of 0.1% ammonia water/ethanol; holding Phase B at 40%; flow rate: 80 milliliter/minute) to afford a target compound: a chiral monomer (compound **143)** with a short peak time and a chiral monomer (compound **144)** with a long peak time.

### Embodiment 143. The chiral monomer (compound 143) with a short peak time after chiral resolution of the compound 142

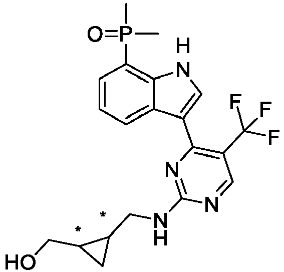

The compound **143** (13.14 mg, white solid). LCMS (ESI): [M+H]⁺ =439.1;
SFC analysis (column: ChiralPak AD-3 (150 × 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 6.744 min, ee = 100%.

¹H NMR (400 MHz, DMSO-*d*₆) δ ppm 11.54 (br s, 1H), 8.66-8.40 (m, 2H), 8.07-7.86 (m, 1H), 7.50 (dd, *J* = 7.1, 12.9 Hz, 1H), 7.32-7.18 (m, 1H), 4.52-4.40 (m, 1H), 3.30-3.19 (m, 4H), 1.82 (d, *J=* 13.5 Hz, 6H), 1.01-0.76 (m, 2H), 0.50-0.30 (m, 2H).

### Embodiment 144. A chiral monomer (compound 144) with a long peak time after chiral resolution of the compound 142

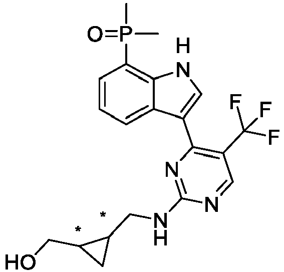

The compound **144** (9.13 mg, white solid). LCMS (ESI): [M+H]⁺ =439.1;
SFC analysis (column: ChiralPak AD-3 (150 × 4.6 mm I.D., 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT = 7.642 min, ee = 100%.

¹H NMR (400 MHz, DMSO-d6) δ ppm 11.56 (br s, 1H), 8.68-8.39 (m, 2H), 8.10-7.84 (m, 2H), 7.50 (dd, *J=* 7.2, 12.9 Hz, 1H), 7.28 (br t, *J=* 7.4 Hz, 1H), 4.47 (br d, *J=* 5.9 Hz, 1H), 3.27 (br d, *J=* 5.3 Hz, 4H), 1.82 (d, *J=* 13.4 Hz, 6H), 1.07-0.81 (m, 2H), 0.49-0.29 (m, 2H).

With reference to the synthetic method of the compound 80, the following compounds were prepared:

### Embodiment 145. Dimethyl(3-(2-((3,3,3-trifluoro-2-hydroxypropyl)amino)-5-(trifluoromethyl)pyrimidin-4-yl)-1H-indole-7-yl)phosphine oxide (compound 145)

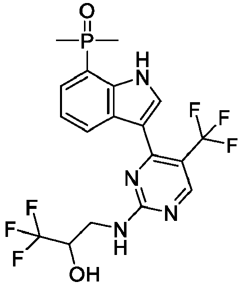

The compound **145** (82.39 mg, white solid). LCMS (ESI): [M+H]⁺ =467.3;
¹H NMR (400 MHz, DMSO-*d*₆) δ ppm 11.57 (br s, 1H), 8.69-8.36 (m, 2H), 8.17-7.89 (m, 2H), 7.50 (dd, *J=* 13.0, 7.0 Hz, 1H), 7.33-7.13 (m, 1H), 6.53 (br s, 1H), 4.28 (br s, 1H), 3.87-3.49 (m, 2H), 1.82 (d, *J* = 13.4 Hz, 6H).

The compound **145** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 25%; flow rate: 60 milliliter/minute) to afford a target compound: a chiral monomer (compound **146)** with a short peak time and a chiral monomer (compound **147)** with a long peak time.

### Embodiment 146. The chiral monomer (compound 146) with a short peak time after chiral resolution of the compound 145

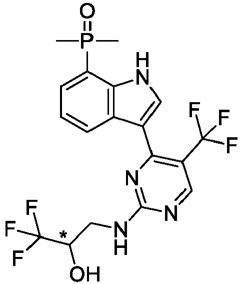

The compound **146** (18.28 mg, white solid). LCMS (ESI): [M+H]⁺= 467.1;
SFC analysis (column: ChiralPak AD-3 (150 * 4.6 mm, 3 um); mobile phases: Phase A of carbon dioxide; and Phase B of 0.05% diethylamine/ethanol; gradient: as for Phase B, from 5% to 40% in 4.5 minutes, then holding Phase B at 5% for 1.5 minutes; flow rate: 2.5 milliliter/minute): RT = 4.114 min; ee = 100%.

¹H NMR (400 MHz, DMSO-*d*6) δ ppm 11.58 (br s, 1H), 8.71-8.40 (m, 2H), 8.19 - 7.88 (m, 2H), 7.50 (dd, *J=* 12.8, 7.0 Hz, 1H), 7.36-7.14 (m, 1H), 6.52 (br t, *J=* 5.3 Hz, 1H), 4.40-4.16 (m, 1H), 3.82-3.67 (m, 1H), 3.58-3.47 (m, 1H), 1.82 (d, *J=* 13.5 Hz, 6H).

### Embodiment 147. A chiral monomer (compound 147) with a long peak time after chiral resolution of the compound 145

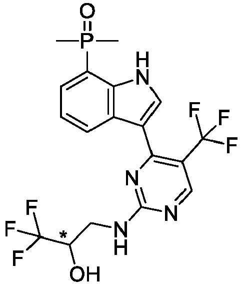

The compound **147** (21.21 mg, white solid). LCMS (ESI): [M+H]⁺ = 467.1;
SFC analysis (column: ChiralPak AD-3 (150 * 4.6 mm, 3 um); mobile phases: Phase A of carbon dioxide; and Phase B of 0.05% diethylamine/ethanol; gradient: as for Phase B, from 5% to 40% in 4.5 minutes, then holding Phase B at 5% for 1.5 minutes; flow rate: 2.5 milliliter/minute): RT = 4.316 min; ee = 99.20%.

¹H NMR (400 MHz, DMSO-*d*₆) δ ppm 11.58 (br s, 1H), 8.69-8.37 (m, 2H), 8.18-7.87 (m, 2H), 7.50 (dd, J = 13.0, 7.0 Hz, 1H), 7.31-7.15 (m, 1H), 6.52 (t, *J* = 5.3 Hz, 1H), 4.41-4.19 (m, 1H), 3.84-3.67 (m, 1H), 3.58-3.49 (m, 1H), 1.82 (d, *J=* 13.5 Hz, 6H).

With reference to the synthetic method of the compound 80, the following compounds were prepared:

### Embodiment 148. (3-(2-((3-hydroxy-2-methylpropyl)amino)-5-(trifluoromethyl) pyrimidin-4-yl)-1H-indole-7-yl)dimethylphosphine oxide (compound 148)

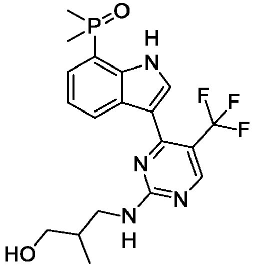

The compound **148** (68 mg, white solid). LCMS (ESI): [M+H]⁺ = 427.1;
¹H NMR (400 MHz, DMSO-*d*₆) δ ppm 11.55 (br s, 1H), 8.63-8.46 (m, 2H), 7.92 (br s, 2H), 7.49 (dd, *J* = 12.9, 7.2 Hz, 1H), 7.26 (td, *J* = 7.7, 2.3 Hz, 1H), 4.72-4.35 (m, 1H), 3.47-3.35 (m, 2H), 3.32-3.18 (m, 2H), 2.01-1.86 (m, 1H), 1.81 (d, *J=* 13.6 Hz, 6H), 0.90 (d, *J* = 6.5 Hz, 3H).

The compound **148** was separated by SFC (column: DAICEL CHIRALPAK IG (250 mm * 30 mm, 10 um); mobile phases: Phase A of carbon dioxide, and Phase B of 0.1% ammonia water/methanol; holding Phase B at 40%; flow rate: 80 milliliter/minute) to afford a chiral monomer compound: a chiral monomer (compound 149) with a short peak time and a chiral monomer (compound 150) with a long peak time.

### Embodiment 149. The chiral monomer (compound 149) with a shorter peak time after chiral resolution of the compound 148

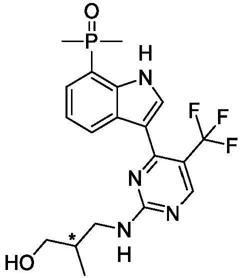

The compound **149** (27.33 mg, white solid). LCMS (ESI): [M+H]⁺= 427.1;
SFC analysis (column: Chiralpak IG-3 (100 mm * 4.6 mm * 3 um); mobile phases: Phase A of carbon dioxide, and Phase B of 0.05% diethylamine/methanol; gradient: as for Phase B, from 5% to 40% in 4 minutes, holding Phase B at 40% for 2.5 minutes, and then holding Phase B at 5% for 2.5 minutes; flow rate: 2.8 milliliter/minute): RT = 4.156 min, ee = 100%

¹H NMR (400 MHz, DMSO-*d*6) δ ppm 11.55 (br s, 1H), 8.63-8.46 (m, 2H), 7.92 (br s, 2H), 7.49 (dd, *J=* 12.9, 7.2 Hz, 1H), 7.26 (td, *J=* 7.7, 2.3 Hz, 1H), 4.72-4.35 (m, 1H), 3.47-3.35 (m, 2H), 3.32-3.18 (m, 2H), 2.01-1.86 (m, 1H), 1.81 (d, *J* = 13.6 Hz, 6H), 0.90 (d, *J* = 6.5 Hz, 3H).

### Embodiment 150. A chiral monomer (compound 150) with a long peak time after chiral resolution of the compound 148

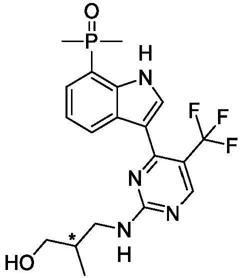

The compound **150** (25.86 mg, white solid). LCMS (ESI): [M+H]⁺ =427.1.

SFC analysis (column: Chiralpak IG-3 (100 mm * 4.6 mm * 3 um); mobile phases: Phase A of carbon dioxide, and Phase B of 0.05% diethylamine/methanol; gradient: as for Phase B, from 5% to 40% in 4 minutes, holding Phase B at 40% for 2.5 minutes, and then holding Phase B at 5% for 2.5 minutes; flow rate: 2.8 milliliter/minute): RT = 4.543 min, ee = 99.7%.

¹H NMR (400 MHz, DMSO-*d*₆) δ ppm 11.29-11.87 (m, 1H), 8.64-8.46 (m, 2H), 7.91 (br s, 2H), 7.49 (dd, *J* = 12.9, 7.4 Hz, 1H), 7.26 (td, *J* = 7.5, 2.3 Hz, 1H), 4.50 (dt, *J* = 13.9, 5.2 Hz, 1H), 3.45-3.35 (m, 2H), 3.32-3.19 (m, 2H), 1.96-1.86 (m, 1H), 1.81 (d, *J* = 13.6 Hz, 6H), 0.90 (d, *J* = 6.8 Hz, 3H).

### Embodiment 151. (3-(2-((1-hydroxycyclopentyl)methyl)amino)-5-(trifluoromethyl) pyrimidin-4-yl)-1hydro-indole-7-yl)dimethylphosphine oxide (compound 151)

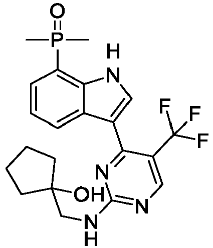

The compound **151** (32.12 mg, white solid). LCMS (ESI): [M+H]⁺ =453.2;
¹H NMR (400 MHz, DMSO-*d*₆) δ ppm 11.55 (br s, 1H), 8.73-8.39 (m, 2H), 7.91 (br d, *J=* 16.1 Hz, 1H), 7.71-7.56 (m, 1H), 7.49 (dd, *J* = 12.8, 6.8 Hz, 1H), 7.26 (br d, *J* = 7.0 Hz, 1H), 4.54 (d, *J=* 8.5 Hz, 1H), 3.55 (br dd, *J=* 15.9, 5.9 Hz, 2H), 1.80 (d, *J=* 13.3 Hz, 6H), 1.73-1.46 (m, 8H).

### Embodiment 152. (3-(6-((1-hydroxycycloheptyl)methyl)amino)-3-(trifluoromethyl) pyrimidin-2-yl)-1hydro-indole-7-yl)dimethylphosphine oxide (compound 152)

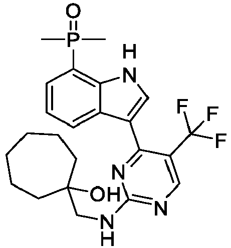

The compound **152** (74.47 mg, white solid). LCMS (ESI): [M+H]⁺ =481.2;
¹H NMR(400 MHz, DMSO-*d*₆) δ ppm 11.19 (br s, 1H), 8.67-8.39 (m, 2H), 7.89 (brd, *J* = 18.8 Hz, 1 H), 7.63-7.38 (m, 2H), 7.31-7.09 (m, 1H), 4.40 (d, *J=* 18.1 Hz, 1H), 3.49-3.36 (m, 2H), 1.78 (d, *J=* 13.55 Hz, 6H), 1.63-1.24 (m, 12H).

### Embodiment 153. (3-(2-(((1R,2R)-2-hydroxycyclobutyl)amino)-5-(trifluoromethyl) pyrimidin-4-yl)-1hydro-indole-7-yl)dimethylphosphine oxide (compound 153)

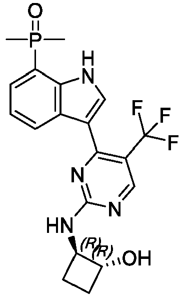

The compound **153** (52.51 mg, white solid). LCMS (ESI): [M+H]⁺ =425.2;
¹H NMR (400 MHz, DMSO-*d*₆) δ ppm 11.54 (br s, 1H), 8.70-8.43 (m, 2H), 8.21 (br t, *J=* 8.2 Hz, 1H), 7.91 (br d, *J* = 18.8Hz, 1H), 7.50 (br dd, *J* = 12.6, 7.28 Hz, 1H), 7.37-7.21 (m, 1H), 5.32 (dd, *J* = 11.3, 7.0 Hz, 1H), 4.43-4.22 (m, 1H), 4.04 (quin, *J* = 7.5 Hz, 1H), 1.97 (q, *J* = 8.0 Hz, 2H), 1.81 (d, *J* = 13.6 Hz, 6H), 1.50-1.28 (m, 2H).

### Embodiment 154. (3-(2-(((1S,2S)-2-hydroxycyclobutyl)amino)-5-(trifluoromethyl) pyrimidin-4-yl)-1hydro-indole-7-yl)dimethylphosphine oxide (compound 154)

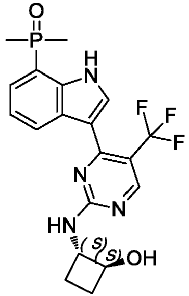

The compound **154** (57.90 mg, white solid). LCMS (ESI): [M+H]⁺= 425.1;
¹H NMR (400 MHz, DMSO-*d*₆) δ ppm 11.55 (br s, 1H), 8.75-8.43 (m, 2H), 8.21 (br t, *J=* 8.2 Hz, 1H), 7.92 (br d, *J* = 18.8 Hz, 1H), 7.49 (br dd, *J* = 12.7, 7.2 Hz, 1H), 7.39-7.18 (m, 1H), 5.32 (dd, *J* = 11.3, 7.0 Hz, 1H), 4.42-4.21 (m, 1H), 4.04 (quin, *J* = 7.5 Hz, 1H), 1.91-2.05 (m, 2H), 1.81 (d, *J* = 13.6 Hz, 6H), 1.50-1.27 (m, 2H).

### Embodiment 155. (3-(2-(((1S,2S)-2-hydroxycyclopentyl)amino)-5-(trifluoromethyl) pyrimidin-4-yl)-1hydro-indole-7-yl)dimethylphosphine oxide (compound 155)

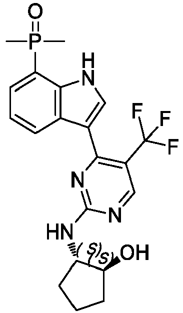

The compound **155** (16.38 mg, white solid). LCMS (ESI): [M+H]⁺ =439.2;
¹H NMR (400 MHz, DMSO-*d*₆) δ ppm 11.52 (br s, 1H), 8.67-8.48 (m, 2H), 7.98-7.80 (m, 2H), 7.49 (dd, *J* = 12.9, 6.9 Hz, 1H), 7.26 (td, *J* = 7.6, 2.3 Hz, 1H), 4.79 (br d, *J* = 10.3 Hz, 1H), 4.25-3.93 (m, 2H), 2.12-1.85 (m, 2H), 1.81 (d, J=13.5 Hz, 6H), 1.66 (br s, 2H), 1.57-1.43 (m, 2H).

### Embodiment 156. (3-(2-((1R,2R)-2-hydroxycyclopentyl)amino)-5-(trifluoromethyl)pyrimidin-4-yl)-1hydro-indole-7-yl)dimethylphosphine oxide (compound 156)

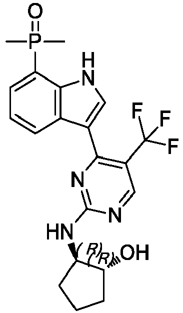

The compound **156** (19.17 mg, white solid). LCMS (ESI): [M+H]⁺ =439.1;
¹H NMR (400 MHz, DMSO-*d*₆) δ ppm 11.54 (br s, 1H), 8.77-8.45 (m, 2H), 8.05-7.82 (m, 2H), 7.58-7.11 (m, 2H), 4.79 (br d, *J=* 10.5 Hz, 1H), 4.29-3.92 (m, 2H), 2.13-1.86 (m, 2H), 1.81 (d, *J* = 13.6 Hz, 6H), 1.67 (br s, 2H), 1.58-1.44 (m, 2H)

### Embodiment 157. (S)-(3-(5-fluoro-2-((1-hydroxypropan-2-yl)amino)pyrimidin-4-yl)-1H-indole-7-yl)dimethylphosphine oxide (compound 157)

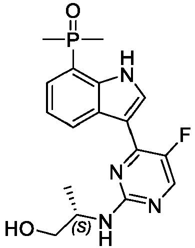

### Step 1: 7-bromo-3-(2-chloro-5-fluoropyrimidin-4-yl)-1H-indole

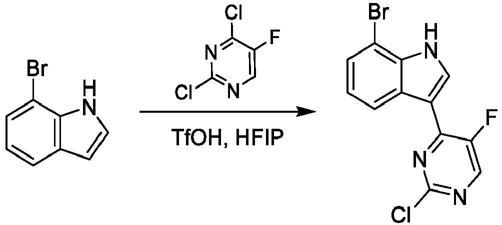

2,4-dichloro-5-fluoropyrimidine (0.85 g, 5.10 mmol) was added to a solution of 7-bromo-1H-indole (1.00 g, 5.10 mmol) in hexafluoroisopropanol (10 mL), and then trifluoromethanesulfonic acid (540 uL, 5.91 mmol) was added dropwise under 0°C. The reaction proceeded with stirring for 16 hours under 60°C. The mixture was concentrated and subjected to beating with water to afford 7-bromo-3-(2-chloro-5-fluoropyrimidin-4-yl)-1hydro-indole (2.10 g, 4.85 mmol, 95% yield), which was a yellow solid. LCMS (ESI): [M+H]⁺ = 326.0.

### Step 2: 7-bromo-3-(5-fluoro-2-(2,2,2-trifluoroethoxy)pyrimidin-4-yl)-1H-indole

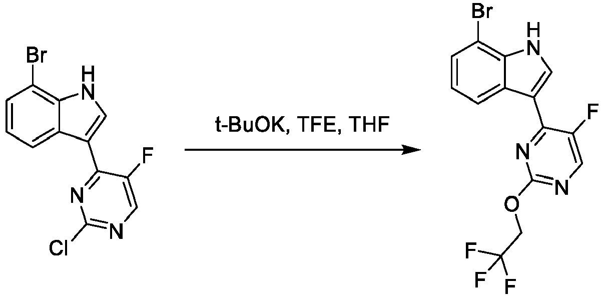

Tetrahydrofuran (20 mL) and potassium tert-butoxide (0.82 g, 7.35 mmol) were added to a solution of 7-bromo-3-(2-chloro-5-fluoropyrimidin-4-yl)-1H-indole (1.00 g, 2.45 mmol) in trifluoroethanol (20 mL), and the solution was stirred for 16 hours under 60°C. The reaction was diluted with water and concentrated under reduced pressure to remove trifluoroethanol and tetrahydrofuran. The mixture was filtered, and the solid was dried in vacuum to afford 7-bromo-3-(5-fluoro-2-(2,2,2-trifluoroethoxy)pyrimidin-4-yl)-1H-indole (0.86 g, 2.16 mmol, 88% yield). LCMS (ESI): [M+H]⁺ = 392.0.

### Step 3: (3-(5-fluoro-2-(2,2,2-trifluoroethoxy)pyrimidin-4-yl)-1H-indole-7-yl)dimethylphosphine oxide

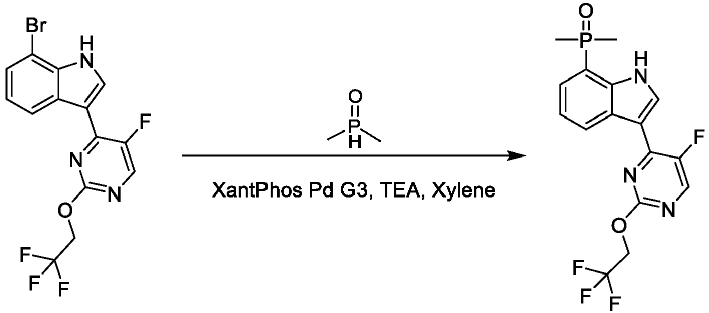

Triethylamine (0.37 g, 3.65 mmol) and dimethylphosphine oxide (0.19 g, 2.44 mmol) were added to a solution of 7-bromo-3-(5-fluoro-2-(2,2,2-trifluoroethoxy)pyrimidin-4-yl)-1H-indole (0.50 g, 1.22 mmol) in xylene (5 mL). Then, a methanesulfonato(9,9-dimethyl-4,5-bis(diphenylphosphino)xanthene)(2-amino-1,1-biphenyl)palladium(II) dichloromethane adduct (60 mg, 0.06 mmol) was added under nitrogen. The mixture was stirred for 16 hour under 140°C. The mixture was purified by flash column chromatography (silica gel, methanol/dichloromethane with 0-18% gradient) to afford (3-(5-fluoro-2-(2,2,2-trifluoroethoxy)pyrimidin-4-yl)-1H-indole-7-yl)dimethylphosphine oxide (0.43 g, 0.94 mmol, 77% yield), which was a yellow solid. LCMS (ESI): [M+H]⁺ = 388.0.

### Step 4: (S)-(3-(5-fluoro-2-((1-hydroxypropan-2-yl)amino)pyrimidin-4-yl)-1H-indole-7-yl)dimethylphosphine oxide

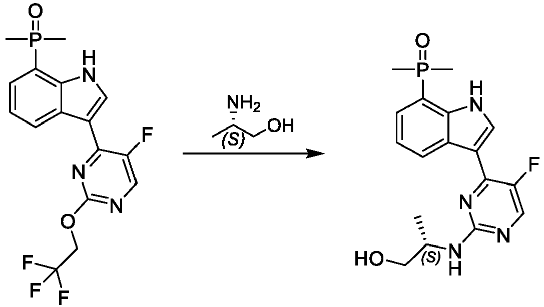

A mixture of (3-(5-fluoro-2-(2,2,2-trifluoroethoxy)pyrimidin-4-yl)-1H-indole-7-yl)dimethylphosphine oxide (100 mg, 0.26 mmol) and (S)-2-aminopropan-1-ol (500 uL) was stirred for 16 hours under 145°C. The mixture was purified by preparative HPLC to afford a white solid compound (16.40 mg, 47 umol, 18% yield). LCMS (ESI): [M+H]⁺ = 363.1;
¹H NMR (400 MHz, CD₃OD) δ ppm 9.02 (d, *J* = 8.3 Hz, 1H), 8.23 (d, *J* = 3.0 Hz, 1H), 8.14 (d, *J* = 4.3 Hz, 1H), 7.52 (dd, *J* = 6.5, 13.6 Hz, 1H), 7.38 (dt, *J* = 2.6, 7.7 Hz, 1H), 4.29-4.14 (m, 1H), 3.72 (d, *J* = 5.5 Hz, 1H), 3.66 (d, *J* = 5.5 Hz, 1H), 1.93 (d, *J* = 13.6 Hz, 6H), 1.33 (d, *J* = 6.5 Hz, 3H).

### Embodiment 158. (S)-(5-fluoro-3-(2-(((1-hydroxypropan-2-yl)amino)-5-(trifluoromethyl)pyrimidin-4-yl)-1H-indole-7-yl)dimethylphosphine oxide (compound 158)

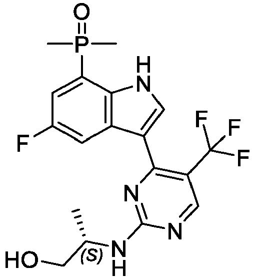

### Step 1: 7-bromo-3-(2-chloro-5-(trifluoromethyl)pyrimidin-4-yl)-5-fluoro-1H-indole

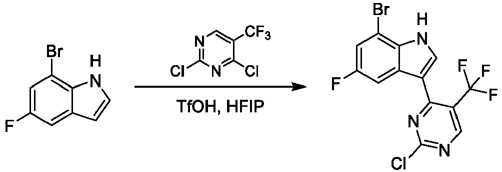

A compound 7-bromo-5-fluoro-1H-indole (250 mg, 1.17 mmol) and 2,4-dichloro-5-(trifluoromethyl)pyrimidine (304 mg, 1.40 mmol) were dissolved in hexafluoroisopropanol (6 mL), and trifluoromethanesulfonic acid (110 uL, 1.28 mmol) was slowly added dropwise under 0°C. The reaction system was warmed up to 60°C to continue the reaction for 16 hours. Cooling to room temperature was carried out, ethyl acetate (2 mL) was added to a reaction solution, the solid was precipitated from the solution, and the solid was filtered, collected, and dried to afford a crude compound 7-bromo-3-(2-chloro-5-(trifluoromethyl)pyrimidin-4-yl)-5-fluoro-1H-indole (380 mg), which was a yellow solid. LCMS (ESI): [M+H]⁺= 394.0.

### Step 2: 7-bromo-5-fluoro-3-(2-(2,2,2-trifluoroethoxy)-5-(trifluoromethyl)pyrimidin-4-yl)-1H-indole

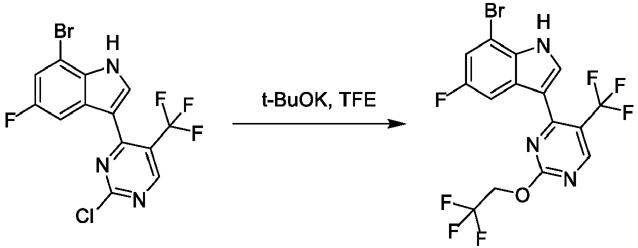

A compound 7-bromo-3-(2-chloro-5-(trifluoromethyl)pyrimidin-4-yl)-5-fluoro-1H-indole (350 mg, 0.89 mmol) was dissolved in trifluoroethanol (8 mL), potassium tert-butoxide (299 mg, 2.66 mmol) was added under 20°C, and stirring was carried out for 16 hours at 60°C. Cooling to room temperature was carried out, the reaction solution was concentrated under reduced pressure, added with water (4 mL), and stirred for 1 hour, a solid was precipitated from the solution, and the solid was filtered, collected, and dried in vacuum to afford a crude compound 7-bromo-5-fluoro-3-(2-(2,2,2-trifluoroethoxy)-5-(trifluoromethyl)pyrimidin-4-yl)-1H-indole (300 mg), which was a white solid. LCMS (ESI): [M+H]⁺= 458.0.

### Step 3: (5-fluoro-3-(2-(2,2,2-trifluoroethoxy)-5-(trifluoromethyl)pyrimidin-4-yl)-1H-indole-7-yl)dimethylphosphine oxide

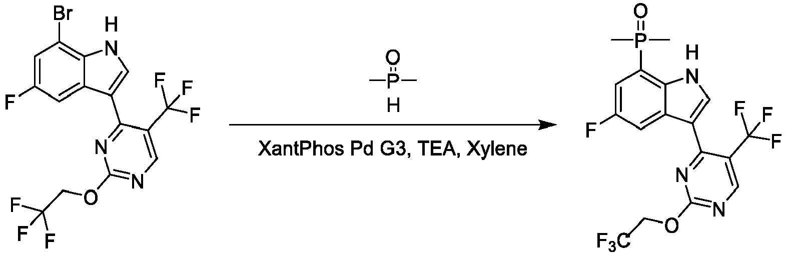

A compound 7-bromo-5-fluoro-3-(2-(2,2,2-trifluoroethoxy)-5-(trifluoromethyl)pyrimidin -4-yl)-1H-indole (270 mg, 0.59 mmol) and dimethylphosphine oxide (68 mg, 0.88 mmol) were dissolved in xylene (5 mL), and under nitrogen protection, triethylamine (410 uL, 2.95 mmol) and a methanesulfonato(9,9-dimethyl-4,5-bis(diphenylphosphino)xanthene)(2-amino-1,1-biphenyl) palladium(II) dichloromethane adduct (31 mg, 30 umol) were added under 20°C. The reaction system was warmed up to 140°C to continue the reaction for 16 hours. Cooling to room temperature and concentration were carried out, and a resulting residue was added with water (3 mL) and extracted with ethyl acetate (4 mL * 2). Organic phases were combined, dried over magnesium sulfate, and concentrated to afford a crude compound (5-fluoro-3-(2-(2,2,2-trifluoroethoxy)-5-(trifluoromethyl)pyrimidin-4-yl)-1H-indole-7-yl)dimethylphosphine oxide (290 mg), which was a yellow solid. LCMS (ESI): [M+H]⁺= 456.1.

### Step 4: (S)-(5-fluoro-3-(2-(((1-hydroxypropan-2-yl)amino)-5-(trifluoromethyl)pyrimidin-4-yl)-1H-indole-7-yl)dimethylphosphine oxide

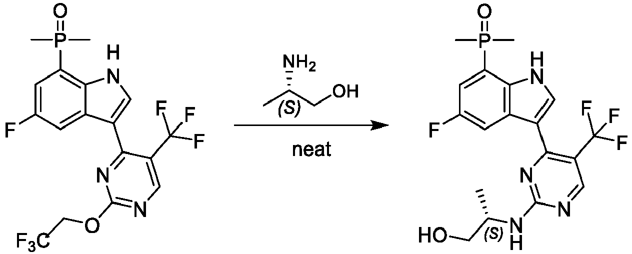

A compound (5-fluoro-3-(2-(2,2,2-trifluoroethoxy)-5-(trifluoromethyl)pyrimidin-4-yl)-1H-indole-7-yl)dimethylphosphine oxide (290 mg, 0.64 mmol) was dissolved in L-aminopropanol (2 mL, 25.48 mmol), and the reaction system was warmed up to 100°C for reaction for 2 hours. Cooling to room temperature was carried out, the reaction solution was concentrated under reduced pressure, and the residue was purified by preparative HPLC to afford (S)-(5-fluoro-3-(2-(((1-hydroxypropan-2-yl)amino)-5-(trifluoromethyl)pyrimidin-4-yl)-1H-indole-7-yl)dimethylphosphine oxide (97.14 mg, 0.23 mmol, 35%), which was a white solid. LCMS (ESI): [M+H]⁺= 431.1.

¹H NMR (400 MHz, DMSO-*d*₆) δ ppm 11.63 (br s, 1H), 8.56 (br d, *J* = 10.6 Hz, 1H), 8.52-8.14 (m, 1H), 8.07-7.95 (m, 1H), 7.88-7.61 (m, 1H), 7.54-7.38 (m, 1H), 4.77 (t, *J=* 5.5 Hz, 1H), 4.14 (dt, *J* = 13.1, 6.5 Hz, 1H), 3.58-3.37 (m, 2H), 1.84 (d, *J* = 13.6 Hz, 6H), 1.19 (d, *J* = 6.6 Hz, 3H).

With reference to the synthetic method of the compound 80, the following compounds were prepared:

### Embodiment 159. (R)-(3-(2-((1-cyclopropyl-2-hydroxyethyl)amino)-5-(trifluoromethyl)pyrimidin-4-yl)-1H-indole-7-yl)dimethylphosphine oxide (compound 159)

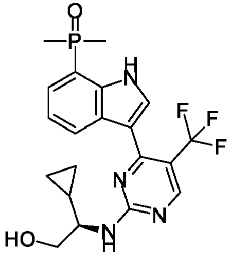

The compound **159** (35.44 mg, white solid). LCMS (ESI): [M+H]⁺= 439.1;
¹H NMR (400 MHz, DMSO-*d*₆) δ ppm 11.53 (br s, 1H), 8.74-8.31 (m, 2H), 8.00-7.86 (m, 1H), 7.79-7.59 (m, 1H), 7.49 (dd, *J=* 13.0, 7.3 Hz, 1H), 7.27 (br d, *J=* 6.2 Hz, 1H), 4.7 (br s, 1H), 3.78 (br s, 1 H), 3.66-3.55 (m, 2H), 1.82 (d, *J* = 13.5 Hz, 6H), 1.04 (br s, 1H), 0.52-0.11 (m, 4H).

### Embodiment 160. (3-(2-((1-(hydroxymethyl)cyclopropyl)methyl)amino)-5-(trifluoromethyl)pyrimidin-4-yl)-1H-indole-7-yl)dimethylphosphine oxide (compound 160)

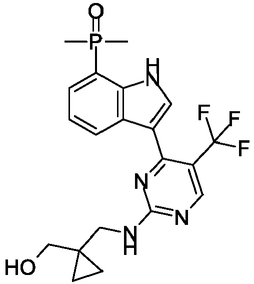

The compound **160** (42.70 mg, white solid). LCMS (ESI): [M+H]⁺= 439.1;
¹H NMR (400 MHz, DMSO-*d*₆) δ ppm 11.55 (br s, 1H), 8.67-8.40 (m, 2H), 8.01-7.73 (m, 2H), 7.50 (dd, *J* = 13.0, 6.5 Hz, 1H), 7.28 (td, *J* = 7.7, 2.4 Hz, 1H), 4.59 (br d, *J* = 5.5 Hz, 1H), 3.61-3.43 (m, 4H), 1.82 (d, *J=* 13.5 Hz, 6H), 0.59-0.25 (m, 4H).

### Embodiment 161. (R)-(3-(2-((1-hydroxy-2-methylbutan-2-yl)amino)-5-(trifluoromethyl)pyrimidin-4-yl)-1H-indole-7-yl)dimethylphosphine oxide (compound 161)

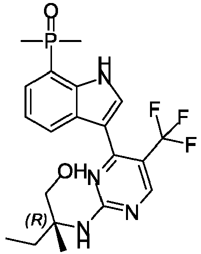

The compound **161** (4.26 mg, white solid). LCMS (ESI): [M+H]⁺= 441.3;
¹H NMR (400 MHz, DMSO-*d*₆) δ ppm 11.53 (br s, 1H), 8.58 (s, 1H), 8.71-7.72 (m, 2H), 7.50 (br dd, *J* = 12.8, 7.3 Hz, 1H), 7.32-7.21 (m, 1H), 7.07 (s, 1H), 4.87 (br s, 1H), 3.67 (br s, 1H), 3.48 (br s, 1H), 1.82 (br d, *J=* 13.3 Hz, 8H), 1.30 (br s, 3H), 0.82 (br t, *J=* 7.3 Hz, 3H).

### Embodiment 162. (3-(2-((2-hydroxy-3-methylbutyl)amino)-5-(trifluoromethyl)pyrimidin-4-yl)-1H-indole-7-yl)dimethylphosphine oxide (compound 162)

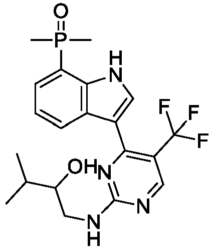

The compound **162** (3.30 mg, white solid). LCMS (ESI): [M+H]⁺= 441.1;
¹H NMR (400 MHz, DMSO-*d*₆) δ ppm 11.55 (br s, 1H), 8.71-8.43 (m, 2H), 7.93 (br d, *J=* 13.1 Hz, 1 H), 7.82-7.64 (m, 1H), 7.50 (dd, *J* = 13.0, 6.5 Hz, 1H), 7.25 (dt, *J* = 14.8, 7.3 Hz, 1H), 3.58-3.46 (m, 2H), 4.72 (br s, 1H), 3.30 (br d, *J=* 5.6 Hz, 1H), 1.82 (d, *J=* 13.5 Hz, 6H), 1.69 (br d, *J=* 6.8 Hz, 1H), 0.96-0.84 (m, 6H).

### Embodiment 163. (S)-(3-(2-((3-hydroxy-3-methylbutan-2-yl)amino)-5-(trifluoromethyl)pyrimidin-4-yl)-1H-indole-7-yl)dimethylphosphine oxide (compound 163)

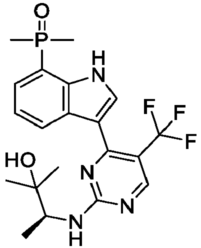

The compound **163** (64.21 mg, white solid). LCMS (ESI): [M+H]⁺= 441.2.

¹H NMR (400 MHz, DMSO-*d*₆) δ ppm 11.54 (br s, 1H), 8.67-8.38 (m, 2H), 7.91 (br d, *J=* 17.9 Hz, 1H), 7.50 (dd, *J* = 7.1, 12.9 Hz, 1H), 7.39-7.22 (m, 2H), 4.49 (br d, *J=* 7.2 Hz, 1H), 4.24-4.08 (m, 1H), 1.82 (d, *J=* 13.5 Hz, 6H), 1.25-1.10 (m, 9H).

### Embodiment 164. (3-(2-((1-hydroxycyclopropyl)methyl)amino)-5-(trifluoromethyl)pyrimidin-4-yl)-1H-indole-7-yl)dimethylphosphine oxide (compound 164)

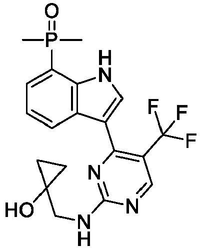

The compound **164** (32.77 mg, white solid). LCMS (ESI): [M+H]⁺= 425.1;
¹H NMR (400 MHz, DMSO-*d*₆) δ ppm 11.54 (br s, 1H), 8.74-8.40 (m, 2H), 8.00-7.70 (m, 2H), 7.50 (dd, *J* = 7.1, 12.9 Hz, 1H), 7.28 (dt, *J* = 1.9, 7.7 Hz, 1H), 5.47 (br d, *J* = 14.3 Hz, 1H), 3.69-3.53 (m, 2H), 1.82 (d, *J=* 13.5 Hz, 6H), 0.58 (br d, *J=* 11.4 Hz, 4H).

### Embodiment 165. (3-(2-(((1S,2S)-2-hydroxycyclohexyl)amino)-5-(trifluoromethyl)pyrimidin-4-yl)-1H-indole-7-yl)dimethylphosphine oxide (compound 165)

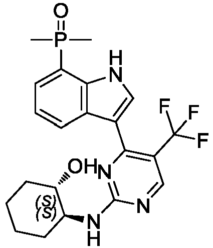

The compound **165** (35.29 mg, white solid). LCMS (ESI): [M+H]⁺ =453.1;
¹H NMR (400 MHz, DMSO-*d*₆) δ ppm 11.54 (br s, 1H), 8.74-8.45 (m, 2H), 7.93 (br s, 1H), 7.67 (br d, *J* = 7.9 Hz, 1H), 7.50 (br dd, *J* = 7.1, 12.6 Hz, 1H), 7.26 (br s, 1H), 4.74 (br s, 1H), 3.93-3.54 (m, 2H), 1.96 (br s, 2H), 1.82 (br d, *J=* 13.4 Hz, 6H), 1.67 (br s, 2H), 1.25 (br s, 4H).

### Embodiment 166. (3-(2-((4-(hydroxymethyl)tetrahydro-2H-pyran-4-yl)amino)-5-(trifluoromethyl)pyrimidin-4-yl)-1H-indole-7-yl)dimethylphosphine oxide (compound 166)

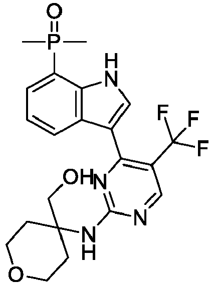

The compound **166** (19.03 mg, white solid). LCMS (ESI): [M+H]⁺= 469.2;
¹H NMR (400 MHz, DMSO-*d*₆) δ ppm 11.51 (br s, 1H), 8.80-7.67 (m, 3H), 7.58-7.16 (m, 3H), 4.83 (br s, 1H), 3.79-3.56 (m, 6H), 2.28 (br s, 2H), 1.82 (d, *J=* 13.4 Hz, 6H), 1.64 (br s, 2H).

### Embodiment 167. (3-(2-((3R,4R)-4-hydroxytetrahydro-2H-pyran-3-yl)amino)-5-(trifluoromethyl)pyrimidin-4-yl)-1H-indole-7-yl)dimethylphosphine oxide (compound 167)

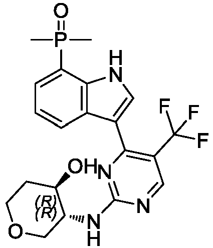

The compound **167** (52.41 mg, white solid). LCMS (ESI): [M+H]⁺= 455.2;
¹H NMR (400 MHz, DMSO-*d*₆) δ ppm 11.55 (br s, 1H), 8.74-8.40 (m, 2H), 8.01-7.87 (m, 1H), 7.74 (br dd, *J* = 7.9, 18.4 Hz, 1H), 7.50 (dd, *J* = 7.2, 13.0 Hz, 1H), 7.33-7.21 (m, 1H), 5.02 (br s, 1H), 4.02-3.79 (m, 3H), 3.68 (br d, *J=* 8.3 Hz, 1H), 3.36-3.28 (m, 1H), 3.10 (br t, *J* = 10.3 Hz, 1H), 2.00-1.90 (m, 1H), 1.82 (d, *J* = 13.4 Hz, 6H), 1.59-1.42 (m, 1H).

### Embodiment 168. (3-(2-((1-(hydroxymethyl)cyclobutyl)amino)-5-(trifluoromethyl)pyrimidin-4-yl)-1H-indole-7-yl)dimethylphosphine oxide (compound 168)

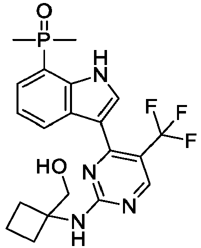

The compound **168** (57.14 mg, white solid). LCMS (ESI): [M+H]⁺= 439.2;
¹H NMR (400 MHz, DMSO-*d*₆) δ ppm 11.52 (br d, *J=* 16.3 Hz, 1H), 8.81-8.13 (m, 2H), 7.99-7.74 (m, 2H), 7.49 (dd, *J* = 7.2, 12.9 Hz, 1H), 7.26 (br t, *J=* 6.9 Hz, 1H), 4.87 (br s, 1H), 3.72 (br d, *J=* 11.1 Hz, 2H), 2.40-2.06 (m, 4H), 1.82 (d, *J=* 13.4 Hz, 8H).

### Embodiment 169. (S)-(3-(2-((1-cyclohexyl-2-hydroxyethyl)amino)-5-(trifluoromethyl)pyrimidin-4-yl)-1H-indole-7-yl)dimethylphosphine oxide (compound 169)

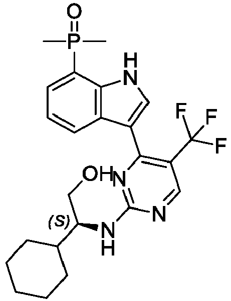

The compound **169** (71.56 mg, white solid). LCMS (ESI): [M+H]⁺= 481.2;
¹H NMR (400 MHz, DMSO-*d*₆) δ ppm 11.54 (br s, 1H), 8.75-8.36 (m, 2H), 8.00-7.83 (m, 1H), 7.65-7.43 (m, 2H), 7.34-7.17 (m, 1H), 4.63 (br s, 1H), 4.09-3.94 (m, 1H), 3.64-3.51 (m, 1H), 3.51-3.48 (m, 1H), 1.88-1.54 (m, 12H), 1.27-0.96 (m, 5H).

### Embodiment 170. (R)-(3-(2-((1-hydroxyhexan-2-yl)amino)-5-(trifluoromethyl)pyrimidin-4-yl)-1H-indole-7-yl)dimethylphosphine oxide (compound 170)

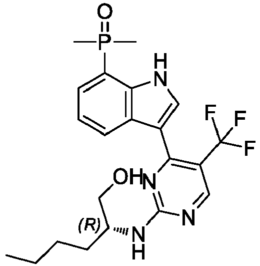

The compound **170** (70.30 mg, white solid). LCMS (ESI): [M+H]⁺= 455.2;
¹H NMR (400 MHz, DMSO-*d*₆) δ ppm 11.54 (br s, 1H), 8.70-8.37 (m, 2H), 8.00-7.83 (m, 1H), 7.67-7.43 (m, 2H), 7.26 (q, *J* = 8.0 Hz, 1H), 4.74 (br s, 1H), 4.09 (br dd, *J* = 6.1, 14.4 Hz, 1H), 3.47 (br dd, *J* = 5.1, 10.4 Hz, 2H), 1.82 (d, *J* = 13.5 Hz, 6H), 1.66 (br d, *J=* 5.0 Hz, 1H), 1.56-1.44 (m, 1H), 1.42-1.20 (m, 4H), 0.86 (br d, *J=* 6.8 Hz, 3H).

### Embodiment 171. (S)-dimethyl(3-(2-((1,1,1-trifluoro-3-hydroxypropan-2-yl)amino)-5-(trifluoromethyl)pyrimidin-4-yl)-1H-indole-7-yl)phosphine oxide (compound 171)

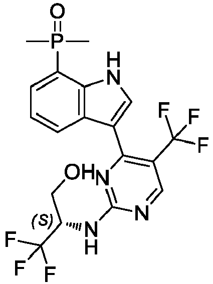

The compound **171** (18.89 mg, white solid). LCMS (ESI): [M+H]⁺= 467.1;
¹H NMR (400 MHz, DMSO-*d*₆) δ ppm 11.70 (br s, 1H), 8.90-8.33 (m, 3H), 8.17-7.96 (m, 1H), 7.61 (dd, *J=* 7.1, 12.9 Hz, 1H), 7.37 (br d, *J=* 6.0 Hz, 1H), 5.34 (br s, 1H), 5.17-5.01 (m, 1H), 3.99-3.73 (m, 2H), 1.91 (d, *J* = 13.4 Hz, 6H).

### Embodiment 172. (R)-(3-(2-((2-hydroxypropyl)amino)-5-(trifluoromethyl)pyrimidin-4-yl)-1H-indole-7-yl)dimethylphosphine oxide (compound 172)

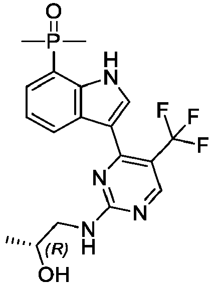

The compound **172** (57.27 mg, white solid). LCMS (ESI): [M+H]⁺= 413.1;
¹H NMR (400 MHz, DMSO-*d*₆) δ ppm 11.53 (br s, 1H), 8.67-8.42 (m, 2H), 8.01-7.73 (m, 2H), 7.50 (dd, *J* = 7.2, 12.9 Hz, 1H), 7.27 (dt, *J* = 2.3, 7.6 Hz, 1H), 4.84-4.70 (m, 1H), 3.88 (br d, *J =* 5.5 Hz, 1H), 3.34 (br d, *J* = 2.0 Hz, 2H), 1.82 (d, *J* = 13.4 Hz, 6H), 1.11 (d, *J* = 6.2 Hz, 3H).

### Embodiment 173. (S)-(3-(2-((2-hydroxypropyl)amino)-5-(trifluoromethyl)pyrimidin-4-yl)-1H-indole-7-yl)dimethylphosphine oxide (compound 173)

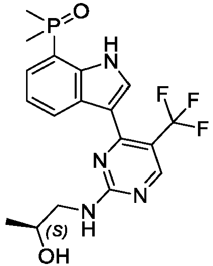

The compound **173** (48.95 mg, white solid). LCMS (ESI): [M+H]⁺= 413.1;
¹H NMR (400 MHz, DMSO-*d*₆) δ ppm 11.53 (br s, 1H), 8.67-8.39 (m, 2H), 7.98-7.73 (m, 2H), 7.50 (dd, *J* =7.3, 12.9 Hz, 1H), 7.27 (dt, *J* =2.1, 7.7 Hz, 1H), 4.85-4.76 (m, 1H), 3.88 (br d, *J* =5.8 Hz, 1H), 3.34 (br d, *J* =6.4 Hz, 2H), 1.82 (d, J=13.5 Hz, 6H), 1.11 (d, *J* =6.1 Hz, 3H)

### Embodiment 174. (3-(2-((3-hydroxybicyclo[1.1.1]pentan-1-yl)amino)-5-(trifluoromethyl)pyrimidin-4-yl)-1H-indole-7-yl)dimethylphosphine oxide (compound 174)

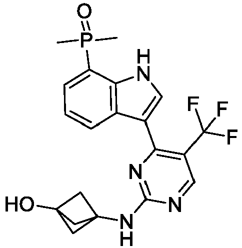

The compound **174** (9.33 mg, white solid). LCMS (ESI): [M+H]⁺= 437.1;
¹H NMR (400 MHz, DMSO-*d*₆) δ ppm 11.51 (br s, 1H), 8.71-8.47 (m, 2H), 8.39-8.05 (m, 1H), 7.99-7.71 (m, 1H), 7.50 (br dd, J=7.1, 12.6 Hz, 1H), 7.38-7.17 (m, 1H), 6.20 (br s, 1H), 2.13 (br d, *J* = 19.3 Hz, 6H), 1.82 (br d, *J* = 13.4 Hz, 6H)

### Embodiment 175. (3-(2-((4-hydroxytetrahydro-2H-pyran-4-yl)methyl)amino)-5-(trifluoromethyl)pyrimidin-4-yl)-1H-indole-7-yl)dimethylphosphine oxide (compound 175)

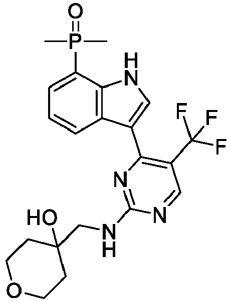

The compound **175** (48.79 mg, white solid). LCMS (ESI): [M+H]⁺= 469.1;
¹H NMR (400 MHz, DMSO-*d*₆) δ ppm 11.54 (br s, 1H), 8.70-8.41 (m, 2H), 7.93 (br d, *J*=18.1 Hz, 1H), 7.77-7.60 (m, 1H), 7.50 (dd, *J*=7.1, 12.9 Hz, 1H), 7.28 (dt, *J*=2.1, 7.6 Hz, 1H), 4.70 (br d, *J*=13.0 Hz, 1H), 3.65-3.46 (m, 6H), 1.82 (d, *J*=13.4 Hz, 6H), 1.67-1.38 (m, 4H)

### Embodiment 176. (R)-(3-(2-((1-hydroxy-3,3-dimethylbutan-2-yl)amino)-5-(trifluoromethyl)pyrimidin-4-yl)-1H-indole-7-yl)dimethylphosphine (compound 176)

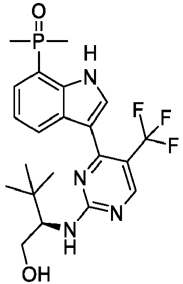

The compound **176** (47.40 mg, white solid). LCMS (ESI): [M+H]⁺= 455.1;
¹H NMR (400 MHz, CD₃OD) δ ppm 8.48-8.63 (m, 2H), 7.93 (br s, 1H), 7.49 (dd, J=13.55, 7.03 Hz, 1H), 7.32 (td, *J*=7.65, 2.51 Hz, 1H), 4.39-4.19 (m, 1H), 3.91 (dd, *J*=11.42, 3.39 Hz, 1H), 3.75-3.56 (m, 1H), 1.91 (d, *J*=13.55 Hz, 6H), 1.02 (s, 9H).

### Embodiment 177. (S)-(3-(5-bromo-2-((1-hydroxypropan-2-yl)amino)pyrimidin-4-yl)-1H-indole-7-yl)dimethylphosphine oxide (compound 177)

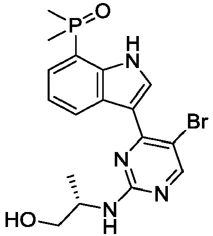

### Step 1: (1H-indole-7-yl)dimethylphosphine oxide

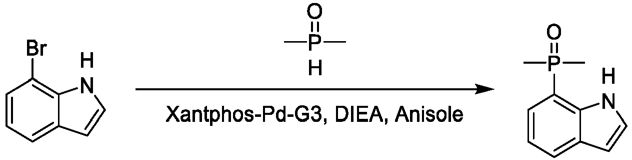

7-bromo-1H-indole (5.00 g, 25.50 mmol), a [9,9-dimethyl-4,5-bis(diphenylphosphino)xanthene][2-amino-1,1-diphenyl]palladium(II) mesylate dichloromethyl adduct (2.60 g, 2.50 mmol), diisopropylethylamine (16.48 g, 127.52 mmol) and dimethylphosphine oxide (3.98 g, 51.01 mmol) were added in anisole (50 mL) under nitrogen protection, and then the mixture was stirred for 16 hours under 145°C. The reaction mixture was spin-dried, and the residue was purified by flash column chromatography (silica gel, tetrahydrofuran/petroleum ether with 0-25% gradient) to afford a white solid compound (1H-indole-7-yl)dimethylphosphine oxide (80% purity, 4.50 g, 23.30 mmol, 73% yield). LCMS (ESI): [M+H]⁺ = 194.1.

### Step 2: (3-(5-bromo-2-chloropyrimidin-4-yl)-1H-indole-7-yl)dimethylphosphine oxide

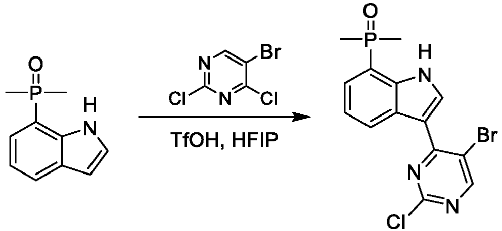

Under 15°C, trifluoromethanesulfonic acid (260 mg, 1.71 mmol) was added to a solution of (1H-indole-7-yl)dimethylphosphine oxide (300 mg, 1.55 mmol) and 2,4-dichloro-5-bromopyrimidine (530 mg, 2.33 mmol) in hexafluoroisopropanol (3 mL). The solution was stirred for 16 hours under 60°C. The solution was subjected to beating with ethyl acetate to afford a yellow solid (3-(5-bromo-2-chloropyrimidin-4-yl)-1H-indole-7-yl)dimethylphosphine oxide (500 mg, 1.30 mmol, 84% yield). LCMS (ESI): [M+H]⁺= 386.0.

### Step 3: (S)-(3-(5-bromo-2-((1-hydroxypropan-2-yl)amino)pyrimidin-4-yl)-1H-indole-7-yl)dimethylphosphine oxide

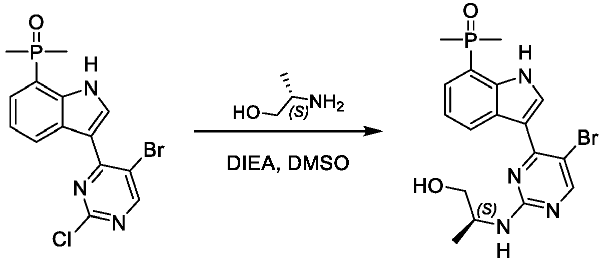

Under normal temperature, (S)-2-amino-1-propanol (0.03 g, 0.39 mmol) and diisopropylethylamine (230 uL, 1.30 mmol) was added to a solution of (3-(5-bromo-2-chloropyrimidin-4-yl)-1H-indole-7-yl)dimethylphosphine oxide (0.10 g, 0.26 mmol) in dimethyl sulfoxide (1 mL). The mixture was stirred for 2 hour under 100°C. The residue was purified by preparative HPLC to afford (S)-(3-(5-bromo-2-((1-hydroxypropan-2-yl)amino)pyrimidin-4-yl)-1H-indole-7-yl)dimethylphosphine oxide (25.49 mg, 59 umol, 23% yield), which was a white solid. LCMS (ESI): [M+H]⁺=425.1;
¹H NMR (400 MHz, DMSO-*d*₆) δ ppm 11.55 (br s, 1H), 8.92-8.30 (m, 3H), 7.49 (dd, *J* = 6.6, 12.9 Hz, 1H), 7.27 (dt, *J* = 2.3, 7.7 Hz, 1H), 7.17-6.88 (m, 1H), 4.76 (br s, 1H), 4.03 (br s, 1H), 3.59-3.47 (m, 2H), 1.81(d, *J* = 13.5 Hz, 6H), 1.18 (d, *J* = 6.6 Hz, 3H).

### Embodiment 178. (S)-(3-(2-(((1-hydroxypropan-2-yl)amino)-5-methylpyrimidin-4-yl)-1H-indole-7-yl)dimethylphosphine oxide (compound 178)

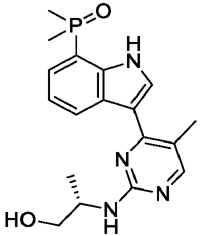

### Step 1: 7-bromo-3-(2-chloro-5-methylpyrimidin-4-yl)-1H-indole

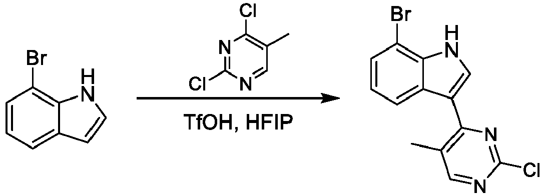

Under 25°C, trifluoromethanesulfonic acid (0.84 g, 5.61 mmol) was added to a solution of 7-bromoindole (1.00 g, 5.10 mmol) and 2,4-dichloro-5-methylpyrimidine (1.00 g, 6.12 mmol) in hexafluoroisopropanol (10 mL). The solution was stirred for 16 hours under 60°C. The reaction solution was subjected to beating with ethyl acetate (20 mL) and filtered to afford a compound 7-bromo-3-(2-chloro-5-methylpyrimidin-4-yl)-1H-indole (1.80 g, 5.10 mmol, 98% yield), which was a yellow solid. LCMS (ESI): [M+H]⁺= 322.0.

### Step 2: 7-bromo-3-(5-methyl-2-(2,2,2-trifluoroethoxy)pyrimidin-4-yl)-1H-indole

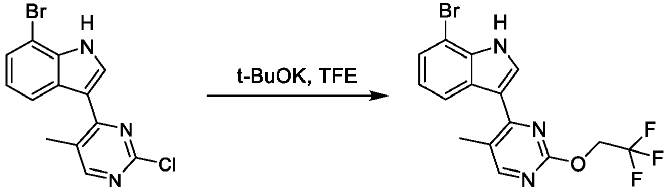

Under 0°C, potassium tert-butoxide (1.04 g, 9.30 mmol) was added to a solution of 7-bromo-3-(2-chloro-5-methylpyrimidin-4-yl)-1H-indole (1.00 g, 3.10 mmol) in trifluoroethanol (10 mL). The solution was stirred for 16 hours under 60°C. The solution was subjected to beating with ethyl acetate (20 mL) and filtered to afford a crude compound 7-bromo-3-(5-methyl-2-(2,2,2-trifluoroethoxy)pyrimidin-4-yl)-1H-indole (1.60 g), which was a yellow solid. LCMS (ESI): [M+H]⁺= 386.0.

### Step 3: dimethyl(3-(5-methyl-2-(2,2,2-trifluoroethoxy)pyrimidin-4-yl)-1H-indole-7-yl)phosphine oxide

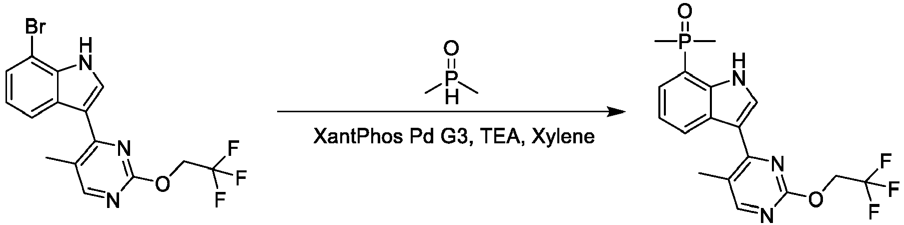

Under a nitrogen atmosphere, a solution of 7-bromo-3-(5-methyl-2-(2,2,2-trifluoroethoxy)pyrimidin-4-yl)-1H-indole (297 mg, 0.77 mmol), dimethylphosphine oxide (119 mg, 1.54 mmol), and triethylamine (536 uL, 3.85 mmol) in xylene (3 mL) was added in a [9,9-dimethyl-4,5-bis(diphenylphosphino)xanthene][2-amino-1,1-diphenyl]palladium(II) methanesulfonate dichloromethyl adduct (80 mg, 0.08 mmol). The reaction solution was stirred for 16 hours at 140°C. The mixture was concentrated to afford a crude product. The residue was purified by flash column chromatography (silica gel, tetrahydrofuran/petroleum ether with 0-20% gradient) to afford a white solid compound dimethyl(3-(5-methyl-2-(2,2,2-trifluoroethoxy)pyrimidin-4-yl)-1H-indole-7-yl)phosphine oxide (80% purity, 300 mg, 0.63 mmol, 81% yield). LCMS (ESI): [M+H]⁺= 384.1.

### Step 4: (S)-(3-(2-(((1-hydroxypropan-2-yl)amino)-5-methylpyrimidin-4-yl)-1H-indole-7-yl)dimethylphosphine oxide

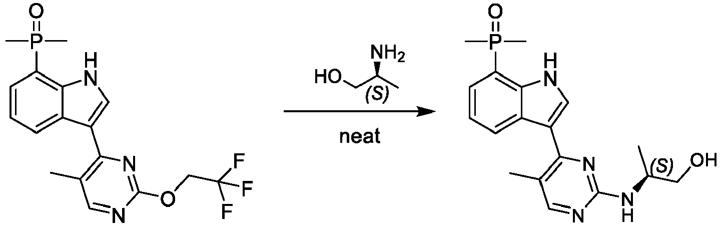

A mixed solution of dimethyl(3-(5-methyl-2-(2,2,2-trifluoroethoxy)pyrimidin-4-yl)-1H-indole-7-yl)phosphine oxide (80% purity, 300 mg, 0.63 mmol) and a compound (S)-2-aminopropan-1-ol (0.65 g, 8.61 mmol) was stirred for 3 hours under 100°C. The solution was diluted with water (10 mL) and extracted with ethyl acetate (20 mL * 3), and organic phases were spin-dried to afford a residue. The residue was purified by preparative HPLC to afford a white solid compound (S)-(3-(2-(((1-hydroxypropan-2-yl)amino)-5-methylpyrimidin-4-yl)-1H-indole-7-yl)dimethylphosphine oxide (17.18 mg, 47 umol, 8% yield). LCMS (ESI): [M+H]⁺= 359.2;
¹H NMR (400 MHz, DMSO-*d*₆) δ ppm 11.42 (br s, 1H), 8.78 (br d, *J=* 7.6 Hz, 1H), 8.09 (s, 1H), 7.95 (s, 1H), 7.55-7.18 (m, 2H), 6.44 (br d, *J=* 7.9 Hz, 1H), 4.13-3.98 (m, 1H), 3.55-3.51 (m, 2H), 2.28 (s, 3H), 1.81 (d, *J =* 13.4 Hz, 6H), 1.18 (d, *J* = 6.6 Hz, 3H).

### Embodiment 179. (S)-4-(7-(dimethylphosphoryl)-1H-indole-3-yl)-2-((1-hydroxypropan-2-yl)amino)pyrimidine-5-carbonitrile (compound 179)

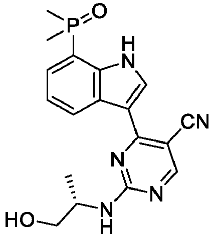

### Step 1: preparation of 4-(7-bromo-1H-indole-3-yl)-2-chloropyrimidine-5-carbonitrile

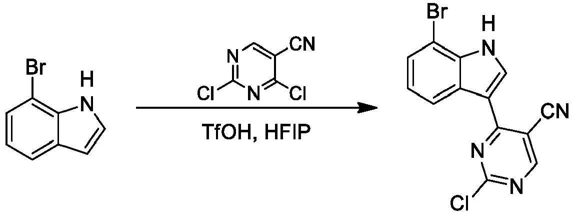

A compound 7-bromo-1H-indole (700 mg, 3.57 mmol) was dissolved in hexafluoroisopropanol (14 mL); then, 2,4-dichloropyrimidine-5-carbonitrile (932 mg, 5.36 mmol) was added, trifluoromethanesulfonic acid (589 mg, 3.93 mmol) was added at 0°C, and the reaction proceeded with stirring for 16 hours at 60°C. After the reaction was completed, the reaction solution was spin-dried and subjected to beating with ethyl acetate (5 mL) to afford 4-(7-bromo-1H-indole-3-yl)-2-chloropyrimidine-5-carbonitrile (950 mg, 2.75 mmol, 77% yield), which was an orange solid. LCMS (ESI): [M+H]⁺= 333.0.

### Step 2: preparation of 4-(7-bromo-1H-indole-3-yl)-2-(2,2,2-trifluoroethoxy)pyrimidine-5-carbonitrile

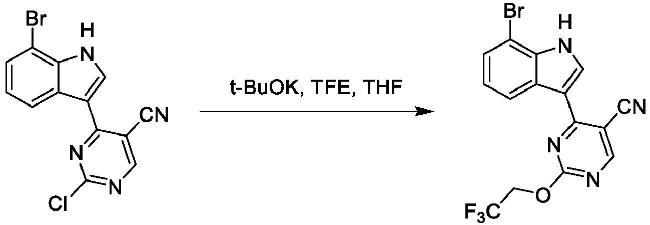

4-(7-bromo-1H-indole-3-yl)-2-chloropyrimidine-5-carbonitrile (500 mg, 1.44 mmol) was dissolved in trifluoroethanol (5 mL) and tetrahydrofuran (1 mL); then, potassium tert-butoxide (484 mg, 4.32 mmol) was added, and the reaction proceeded with stirring for 16 hours at 60°C. After the reaction was completed, a reaction solution was spin-dried; then, the crude product was subjected to beating with water (5 mL) to afford 4-(7-bromo-1H-indole-3-yl)-2-(2,2,2-trifluoroethoxy)pyrimidine-5-carbonitrile (400 mg, 0.98 mmol, 68% yield), which was a yellow solid. LCMS (ESI): [M+H]⁺= 398.8.

### Step 3: preparation of 4-(7-(dimethylphosphoryl)-1H-indole-3-yl)-2-(2,2,2-trifluoroethoxy)pyrimidine-5-carbonitrile

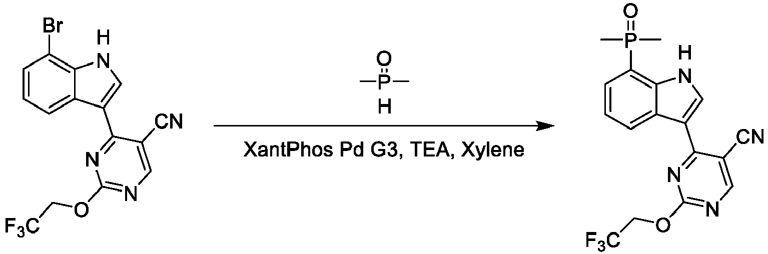

In a glove box, 4-(7-bromo-1H-indole-3-yl)-2-(2,2,2-trifluoroethoxy)pyrimidine-5-carbonitrile (350 mg, 0.87 mmol) was dissolved in xylene (3.5 mL); then, dimethylphosphine oxide (136 mg, 1.75 mmol), triethylamine (267 mg, 2.64 mmol) and a [9,9-dimethyl-4,5-bis(diphenylphosphino)xanthene][2-amino-1,1-diphenyl]palladium(II) methanesulfonate dichloromethyl adduct (84 mg, 0.09 mmol) were added, and the reaction proceeded with stirring for 16 hours at 145°C. After the reaction was completed, the reaction solution was spin-dried to remove a solvent, and the residue was purified by flash column chromatography (silica gel, methanol/dichloromethane with 0-5% gradient) to afford 4-(7-(dimethylphosphoryl)-1H-indole-3-yl)-2-(2,2,2-trifluoroethoxy)pyrimidine-5-carbonitrile (70% purity, 300 mg, 0.52 mmol, 60% yield), which was an umber solid. LCMS (ESI): [M+H]⁺= 395.1.

### Step 4: preparation of (S)-4-(7-(dimethylphosphoryl)-1H-indole-3-yl)-2-((1-hydroxypropan-2-yl)amino)pyrimidine-5-carbonitrile

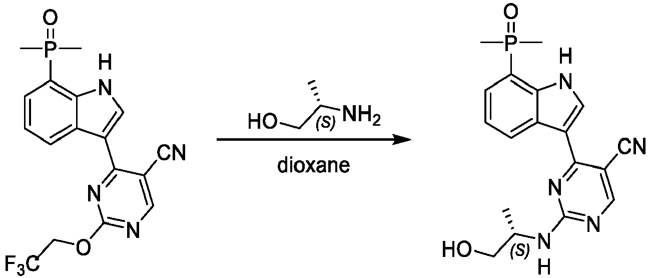

4-(7-(dimethylphosphoryl)-1H-indole-3-yl)-2-(2,2,2-trifluoroethoxy)pyrimidine-5-carbonitrile (70% purity, 100 mg, 0.18 mmol) was dissolved in dioxane (1 mL); then, (S)-2-aminopropan-1-ol (133 mg, 1.77 mmol) was added, and the reaction proceeded with stirring for 16 hours at 100°C. After the reaction was completed, the reaction solution was spin-dried, and the residue was purified by preparative HPLC to afford (S)-4-(7-(dimethylphosphoryl)-1H-indole-3-yl)-2-((1-hydroxypropan-2-yl)amino)pyrimidine-5-carbonitrile (33 mg, 0.09 mmol, 50% yield), which was a white solid. LCMS (ESI): [M+H]⁺= 370.1.

¹H NMR (400 MHz, DMSO-*d*₆) δ ppm 11.73 (br s, 1 H), 8.95-8.54 (m, 3 H), 8.06-7.83 (m, 1 H), 7.53 (dd, *J* = 12.80, 7.28 Hz, 1 H), 7.37-7.25 (m, 1 H), 4.82 (dt, *J* = 10.73, 5.55 Hz, 1 H), 4.32-4.03 (m, 1 H), 3.63-3.39 (m, 2 H), 1.81 (d, *J* = 13.30 Hz, 6 H), 1.21 (dd, *J* = 16.06, 6.78 Hz, 3 H).

### Embodiment 180. (S)-4-(7-(dimethylphosphoryl)-1H-indole-3-yl)-2-((1-hydroxypropan-2-yl)amino)pyrimidine-5-carboxamide (compound 180)

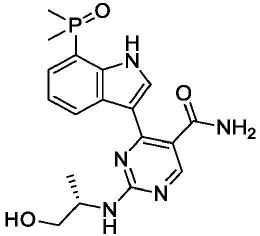

### Step 1: preparation of 2-chloro-4-(7-(dimethylphosphoryl)-1H-indole-3-yl)pyrimidine-5-carboxamide

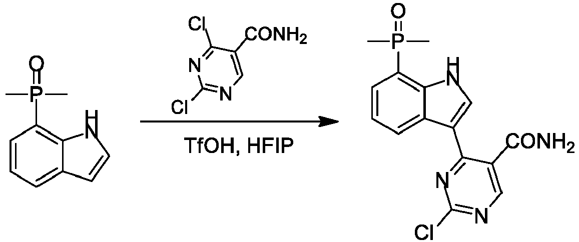

A compound (1H-indole-7-yl)dimethylphosphine oxide (200 mg, 0.83 mmol) was dissolved in hexafluoroisopropanol (2 mL); then, 2,4-dichloropyrimidine-5-carboxamide (170 mg, 0.91 mmol) was added, trifluoromethanesulfonic acid (140 mg, 0.91 mmol) was added at 0°C, and the reaction proceeded with stirring for 16 hours at 60°C. After the reaction was completed, the reaction solution was spin-dried and subjected to beating with ethyl acetate (5 mL) to afford a crude compound 2-chloro-4-(7-(dimethylphosphoryl)-1H-indole-3-yl)pyrimidine-5-carboxamide (600 mg), which was an orange solid. LCMS (ESI): [M+H]⁺= 349.1.

### Step 2: preparation of ((S)-4-(7-(dimethylphosphoryl)-1H-indole-3-yl)-2-((1-hydroxypropan-2-yl)amino)pyrimidine-5-carboxamide

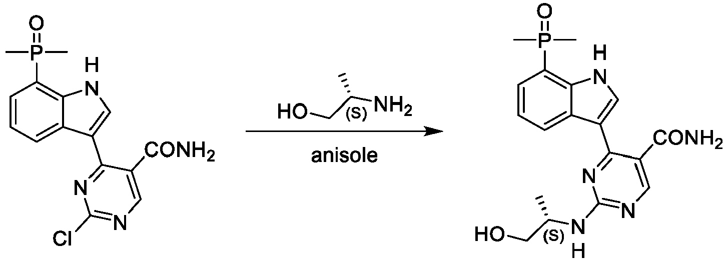

2-chloro-4-(7-(dimethylphosphoryl)-1H-indole-3-yl)pyrimidine-5-carboxamide (35% purity, 600 mg, 0.60 mmol) was dissolved in anisole (2 mL); then, (S)-2-aminopropan-1-ol (452 mg, 6.02 mmol) was added, and the reaction proceeded with stirring for 16 hours at 145°C. After the reaction was completed, the reaction solution was spin-dried, and the residue was purified by preparative HPLC to afford ((S)-4-(7-(dimethylphosphoryl)-1H-indole-3-yl)-2-((1-hydroxypropan-2-yl)amino) pyrimidine-5-carboxamide (33.77 mg, 87 umol, 14% yield), which was a white solid. LCMS (ESI): [M+H]⁺= 388.1.

¹H NMR (400 MHz, DMSO-*d*₆) δ ppm 11.76-11.02 (m, 1 H), 8.67 (br s, 1 H), 8.20 (s, 1 H), 8.01 (s, 1 H), 7.79 (br s, 1 H), 7.53-7.36 (m, 2 H), 7.24 (td, J=7.65, 2.3 Hz, 1 H), 7.02 (br s, 1 H), 4.74 (t, *J* = 5.5 Hz, 1 H), 4.10 (br s, 1 H), 3.54 (dt, *J* = 10.5, 5.4 Hz, 1 H), 3.39 (br d, *J* = 4.8 Hz, 1H), 1.80 (d, *J* = 13.3 Hz, 6 H), 1.19 (d, *J =* 6.5 Hz, 3 H).

### Embodiment 181. (S)-2-((4-(6-(3,6-dihydro-2H-pyran-4-yl)-1H-pyrrolo[2,3-b]pyridin-3-yl)-5-(trifluoromethyl)pyrimidin-2-yl)amino)propan-1-ol (compound 181)

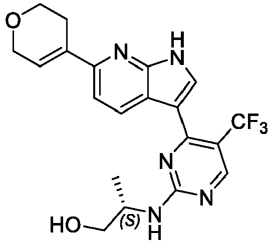

### Step 1: 6-chloro-1-(benzenesulfonyl)-3-(2-(2,2,2-trifluoroethoxy)-5-(trifluoromethyl) pyrimidin-4-yl)-1H-pyrrolo[2,3-b]pyridine

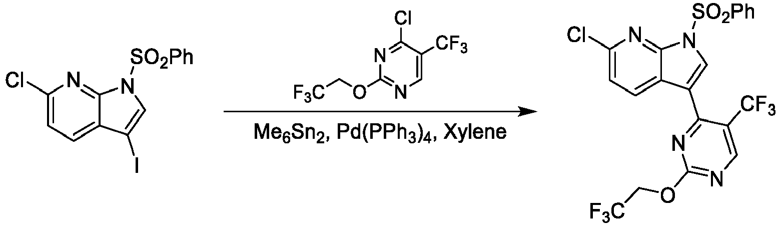

A compound 6-chloro-3-iodo-1-(benzenesulfonyl)-1H-pyrrolo[2,3-b]pyridine (5.00 g, 11.94 mmol) was added in xylene (50 mL); then, a compound 4-chloro-2-(2,2,2-trifluoroethoxy)-5-(trifluoromethyl)pyrimidine (3.35 g, 11.94 mmol) and hexamethyldistannane (5.09 g, 15.53 mmol) were added; and then, tetrakis(triphenylphosphine)palladium (1.38 g, 1.19 mmol) was added under a nitrogen environment. The reaction proceeded with stirring for 2 hours at 100°C, which was then moved to 140°C for a reaction for 16 hours. After the reaction was completed, organic phases were added in potassium fluoride (5 g), stirred for 1 h at 25°C, concentrated and mixed. The residue was purified by flash column chromatography (silica gel, ethyl acetate/petroleum ether with 0-10% gradient) to afford a compound 6-chloro-1-(benzenesulfonyl)-3-(2-(2,2,2-trifluoroethoxy)-5-(trifluoromethyl)pyrimidin-4-yl)-1H-pyrrolo[2,3-b]pyridine (3.74 g, 6.92 mmol, 58% yield), which was a yellow solid. LCMS (ESI): [M+H]⁺ = 537.0.

### Step 2: (S)-2-((4-(6-chloro-1H-pyrrolo[2,3-b]pyridin-3-yl]-5-(trifluoromethyl)pyrimidin-2-yl)amino)propan-1-ol

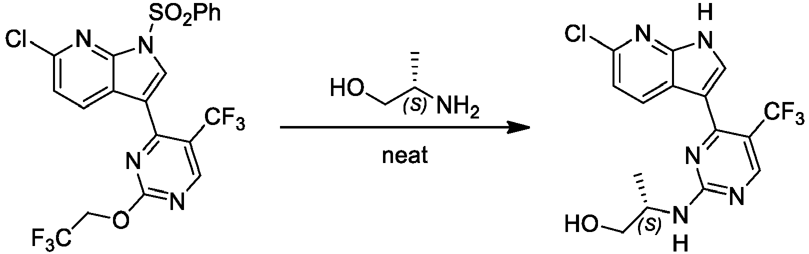

6-chloro-1-(benzenesulfonyl)-3-(2-(2,2,2-trifluoroethoxy)-5-(trifluoromethyl)pyrimidin-4-yl)-1H-pyrrolo[2,3-b]pyridine (2.00 g, 3.35 mmol) was added in (S)-2-aminopropan-1-ol (10 mL). Then, the mixture was stirred for 16 hours under 145°C. The residue was purified by flash column chromatography (silica gel, methanol/dichloromethane with 0-5% gradient) to afford (S)-2-((4-(6-chloro-1H-pyrrolo[2,3-b]pyridin-3-yl]-5-(trifluoromethyl)pyrimidin-2-yl)amino)propan-1-ol (1.02 g, 2.31 mmol, 69% yield). LCMS (ESI): [M+H]⁺ = 372.1.

### Step 3: (S)-2-((4-(6-(3,6-dihydro-2H-pyran-4-yl)-1H-pyrrolo[2,3-b]pyridin-3-yl)-5-(trifluoromethyl)pyrimidin-2-yl)amino)propan-1-ol

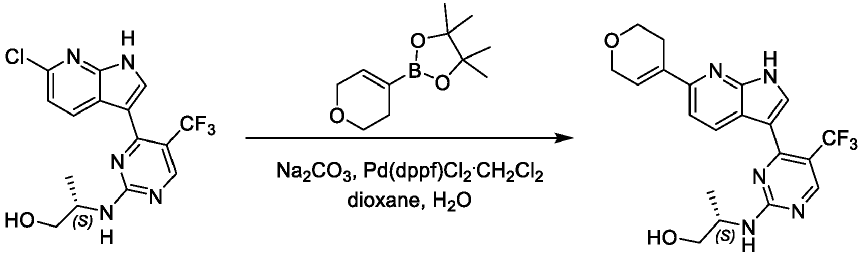

(S)-2-((4-(6-chloro-1H-pyrrolo[2,3-b]pyridin-3-yl]-5-(trifluoromethyl)pyrimidin-2-yl)amino)propan-1-ol (200 mg, 0.48 mmol) was added in 1,4-dioxane (2 mL); H₂O (40 uL), sodium carbonate (154 mg, 1.45 mmol) and 3,6-dihydro-2H-pyran-4-boronic acid pinacol ester (153 mg, 0.73 mmol) were added; then, a 1,1-bis(diphenylphosphino)ferrocene palladium dichloride dichloromethane mixture (20 mg, 0.02 mmol) was added under nitrogen, and the mixture was stirred for 16 hours under 100°C. The residue was purified by preparative HPLC to afford a compound (S)-2-((4-(6-(3,6-dihydro-2H-pyran-4-yl)-1H-pyrrolo[2,3-b]pyridin-3-yl)-5-(trifluoromethyl)pyrimidin-2-yl)amino)propan-1-ol (54.86 mg, 0.13 mmol, 27% yield). LCMS (ESI): [M+H]⁺ = 420.2.

1H NMR (400 MHz, CD₃OD) δ ppm 8.82-8.46 (m, 2H), 7.97 (s, 1H), 7.47 (br d, *J* = 8.5 Hz, 1H), 6.70 (br s, 1H), 4.39 (q, *J* = 2.6 Hz, 2H), 4.34-4.19 (m, 1H), 3.98 (t, *J* = 5.5 Hz, 2H), 3.74-3.61 (m, 2H), 2.75 (br d, *J=* 2.0 Hz, 2H), 2.68 (s, 3H), 1.32 (d, *J=* 6.5 Hz, 3H).

With reference to the synthetic method of the compound 181, the following compounds were prepared:

### Embodiment 182. (2S)-2-((4-(6-(tetrahydro-2H-pyran-2-yl)-1H-pyrrolo[2,3-b]pyridin-3-yl)-5-(trifluoromethyl)pyrimidin-2-yl)amino)propanol (compound 182)

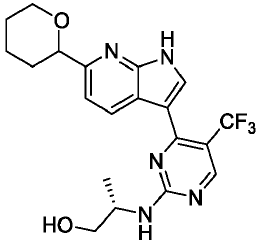

The compound **182** (90 mg, white solid). LCMS (ESI): [M+H]⁺=422.2.

¹H NMR (400 MHz, CD₃OD) δ ppm 8.88-8.66 (m, 1H), 8.53 (br s, 1H), 7.97 (s, 1H), 7.37 (d, *J=* 8.5 Hz, 1H), 4.59-4.51 (m, 1H), 4.45-4.22 (m, 1H), 4.21-4.12 (m, 1H), 3.77-3.60 (m, 3H), 2.06-1.96 (m, 2H), 1.83-1.61 (m, 4H), 1.31 (d, *J* = 6.5 Hz, 3H).

The compound **182** was separated by SFC (column: DAICEL CHIRALCEL OJ (250 mm * 30 mm, 10 um); mobile phases: Phase A of carbon dioxide, and Phase B of 0.05% diethylamine/ethanol; holding 50% of Phase B; flow rate: 80 mL/min) to afford compounds: a chiral monomer compound (compound **183)** with a short peak time and a chiral monomer compound (compound **184)** with a long peak time

### Embodiment 183. The chiral monomer compound (compound 183) with a short peak time after SFC separation of the compound 182

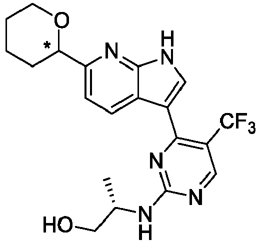

The compound **183** (26.62 mg, white solid). LCMS (ESI): [M+H]⁺= 422.2;
SFC analysis (column: Chiralcel OJ-3 (100 mm * 4.6 mm, 3 um); mobile phases: Phase A of carbon dioxide, and Phase B of 0.05% diethylamine/ethanol; gradient: holding Phase B at 40%; flow rate: 2.8 milliliter/minute): RT =1.017 min, de = 100%.
¹H NMR (400 MHz, CD₃OD) δ ppm 8.88-8.66 (m, 1H), 8.53 (br s, 1H), 7.97 (s, 1H), 7.37 (d, *J=* 8.5 Hz, 1H), 4.59-4.51 (m, 1H), 4.45-4.22 (m, 1H), 4.21-4.12 (m, 1H), 3.77-3.60 (m, 3H), 2.06-1.96 (m, 2H), 1.83-1.61 (m, 4H), 1.31 (d, *J* = 6.5 Hz, 3H)

### Embodiment 184. The chiral monomer compound (compound 184) with a long peak time after SFC separation of the compound 182

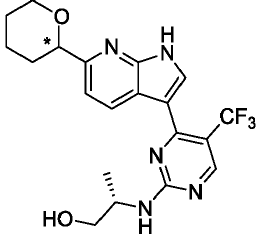

The compound **184** (23.32 mg, white solid). LCMS (ESI): [M+H]⁺= 422.2;
SFC analysis (column: Chiralcel OJ-3 (100 mm * 4.6 mm, 3 um); mobile phases: Phase A of carbon dioxide, and Phase B of 0.05% diethylamine/ethanol; gradient: holding Phase B at 40%; flow rate: 2.8 milliliter/minute): RT =2.833 min, de = 98.30%.

¹H NMR (400 MHz, CD₃OD) δ ppm 8.88-8.66 (m, 1H), 8.53 (br s, 1H), 7.97 (s, 1H), 7.37 (d, *J=* 8.5 Hz, 1H), 4.59-4.51 (m, 1H), 4.45-4.22 (m, 1H), 4.21-4.12 (m, 1H), 3.77-3.60 (m, 3H), 2.06-1.96 (m, 2H), 1.83-1.61 (m, 4H), 1.31 (d, *J* = 6.5 Hz, 3H)

### Embodiment 185. (S)-2-((4-(6-(tetrahydro-2H-pyran-4-yl)-1H-pyrrolo[2,3-b]pyridin-3-yl)-5-(trifluoromethyl) pyrimidin-2-yl) amino)propan-1-ol (compound 185)

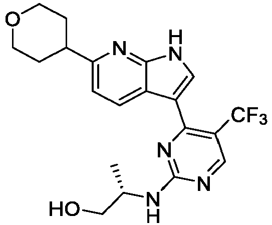

The compound **185** (23.84 mg, white solid). LCMS (ESI): [M+H]⁺= 422.2.
¹H NMR (400 MHz, CD₃OD) δ ppm 8.70 (br s, 1H), 8.52 (br s, 1H), 7.94 (s, 1H), 7.20 (br d, *J=* 8.0 Hz, 1H), 4.37 (br s, 1H), 4.10 (dd, *J=* 3.3, 11.3 Hz, 2H), 3.71-3.60 (m, 4H), 3.08 (tt, *J* = 3.7, 11.7 Hz, 1H), 2.07-1.95 (m, 2H), 1.94-1.87 (m, 2H), 1.32 (d, *J* = 6.8 Hz, 3H)

### Embodiment 186. (S)-(3-(2-(((1-hydroxypropan-2-yl)amino)pyrimidin-4-yl)-1H-indole-7-yl)dimethylphosphine oxide (compound 186)

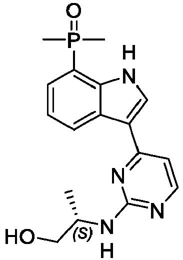

### Step 1: 7-bromo-3-(2-chloropyrimidin-4-yl)-1H-indole

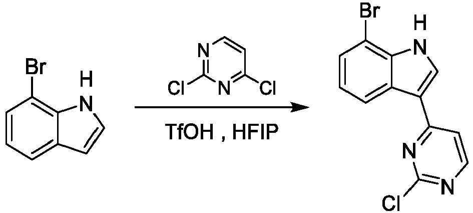

7-bromo-1H-indole (0.90 g, 4.59 mmol) and 2,4-dichloropyrimidine (1.03 g, 6.89 mmol) were dissolved in hexafluoroisopropanol (7 mL), and trifluoromethanesulfonic acid (450 uL, 5.05 mmol) was added dropwise at 0°C. The reaction solution was reacted for 16 hours at 60°C, ethyl acetate (7 mL) was added for beating after cooling to room temperature, and filtration was carried out; and the solid was dried in vacuum to afford a compound 7-bromo-3-(2-chloropyrimidin-4-yl)-1H-indole (1.73 g, 3.90 mmol, 85% yield), which was a yellow solid. LCMS (ESI): [M+H]⁺= 308.0.

### Step 2: 7-bromo-3-(2-(2,2,2-trifluoroethoxy)pyrimidin-4-yl)-1H-indole

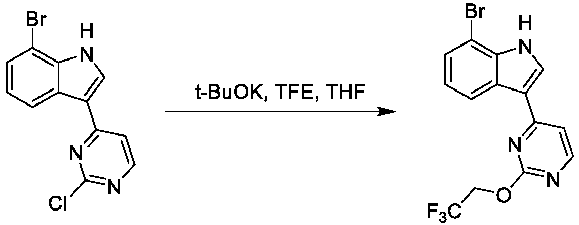

7-bromo-3-(2-chloropyrimidin-4-yl)-1H-indole (1.20 g, 2.33 mmol) and potassium tert-butoxide (2.18 g, 11.67 mmol) were added in a mixed solution of trifluoroethanol (8 mL) and tetrahydrofuran (8 mL), and the temperature was raised to 60°C for a reaction for 16 hours. The reaction solution was concentrated, the residue was washed with water and filtered, and the solid was dried in vacuum to afford a compound 7-bromo-3-(2-(2,2,2-trifluoroethoxy)pyrimidin-4-yl)-1H-indole (0.67 g, 1.79 mmol, 77% yield), which was a gray solid. LCMS (ESI): [M+H]⁺= 372.0.

### Step 3: dimethyl(3-(2-(2-(2,2,2-trifluoroethoxy)pyrimidin-4-yl)-1H-indole-7-yl)phosphine oxide

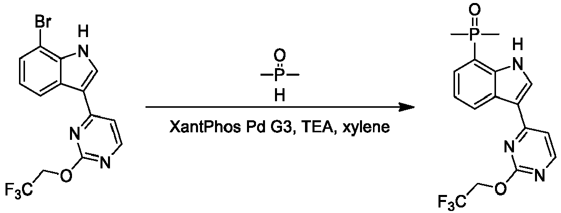

In a glove box, 7-bromo-3-(2-(2,2,2-trifluoroethoxy)pyrimidin-4-yl)-1H-indole (0.30 g, 0.81 mmol), dimethylphosphine oxide (0.19 g, 2.42 mmol) and triethylamine (340 uL, 2.42 mmol) were dissolved in xylene (2 mL), and a methanesulfonato[9,9-dimethyl-4,5-bis(diphenylphosphino)xanthene][2-amino-1,1-biphenyl]palladium(II) dichloromethane adduct (0.08 g, 0.08 mmol) was added in the reaction solution, and the temperature was raised to 140°C for a reaction for 16 hours. The reaction solution was concentrated to afford a crude compound dimethyl(3-(2-(2-(2,2,2-trifluoroethoxy)pyrimidin-4-yl)-1H-indole-7-yl)phosphine oxide (0.27 g). LCMS (ESI): [M+H]⁺= 370.1.

### Step 4: (S)-(3-(2-(((1-hydroxypropan-2-yl)amino)pyrimidin-4-yl)-1H-indole-7-yl)dimethylphosphine oxide e

Dimethyl(3-(2-(2-(2,2,2-trifluoroethoxy)pyrimidin-4-yl)-1H-indole-7-yl)phosphine oxide (140 mg, 0.04 mmol) and (S)-2-amino-1-propanol (1 mL) were added in a reaction flask, and the temperature was raised to 100°C for a reaction for 2 hours. The residue was purified by preparative HPLC to afford (S)-(3-(2-(((1-hydroxypropan-2-yl)amino)pyrimidin-4-yl)-1H-indole-7-yl)dimethylphosphine oxide (5.03 mg, 15 umol, 38% yield), which was an umber solid. LCMS (ESI): [M+H]⁺= 345.0.

¹H NMR (400 MHz, CD₃OD) δ ppm 8.82 (d, *J=* 8.1 Hz, 1H), 8.24 (s, 1H), 8.13 (br s, 1H), 7.61-7.44 (m, 1H), 7.38 (t, *J* = 7.3 Hz, 1H), 7.11 (d, *J* = 5.6 Hz, 1H), 4.29 (br s, 1H), 3.71 (t, *J* = 6.0 Hz, 2H), 1.93 (d, *J* = 13.4 Hz, 6H), 1.35 (d, *J* = 6.6 Hz, 3H).

### Embodiments 187 and 188. (3-(2-(cis-4,4-difluoro-2-hydroxycyclopentylamino)-5-(trifluoromethyl)pyrimidin-4-yl)-1H-indole-7-yl)dimethylphosphine oxide (compound 187) and (3-(2-(trans-4,4-difluoro-2-hydroxycyclopentylamino)-5-(trifluoromethyl)pyrimidin-4-yl)-1H-indole-7-yl)dimethylphosphine oxide (compound 188)

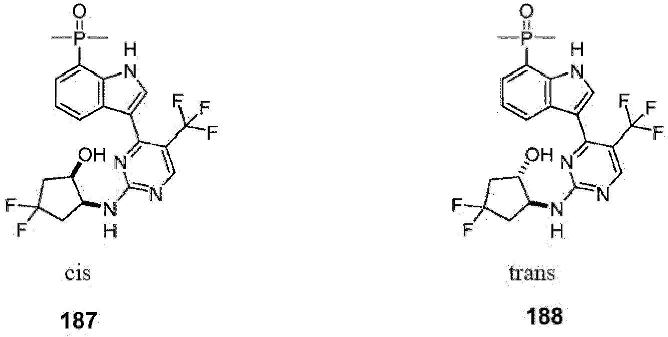

### Step 1: tert-butyl-(cyclopent-3-en-1-yloxy)-diphenylsilane

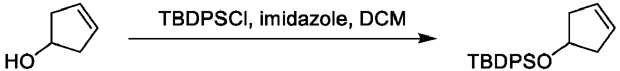

Under 0°C, tert-butyldiphenylchlorosilane (20.08 g, 73.05 mmol) and imidazole (11.13 g, 163.45 mmol) were added to a solution of 3-cyclopenten-1-ol (5.00 g, 59.44 mmol) in dichloromethane (300 mL). After the addition was completed, the mixture was stirred for 16 hours under 20^{°C}. The mixture was diluted with water (400 mL) and extracted with ethyl acetate (600 mL * 3). An organic layer was dried over sodium sulfate, filtered, and then concentrated to afford ayellow oily compound tert-butyl-(cyclopent-3-en-1-yloxy)-diphenylsilane (20.00 g, 55.80 mmol, 94 % yield).

¹H NMR (400 MHz, DMSO-*d*₆) *δ* ppm 7.71-7.57 (m, 4H), 7.49-7.35 (m, 6H), 5.66-5.59 (s, 2H), 4.52 (tt, *J* = 3.5, 6.8 Hz, 1H), 2.41 (dd, *J* = 6.7, 15.4 Hz, 2H), 2.37-2.21 (m, 2H), 1.02-0.95 (s, 9H).

### Step 2: (6-oxabicyclo[3.1.0]hex-3-yloxy)-tert-butyl-diphenylsilane

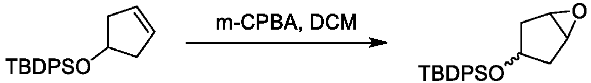

Under 0°C, m-chloroperoxybenzoic acid (85% purity, 15.64 g, 77.02 mmol) was slowly added to a solution of tert-butyl-(cyclopent-3-en-1-yloxy)-diphenylsilane (20.00 g, 55.80 mmol) in dichloromethane (300 mL). The mixture was stirred for 16 hour under 20^{°}C. The mixture was diluted with saturated sodium bicarbonate (400 mL), and extracted with dichloromethane (400 mL * 3). An organic layer was dried over anhydrous sodium sulfate, filtered, and then concentrated. The residue was purified by flash column chromatography (silica gel, tetrahydrofuran/dichloromethane with 0-5% gradient) to afford a yellow oily compound (6-oxabicyclo[3.1.0]hex-3-yloxy)-tert-butyl-diphenylsilane (20.00 g, 54.50 mmol, 94% yield). LCMS (ESI): [M+H]⁺= 338.3.

### Step 3: 2-(benzylamino)-4-((tert-butyldiphenylsilyl)oxy)cyclopentanol

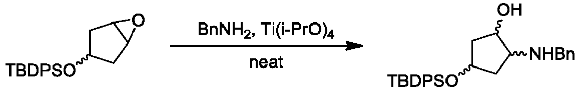

Tetraisopropyl titanate (1.86 g, 6.54 mmol) was added to a solution of (6-oxabicyclo[3.1.0]hex-3-yloxy)-tert-butyl-diphenylsilane (20.00 g, 54.50 mmol) in benzylamine (200 mL). The mixture was stirred for 16 hour under 130^{°}C. The mixture was concentrated in vacuum, and the residue was purified by flash column chromatography (silica gel, tetrahydrofuran/dichloromethane with 0-20% gradient) to afford a yellow oily compound 2-(benzylamino)-4-((tert-butyldiphenylsilyl)oxy)cyclopentanol (27.00 g, 48.50 mmol, 89% yield). LCMS (ESI): [M+H]⁺= 446.3.

### Step 4: cyclopentyl 2-(N-benzylacetamido)-4-((tert-butyldiphenylsilyl)oxy)acetate

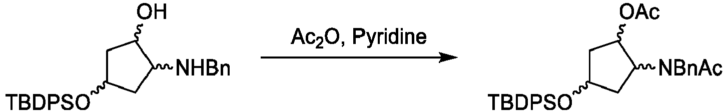

Acetic anhydride (34.45 g, 291.69 mmol) was added to a solution of 2-(benzylamino)-4-((tert-butyldiphenylsilyl)oxy)cyclopentanol (26.00 g, 43.75 mmol) in pyridine (200 mL). The mixture was stirred for 16 hour under 75^{°}C. The mixture was concentrated in vacuum, and the residue was purified by flash column chromatography (silica gel, tetrahydrofuran/dichloromethane with 0-10% gradient) to afford a yellow oily compound cyclopentyl 2-(N-benzylacetamido)-4-((tert-butyldiphenylsilyl)oxy)acetate (11.00 g, 15.75 mmol, 36% yield). LCMS (ESI): [M+H]⁺ = 530.3.

### Step 5: N-benzyl-N-(4-((tert-butyldiphenylsilyl)oxy)-2-hydroxycyclopentyl)acetamide

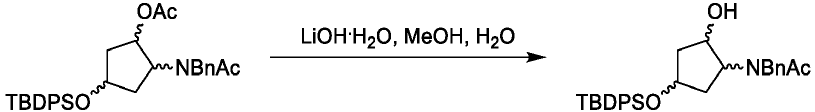

Lithium hydroxide monohydrate (0.24 g, 5.66 mmol) was added to a solution of cyclopentyl 2-(N-benzylacetamido)-4-((tert-butyldiphenylsilyl)oxy)acetate (2.00 g, 3.78 mmol) in methanol (16 mL) and water ( 4 mL). The mixture was stirred for 16 hour under 25°C. The mixture was concentrated in vacuum to afford a residue. The residue was diluted with water (5 mL), and extracted with dichloromethane (5 mL * 3). Organic phases were dried over sodium sulfate and filtered, and the filtrate was concentrated in vacuum to afford a yellow oily compound N-benzyl-N-(4-((tert-butyldiphenylsilyl)oxy)-2-hydroxycyclopentyl)acetamide crude product (1.70 g, 3.14 mmol, 83% yield). LCMS (ESI): [M+H]⁺= 488.3.

### Step 6: N-benzyl-N-(2-(benzyloxy)-4-((tert-butyldiphenylsilyl)oxy)cyclopentyl)acetamide

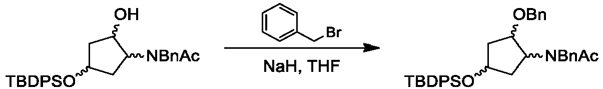

Under 0°C, sodium hydride (60% purity; 0.17 g, 4.25 mmol) was added to a solution of N-benzyl-N-(4-((tert-butyldiphenylsilyl)oxy)-2-hydroxycyclopentyl)acetamide (1.70 g, 3.14 mmol) in tetrahydrofuran ( 40 mL), and stirring was carried out for 15 minutes. Then, benzyl bromide (1.34 g, 7.84 mmol) was added. The mixture was stirred for 16 hours under 25°C. The mixture was diluted with an aqueous ammonium chloride solution (10 mL), and extracted with tetrahydrofuran (20 mL * 3). Organic phases were dried over sodium sulfate and filtered, and the filtrate was concentrated in vacuum to afford a crude compound N-benzyl-N-(2-(benzyloxy)-4-((tert-butyldiphenylsilyl)oxy)cyclopentyl)acetamide (2.50 g), which was a yellow oily liquid. LCMS (ESI): [M+H]⁺= 578.4.

### Step 7: N-benzyl-N-(2-(benzyloxy)-4-hydroxycyclopentyl)acetamide

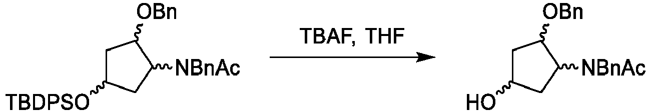

Tetrabutylammonium fluoride (5.94 g, 22.71 mmol) was added to a solution of N-benzyl-N-(2-(benzyloxy)-4-((tert-butyldiphenylsilyl)oxy)cyclopentyl)acetamide (2.50 g, 4.33 mmol) in tetrahydrofuran (50 mL). The mixture was stirred for 16 hour under 25°C. The mixture was diluted with water (50 mL), and extracted with dichloromethane (50 mL * 3). An organic layer was concentrated to afford a residue. The residue was purified by flash column chromatography (silica gel, tetrahydrofuran/dichloromethane with 0-18% gradient) to afford a colorless oily N-benzyl-N-(2-(benzyloxy)-4-hydroxycyclopentyl)acetamide (0.78 g, 2.30 mmol, 53% yield). LCMS (ESI): [M+H]⁺ = 340.2.

### Step 8: N-benzyl-N-(2-(benzyloxy)-4-oxocyclopentyl)acetamide

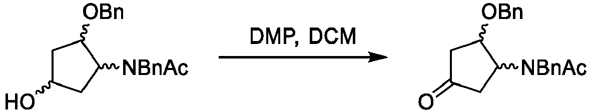

Under 0°C, Dess-Martin Periodinane (1.40 g, 3.23 mmol) was added to a solution of N-benzyl-N-(2-(benzyloxy)-4-hydroxycyclopentyl)acetamide (783 mg, 2.30 mmol) in dichloromethane (10 mL). The mixture was stirred for 16 hour under 25°C. The residue was purified by flash column chromatography (silica gel, tetrahydrofuran/dichloromethane with 0-8% gradient) to afford a yellow oily compound N-benzyl-N-(2-(benzyloxy)-4-oxocyclopentyl)acetamide (521 mg, 1.54 mmol, 67% yield). LCMS (ESI): [M+H]⁺= 338.2.

### Step 9: N-benzyl-N-(2-(benzyloxy)-4,4-difluorocyclopentyl)acetamide

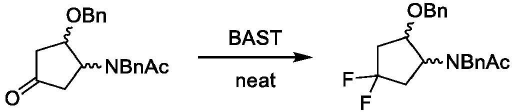

Under 0°C, N-benzyl-N-(2-(benzyloxy)-4-oxocyclopentyl)acetamide (400 mg, 1.19 mmol) was added in bis(2-methoxyethyl)aminosulfur trifluoride (4 mL). The mixture was stirred for 16 hour under 25°C. The residue was purified by flash column chromatography (silica gel, tetrahydrofuran/dichloromethane with 0-10% gradient) to afford an umber oily compound N-benzyl-N-(2-(benzyloxy)-4,4-difluorocyclopentyl)acetamide (220 mg, 0.61 mmol, 51% yield). LCMS (ESI): [M+H]⁺ =360.2.

### Step 10: 2-(benzylamino)-4,4-difluorocyclopentanol

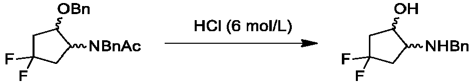

A solution of N-benzyl-N-(2-(benzyloxy)-4,4-difluorocyclopentyl)acetamide (220 mg, 0.61 mmol) in hydrochloric acid (6 M, 6 mL) was stirred for 16 hours under 100°C. The mixture was washed with ethyl acetate (1 mL), and organic phases were extracted with water (1 mL * 2). Combined aqueous phases were concentrated in vacuum to afford a crude compound 2-(benzylamino)-4,4-difluorocyclopentanol (200 mg), which was a white solid. LCMS (ESI): [M+H]⁺= 228.1.

### Step 11: 2-amino-4,4-difluorocyclopentanol

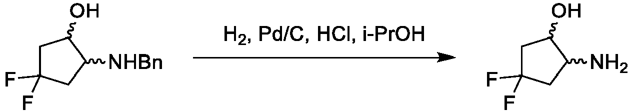

Dry palladium-on-carbon (10% content, 47 mg) and hydrochloric acid (6 M, 73 uL, 0.44 mmol) were added to a solution of 2-(benzylamino)-4,4-difluorocyclopentanol (50% purity, 200 mg, 0.44 mmol) in isopropanol (10 mL). In a 50 psi hydrogen atmosphere, the reaction proceeded with stirring for 16 hours under 50^{°}C. After the reaction was completed, the dry palladium-on-carbon was filtered and recovered. The filtrate was concentrated in vacuum to afford a crude compound 2-amino-4,4-difluorocyclopentanol (50 mg), which was a white solid. LCMS (ESI): [M+H]⁺ = 138.1.

### Step 12: (3-(2-(trans-4,4-difluoro-2-hydroxycyclopentylamino)-5-(trifluoromethyl) pyrimidin-4-yl)-1H-indole-7-yl)dimethylphosphine oxide and (3-(2-(cis-4,4-difluoro-2-hydroxycyclopentylamino)-5-(trifluoromethyl)pyrimidin-4-yl)-1H-indole-7-yl)dimethylphosphine oxide

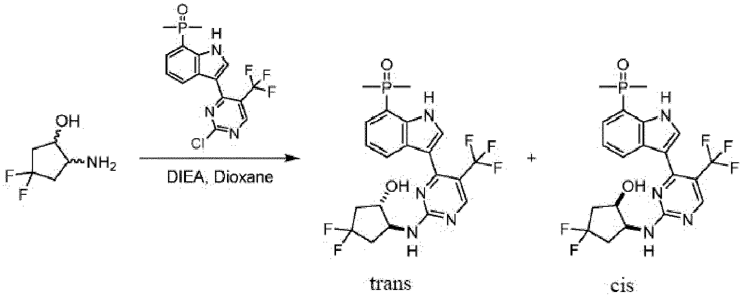

Diisopropylethylamine (8.00 g, 61.92 mmol) was added to a solution of (3-(2-chloro-5-(trifluoromethyl)pyrimidin-4-yl)-1H-indole-7-yl)dimethylphosphine oxide (2.50 g, 6.69 mmol) and 2-amino-4,4-difluorocyclopentanol (50% purity, 1.80 g, 6.56 mmol) in dioxane (20 mL). The reaction proceeded with stirring for 12 hours under 100°C. The mixture was concentrated in vacuum to afford a residue. The residue was purified by flash column chromatography (C18, acetonitrile/water with 0-33% gradient) to afford a peak 1: (3-(2-(trans-4,4-difluoro-2-hydroxycyclopentylamino)-5-(trifluoromethyl)pyrimidin-4-yl)-1H-indole-7-yl)dimethylphosphine oxide(710 mg, 1.50 mmol, 23% yield) and a peak 2: (3-(2-(cis-4,4-difluoro-2-hydroxycyclopentylamino)-5-(trifluoromethyl)pyrimidin-4-yl)-1H-indole-7-yl)dimethylphosphine oxide (400 mg, 0.84 mmol, 13% yield), both of which were white solid compounds. Through 2D-NMR analysis, the peak 1 was a trans isomer and the peak 2 was a cis isomer. Through two-dimensional NMR analysis, the peak 1 was a trans isomer **188,** and the peak 2 was a cis isomer **187.**

The peak 2: the cis compound **187:** LCMS (ESI): [M+H]⁺= 475.1.
¹H NMR (400 MHz, CD₃OD) δ ppm 8.58 (m, 2H), 7.99 (br s, 1H), 7.51 (dd, *J* = 6.9, 13.7 Hz, 1H), 7.34 (dt, *J=* 2.5, 7.7 Hz, 1H), 4.62 (m, 1H), 4.46 (br s, 1H), 2.65-2.23 (m, 4H), 1.93 (d, *J=* 13.3 Hz, 6H)

The peak 1: the trans compound **188:** LCMS (ESI): [M+H]⁺= 475.1.
H NMR (400 MHz, CD₃OD) δ ppm 8.61 (br s, 2H), 7.98 (s, 1H), 7.51 (dd, *J* = 7.3, 13.6 Hz, 1H), 7.34 (dt, *J* = 2.5, 7.7 Hz, 1H), 4.67 - 4.41 (m, 1H), 4.32 (q, *J* = 7.4 Hz, 1H), 2.83 - 2.55 (m, 2H), 2.24 - 2.06 (m, 2H), 1.93 (d, *J* = 13.3 Hz, 6H)

The cis compound **187** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 40%; flow rate: 80 milliliter/minute) to afford a chiral monomer compound **189** with a short peak time and a chiral monomer compound **190** with a long peak time.

### Embodiment 189. The chiral monomer compound (compound 189) with a short peak time after SFC separation of the compound 187

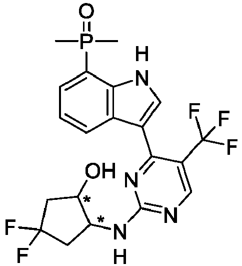

The compound **189** (16.60 mg, white solid). LCMS (ESI): [M+H]⁺ = 475.2;
SFC analysis (column: Chiralpak AD-3 (50 mm * 4.6 mm, 3 um); mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT =1.615 min, ee = 99.78%.

¹H NMR (400 MHz, CD₃OD) δ ppm 8.59 (br s, 2H), 7.98 (br s, 1H), 7.51 (dd, *J* = 7.3, 13.6 Hz, 1H), 7.34 (m, 1H), 4.62 (s, 1H), 4.46 (br s, 1H), 2.67-2.24 (m, 4H), 1.93 (d, *J=* 13.6 Hz, 6H).

### Embodiment 190, a chiral monomer compound (compound 190) with a short peak time after SFC separation of the compound 187

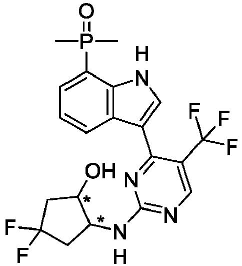

The compound **190** (18.09 mg, white solid). LCMS (ESI): [M+H]⁺ = 475.2;
SFC analysis (column: Chiralpak AD-3 (50 mm * 4.6 mm, 3 um); mobile phases: Phase A of carbon dioxide, and Phase B of 0.05% diethylamine/ethanol; gradient: as for Phase B, from 5% to 40% in 2 minutes, holding Phase B at 40% for 1.2 minutes, and then holding Phase B at 5% for 0.8 minutes; flow rate: 4 milliliter/minute): RT =1.917 min, ee = 99.74%.

¹H NMR (400 MHz, CD₃OD) δ ppm 8.59 (br s, 2H), 7.98 (br s, 1H), 7.51 (dd, *J* = 7.3, 13.6 Hz, 1H), 7.34 (m, 1H), 4.62 (s, 1H), 4.46 (br s, 1H), 2.67-2.24 (m, 4H), 1.93 (d, *J=* 13.6 Hz, 6H).

The trans compound **188** was separated by SFC (column: DAICEL CHIRALPAK AD (250 mm * 30 mm, 10 um); mobile phases: Phase A of carbon dioxide, and Phase B of 0.1% ammonia water/ethanol; holding Phase B at 35%; flow rate: 60 milliliter/minute) to afford a chiral monomer compound **191** with a short peak time and a chiral monomer compound **192** with a long peak time.

### Embodiment 191, a chiral monomer compound (compound 191) with a short peak time after SFC separation of the compound 188

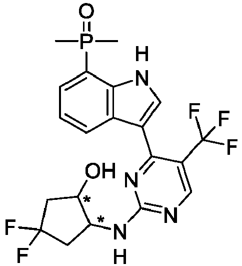

The compound **191** (138.93 mg, white solid). LCMS (ESI): [M+H]⁺ = 475.1;
SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT =5.293 min, ee = 100%.

¹H NMR (400 MHz, DMSO-*d*₆) δ ppm 11.58 (br s, 1H), 8.72-8.45 (m, 2H), 8.16-8.07 (m, 1H), 7.95 (br d, *J=* 17.8 Hz, 1H), 7.51 (dd, *J* = 7.2, 12.9 Hz, 1H), 7.27 (br d, *J* = 7.3 Hz, 1H), 5.45 (br s, 1H), 4.47-4.30 (m, 1H), 4.30-4.21 (m, 1H), 2.71-2.54 (m, 2H), 2.23-1.96 (m, 2H), 1.82 (d, *J* = 13.3 Hz, 6H).

### Embodiment 192, a chiral monomer compound (compound 192) with a short peak time after SFC separation of the compound 188

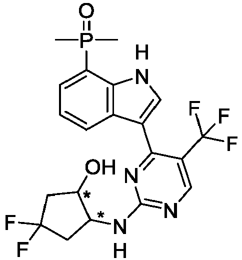

The compound **192** (138.55 mg, white solid). LCMS (ESI): [M+H]⁺ = 475.1;
SFC analysis (column: Chiralpak AD-3 (150 mm * 4.6 mm, 3 um); mobile phases: Phase A of carbon dioxide, and Phase B of 0.05% diethylamine/ethanol; gradient: as for Phase B, from 5% to 40% in 5.5 minutes, holding Phase B at 40% for 3 minutes, and then holding Phase B at 5% for 1.5 minutes; flow rate: 2.5 milliliter/minute): RT =5.960 min, ee = 100%.

¹H NMR (400 MHz, DMSO-*d₆*) δ ppm 11.54 (br s, 1H), 8.70 - 8.47 (m, 2H), 8.12 (br s, 1H), 7.94 (br d, *J* = 17.3 Hz, 1H), 7.51 (dd, *J* = 7.2, 12.7 Hz, 1H), 7.27 (br d, *J* = 7.5 Hz, 1H), 5.45 (br s, 1H), 4.49 - 4.19 (m, 2H), 2.70 - 2.55 (m, 2H), 2.10 (m, 2H), 1.82 (d, *J* = 13.3 Hz, 6H).

### Embodiment 193 Synthesis of (S)-(3-(2-((1-hydroxypropan-2-yl)amino)-5-(trifluoromethyl)pyrimidin-4-yl)-1H-indazol-7-yl)dimethylphosphine oxide (compound 193)

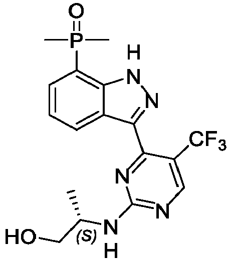

### Step 1: 7-bromo-3-iodo-1H-indazole

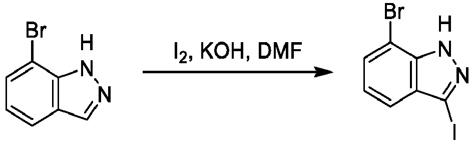

Under 0°C, potassium hydroxide (3.56 g, 63.44 mmol) was added to a solution of 7-bromo-1H-indazole (5.00 g, 25.38 mmol) in dimethylformamide (25 mL), and then a solution of elementary iodine (7.73 g, 30.45 mmol) in dimethylformamide (25 mL) was added dropwise. The reaction proceeded with stirring for 16 hours under 25°C. The mixture was diluted with water (80 mL) and extracted with dichloromethane (80 mL * 4). Organic phases were concentrated in vacuum to afford a residue, and the residue was subjected to beating with water (20 mL) and filtered, and the solid was dried in vacuum to afford a yellow solid 7-bromo-3-iodo-1H-indazole (7.95 g, 23.35 mmol, 92% yield). LCMS (ESI): [M+H]⁺= 324.8;

### Step 2: 7-bromo-3-(2-chloro-5-(trifluoromethyl)pyrimidin-4-yl)-1H-indazole

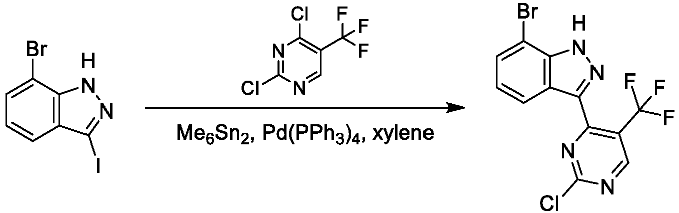

Under a nitrogen environment, tetrakis(triphenylphosphine)palladium (1.36 g, 1.18 mmol) was added to a solution of 7-bromo-3-iodo-1H-indazole (4.00 g, 11.77 mmol), 2,4-dichloro-5-(trifluoromethyl)pyrimidine (3.32 g, 15.30 mmol) and hexamethyldistannane (5.01 g, 15.30 mmol) in xylene (40 mL). The reaction proceeded with stirring for 16 hours under 100°C. The mixture was diluted with water (100 mL) and extracted with dichloromethane (100 mL * 3). Organic phases were concentrated in vacuum to afford a residue. The residue was purified by flash column chromatography (silica gel, ethyl acetate/petroleum ether with 0-15% gradient) to afford a yellow solid compound 7-bromo-3-(2-chloro-5-(trifluoromethyl)pyrimidin-4-yl)-1H-indazole (0.30 g, 0.82 mmol, 7% yield). LCMS (ESI): [M+H]⁺= 378.9;

### Step 3: (S)-2-((4-(7-bromo-1H-indazol-3-yl)-5-(trifluoromethyl)pyrimidin-2-yl)amino)propan-1 -ol

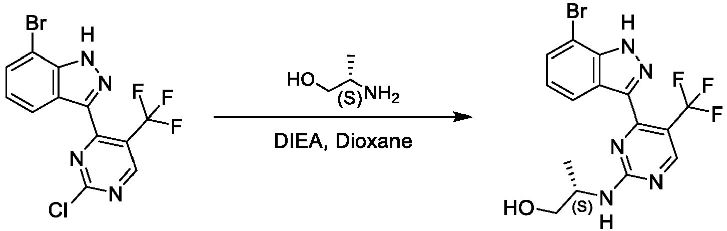

Diisopropylethylamine (575 mg, 4.45 mmol) was added to a solution of 7-bromo-3-(2-chloro-5-(trifluoromethyl)pyrimidin-4-yl)-1H-indazole (280 mg, 0.74 mmol) and (S)-2-amino-propan-1-ol (836 mg, 11.12 mmol) in 1,4-dioxane (3 mL). The reaction proceeded with stirring for 16 hours under 100°C. The mixture was diluted with water (5 mL) and extracted with ethyl acetate (5 mL * 3). Organic phases were dried over anhydrous magnesium sulfate and filtered, and the filtrate was concentrated in vacuum to afford a crude compound (S)-2-((4-(7-bromo-1H-indazol-3-yl)-5-(trifluoromethyl)pyrimidin-2-yl)amino)propan-1-ol (464 mg), which was a yellow oily liquid. LCMS (ESI): [M+H]⁺ = 416.0;

### Step 4: (S)-(3-(2-((1-hydroxypropan-2-yl)amino)-5-(trifluoromethyl)pyrimidin-4-yl)-1H-indazol-7-yl)dimethylphosphine oxide

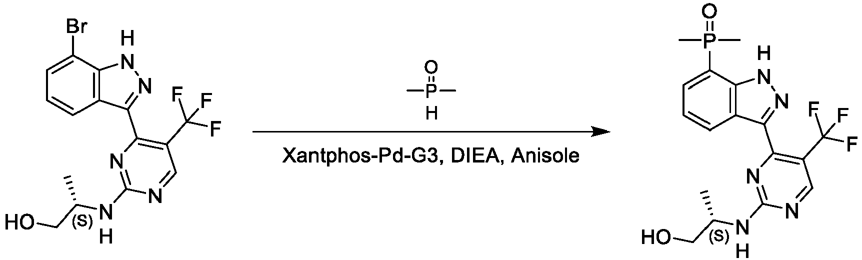

Diisopropylethylamine (311 mg, 2.40 mmol) and dimethylphosphine oxide (74 mg, 0.96 mmol) were added to a solution of (S)-2-((4-(7-bromo-1H-indazol-3-yl)-5-(trifluoromethyl)pyrimidin-2-yl)amino)propan-1-ol (200 mg, 0.48 mmol) in anisole (3 mL). Then, under the nitrogen environment, a methanesulfonato[9,9-dimethyl-4,5-bis(diphenylphosphino)xanthene][2-amino-1, 1-biphenyl]palladium(II) dichloromethane adduct (46 mg, 0.05 mmol) was added. The mixture was stirred for 6 hours at 140°C. The mixture was an umber suspension. The mixture was diluted with water (2 mL) and extracted with ethyl acetate (2 mL * 3). Organic phases were concentrated in vacuum to afford a residue, and the residue was purified by preparative HPLC to afford a white solid compound (S)-(3-(2-((1-hydroxypropan-2-yl)amino)-5-(trifluoromethyl)pyrimidin-4-yl)-1H-indazol-7-yl)dimethylphosphine oxide (46.05 mg, 0.11 mmol, 23% yield).

LCMS (ESI): [M+H]⁺= 414.1.

¹H NMR (400 MHz, DMSO-*d₆*) δ ppm 13.71 (br s, 1H), 8.72 -8.45 (m, 2H), 8.02-7.75 (m, 2H), 7.42 (br t, *J* = 7.4 Hz, 1H), 4.80 (t, *J* = 5.5 Hz, 1H), 4.22-4.08 (m, 1H), 3.54 (td, *J* = 5.4, 10.6 Hz, 1H), 3.43 (br d, *J* = 5.3 Hz, 1H), 1.86 (d, *J* = 13.3 Hz, 6H), 1.21 (d, *J* = 6.5 Hz, 3H).

### Embodiment 194 Synthesis of (3-(2-((1,3-dihydroxypropan-2-yl)amino)-5-(trifluoromethyl)pyrimidin-4-yl)-1H-indole-7-yl)dimethylphosphine oxide (compound 194)

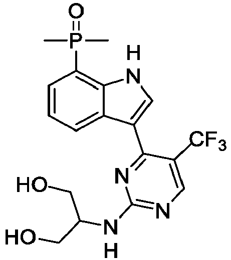

2-aminopropan-1,3-diol (37 mg, 0.40 mmol) and N,N-diisopropylethylamine (208 mg, 1.61 mmol) were added to a solution of (3-(2-chloro-5-(trifluoromethyl)pyrimidin-4-yl)-1H-indole-7-yl)dimethylphosphine oxide (100 mg, 0.27 mmol) in dioxane (1 mL). The mixture was stirred for 2 hours under 100°C. The reaction solution was added with an aqueous solution (2 mL), and extracted with ethyl acetate (3 * 2 mL). Organic phases were concentrated under reduced pressure, and the residue was purified by preparative HPLC to afford a white solid compound (3-(2-((1,3-dihydroxypropan-2-yl)amino)-5-(trifluoromethyl)pyrimidin-4-yl)-1H-indole-7-yl)dimethylphosphine oxide (59.87 mg, 0.14 mmol, 52% yield). LCMS (ESI): [M+H]⁺ = 429.1.

¹H NMR (400 MHz, DMSO-*d₆*) δ ppm 11.55 (br s, 1H), 8.73 - 8.40 (m, 2H), 8.01 - 7.85 (m, 1H), 7.56 - 7.39 (m, 2H), 7.27 (br t, *J*=6.8 Hz, 1H), 4.72 (br s, 2H), 4.21 - 4.01 (m, 1H), 3.58 (br s, 4H), 1.82 (d, *J*=13.6 Hz, 6H)

### Embodiment 195. (S)-(6-fluoro-3-(2-(((1-hydroxypropan-2-yl)amino)-5-(trifluoromethyl)pyrimidin-4-yl)-1H-indole-7-yl)dimethylphosphine oxide (compound 195)

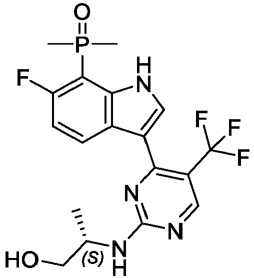

### Step 1: 7-bromo-6-fluoro-1H-indole

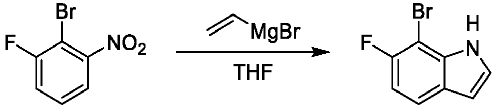

Vinylmagnesium bromide (1 M tetrahydrofuran solution, 82 mL, 82 mmol) was added to a solution of a compound 2-bromo-1-fluoro-3-nitrobenzene (6.00 g, 27.27 mmol) in tetrahydrofuran (36 mL) under -78°C. The reaction proceeded with stirring for 12 hours under 25°C. The reaction solution was poured into a saturated ammonium chloride solution (100 mL) and extracted with ethyl acetate (100 mL * 3), combined organic phases were dried over anhydrous sodium sulfate and filtered, and the filtrate was concentrated under reduced pressure. The residue was purified by flash column chromatography (silica gel, ethyl acetate/petroleum ether with 0-30% gradient) to afford an umber oily compound 7-bromo-6-fluoro-1H-indole (0.82 g, 3.83 mmol, 14% yield).

¹H NMR (400 MHz, CDCl₃) δ ppm 8.34 (br s, 1H), 7.56-7.48 (m, 1H), 7.27-7.25 (m, 1H), 6.97 (dd, *J* = 8.7, 9.3 Hz, 1H), 6.62 (dd, *J* = 2.2, 3.2 Hz, 1H)

### Step 2: 7-bromo-3-(2-chloro-5-(trifluoromethyl)pyrimidin-4-yl)-6-fluoro-1H-indole

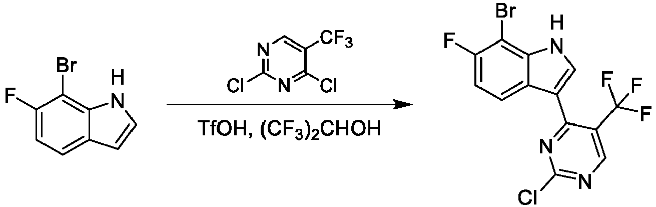

A solution of a compound 7-bromo-6-fluoro-1H-indole (810 mg, 3.78 mmol) and 2,4-dichloro-5-(trifluoromethyl)pyrimidine (985 mg, 4.54 mmol) in hexafluoroisopropanol (8.1 mL) was cooled to 0°C, trifluoromethanesulfonic acid (370 uL, 4.16 mmol) was added, and then stirring was carried out for 12 hours under nitrogen protection at 60°C. The reaction solution was subjected to beating with ethyl acetate (8 mL) and filtered, and the solid was dried in vacuum to afford a white solid compound 7-bromo-3-(2-chloro-5-(trifluoromethyl)pyrimidin-4-yl)-6-fluoro-1H-indole (1270 mg, 3.22 mmol, 85% yield). LCMS (ESI): [M+H]⁺ = 395.9.

### Step 3: 7-bromo-6-fluoro-3-(2-(2,2,2-trifluoroethoxy)-5-(trifluoromethyl)pyrimidin-4-yl)-1H-indole

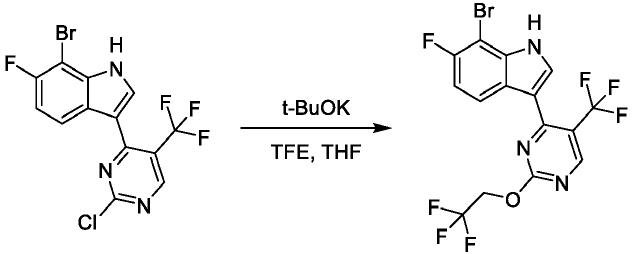

A solution of a compound 7-bromo-3-(2-chloro-5-(trifluoromethyl)pyrimidin-4-yl)-6-fluoro-1H-indole (1250 mg, 3.17 mmol) and trifluoroethanol (12.5 mL) in tetrahydrofuran (25 mL) was cooled to 0°C, potassium tert-butoxide (1067 mg, 9.50 mmol) was added, and then stirring was carried out for 12 hours under nitrogen protection at 60°C. The reaction solution was concentrated under reduced pressure. The residue was subjected to beating with water (2 mL) and filtered, and the solid was dried in vacuum to afford a white solid compound 7-bromo-6-fluoro-3-(2-(2,2,2-trifluoroethoxy)-5-(trifluoromethyl)pyrimidin-4-yl)-1H-indole (1080 mg, 2.36 mmol, 74% yield). LCMS (ESI): [M+H]⁺ = 459.9.

### Step 4: (6-fluoro-3-(2-(2,2,2-trifluoroethoxy)-5-(trifluoromethyl)pyrimidin-4-yl)-1H-indole-7-yl)dimethylphosphine oxide

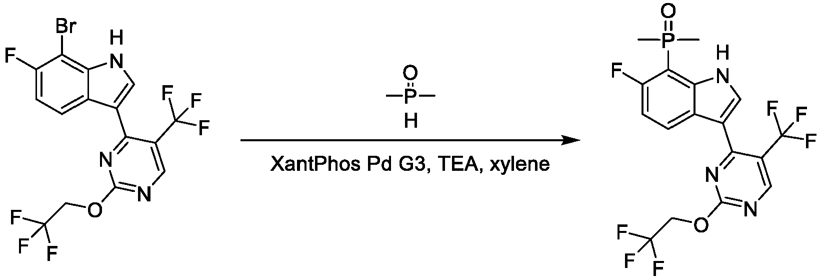

In a glove box, a methanesulfonato[9,9-dimethyl-4,5-bis(diphenylphosphino)xanthene][2-amino-1,1-biphenyl]palladium(II) dichloromethane adduct (158 mg, 0.15 mmol) was added to a solution of a compound 7-bromo-6-fluoro-3-(2-(2,2,2-trifluoroethoxy)-5-(trifluoromethyl)pyrimidin-4-yl)-1H-indole (700 mg, 1.53 mmol), triethylamine (760 uL, 4.58 mmol) and dimethylphosphine oxide (235 mg, 3.06 mmol) in xylene (14 mL). The reaction proceeded with stirring for 12 hours under nitrogen under 140°C. Cooling to room temperature was carried out, and the reaction solution was concentrated under reduced pressure. The residue was subjected to beating with water (2 mL) plus methyl tert-butyl ether (2 mL), and filtered, and the solid was dried in vacuum to afford a yellow solid compound (6-fluoro-3-(2-(2,2,2-trifluoroethoxy)-5-(trifluoromethyl)pyrimidin-4-yl)-1H-indole-7-yl)dimethylphosphine oxide (88% purity, 710 mg, 1.37 mmol, 89% yield). LCMS (ESI): [M+H]⁺ = 456.1.

### Step 5: (S)-(6-fluoro-3-(2-((1-hydroxypropan-2-yl)amino)-5-(trifluoromethyl)pyrimidin-4-yl)-1H-indole-7-yl)dimethylphosphine oxide

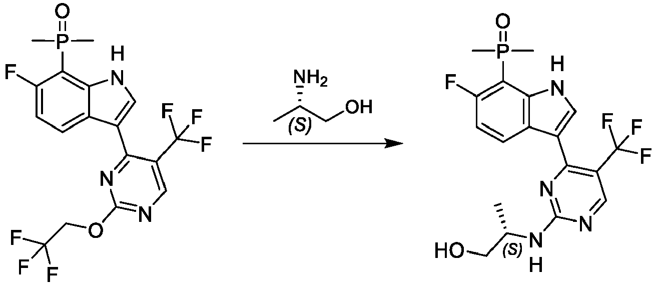

A compound (6-fluoro-3-(2-(2,2,2-trifluoroethoxy)-5-(trifluoromethyl)pyrimidin-4-yl)-1H-indole-7-yl)dimethylphosphine oxide (88% purity, 710 mg, 1.37 mmol) was dissolved in (S)-2-aminopropan-1-ol (1.2 g, 15.6 mmol). The reaction proceeded with stirring for 2 hours under nitrogen protection at 100°C. The mixture was purified by flash column chromatography (C18, acetonitrile/water with 0-50% gradient) to afford a white solid compound (S)-(6-fluoro-3-(2-((1-hydroxypropan-2-yl)amino)-5-(trifluoromethyl)pyrimidin-4-yl)-1H-indole-7-yl)dimethylphosphine oxide (360 mg, 0.84 mmol, 61% yield). LCMS (ESI): [M+H]⁺ = 431.1
¹H NMR (400 MHz, CD₃OD) δ ppm 8.54 (br s, 2H), 7.96 (s, 1H), 7.21-6.95 (m, 1H), 4.30 (br s, 1H), 3.75-3.56 (m, 2H), 1.97 (dd, *J* = 1.0, 13.9 Hz, 6H), 1.30 (d, *J* = 6.7 Hz, 3H).

A control compound: 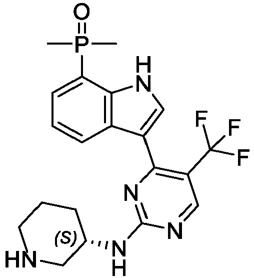 (compound 353 from WO2018013867)

### Effect Embodiment 1: In-vitro enzymatic inhibitory activity testing of compounds CDK7, CDK2, CDK9, and CDK12

An assay was carried out in a U-bottom 384-well plate (coming, 4512#), and the reaction temperature was 27°C. CDK7/CyclinH was diluted in an assay buffer (20 mM MES PH 6.75, 0.01% Tween 20, 50 ug/mL BSA, 6 mM MgCl2) to afford a corresponding enzyme solution with the 2.4 × concentration. CDK2/CyclinE1 was diluted in an assay buffer (20 mM MES PH 6.75, 0.01% Tween 20, 50 ug/mL BSA, 6 mM MgCl2) to afford a corresponding enzyme solution with the 2.4 × concentration. CDK9/CyclinT1 was diluted in an assay buffer (20 mM MES PH 6.75, 0.01% Tween 20, 50 ug/mL BSA, 10 mM MgCl2) to afford a corresponding enzyme solution with the 2.4 × concentration. CDK12/CyclinK was diluted in an assay buffer (80 mM MES PH 6.5, 0.01% Tween 20, 50 ug/mL BSA, 10 mM MgCl2) to afford a corresponding enzyme solution with the 2.4 × concentration. A compound was dissolved in dimethyl sulfoxide (DMSO) at a concentration of 10 mM. During use, the compound was diluted with DMSO into 10 concentration gradients from 25 nM to 500 uM, and respectively diluted by 8.3 folds in the assay buffer to afford a compound solution with a 6 × concentration. A polypeptide substrate and ATP were diluted in the assay buffer to afford a polypeptide substrate and ATP mixed solution with a 2.4 × concentration. 2 ul of a test compound solution was mixed with 5 ul of the enzyme solution; after incubation for 10 min, 5 ul of the polypeptide substrate and ATP mixed solution was added; incubation was carried out for 180 min at 27°C; and then, the reaction was terminated by adding 4 uL of EDTA with a concentration of 120 mM to each sample. The compound solution was replaced with the assay buffer containing 20 uM staurosporine to serve as a 100% inhibition control, the compound solution was replaced with the DMSO to serve as a 0% inhibition control, and there were at least 2 parallel controls for each test. Specifically, a CDK7/cyclinH/MAT1 complex (6 nM) and a "5-FAMCD K 7 t i d e" peptide substrate (2 µM, synthetic fluorophore-labeled peptide with the following sequence: 5-FAM -YSPTSPSYSPTSPSYSPTSPSKKKK, wherein "5-FAM" refers to 5-carboxyfluorescein) were used for a CDK7 inhibition assay. A CDK9/cyclinT1 complex (8 nM) and a "5-FAM-CDK9tide" peptide substrate (2 µM, synthetic fluorophore-labeled peptide with the following sequence: 5-FAM-GSRTPMY-NH2, wherein 5-FAM was as defined above and NH2 represented C-terminal amide) were used for a CDK9 inhibition assay. A CDK12 (aa686-1082)/cyclinK complex (50 nM) and "5-FAM-CDK9tide" (2 µM) as defined above were used for a CDK12 inhibition assay. A CDK2/cyclinE1 complex (0.5 nM) and "5-FAM-CDK7tide" (2 µM) as defined above were used for CDK2 inhibition assay.

A reaction mixture was analyzed on Caliper EZ Reader II by electrophoretic separation of a fluorogenic substrate and a phosphorylated product. Data were calculated by using GraphPad Prism version 6.0, and IC50 values were adjusted and obtained by using a nonlinear regression model of a dose response curve.

These assayed IC50 results are shown in the following Table 1, wherein "A" indicates that the calculated IC50 is less than 20 nM; "B" indicates that the calculated IC50 is 20 nM to less than 200 nM; "C" indicates that the calculated IC50 is 200 nM to less than 5 µM; "D" indicates that the calculated IC50 is 5 µM to less than 50 µM; and "E" indicates that the calculated IC50 is greater than or equal to 50 µM.

**Table 1**

| Compound number | CDK7 | CDK2 | CDK9 | CDK12 |
|---|---|---|---|---|
| Staurosporine | C | B | C | C |
| 35 | A | C | C | C |
| 36 | A | D | D | C |
| 37 | B | D | D | D |
| 38 | B | D | D | D |
| 39 | A | D | D | D |
| 40 | B | D | D | D |
| 41 | B | D | D | D |
| 42 | B | D | D | D |
| 45 | B | D | D | D |
| 46 | C | D | D | D |
| 47 | B | D | D | D |
| 48 | B | C | D | C |
| 49 | C | D | E | D |
| 50 | C | D | E | D |
| 51 | C | D | D | D |
| 52 | B | D | D | D |
| 54 | B | D | E | D |
| 55 | B | D | E | E |
| 56 | C | E | E | E |
| 57 | C | E | E | E |
| 58 | B | D | E | E |
| 59 | A | D | D | E |
| 61 | A | D | D | D |
| 62 | B | E | E | D |
| 63 | B | E | E | D |
| 64 | B | E | E | D |
| 65 | A | D | D | D |
| 66 | B | C | D | C |
| 67 | A | D | D | D |
| 68 | B | D | D | D |
| 76 | B | E | E | E |
| 77 | B | E | E | E |
| 78 | A | D | D | D |
| 79 | A | C | E | E |
| 80 | A | C | D | D |
| 81 | A | C | C | C |
| 82 | A | C | E | D |
| 83 | A | C | D | D |
| 84 | A | B | D | C |
| 85 | A | D | E | E |
| 86 | A | C | E | E |
| 87 | A | C | E | E |
| 88 | A | C | E | E |
| 89 | A | C | E | E |
| 90 | B | D | D | E |
| 91 | A | D | E | D |
| 92 | A | D | D | D |
| 93 | A | C | E | D |
| 94 | B | D | E | E |
| 95 | A | D | E | E |
| 96 | B | D | E | E |
| 97 | A | C | E | E |
| 98 | A | C | D | D |
| 99 | A | C | D | C |
| 100 | A | C | D | C |
| 101 | B | D | C | C |
| 102 | A | D | E | E |
| 103 | B | C | E | E |
| 104 | B | C | E | E |
| 105 | B | D | E | E |
| 106 | B | C | D | D |
| 108 | A | C | D | C |
| 109 | B | D | E | E |
| 110 | A | D | E | D |
| 111 | B | D | E | E |
| 112 | B | D | E | E |
| 113 | B | D | E | E |
| 114 | A | C | E | E |
| 115 | B | D | E | E |
| 116 | B | D | E | E |
| 117 | A | D | E | E |
| 118 | A | D | E | E |
| 119 | B | D | E | E |
| 121 | B | D | E | E |
| 122 | B | D | E | E |
| 124 | A | D | E | E |
| 125 | B | D | E | E |
| 126 | A | C | E | E |
| 127 | A | C | E | E |
| 128 | A | D | E | E |
| 129 | A | D | E | E |
| 130 | A | D | E | E |
| 131 | A | --- | --- | --- |
| 132 | A | --- | --- | --- |
| 133 | A | --- | --- | --- |
| 134 | A | --- | --- | --- |
| 135 | A | C | D | C |
| 136 | B | E | E | E |
| 137 | A | C | D | C |
| 139 | B | D | E | E |
| 140 | B | D | E | E |
| 141 | A | C | E | E |
| 143 | A | D | E | E |
| 144 | B | D | E | E |
| 145 | B | E | E | E |
| 146 | A | D | E | E |
| 147 | B | E | E | E |
| 149 | A | C | E | E |
| 150 | A | C | E | E |
| 151 | B | D | E | E |
| 152 | B | --- | --- | --- |
| 153 | A | C | E | E |
| 154 | A | C | D | D |
| 155 | A | C | D | D |
| 156 | B | D | D | D |
| 157 | C | C | E | E |
| 158 | A | C | E | E |
| 159 | A | D | E | E |
| 160 | B | D | E | E |
| 161 | A | D | E | E |
| 162 | B | D | E | E |
| 163 | B | D | E | E |
| 164 | A | D | E | E |
| 165 | B | D | E | E |
| 166 | B | E | E | E |
| 167 | B | E | E | E |
| 168 | A | D | E | E |
| 169 | B | --- | --- | --- |
| 170 | B | --- | --- | --- |
| 172 | B | C | E | E |
| 173 | A | D | E | E |
| 174 | A | C | D | C |
| 175 | B | D | E | E |
| 177 | A | C | D | D |
| 178 | B | D | E | E |
| 179 | A | C | E | D |
| 180 | D | E | E | E |
| 181 | B | C | E | D |
| 183 | B | --- | --- | --- |
| 184 | B | --- | --- | --- |
| 186 | D | E | E | E |
| 187 | B | --- | --- | --- |
| 188 | A | --- | --- | --- |
| 189 | B | --- | --- | --- |
| 190 | B | --- | --- | --- |
| 191 | A | C | --- | --- |
| 192 | B | D | --- | --- |
| 193 | B | --- | --- | --- |
| 194 | A | D | E | E |
| 195 | A | C | E | D |

| | | | | |
|---|---|---|---|---|
| Note: --- indicates that this test has not been carried out | | | | |

It can be seen from the above table that, through in-vitro biological activity screening, with staurosporine used as a control substance, the compounds synthesized in the present application have good inhibitory ability to CDK7 kinase and have good selectivity. The compounds are expected to be developed into drugs for regulating CDK7 kinase activity or treating CDK7-related diseases.

### Effect Embodiment 2: Cell biological activity detection

A2780 and HCC70 cells were subjected to trypsin digestion treatment, and cell suspensions were respectively transferred to 15 mL centrifuge tubes and centrifuged for 5 min at 800 rpm; supernatant was discarded, and resuspension was carried out in a fresh medium (RPMI 1640 + 10% FBS); after counting, cells were seeded into a 384-well plate at 2000/well (50 µL of 1640 + 10% FBS medium was added to the 2nd column and the 23rd column of the 384-well plate, and 50 µL of DPBS was added to the surrounding wells). Incubation was carried out overnight in an incubator (37°C, 5% CO2).

The compound was added to the well plates the next day. The compound had the highest concentration of 10 µM and was diluted at 1:4, and there were a total of 9 concentrations; and a positive compound Paclitaxel had the highest concentration of 1 µM and was diluted at 1:3, and there were a total of 9 concentrations. The DMSO content in each well was unified to 0.2%. The cell plates were centrifuged for 30 seconds at 800 rpm and placed in an incubator (37°C, 5% CO2) for incubation for 72 hours. On the fourth day, a Cyquant reagent (3X) was formulated according to kit instructions, and formulation was carried out for each 384-well plate according to the following proportions: DPBS 11.568 mL, CyQuant^{®} Direct nucleic acid stain 72 µL, and CyQuant^{®} Direct background suppressor 360 µL. After uniform mixing, the reagent was placed at room temperature for further use. The cell plates were taken out and equilibrated for 30 min at room temperature, and 25 µL of the Cyquant reagent (3X) per well was dispensed into the cells of the 384-well plates by Multi-drop, and incubation was carried out for more than 60 minutes at 37°C. Acumen plate reading (Acumen setting: an excitation wavelength of 488 nm). IC50 results were analyzed by XLFIT5 from IDBS Company.

These assay results are shown in the following Table 2, wherein "A" indicates that the calculated IC50 is less than 20 nM; "B" indicates that the calculated IC50 is 20 nM to less than 200 nM; "C" indicates that the calculated IC50 is 200 nM to less than 5 µM; "D " indicates that the calculated IC50 is 5 µM to less than 50 µM; and "E" indicates that the calculated IC50 is greater than or equal to 50 µM.

**Table 2**

| Compound number | HCC70 | A2780 |
|---|---|---|
| 35 | B | B |
| 36 | --- | A |
| 37 | C | C |
| 39 | B | B |
| 40 | C | B |
| 41 | C | B |
| 46 | C | C |
| 47 | C | C |
| 48 | C | C |
| 49 | C | C |
| 50 | C | C |
| 51 | C | C |
| 52 | C | C |
| 53 | C | C |
| 54 | C | C |
| 55 | C | B |
| 56 | D | D |
| 57 | C | C |
| 58 | B | C |
| 59 | B | B |
| 61 | B | C |
| 62 | C | C |
| 63 | C | C |
| 64 | C | C |
| 65 | B | B |
| 66 | A | B |
| 67 | B | B |
| 68 | C | C |
| 76 | B | B |
| 77 | B | B |
| 78 | A | A |
| 79 | B | A |
| 80 | A | A |
| 81 | A | A |
| 82 | A | A |
| 83 | A | A |
| 84 | A | A |
| 85 | B | A |
| 86 | B | A |
| 87 | A | A |
| 88 | B | A |
| 89 | A | A |
| 90 | B | B |
| 91 | B | A |
| 92 | B | A |
| 93 | A | A |
| 94 | B | A |
| 95 | B | B |
| 96 | A | A |
| 97 | B | A |
| 98 | A | A |
| 99 | A | A |
| 100 | A | A |
| 101 | C | B |
| 102 | A | A |
| 103 | B | B |
| 105 | B | B |
| 108 | A | A |
| 109 | B | B |
| 110 | B | A |
| 111 | B | A |
| 114 | A | A |
| 115 | B | B |
| 116 | B | B |
| 117 | A | A |
| 118 | A | A |
| 119 | B | B |
| 121 | B | C |
| 122 | B | C |
| 124 | B | B |
| 125 | B | C |
| 126 | B | B |
| 127 | A | A |
| 128 | B | A |
| 129 | B | B |
| 130 | B | B |
| 135 | A | A |
| 137 | A | A |
| 141 | B | B |
| 143 | B | B |
| 144 | B | B |
| 145 | B | B |
| 146 | B | B |
| 147 | C | B |
| 149 | A | B |
| 150 | A | B |
| 151 | B | B |
| 153 | A | B |
| 154 | A | A |
| 155 | A | A |
| 156 | A | B |
| 158 | A | A |
| 159 | B | B |
| 160 | C | C |
| 161 | C | C |
| 162 | C | B |
| 163 | C | B |
| 164 | B | B |
| 165 | B | B |
| 166 | C | C |
| 167 | C | C |
| 168 | B | B |
| 172 | B | B |
| 173 | B | B |
| 174 | A | A |
| 175 | B | C |
| 176 | A | - |
| 177 | A | A |
| 178 | C | B |
| 179 | B | B |
| 181 | C | B |
| 191 | B | A |
| 194 | B | A |
| 195 | A | A |

| | | |
|---|---|---|
| Note: --- indicates that this test has not been carried out. | | |

It can be seen from Table 2 that the compounds of the present invention have excellent inhibitory effects on human breast cancer cells HCC70 and ovarian cancer A2780.

### Effect Embodiment 3: Caco-2 membrane permeability and efflux rate

The recovered Caco-2 cell suspension was seeded into a TRANSWELL plate and cultured for 14-18 days. After being cultured for 14 days, the cells were fused and differentiated to be ready for a transport experiment. The Caco-2 cell plate was taken out from the incubator. Stock solutions of a to-be-tested compound and a control compound were formulated with DMSO and diluted with an HBSS (10 mM HEPES, pH 7.4) buffer to a final concentration of 5 µM. A test compound solution and a control drug solution were added to each well (top end) of an insert culture plate, and the HBSS (10 mM HEPES, pH 7.4) buffer was added to each well of a test compound receiving plate (basal end). When measuring the transport rate of the compound from the basal end to the top end, the test compound solution and the control drug solution were added to each well of the receiving plate (basal end), the HBSS (10 mM HEPES, pH 7.4) buffer solution was added to each well (top end) of the insert culture plate for the test compound solution, and incubation was carried out for 2 hours in a CO₂ incubator at 37°C. After the transport experiment was completed, vortexing was carried out for 10 minutes at 1000 rpm. After sample quenching at each time point, LC/MS/MS analysis was carried out. A fluorescence value was measured after a two-hour transport experiment, a lucifer yellow solution was added in a transwell chamber (top end), a transport buffer solution was added in the basal end, and incubation was carried out for 30 minutes in the CO₂ incubator at 37°C. The solution was taken out directly from the top end and the basal end (using basolateral wells) and transferred to a new 96-well plate. A cell fluorescence value was measured by a microplate reader (to detect membrane integrity), an excitation wavelength was 485 nM, and an emission wavelength was 530 nM. All data were calculated by using Microsoft Excel, and a peak area was calculated according to chromatograms. An apparent permeability coefficient (Papp, unit: 10-6, cm/s) and an efflux rate (ER) of the compounds in Caco-2 cells were calculated by the specific concentrations of a receiving end and an administering end.

The assay results are shown in the following Table 3.

**Table 3**

| **Compound number** | **Papp(A-B) (10⁻⁶, cm/s)** | **P app(B-A) (10⁻⁶, cm/s)** | **ER** |
|---|---|---|---|
| 79 | 5.1 | 22.34 | 4.38 |
| 82 | 5.32 | 23.55 | 3.95 |
| 83 | 1.99 | 32.43 | 16.3 |
| 85 | 7.69 | 21.31 | 2.77 |
| 86 | 1.44 | 29.48 | 20.5 |
| 87 | 0.68 | 25.1 | 37.1 |
| 88 | 3.76 | 29.24 | 7.77 |
| 89 | 2.13 | 30.59 | 14.3 |
| 91 | 2.64 | 27.27 | 10.3 |
| 93 | 3.53 | 26.96 | 7.64 |
| 94 | 2.51 | 31.38 | 12.5 |
| 96 | 1.86 | 26.09 | 14.1 |
| 97 | 1.55 | 32.27 | 20.8 |
| 102 | 3.43 | 26.76 | 7.8 |
| 103 | 6.7 | 36.08 | 5.38 |
| 105 | 2.99 | 21.91 | 7.32 |
| 114 | 0.8 | 25.4 | 31.9 |
| 118 | 2.11 | 33.76 | 16 |
| 119 | 2.23 | 34.09 | 15.3 |
| 126 | 3.32 | 24.58 | 7.41 |
| 127 | 3.82 | 22.37 | 5.86 |
| 129 | 4.8 | 32.42 | 6.76 |
| 130 | 5.82 | 35.08 | 6.03 |
| 137 | 2.79 | 34.59 | 12.4 |
| 143 | 2.44 | 32.48 | 13.3 |
| 144 | 2.89 | 36.73 | 12.7 |
| 149 | 4.2 | 32.76 | 7.8 |
| 150 | 4.68 | 27.87 | 5.95 |
| 153 | 4.17 | 24.75 | 5.94 |
| 154 | 4.42 | 26.41 | 5.97 |
| 155 | 5.57 | 24 | 4.31 |
| 156 | 5.32 | 20.58 | 3.87 |
| 158 | 5.07 | 28.65 | 5.65 |
| 168 | 7.99 | 17.07 | 2.14 |
| 174 | 2.03 | 34.79 | 17.1 |
| 179 | 0.51 | 33.52 | 65.9 |
| 195 | 10.3 | 24.38 | 2.36 |
| A control compound WO2018013867 (353) | 0.23 | 15.91 | 70.69 |

It can be seen from Table 3 that the compounds of the present invention have good Caco-2 membrane permeability and low efflux rate.

Although the embodiments of the present invention have been described above, those skilled in the art should understand that these are only illustrated, and various alterations or modifications can be made to these embodiments without departing from the principle and essence of the present invention. Accordingly, the protection scope of the present invention is defined by the appended claims.

## Claims

1. A compound as represented by formula I-A, a stereoisomer thereof, a diastereoisomer thereof, or a pharmaceutically acceptable salt of any one of the described substances, or a crystalline form or a solvate of any one of the described substances: 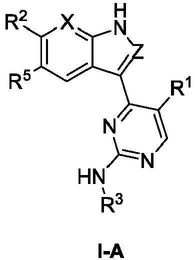
R¹ is H, CF₃, CHF₂, F, Cl, Br, C₁-C₆ alkyl, -C(=O)NH₂, or CN; preferably CF₃, CHF₂, F, Cl, Br; more preferably CF₃;
R⁵ is H, halogen, C₁-C₆ alkyl, or C₁-C₆ alkoxy; preferably H;
X is N or C(R⁴), and R⁴ is -P(=O)Me₂;
Z is N or CH;
R² is H, halogen, "5- to 10-membered heteroaryl having 1-4 heteroatoms selected from one or more of N, O, and S," "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, "4- to 12-membered heterocycloalkenyl having 1-4 heteroatoms selected from one or more of N, O, and S," "4- to 12-membered heterocycloalkenyl having 1-4 heteroatoms selected from one or more of N, O, and S" and substituted with one or more R^{a-3}, C₂-C₆ alkynyl, C₁-C₆ alkyl, C₁-C₆ alkoxy, or CN;
R^{a-1}, R^{a-2}, and R^{a-3} are independently CN, oxo, C₁-C₆ alkyl substituted with one or more R^{a-1-1}, NH₂, OH, or C₁-C₆ alkyl; R^{a-1-1} is independently CN, OH, or halogen;
R³ is C₃-C₈ cycloalkyl substituted with one or more R^{b-1}, C₁-C₆ alkyl substituted with one or more R^{b-2}, 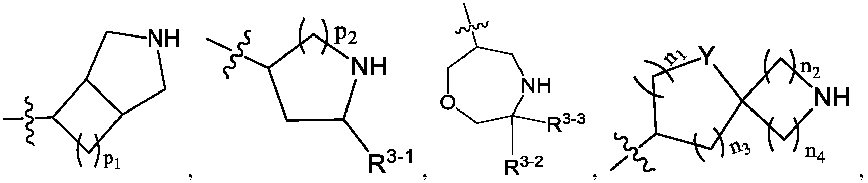 "4- to 12-membered heterocycloalkyl having 1-4 heteroatoms being O" and substituted with one or more R^{b-3}, 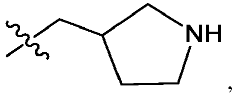 or "5- to 10-membered heteroaryl having 1-4 heteroatoms selected from one or more of N, O, and S";
R^{b-1} is independently halogen, OH, -NR^{b-1-1}R^{b-1-2}, C₁-C₆ alkyl, or "C₁-C₆ alkyl substituted with one or more R^{b-1-3}";
R^{b-1-1} and R^{b-1-2} are independently H or C₁-C₆ alkyl (for example, methyl);
R^{b-1-3} is independently OH or NR^{b-1-4}R^{b-1-5}; R^{b-1-4} and R^{b-1-5} are independently H or C₁-C₆ alkyl (for example, methyl);
R^{b-2} is independently OH, halogen, C₃-C₈ cycloalkyl, C₃-C₈ cycloalkyl substituted with one or more R^{b-2-1}, 4- to 12-membered heterocycloalkyl having 1-4 heteroatoms being O, or "4- to 12-membered heterocycloalkyl having 1-4 heteroatoms being O and substituted with one or more R^{b-2-2}_{";}
R^{b-2-1} and R^{b-2-2} are independently OH, C₁-C₆ alkyl (for example, methyl), or "C₁-C₆ alkyl (for example, methyl) substituted with one or more OH";
pi is 0, 1, 2, or 3 (preferably 0 or 3);
p₂ is 2 or 3, R³⁻¹ is H or C₁-C₆ alkyl (for example, methyl);
R³⁻² and R³⁻³ are independently H, C₁-C₆ alkyl (for example, methyl), or "C₁-C₆ alkyl substituted with one or more halogens";
Y is O or CH₂, n₁ is 1 or 2, n₂, n₃, and n₄ are independently 0, 1, 2, or 3, and n₂ and n₄ are not 0 at the same time;
R^{b-3} is independently halogen, OH, C₁-C₆ alkyl (for example, methyl), or "C₁-C₆ alkyl (for example, methyl) substituted with one or more OH";
or R³ represents -(CR^{M1}R^{M2})ₘ-(L)ₛ-(CR^{N1}R^{N2})ₜ-M;
wherein R^{M1}, R^{M2}, R^{N1}, and R^{N2} each independently represent hydrogen, and C₁-C₆ alkyl, and C₃-C₆ cycloalkyl substituted with 0, 1, 2 selected from hydroxyl, C₁-C₆ alkyl, and halogen; or R^{M1}, R^{M2}, R^{N1}, and R^{N2} each independently form a 3- to 6-membered ring together with carbon atoms to which they are jointly attached, and the ring optionally contains 0, 1, 2 heteroatoms selected from O, N, and S; further, the ring can be furthermore optionally substituted with 0, 1, 2 substituents selected from halogen, C₁-C₆ alkyl, and hydroxyl;
wherein L represents -CR^{Q1}R^{Q2}- or -C₃-C₆ cycloalkyl-; wherein R^{Q1} and R^{Q2} each independently represent hydrogen, and C₁-C₆ alkyl, and C₃-C₆ cycloalkyl substituted with 0, 1, 2 selected from hydroxyl, C₁-C₆ alkyl, and halogen; or R^{Q1} and R^{Q2} each independently form a 3- to 6-membered ring together with carbon atoms to which they are jointly attached, and the ring optionally contains 0, 1, 2 heteroatoms selected from O, N, and S; further, the ring can be furthermore optionally substituted with 0, 1, 2 substituents selected from halogen, C₁-C₆ alkyl, and hydroxyl;
wherein M represents hydrogen, hydroxyl, or C₁-C₆ alkyl and C₃-C₆ cycloalkyl substituted with 0, 1, 2 selected from hydroxyl, C₁-C₆ alkyl, and halogen;
wherein m, s, and t each independently represent 0, 1, 2, 3;
wherein at least one group in -(CR^{M1}R^{M2})ₘ-(L)ₛ-(CR^{N1}R^{N2})ₜ-M is substituted with hydroxyl;
the premise is that the compound as represented by formula I-A does not include: 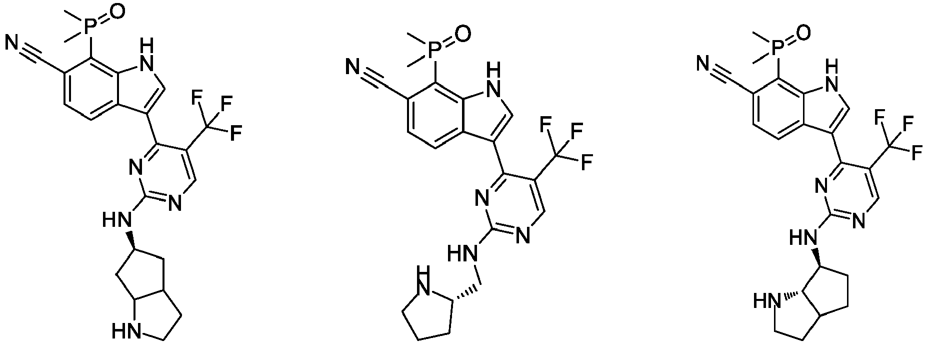 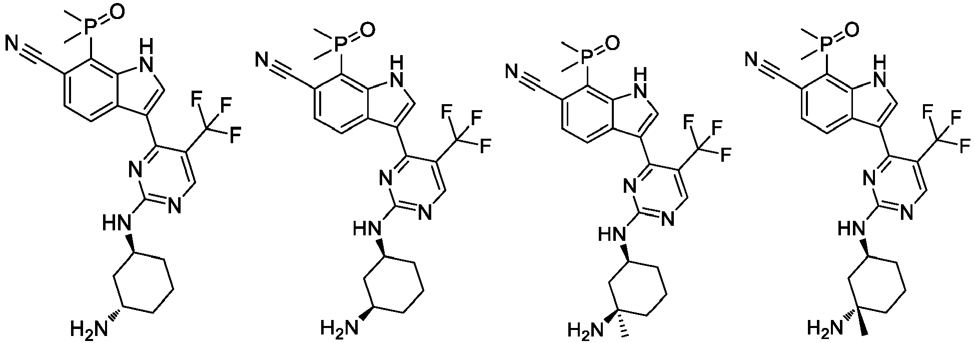 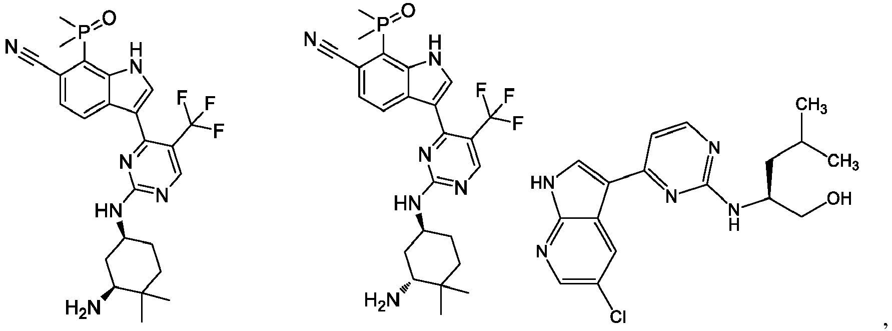 or a pharmaceutically acceptable salt, a stereoisomer, a tautomer, a hydrate, a solvate, an isotopic compound, or a prodrug of any one of the above-mentioned substances.

2. The compound as represented by formula I-A, the stereoisomer thereof, the diastereoisomer thereof, or the pharmaceutically acceptable salt of any one of the described substances, or the crystalline form or the solvate of any one of the described substances according to Claim 1, **characterized in that** the compound as represented by formula I-A is 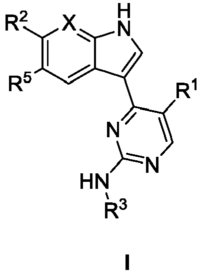 wherein,
R¹ is CF₃, F, Cl, Br, or CN;
R⁵ is H or halogen;
X is N or C(R⁴), and R⁴ is -P(=O)Me₂;
R² is H, halogen, "5- to 10-membered heteroaryl having 1-4 heteroatoms selected from one or more of N, O, and S," "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, C₂-C₆ alkynyl, or CN;
R^{a-1} and R^{a-2} are independently CN, oxo, C₁-C₆ alkyl substituted with one or more R^{a-1-1}, NH₂, OH, or C₁-C₆ alkyl; R^{a-1-1} is independently CN, OH, or halogen;
R³ is C₃-C₈ cycloalkyl substituted with one or more R^{b-1}, C₁-C₆ alkyl substituted with one or more R^{b-2}, 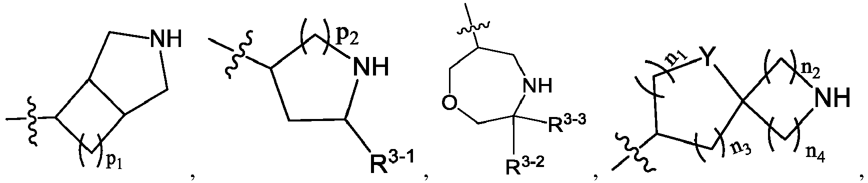 "4- to 12-membered heterocycloalkyl having 1-4 heteroatoms being O" and substituted with one or more OH, 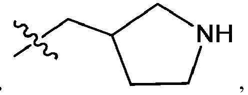 or "5- to 10-membered heteroaryl having 1-4 heteroatoms selected from one or more of N, O, and S";
R^{b-1} is independently halogen, OH, -NR^{b-1-1}R^{b-1-2}, or "C₁-C₆ alkyl substituted with one or more R^{b-1-3}";
R^{b-1-1} and R^{b-1-2} are independently H or C₁-C₆ alkyl;
R^{b-1-3} is independently OH or NR^{b-1-4}R^{b-1-5}; R^{b-1-4} and R^{b-1-5} are independently H or C₁-C₆ alkyl;
R^{b-2} is independently OH, halogen, or "C₃-C₈ cycloalkyl substituted with one or more OH";
pi is 0, 1, 2, or 3;
p₂ is 2 or 3, and R³⁻¹ is H or C₁-C₆ alkyl;
R³⁻² and R³⁻³ are independently H, C₁-C₆ alkyl, or "C₁-C₆ alkyl substituted with one or more halogens";
Y is O or CH₂, n₁ is 1 or 2, n₂, n₃, and n₄ are independently 0, 1, 2, or 3, and n₂ and n₄ are not 0 at the same time.

3. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-2, **characterized in that** R¹ is CF₃, CHF₂, F, Cl, Br, C₁-C₃ alkyl (for example, methyl), -C(=O)NH₂, or CN.

4. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-3, **characterized in that** the R⁵ represents H, F, Cl, Br, I, C₁-C₃ alkyl (for example, methyl), or C₁-C₃ alkoxy (for example, methoxy).

5. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-4, **characterized in that** the R² each independently represents H, F, Cl, Br, I, 5- to 6-membered heteroaryl having 1-4 heteroatoms selected from one or more of N and O, "5- to 6-membered heteroaryl having 1-4 heteroatoms selected from one or more of N and O" and substituted with one or 2 R^{a-1}, 4- to 12-membered heterocycloalkyl having 1-4 heteroatoms (preferably 1-2 heteroatoms) selected from one or more of N, O, and S, "4- to 12-membered heterocycloalkyl having 1-4 heteroatoms (preferably 1-2 heteroatoms) selected from one or more of N, O, and S" and substituted with 1 or 2 R^{a-2}, 4- to -12-membered heterocycloalkenyl having 1-2 heteroatoms selected from one or more of N, O, and S, "4- to -12-membered heterocycloalkenyl having 1-2 heteroatoms selected from one or more of N, O, and S" and substituted with one or more R^{a-3}, C₂-C₆ alkynyl, C₁-C₃ alkyl (for example, methyl), C₁-C₃ alkoxy (for example, methoxy), or CN, wherein the 4- to 12-membered heterocycloalkyl is a monocyclic ring, a bicyclic ring, or a bridged ring, and the bicyclic ring includes a spiro ring or a fused ring.

6. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-5, **characterized in that** R^{a-1}, R^{a-2}, and R^{a-3} are independently CN, oxo, C₁-C₃ alkyl (for example, methyl) substituted with one or more R^{a-1-1}, NH₂, OH, or C₁-C₃ alkyl (for example, methyl); wherein the R^{a-1-1} is independently CN, OH, F, Cl, Br, or I.

7. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims -6, **characterized in that** the R³ is C₃-C₆ cycloalkyl substituted with 1, 2, or 3 R^{b-1}, methyl substituted with one or more R^{b-2}, ethyl substituted with one or more R^{b-2}, n-propyl substituted with one or more R^{b-2}, isopropyl substituted with one or more R^{b-2}, n-butyl substituted with one or more R^{b-2}, isobutyl substituted with one or more R^{b-2}, sec-butyl substituted with one or more R^{b-2}, 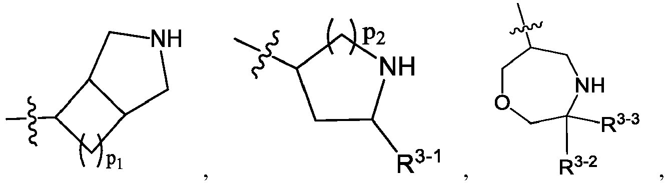 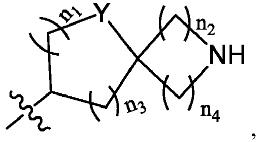 "4- to 6-membered heterocycloalkyl having 1-2 heteroatoms being O" and substituted with 1 or 2 R^{b-3}, 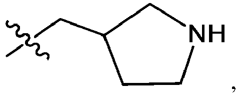 or "5- to 6-membered heteroaryl having 1-4 heteroatoms selected from one or more of N."

8. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-7, **characterized in that** R^{b-1} is independently F, Cl, Br, I, OH, -NR^{b-1-1}R^{b-1-2}, C₁-C₃ alkyl (for example, methyl), or "C₁-C₃ alkyl (for example, methyl) substituted with one or more R^{b-1-3}."

9. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-8, **characterized in that** R^{b-1-1} and R^{b-1-2} are independently H or C₁-C₃ alkyl;
R^{b-1-4} and R^{b-1-5} are independently H or C₁-C₃ alkyl.

10. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-9, **characterized in that** R^{b-2} is independently OH, F, Cl, Br, I, C₃-C₇ cycloalkyl or C₃-C₇ cycloalkyl substituted with 1 or 2 R^{b-2-1}, 4- to 6-membered heterocycloalkyl having 1 heteroatom being O, or 4- to 6-membered heterocycloalkyl having 1 heteroatom being O and substituted with 1 or 2 R^{b-2-2};
wherein R^{b-2-1} and R^{b-2-2} are independently OH, C₁-C₃ alkyl, or C₁-C₃ alkyl substituted with one or more OH.

11. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-10, **characterized in that** R³⁻¹ is H or C₁-C₃ alkyl.

12. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-11, **characterized in that** R³⁻² and R³⁻³ are independently H, C₁-C₃ alkyl, or "C₁-C₃ alkyl substituted with one or more halogens."

13. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-12, **characterized in that** R^{b-3} is independently F, Cl, Br, I, OH, C₁-C₃ alkyl, or C₁-C₃ alkyl substituted with one or more OH.

14. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-13, **characterized in that**, in R³, 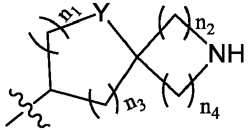 is

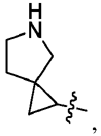

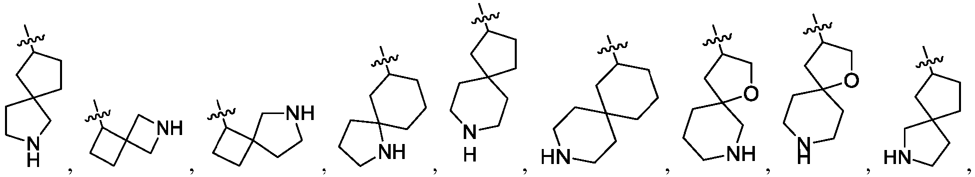

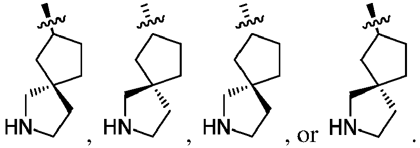

15. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-14, **characterized in that**, in R², the C₁-C₃ alkyl is methyl, ethyl, n-propyl, or isopropyl;
the C₁-C₃ alkoxy is methoxy, ethoxy, n-propoxy, or isopropoxy;
the "5- to 10-membered heteroaryl having 1-4 heteroatoms selected from one or more of N, O, and S" is 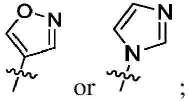
the "5- to 10-membered heteroaryl having 1-4 heteroatoms selected from one or more of N, O, and S" and substituted with one or more R^{a-1} is 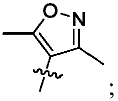
the "4- to 12-membered heterocycloalkyl having 1-4 heteroatoms selected from one or more of N, O, and S" is 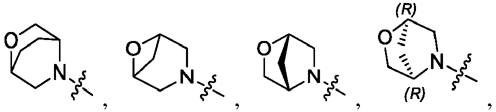 azetidinyl, oxazepanyl, tetrahydrofuranyl, tetrahydropyranyl, piperidyl, pyrrolidinyl, piperazinyl, thiomorpholinyl, or morpholinyl;
the "4- to 12-membered heterocycloalkyl having 1-4 heteroatoms selected from one or more of N, O, and S" and substituted with one or more R^{a-2} is morpholinyl substituted with 1 or 2 R^{a-2}, azetidinyl substituted with 1 or 2 R^{a-2}, tetrahydrofuranyl substituted with 1 or 2 R^{a-2}, tetrahydropyranyl substituted with 1 or 2 R^{a-2}, piperidyl substituted with 1 or 2 R^{a-2}, pyrrolidinyl substituted with 1 or 2 R^{a-2}, piperazinyl substituted with 1 or 2 R^{a-2}, thiomorpholinyl substituted with 1 or 2 R^{a-2};
the "4- to 12-membered heterocycloalkenyl having 1-4 heteroatoms selected from one or more of N, O, and S" is dihydrofuranyl.

16. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-15, **characterized in that**, in R^{a-1}, R^{a-2}, and R^{a-3}, the C₁-C₆ alkyl is independently methyl, ethyl, n-propyl, or isopropyl;
the C₁-C₆ alkyl substituted with one or more R^{a-1-1} is independently methyl, ethyl, n-propyl, or isopropyl substituted with one or more R^{a-1-1};
in R^{a-1-1}, the halogen is F.

17. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-16, **characterized in that**, in R³, the C₃-C₈ cycloalkyl substituted with one or more R^{b-1} is cyclopropyl substituted with 1, 2, or 3 R^{b-1}, cyclobutyl substituted with 1, 2, or 3 R^{b-1}, cyclopentyl substituted with 1, 2, or 3 R^{b-1};
in R^{b-1}, the halogen is F, the C₁-C₆ alkyl is methyl, ethyl, n-propyl, or isopropyl, and the "C₁-C₆ alkyl substituted with one or more R^{b-1-3}" is methyl, ethyl, n-propyl, or isopropyl substituted with one or more R^{b-1-3},

18. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-17, **characterized in that**, in R^{b-1-1} and R^{b-1-2}, the C₁-C₆ alkyl is independently methyl, ethyl, n-propyl, or isopropyl;
in R^{b-1-4} and R^{b-1-5}, the C₁-C₆ alkyl is independently methyl, ethyl, n-propyl, or isopropyl.

19. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-18, **characterized in that**, in R^{b-2}, the halogen is F; the C₃-C₈ cycloalkyl is cyclopropyl, cyclobutyl, cyclopentyl, cyclohexyl, or cycloheptyl; the C₃-C₈ cycloalkyl substituted with one or more R^{b-2-1} is cyclopropyl substituted with 1 or 2 R^{b-2-1}, cyclobutyl substituted with 1 or 2 R^{b-2-1}, cyclopentyl substituted with 1 or 2 R^{b-2-1}, cyclohexyl substituted with 1 or 2 R^{b-2-1}, or cycloheptyl substituted with 1 or 2 R^{b-2-1}; the "4- to 12-membered heterocycloalkyl having 1-4 heteroatoms being O" is tetrahydrofuranyl or tetrahydropyranyl; the "4- to 12-membered heterocycloalkyl having 1-4 heteroatoms being O and substituted with one or more R^{b-2-2}" is "tetrahydrofuranyl substituted with 1 or 2 R^{b-2-2}" or "tetrahydropyranyl substituted with 1 or 2 R^{b-2-2}";
in R^{b-2-1} and R^{b-2-2}, the C₁-C₆ alkyl is independently methyl, ethyl, n-propyl, or isopropyl; the "C₁-C₆ alkyl substituted with one or more OH" is independently "methyl, ethyl, n-propyl, or isopropyl substituted with one or more OH."

20. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-19, **characterized in that**, in R³, the C₁-C₆ alkyl substituted with one or more R^{b-2} is

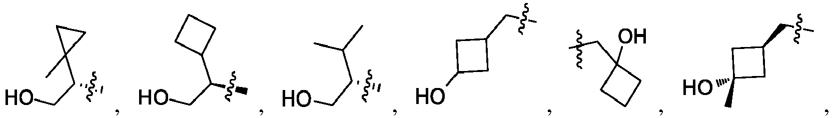

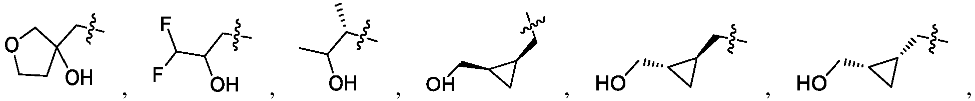

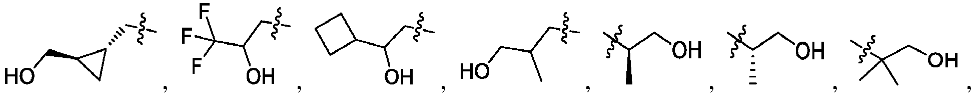

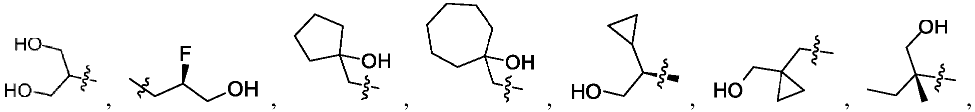

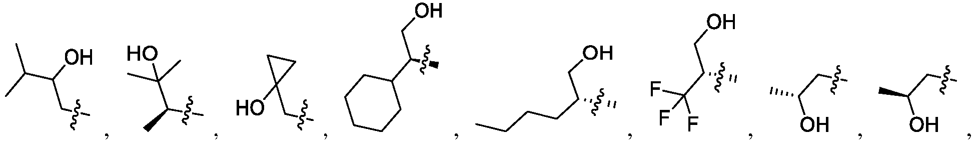

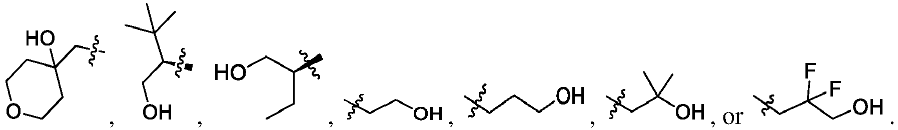

21. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-20, **characterized in that**, in R³⁻¹, R³⁻² and R³⁻³, the C₁-C₆ alkyl is independently methyl, ethyl, n-propyl, or isopropyl.

22. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-21, **characterized in that**, in R³, the "4- to 12-membered heterocycloalkyl having 1-4 heteroatoms being O" and substituted with one or more R^{b-3} is oxetanyl substituted with 1 or 2 R^{b-3}, tetrahydrofuranyl substituted with 1 or 2 R^{b-3}, or "tetrahydropyranyl substituted with 1 or 2 R^{b-3}";
wherein, in R^{b-3}, the C₁-C₆ alkyl substituted with one or more OH is methyl, ethyl, n-propyl, or isopropyl substituted with one or more OH.

23. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-22, **characterized in that**, in R³, the "5- to 10-membered heteroaryl having 1-4 heteroatoms selected from one or more of N, O, and S" is pyrazolyl.

24. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-23, **characterized in that**, in R², the "4- to 12-membered heterocycloalkyl having 1-4 heteroatoms selected from one or more of N, O, and S" is 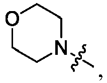 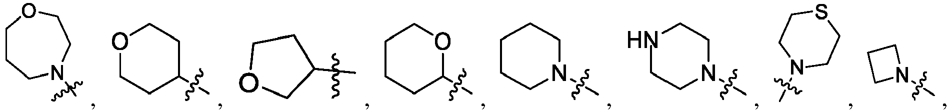 or 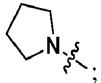
The "4- to 12-membered heterocycloalkyl having 1-4 heteroatoms selected from one or more of N, O, and S" and substituted with one or more R^{a-2} is 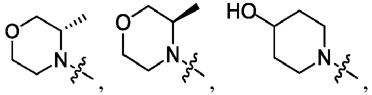 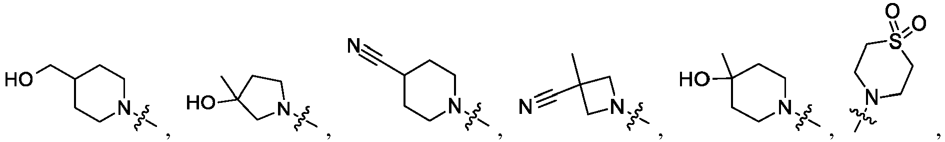 or 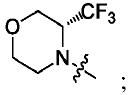
the "4- to 12-membered heterocycloalkenyl having 1-4 heteroatoms selected from one or more of N, O, and S" is

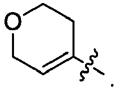

25. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-24, **characterized in that**, in R³, the C₃-C₈ cycloalkyl substituted with one or more R^{b-1} is 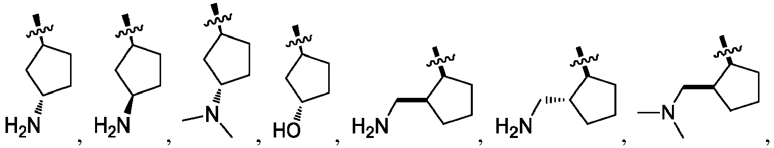 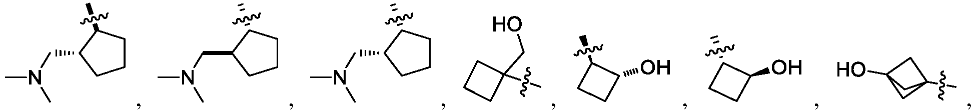 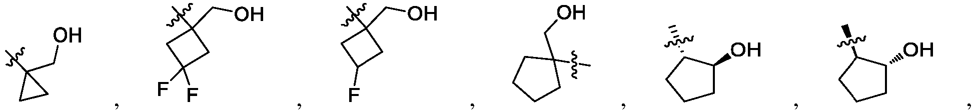 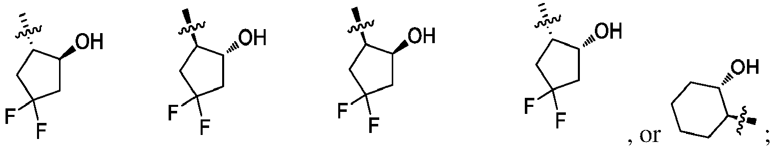
the "4- to 12-membered heterocycloalkyl having 1-4 heteroatoms being O" and substituted with one or more R^{b-3} is 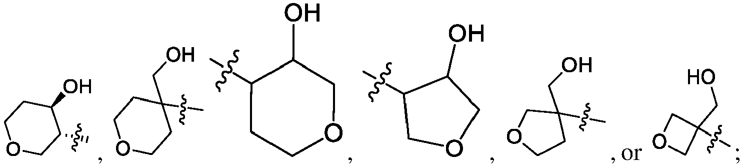
the "5- to 10-membered heteroaryl having 1-4 heteroatoms selected from one or more of N, O, and S" is

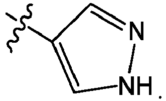

26. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-25, **characterized in that**, in R^{b-2}, the C₃-C₈ cycloalkyl substituted with one or more R^{b-2-1} is 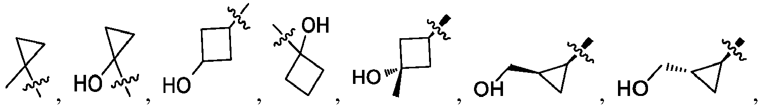 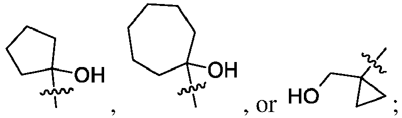
the "4- to 12-membered heterocycloalkyl having 1-4 heteroatoms being O" is 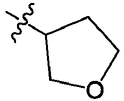 or 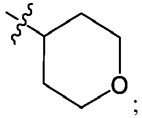
the "4- to 12-membered heterocycloalkyl having 1-4 heteroatoms being O and substituted with one or more R^{b-2-2}" is

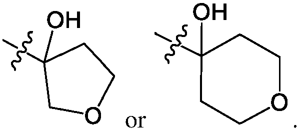

27. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-26, **characterized in that** R¹ is CF₃, Cl, Br or CN.

28. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-27, **characterized in that** X is C(R⁴), and R⁴ is -P(=O)Me₂.

29. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-28, **characterized in that** Z is CH.

30. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-29, **characterized in that**, when Y is O, n₁ is 1, and n₃ is 0 or 1.

31. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-30, **characterized in that**, when the number of R^{b-1} is one, R^{b-1} is OH, - NR^{b-1-1}R^{b-1-2}, or "C₁-C₆ alkyl substituted with one or more R^{b-1-3}"; when the number of R^{b-1} is plural, at least one R^{b-1} is OH or "C₁-C₆ alkyl substituted with one or more OH."

32. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-31, **characterized in that**, when the number of R^{b-2} is one, R^{b-2} is OH, C₃-C₈ cycloalkyl substituted with one or more R^{b-2-1}, or "4- to 12-membered heterocycloalkyl having 1-4 heteroatoms being O and substituted with one or more R^{b-2-2}"; when the number of R^{b-2} is plural, at least one R^{b-2} is OH, C₃-C₈ cycloalkyl substituted with one or more R ^{b-2-1}, or "4- to 12-membered heterocycloalkyl having 1-4 heteroatoms being O and substituted with one or more R^{b-2-2}"; wherein, when the number of R^{b-2-1} is one, R^{b-2-1} is OH or "C₁-C₆ alkyl substituted with one or more OH"; when the number of R^{b-2-1} is plural, at least one R^{b-2-1} is OH or "C₁-C₆ alkyl substituted with one or more OH"; when the number of R^{b-2-2} is one, R^{b-2-2} is OH or "C₁-C₆ alkyl substituted with one or more OH"; when the number of R^{b-2-2} is plural, at least one R^{b-2-2} is OH or "C₁-C₆ alkyl substituted with one or more OH."

33. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-32, **characterized in that**, when the number of R^{b-3} is one, R^{b-3} is OH or "C₁-C₆ alkyl substituted with one or more OH; when the number of R^{b-3} is plural, at least one R^{b-3} is OH or "C₁-C₆ alkyl substituted with one or more OH."

34. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-33, **characterized in that** R² is H, halogen, "5- to 10-membered heteroaryl having 1-4 heteroatoms selected from one or more of N, O, and S" and substituted with one or more R^{a-1}, "4- to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, "4- to 12-membered heterocycloalkenyl having 1-4 heteroatoms selected from one or more of N, O, and S," C₁-C₆ alkoxy, or CN; wherein R^{a-1} and R^{a-2} are as defined in Claim 1 or 2.

35. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-34, **characterized in that** R³ is C₁-C₆ alkyl substituted with one or more R^{b-2}, 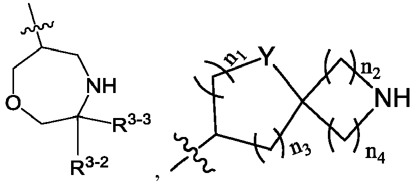 , or "4- to 12-membered heterocycloalkyl having 1-4 heteroatoms being O" and substituted with one or more R^{b-3}, wherein n₁, n₂, n₃, n₄, Y, R³⁻², R³⁻³, R^{b-2}, and R^{b-3} are as defined in Claim 1 or 2.

36. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-35, **characterized in that** R² is H, F, CN, -OCH₃,

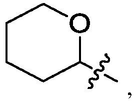

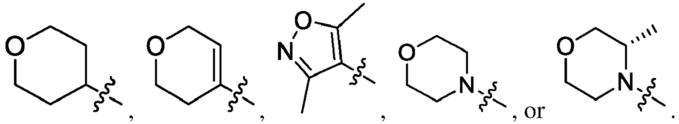

37. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-36, **characterized in that** R³ is 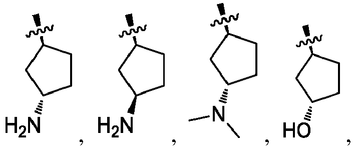 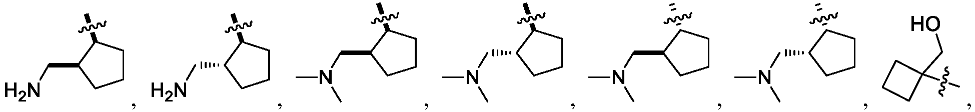 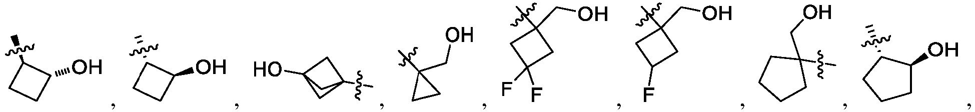 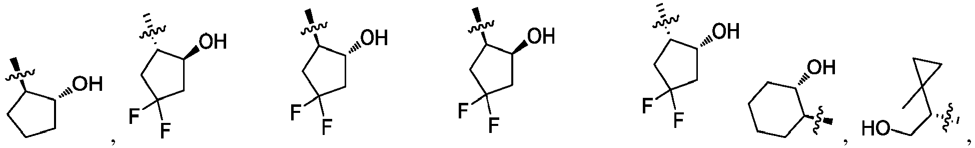 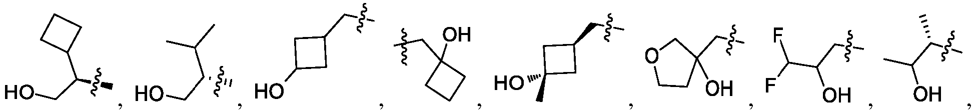 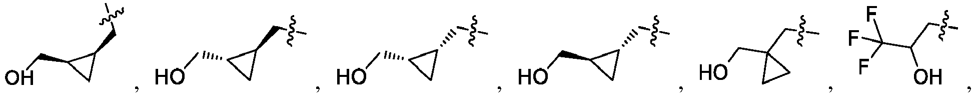 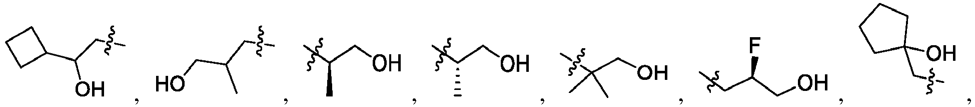 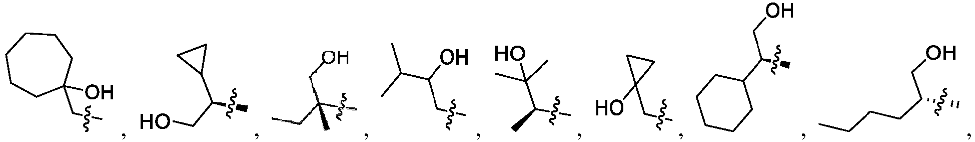 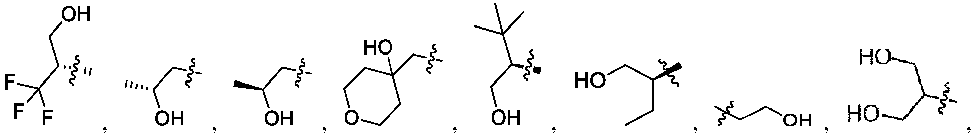 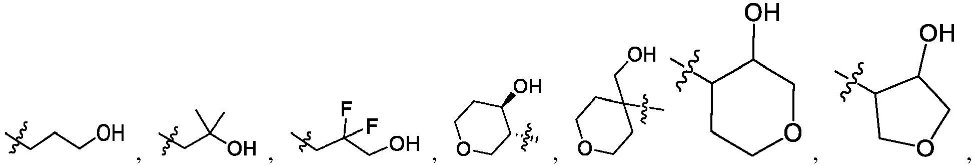 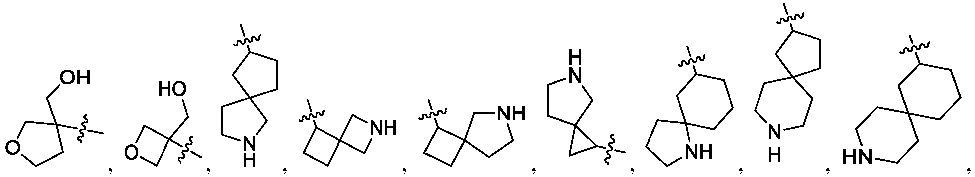 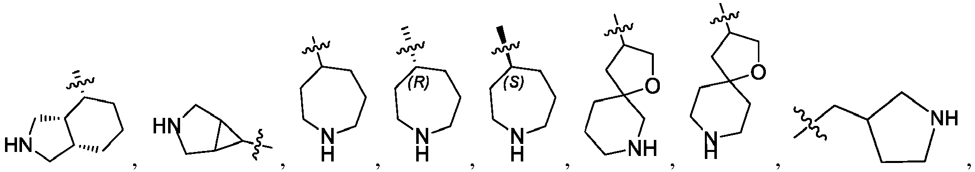 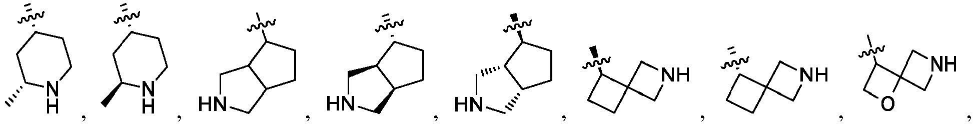 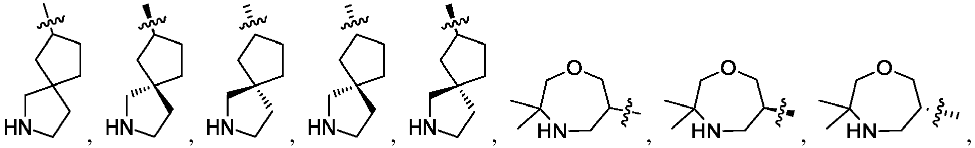 or

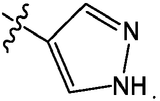

38. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-37, **characterized in that**, when X is C(R⁴) and R⁴ is -P(=O)Me₂, R² is H, halogen, C₁-C₆ alkoxy, or CN; when X is N, R² is "5- to 10-membered heteroaryl having 1-4 heteroatoms selected from one or more of N, O, and S" and substituted with one or more R^{a-1}, "4-to 12-membered heterocycloalkyl having 1-4 heteroatoms selected from one or more of N, O, and S," "4- to 12-membered heterocycloalkyl having 1-4 heteroatoms selected from one or more of N, O, and S" and substituted with one or more R^{a-2}, "4- to 12-membered heterocycloalkenyl having 1-4 heteroatoms selected from one or more of N, O, and S," or CN, wherein R^{a-1} and R^{a-2} are as defined in Claim 1 or 2.

39. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-38, **characterized in that**, when X is C(R⁴) and R⁴ is -P(=O)Me₂, R³ is C₃-C₈ cycloalkyl substituted with one or more R^{b-1}, 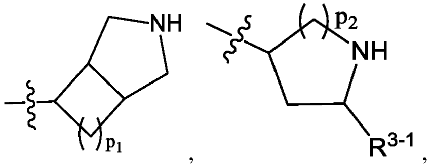 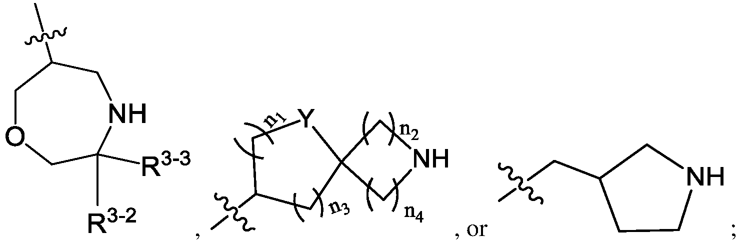 wherein R^{b-1}, R³⁻¹, R³⁻², R³⁻³, Y, p₁, n₁, n₂, n₃, and n₄ are as defined in Claim 1 or 2.

40. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-39, **characterized in that**, when X is N, R³ is C₃-C₈ cycloalkyl substituted with one or more R^{b-1}, C₁-C₆ alkyl substituted with one or more R^{b-2}, 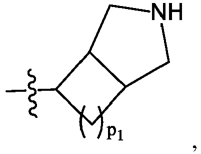 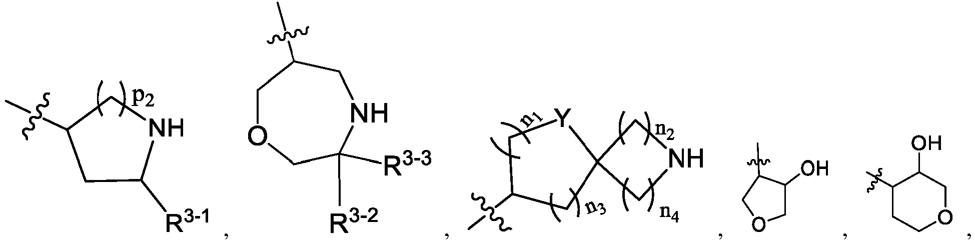 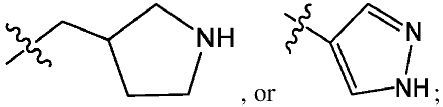 wherein R^{b-1}, R³⁻¹, R³⁻², R³⁻³, Y, p₁, p₂, n₁, n₂, n₃, and n₄ are as defined in Claim 1 or 2.

41. The compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-40, **characterized in that**, R³ is C₃-C₈ cycloalkyl substituted with one or more R^{b-1}, C₁-C₆ alkyl substituted with one or more R^{b-2}, 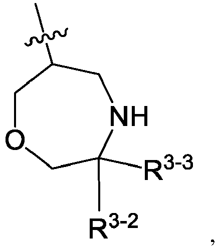 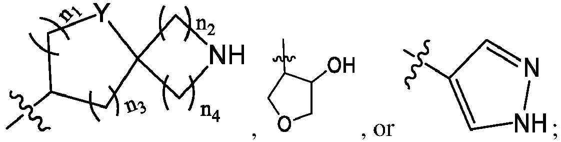 wherein R^{b-1}, R^{b-2}, R³⁻² , R³⁻³, Y, n₁, n₂, n₃, and n₄ are as defined in Claim 1 or 2; when Y is O, n₁ is 1, and n₃ is 0.

42. The compound as represented by formula I-A, the stereoisomer thereof, the diastereoisomer thereof, or the pharmaceutically acceptable salt of any one of the described substances, or the crystalline form or the solvate of any one of the described substances according to Claims 1- 41, **characterized in that** the compound as represented by formula I-A is any one of the following compounds:

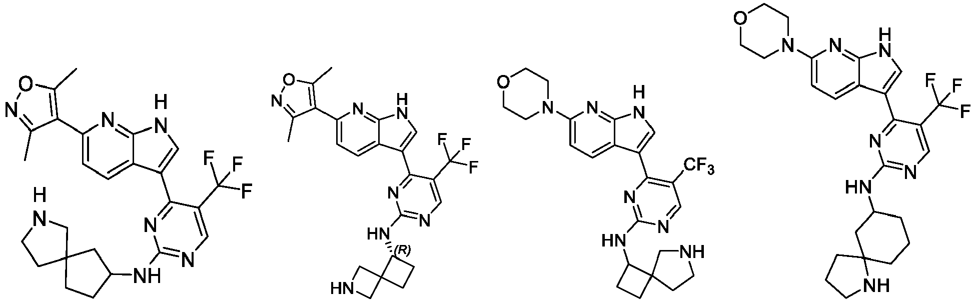

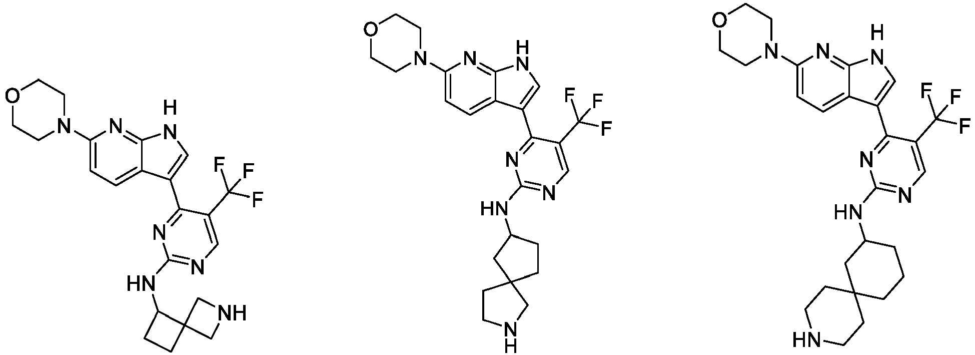

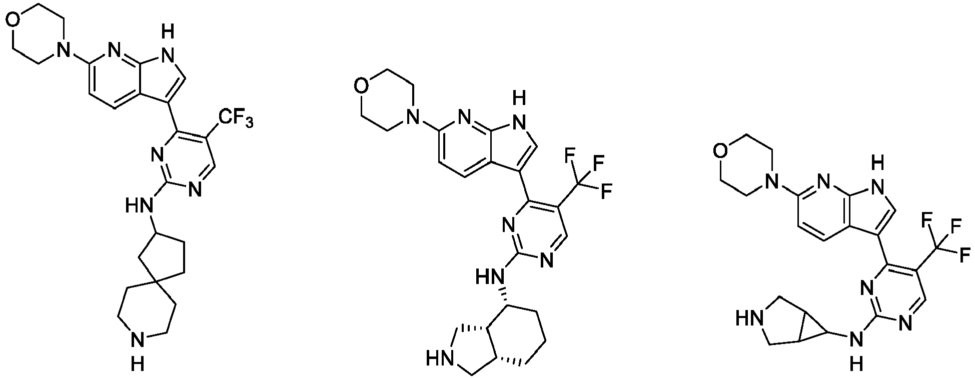

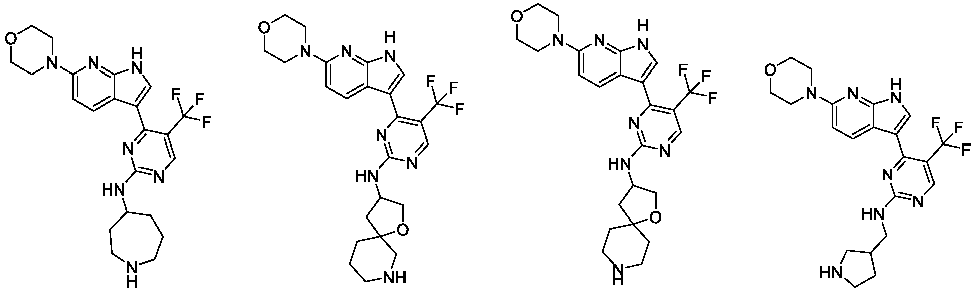

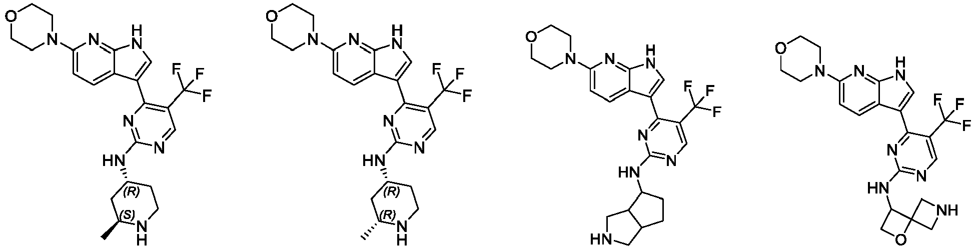

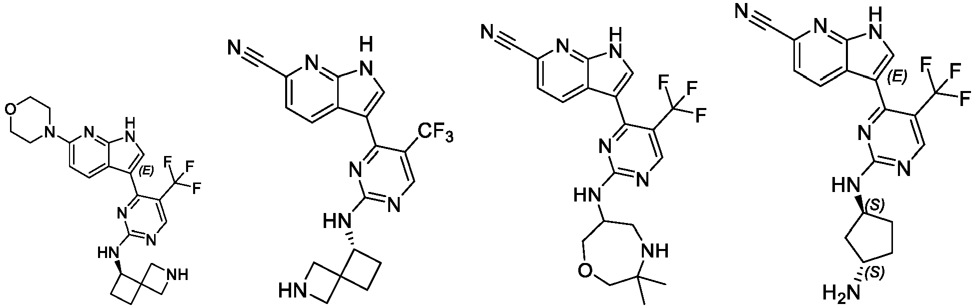

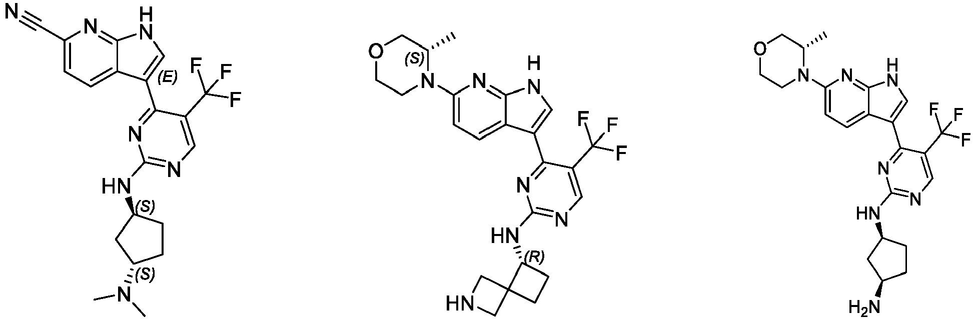

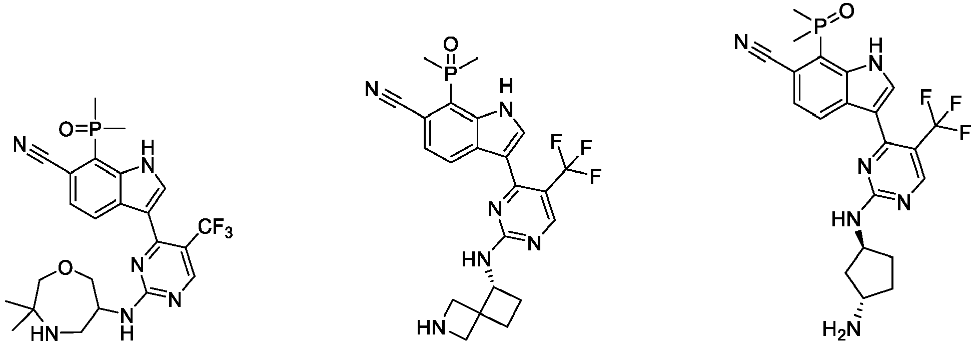

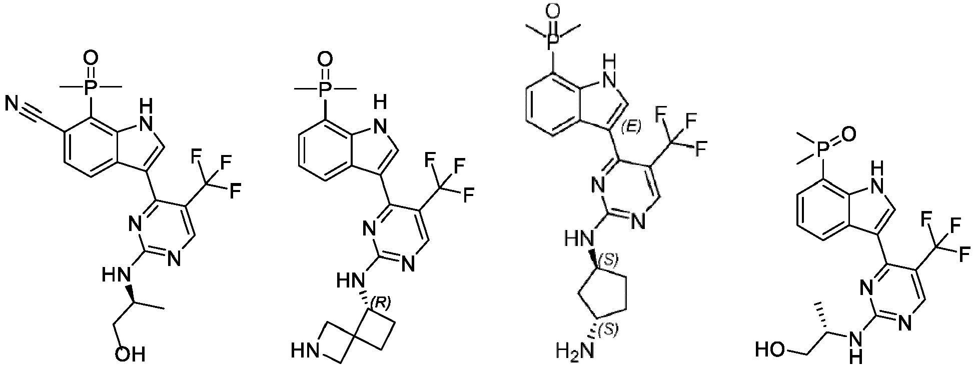

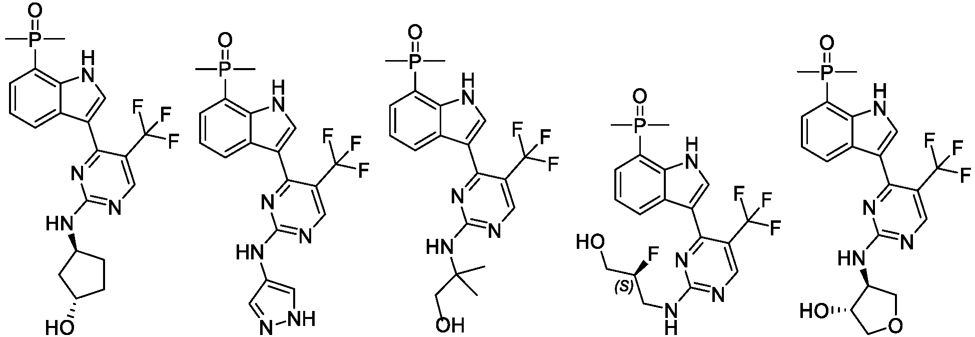

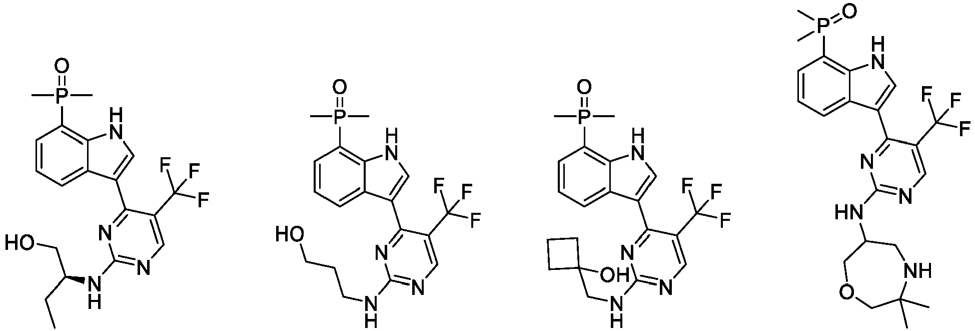

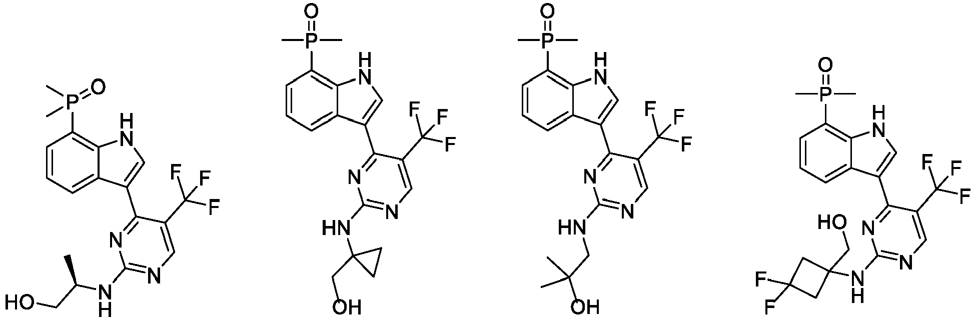

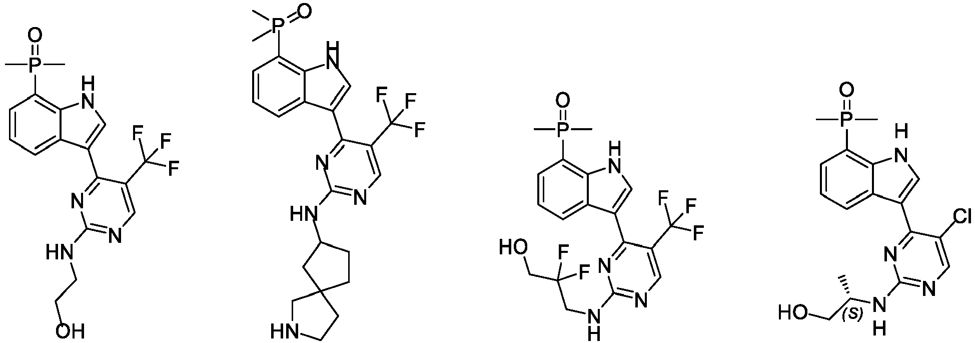

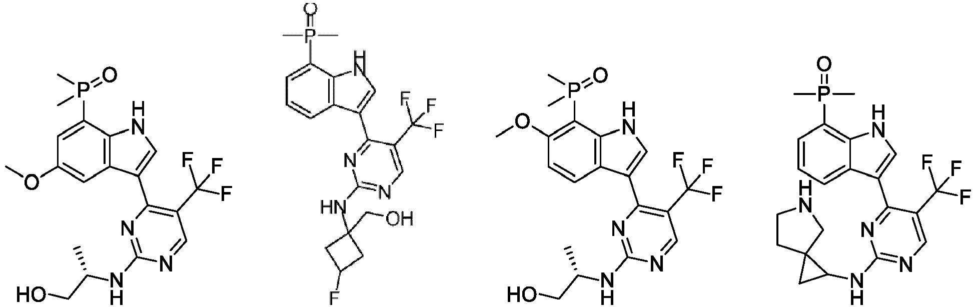

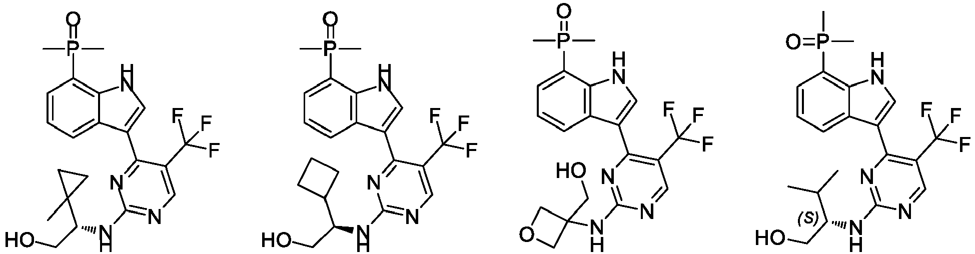

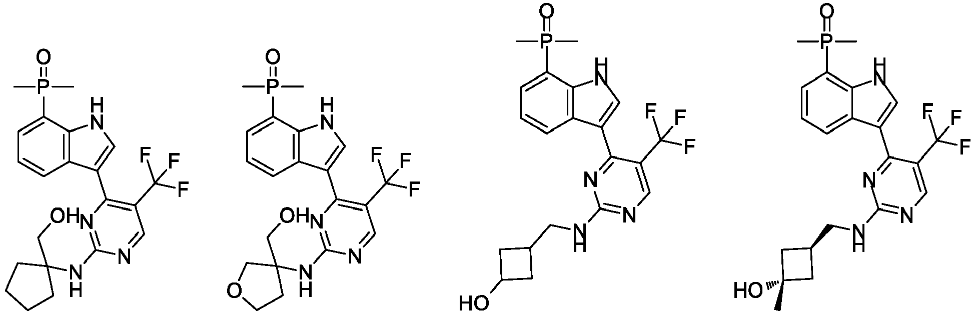

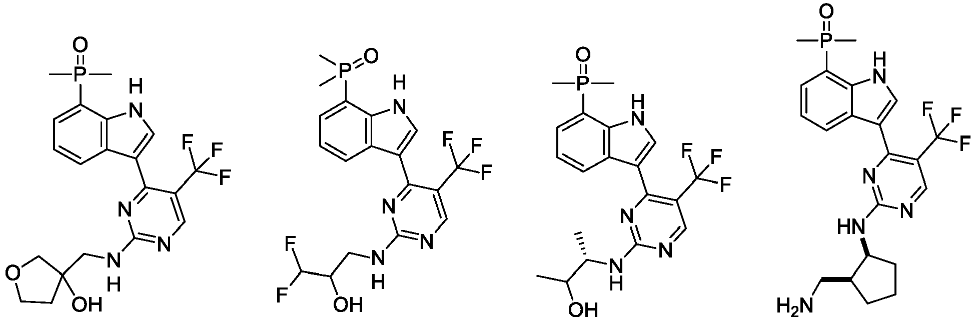

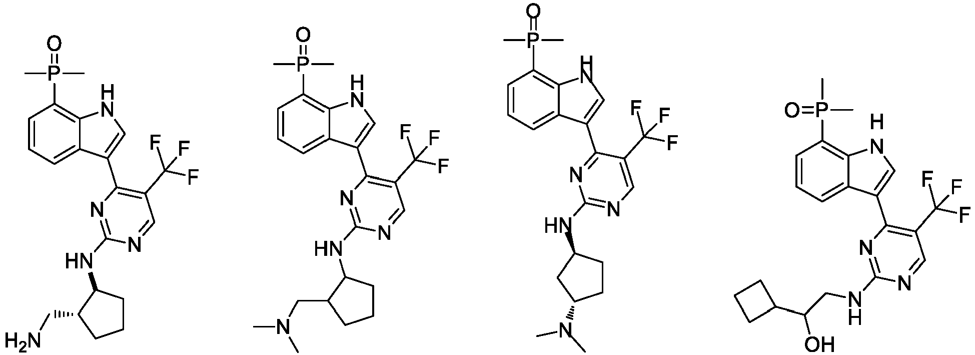

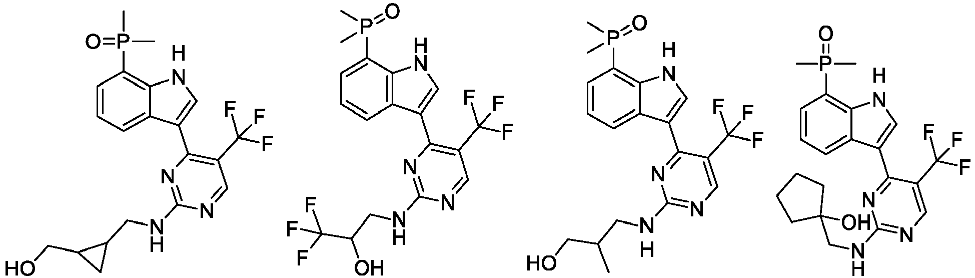

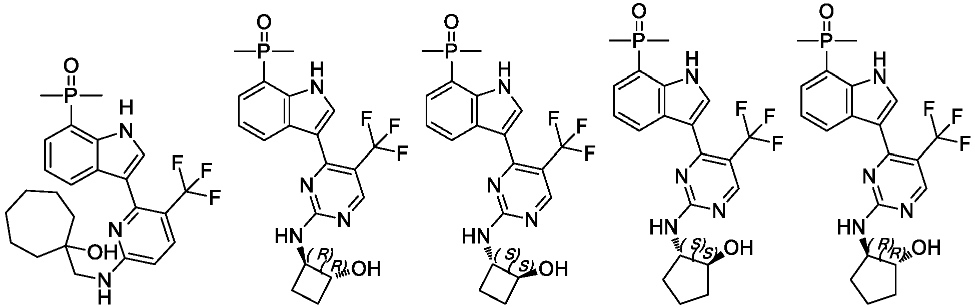

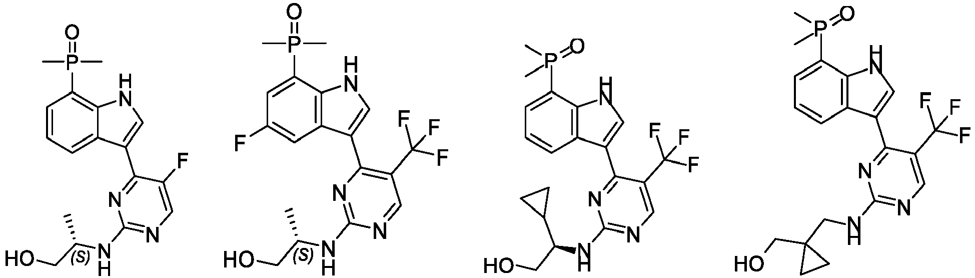

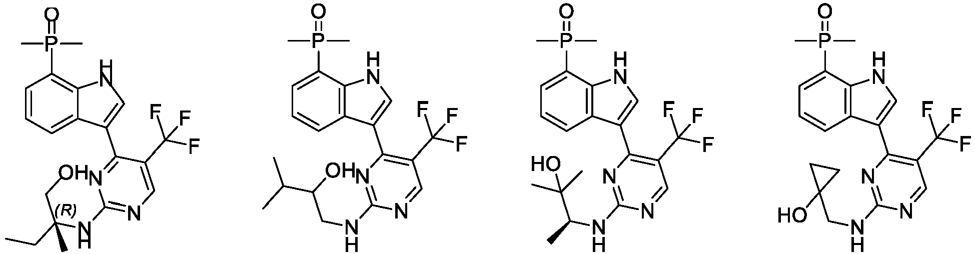

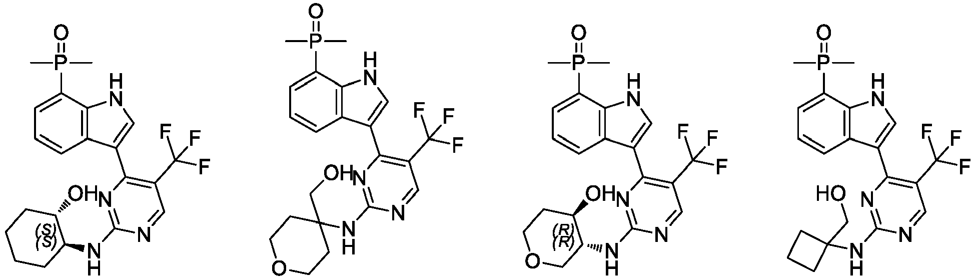

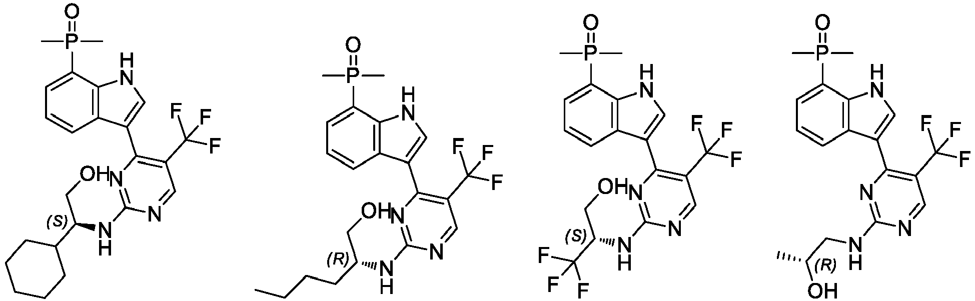

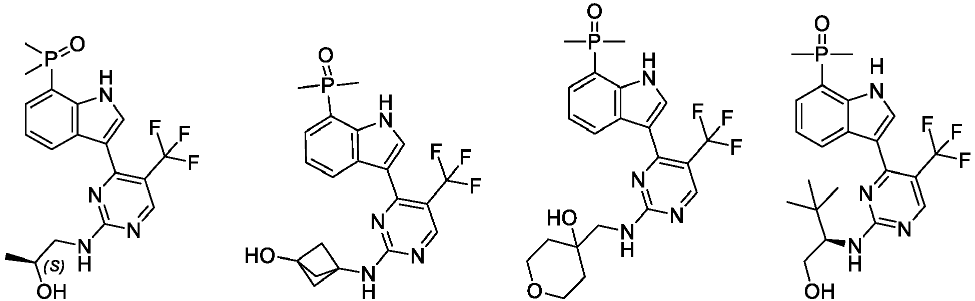

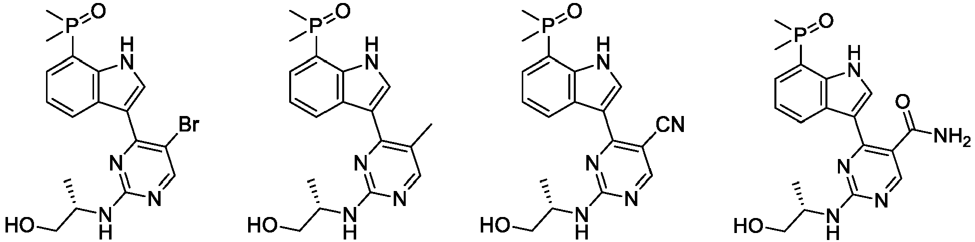

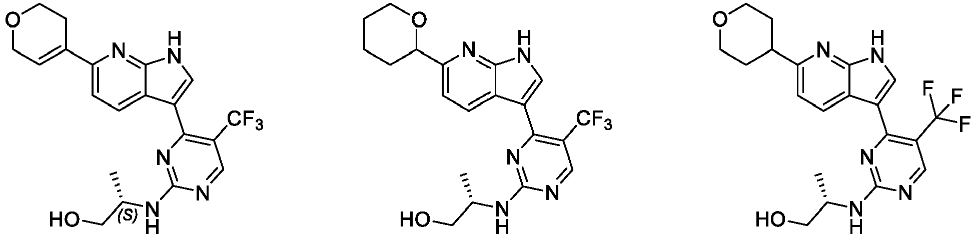

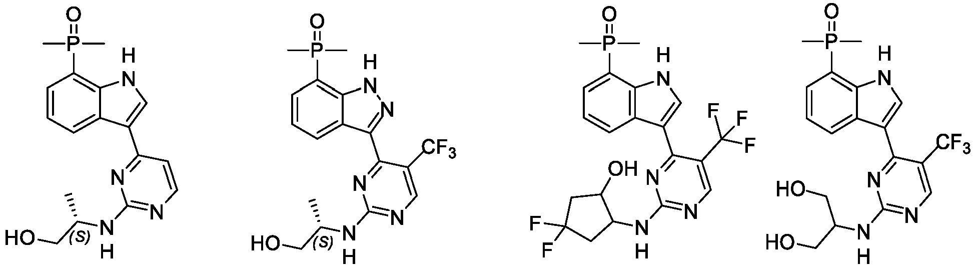

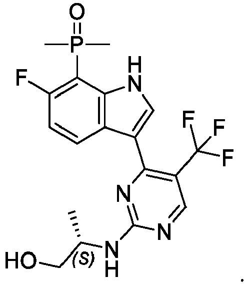

43. A pharmaceutical composition, comprising a compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-42, and a pharmaceutical excipient.

44. Use of a compound as represented by formula I-A, the stereoisomer thereof, the diastereoisomer thereof, or the pharmaceutically acceptable salt of any one of the described substances, or the crystalline form or the solvate of any one of the described substances according to at least one of Claims 1-42, or the pharmaceutical composition according to Claim 43 for preventing and/or treating proliferative diseases; preferably, the proliferative diseases being cancers (for example, leukemia, acute myeloid leukemia, acute lymphoblastic leukemia, breast cancer, ovarian cancer, brain cancer, lung cancer, liver cancer, small cell lung cancer, melanoma, bladder cancer, colon cancer, esophageal cancer, bone cancer, neuroblastoma, ovarian cancer, pancreatic cancer, prostate cancer, testicular cancer epithelial sarcoma, soft tissue sarcoma, multiple myeloma), benign neoplasms, angiogenesis, inflammatory diseases, autoinflammatory diseases, and autoimmune diseases.
